# EUROPEAN PATENT APPLICATION

(11) **EP 4 663 693 A1**
(43) Date of publication of application: **17.12.2025**
(21) Application number: 24753362.3
(22) Date of filing: 06.02.2024
(51) Int. Cl.: C08J 11/16, C07C 37/50, C07C 39/16, C07C 41/16, C07C 43/205, C07C 315/04, C07C 317/22, C07D 209/48, C08K 5/37, C08K 5/5399, C08L 101/00

(54) **METHOD FOR DECOMPOSING COMPOUND**

(30) Priority: 07.02.2023 JP 2023016683; 07.02.2023 JP 2023016684
(71) Applicant: National Institute of Advanced Industrial Science and Technology, Chiyoda-ku Tokyo 100-8921 (JP)
(72) Inventor: MINAMI, Yasunori, Tsukuba-shi, Ibaraki 305-8560 (JP); NAKAJIMA, Yumiko, Tsukuba-shi, Ibaraki 305-8560 (JP); SATO, Kazuhiko, Tsukuba-shi, Ibaraki 305-8560 (JP)
(74) Representative: Groth, Wieland
(86) International application number: PCT/JP2024/003977
(87) International publication number: WO 2024/166919

(57) **Abstract**

[Problems] To provide a novel method for depolymerizing a super engineering plastic such as polysulfone.

[Solution to Problem] A method for decomposing a compound, including a decomposition step of reacting a first compound represented by the following general formula (1) and a second compound represented by the following general formula (8) or a hydroxide in the co-presence of a base (in the formula, n₁ is an integer of 2 or greater; Z¹¹ and Z¹² are each independently a group other than a hydrogen atom; m₁₁ and m₁₂ are each independently an integer of 0 to 4; Ar¹ is represented by the following general formula (91), (92) or (93); and R⁸ is an alkyl group, an aryl group, or an aralkyl group; X¹¹, X¹², X²¹, X³¹, and X³² are each independently a group other than a hydrogen atom; and l₁₁, l₁₂, l₂₁, l₃₁, and l₃₂ are each independently an integer of 0 to 4.).

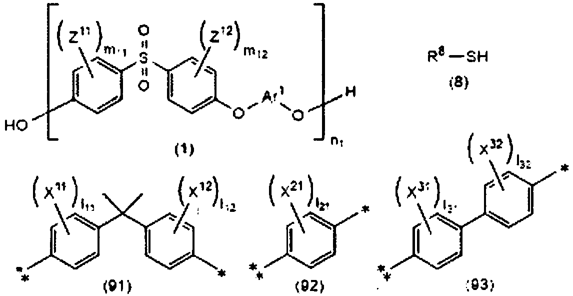

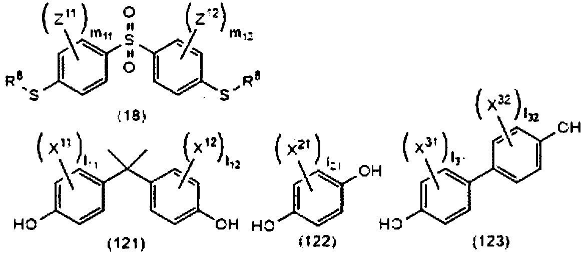

## Description

### Technical Field

The present invention relates to a method for depolymerizing (decomposing) a compound.

### Background Art

Polysulfones, super engineering plastics, are thermoplastic resins that not only have excellent heat resistance, a wide range of operating temperatures, hydrolysis resistance, chemical resistance, and electrical properties, but also have a mechanical strength, transparency, moldability, and weather resistance, and the like, rendering them ideal as processable materials as well. Due to these properties, polysulfones have a wide range of applications, including medical and food materials such as medical biomembrane substitutes, artificial kidneys, medical appliances, food containers, and cooker parts, as well as electronic equipment components such as connectors and printed circuit boards. Highly stable thermoplastic resins similar to polysulfone have been known to be polyether ether sulfone, polyphenyl sulfone, and the like, and all of them have excellent properties as materials, as with polysulfone. Furthermore, they are also characterized in that their main chains are partially configured of a bisphenol or hydroquinone that is also widely used in other organic materials, and are also highly valuable as molecular materials. As of 2019, the total production volume of polysulfone and polyphenyl sulfone was 26,600 tons, and the production volume of polyether sulfone was 16,870 tons, which seems, at first glance, small, however, considering that these are expensive resins, the production volume is by no means small. These super engineering plastics are, in the background of the above-described excellent properties, indispensable materials in industry, both now and in the future.

However, the high stability of these super engineering plastics leads to extreme difficulty in their disposal and recycling. For example, they contain many benzene ring backbones in their molecular structure, making it difficult to be decomposed by microorganisms. Therefore, they impose a heavy burden on the environment and may pose major problems in the future. In fact, the only some chemical recycling technologies for super engineering plastics that have been disclosed so far are those related to decomposition of polysulfones into smaller molecules (see Patent Literature 1) and decomposition of polyphenylene sulfide and polyether sulfone into a monomer unit and oligomer unit (see Patent Literature 2 and Non-Patent Literatures 1 to 6). Of these, the chemical recycling technology for polyether sulfone uses subcritical water, but is not suitable for large-scale implementation. On the other hand, a related reaction therewith has been known, whereby a methoxy group is substituted with an amino group in a para-methoxy-substituted benzophenone, which is one of constituent units of polyether ether ketone, using an organic superbase catalyst and an arylamine (see Non-Patent Literature 7), however, there are no examples of this method being applied to depolymerization of polysulfone, polyphenyl sulfone, polyether sulfone, and the like.

Therefore, if things continue as they are, not only will this impose a heavy burden on the environment, but will also be unable to address a future where use of non-recyclable plastics will be prohibited. Furthermore, since these super engineering plastics are high-priced products, the disposal thereof itself involves a large economic loss. Thus, development of new method for depolymerizing super engineering plastics has been desired.

### Citation List

### Patent Literature

Patent Literature 1: WO 2008/004642
Patent Literature 2: JP 2013-249324 A

### Non Patent Literature

Non Patent Literature 1: Yu, Z. L.; Miao, G.X.; Chen, Y. R. Macromol Chem Phys 1996, 197,4061.
Non Patent Literature 2: Wang, S. J.; Bian, S. G.; Yan, H.; Xiao, M.; Meng, Y. Z. J. App. Poly. Sci. 2008, 110, 4049.
Non Patent Literature 3: Lian, Z.; Bhawal, B. N.; Morandi, B. Science 2017, 356, 1059.
Non Patent Literature 4: Minami, Y.; Matsuyama, N.; Matsuo, Y.; Matsuo, Y.; Sato, K.; Nakajima, Y. Synthesis 2021, 53, 3351.
Non Patent Literature 5: Delcaillau, T.; Woenckhaus-Alvarez, A.; Morandi, B. Org. Lett. 2021, 23, 7018.
Non Patent Literature 6: Minami, Y.; Matsuyama, N.; Takeichi, Y.; Watanabe, R.; Mathew, S.; Nakajima, Y. Communications Chemistry, 2023, 6, 14. DOI: 10.1038/s42004-023-00814-8.
Non Patent Literature 7: Shigeno, M.; Hayashi, K.; Nozawa-Kumada, K.; Kondo, Y. Org. Lett. 2019, 21, 5505.

### Summary of Invention

### Technical Problem

An object of the present invention is to provide a novel method for depolymerizing (decomposing) a super engineering plastic such as polysulfone. Solution to Problem

In order to solve the above problems, the present invention adopts the following configuration.
[1] A method for decomposing a compound, comprising a decomposition step of reacting a first compound represented by the following general formula (1): 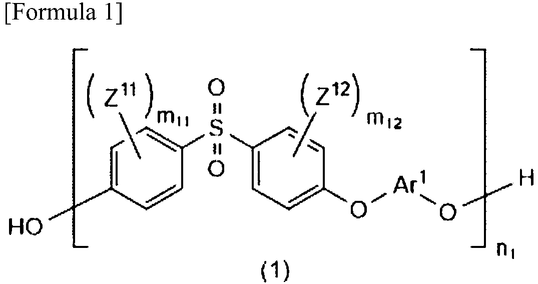
   (wherein n₁ is an integer of 2 or greater (preferably 10 to 200); Z¹¹ and Z¹² are each independently a group other than a hydrogen atom; m₁₁ and m₁₂ are each independently an integer of 0 to 4, and when m₁₁ is an integer of 1 or greater, n₁ × m₁₁ Z¹¹ groups are optionally the same or different from each other, and when m₁₂ is an integer of 1 or greater, n₁ × m₁₂ Z¹² groups are optionally the same or different from each other; Ar¹ is represented by the following general formula (91), (92) or (93) 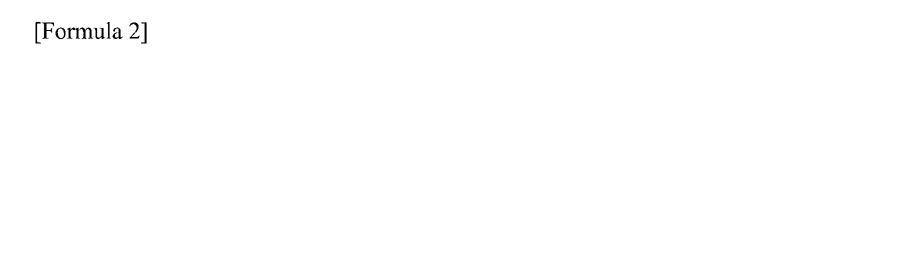 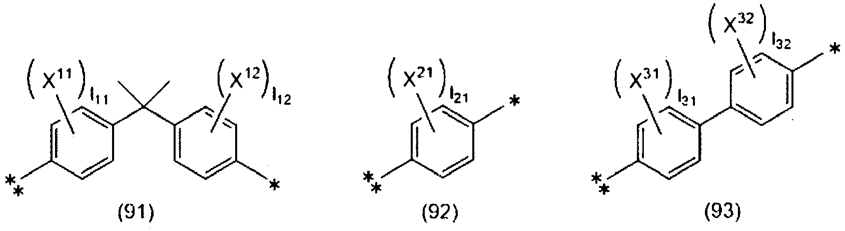
   (wherein X¹¹, X¹², X²¹, X³¹, and X³² are each independently a group other than a hydrogen atom; l₁₁, l₁₂, l₂₁, l₃₁, and l₃₂ are each independently an integer of 0 to 4, and when l₁₁ is an integer of 1 or greater, n₁ × l₁₁ X¹¹ groups are optionally the same or different from each other, when l₁₂ is an integer of 1 or greater, n₁ × l₁₂ X¹² groups are optionally the same or different from each other, when l₂₁ is an integer of 1 or greater, n₁ × l₂₁ X²¹ groups are optionally the same or different from each other, when l₃₁ is an integer of 1 or greater, n₁ × l₃₁ X³¹ groups are optionally the same or different from each other, and when l₃₂ is an integer of 1 or greater, n₁ × l₃₂ X³² groups are optionally the same or different from each other.), and the bond attached with the sign * and the bond attached with the sign ** in the general formulae (91), (92) and (93) above are each formed to an oxygen atom in the general formula (1).)
      with
      a second compound represented by the following general formula (8):

      R⁸-SH (8)
   (wherein R⁸ is an alkyl group, an aryl group, or an aralkyl group, and one or two or more hydrogen atoms in the alkyl group, aryl group, or aralkyl group are optionally substituted with a halogen atom, a hydroxyl group, a mercapto group, or a trialkoxysilyl group, and when the alkyl group, aryl group, or aralkyl group has one or two or more trimethylene groups therein, a central methylene group in the trimethylene group is optionally substituted with an oxycarbonyl group or a carbonyloxy group.), or a hydroxide, in the co-presence of a base to decomposed the first compound.
[2] A method for decomposing a compound, comprising a decomposition step of reacting a third compound represented by the following general formula (2): 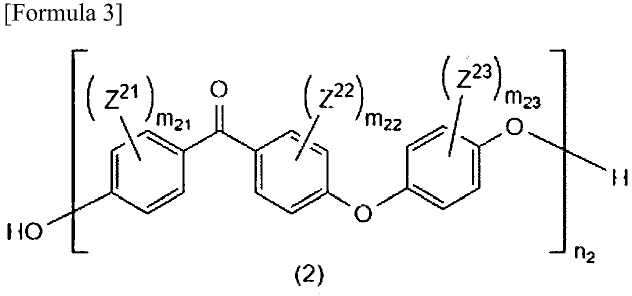
   (wherein n₂ is an integer of 2 or greater (preferably 10 to 200); Z²¹, Z²², and Z²³ are each independently a group other than a hydrogen atom; m₂₁, m₂₂, and m₂₃ are each independently an integer of 0 to 4, and when m₂₁ is an integer of 1 or greater, n₂ × m₂₁ Z²¹ groups are optionally the same or different from each other, when m₂₂ is an integer of 1 or greater, n₂ × m₂₂ Z²² groups are optionally the same or different from each other, and when m₂₃ is an integer of 1 or greater, n₂ × m₂₃ Z²³ groups are optionally the same or different from each other.)
      with
      a second compound represented by the following general formula (8):

      R⁸-SH (8)
   (wherein R⁸ is an alkyl group, an aryl group, or an aralkyl group, and one or two or more hydrogen atoms in the alkyl group, aryl group, or aralkyl group are optionally substituted with a halogen atom, a hydroxyl group, a mercapto group, or a trialkoxysilyl group, and when the alkyl group, aryl group, or aralkyl group has one or two or more trimethylene groups therein, a central methylene group in the trimethylene group is optionally substituted with an oxycarbonyl group or a carbonyloxy group.), or a hydroxide, in the co-presence of a base to obtain the third compound.
[3] A method for decomposing a compound, comprising a decomposition step of reacting a fourth compound represented by the following general formula (4): 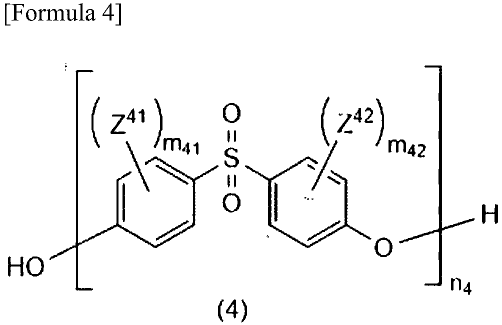
   (wherein n₄ is an integer of 2 or greater (preferably 10 to 200); Z⁴¹ and Z⁴² are each independently a group other than a hydrogen atom; m₄₁ and m₄₂ are each independently an integer of 0 to 4, and when m₄₁ is an integer of 1 or greater, n₄ × m₄₁ Z⁴¹ groups are optionally the same or different from each other, and when m₄₂ is an integer of 1 or greater, n₄ × m₄₂ Z⁴² groups are optionally the same or different from each other.)
      with
      a second compound represented by the following general formula (8):

      R⁸-SH (8)
   (wherein R⁸ is an alkyl group, an aryl group, or an aralkyl group, and one or two or more hydrogen atoms in the alkyl group, aryl group, or aralkyl group are optionally substituted with a halogen atom, a hydroxyl group, a mercapto group, or a trialkoxysilyl group, and when the alkyl group, aryl group, or aralkyl group has one or two or more trimethylene groups therein, a central methylene group in the trimethylene group is optionally substituted with an oxycarbonyl group or a carbonyloxy group.) in the co-presence of a base to decompose the fourth compound.
[4] The method for depolymerizing a compound according to any one of [1] to [3], comprising using at least a phosphazene base or sodium tert-butoxide as a base.
[5] The method for decomposing a compound according to any one of [1] to [3], wherein the compound represented by the general formula (8) above is one or two or more selected from the group consisting of an alkyl mercaptan having 1 to 15 carbon atoms, an aryl mercaptan having 6 to 12 carbon atoms, an aralkyl mercaptan having 7 to 14 carbon atoms, a halogenated alkyl mercaptan having 1 to 15 carbon atoms, a halogenated aryl mercaptan having 6 to 12 carbon atoms, a hydroxyalkyl mercaptan having 1 to 15 carbon atoms, a polymercaptoalkane having 1 to 15 carbon atoms, a (mercaptoalkyl)trialkoxysilane having 4 to 15 carbon atoms, and an alkoxycarbonylalkanethiol having 3 to 15 carbon atoms.
[6] The method for decomposing a compound according to any one of 1 to 3, further comprising using a dehydrating agent in the decomposition step.
[7] The method for decomposing a compound according to any one of [1] to [3], wherein the hydroxide is an alkali metal hydroxide.
[8] The method for decomposing a compound according to [6], wherein the dehydrating agent is one or two or more selected from the group consisting of sodium hydride, potassium hydride, calcium hydride, calcium oxide, cesium chloride, calcium chloride, magnesium sulfate, and zeolite.
[9] A method for producing an ether compound, comprising a step of etherifying one or more phenolic hydroxyl groups or one or more groups in which the phenolic hydroxyl group has formed its salt, *in* one or two or more selected from the group consisting of a compound represented by the following general formula (11): 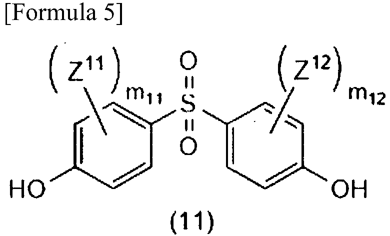
   (wherein Z¹¹, Z¹², m₁₁, and m₁₂ are the same as described above.) or a salt thereof,
   a compound represented by the following general formula (121): 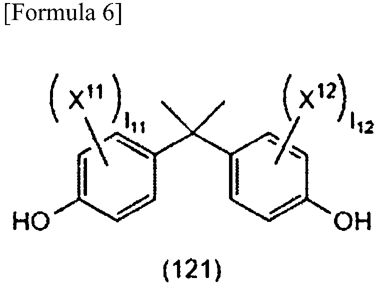
   (wherein X¹¹, X¹², l₁₁ and l₁₂ are the same as described above.) and a salt thereof,
   a compound represented by the following general formula (122): 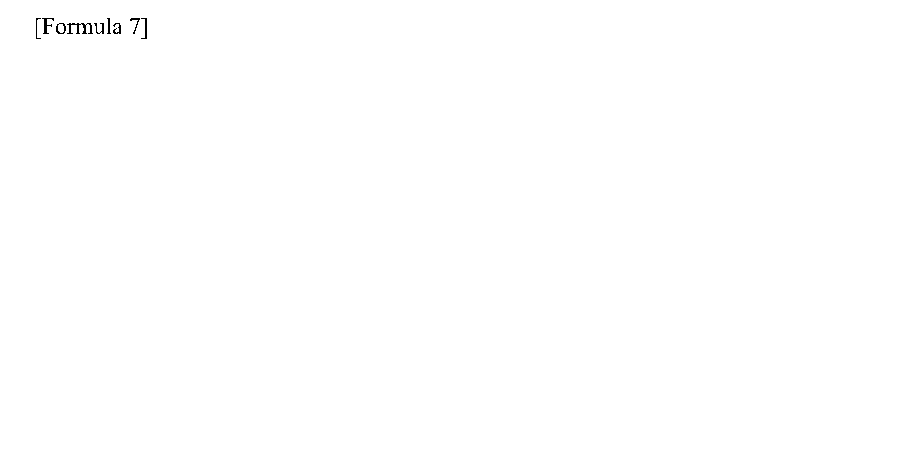 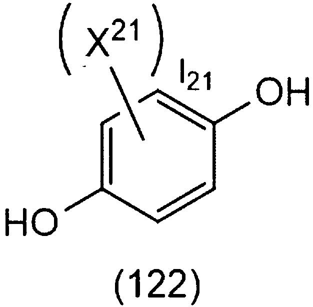
   (wherein X²¹ and l₂₁ are the same as described above.) and a salt thereof, as well as
   a compound represented by the following general formula (123): 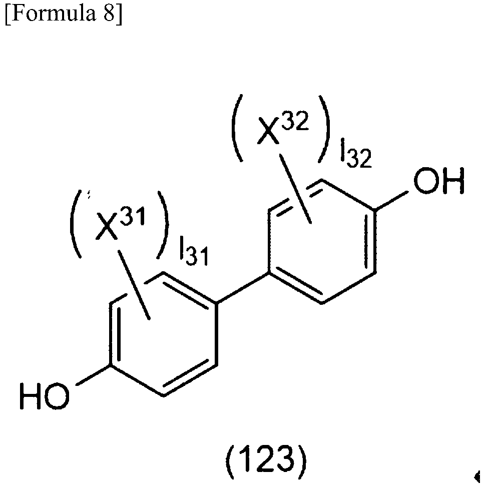
   (wherein X³¹, X³², l₃₁ and l₃₂ are the same as described above.) and a salt thereof,
   one or two or more selected from a compound represented by the following general formula (21): 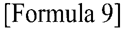 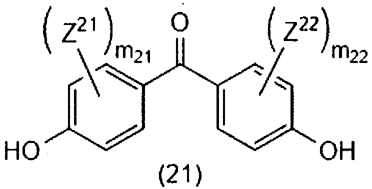
   (wherein Z²¹, Z²², m₂₁, and m₂₂ are the same as described above.) or a salt thereof, and
   one or two or more selected from a compound represented by the following general formula (22): 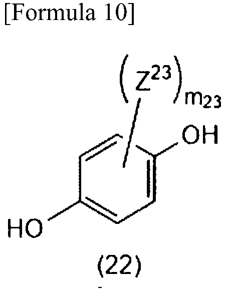
   (wherein Z²³ and m₂₃ are the same as those described above.) and a salt thereof,
   or one or two or more selected from the group consisting of a compound represented by the following general formula (41): 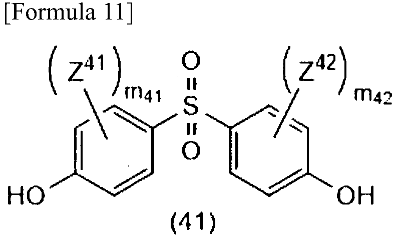
   (wherein Z⁴¹, Z⁴², m₄₁, and m₄₂ are the same as described above.) and a salt thereof,
   which have been obtained by the method for decomposing a compound according to [1] to [3], to obtain the ether compound.
[12] A method for decomposing a compound, comprising a decomposition step of reacting a fifth compound represented by the following general formula (3): 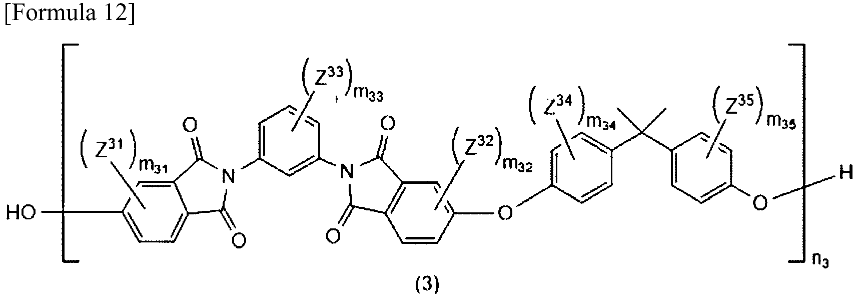
   (wherein, n₃ is an integer of 2 or greater (preferably 10 to 200); Z³¹, Z³², Z³³, Z³⁴, and Z³⁵ are each independently a group other than a hydrogen atom; m₃₁ and m₃₂ are each independently an integer of 0 to 3, and m₃₃, m₃₄, and m₃₅ are each independently an integer of 0 to 4, and when m₃₁ is an integer of 1 or greater, n₃ × m₃₁ Z³¹ groups are optionally be the same or different from each other, when m₃₂ is an integer of 1 or greater, n₃ × m₃₂ Z³² groups are the same or different from each other, when m₃₃ is an integer of 1 or greater, n₃ × m₃₃ Z³³ groups are optionally the same or different from each other, when m₃₄ is an integer of 1 or greater, n₃ × m₃₄ Z³⁴ groups are optionally the same or different from each other, and when m₃₅ is an integer of 1 or greater, n₃ × m₃₅ Z³⁵ groups are optionally the same or different from each other.)
      with
      a second compound represented by the following general formula (8):

      R⁸-SH (8)
   (wherein R⁸ is an alkyl group, an aryl group, or an aralkyl group, and one or two or more hydrogen atoms in the alkyl group, aryl group, or aralkyl group is optionally substituted with a halogen atom, a hydroxyl group, a mercapto group, or a trialkoxysilyl group, and when the alkyl group, aryl group, or aralkyl group has one or two or more trimethylene groups therein, a central methylene group in the trimethylene group is optionally substituted with an oxycarbonyl group or a carbonyloxy group.) in the co-presence of a base to decomposed the fifth compound.

### Advantageous Effects of Invention

According to the present invention, a novel method for depolymerizing (decomposing) a super engineering plastic such as polysulfone is provided.

### Brief Description of Drawings

Fig. 1 is the results of ¹H NMR analysis of a reaction mixture in Example 1 of embodiment 2.
Fig. 2 is the results of ¹H NMR analysis of a reaction mixture in Example 2 of embodiment 2.
Fig. 3 is the results of ¹H NMR analysis of a reaction mixture in Example 3 of embodiment 2.
Fig. 4 is the results of ¹H NMR analysis of a reaction mixture in Example 4 of embodiment 2.
Fig. 5 is the results of ¹H NMR analysis of a reaction mixture in Example 5 of embodiment 2.
Fig. 6 is the results of ¹H NMR analysis of a reaction mixture in Example 6 of embodiment 2.
Fig. 7 is the results of ¹H NMR analysis of a reaction mixture in Example 7 of embodiment 2.
Fig. 8 is the results of ¹H NMR analysis of a reaction mixture in Example 8 of embodiment 2.
Fig. 9 is the results of ¹H NMR analysis of a reaction mixture in Example 9 of embodiment 2.
Fig. 10 is the results of ¹H NMR analysis of a reaction mixture in Example 10 of embodiment 2.
Fig. 11 is the results of ¹H NMR analysis of a reaction mixture in Example 11 of embodiment 2.
Fig. 12 is the results of ¹H NMR analysis of a reaction mixture in Example 12 of embodiment 2.
Fig. 13 is the results of ¹H NMR analysis of bisphenol S in Example 13 of embodiment 2.
Fig. 14 is the results of ¹H NMR analysis of bisphenol A in Example 13 of embodiment 2.
Fig. 15 is the results of ¹H NMR analysis of a target substance in Example 14 of embodiment 2.
Fig. 16 is the results of ¹H NMR analysis of a target substance in Example 15 of embodiment 2.
Fig. 17 is the results of ¹H NMR analysis of a target substance in Example 16 of embodiment 2.
Fig. 18 is the results of ¹H NMR analysis of bisphenol S in Example 17 of embodiment 2.
19 is the results of ¹H NMR analysis of a target substance in Example 18 of embodiment 2.
Fig. 20 is the results of ¹H NMR analysis of bisphenol S in Example 19 of embodiment 2.
Fig. 21 is the results of ¹H NMR analysis of 4,4'-dihydroxybiphenyl in Example 19 of embodiment 2.
Fig. 22 is the results of ¹H NMR analysis of bisphenol S in Example 20 of embodiment 2.
Fig. 23 is the results of ¹H NMR analysis of a target substance in Example 21 of embodiment 2.
Fig. 24 is the results of ¹H NMR analysis of 4-((4-(4-hydroxyphenoxy)phenyl)sulfonyl)phenol in Example 22 of embodiment 2.
Fig. 25 is the results of ¹H NMR analysis of 4,4'-dihydroxybenzophenone in Example 23 of embodiment 2.
Fig. 26 is the results of ¹H NMR analysis of 4-((4-(4-hydroxyphenoxy)phenyl)sulfonyl)phenol in Example 23 of embodiment 2.

### Description of Embodiments

An embodiment of the method for decomposing a compound according to the present invention will be described below. Incidentally, the present invention is not limited to the following embodiments. Moreover, the contents of Japanese Patent Application No. 2023-016683 and Japanese Patent Application No. 2023-016684 are incorporated herein by reference as part of the present description.

In the present description, the unit of concentration "M" means "mol/L".

In the present description, when a compound is represented by a general formula or other formulae (non-generalized formulae; as used herein, they may be each simply referred to as "formula"), a sign may be attached with the general formula or other formulae. In such a case, the compound may be given a name with the sign attached. For example, a compound represented by the general formula (1) described below may be referred to as "compound (1)" as used herein.

### (Embodiment 1)

### ⊚ Depolymerization method of compound

### <<Depolymerization method (i)>>

The method for depolymerizing (decomposing) a compound according to one embodiment of the present invention comprises a depolymerization step (decomposition step) of reacting a compound (compound (1)) represented by the following general formula (1): 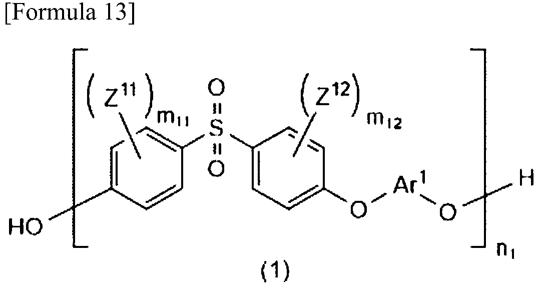
(wherein n₁ is an integer of 2 or greater (preferably 10 to 200); Z¹¹ and Z¹² are each independently a group other than a hydrogen atom; m₁₁ and m₁₂ are each independently an integer of 0 to 4, and when m₁₁ is an integer of 1 or greater, n₁ × m₁₁ Z¹¹ groups may be the same or different from each other, and when m₁₂ is an integer of 1 or greater, n₁ × m₁₂ Z¹² groups may be the same or different from each other; Ar¹ is represented by the following general formula (91), (92) or (93), and 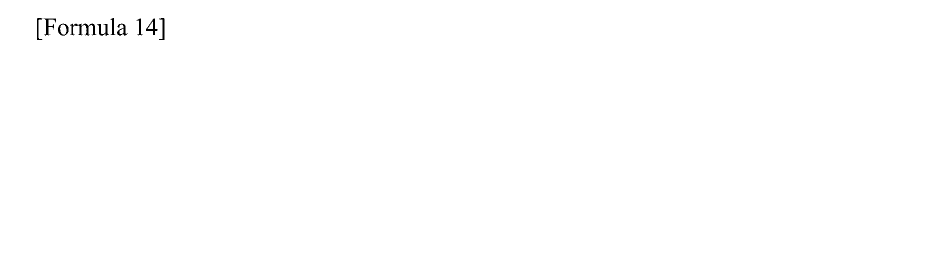 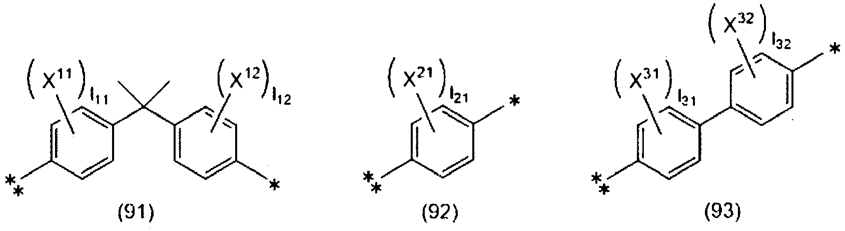
(wherein X¹¹, X¹², X²¹, X³¹, and X³² are each independently a group other than a hydrogen atom; l₁₁, l₁₂, l₂₁, l₃₁, and l₃₂ are each independently an integer of 0 to 4, and when l₁₁ is an integer of 1 or greater, n₁ × l₁₁ X¹¹ groups may be the same or different from each other, when l₁₂ is an integer of 1 or greater, n₁ × l₁₂ X¹² groups may be the same or different from each other, when l₂₁ is an integer of 1 or greater, n₁ × l₂₁ X²¹ groups may be the same or different from each other, when l₃₁ is an integer of 1 or greater, n₁ × l₃₁ X³¹ groups may be the same or different from each other, and when l₃₂ is an integer of 1 or greater, n₁ × l₃₂ X³² groups may be the same or different from each other.), and the bond attached with the sign * and the bond attached with the sign ** in the general formulae (91), (92) and (93) are each formed to an oxygen atom in the general formula (1).)
   with a compound represented by the following general formula (8) (may be referred to as "compound (8)" as used herein):

   R⁸-SH (8)
(wherein R⁸ is an alkyl group, an aryl group, or an aralkyl group, and one or two or more hydrogen atoms in the alkyl group, aryl group, or aralkyl group may be substituted with a halogen atom, a hydroxyl group, a mercapto group, or a trialkoxysilyl group, and when the alkyl group, aryl group, or aralkyl group has one or two or more trimethylene groups therein, a central methylene group in the trimethylene group may be substituted with an oxycarbonyl group or a carbonyloxy group.) in the co-presence of a base to obtain a compound represented by the following general formula (18) (may be referred to as "compound (18)" as used herein): 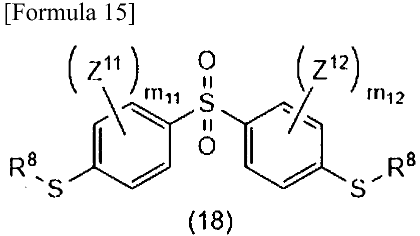
(wherein Z¹¹, Z¹², m₁₁, m₁₂, and R⁸ are the same as those described above.) and a compound represented by the following general formula (121), (122), or (123) (may be each referred to as "compound (121)," "compound (122)," or "compound (123)"as used herein) 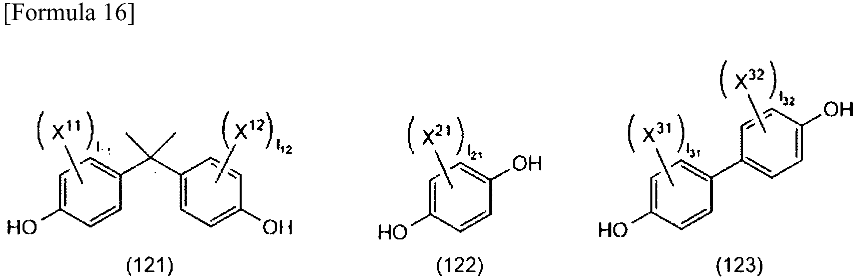
(wherein, X¹¹, X¹², X²¹, X³¹, X³², l₁₁, l₁₂, l₂₁, l₃₁ and l₃₂ are the same as described above.). As used herein, the method for depolymerizing (decomposing) a compound of the present embodiment may be referred to as "depolymerization method (i)".

The depolymerization method of the present embodiment (depolymerization method (i)) is a novel method for depolymerizing compound (1), which includes super engineering plastics.

The compound (1) has a benzene ring backbone to which an electron-withdrawing sulfonyl group (-SO₂-) is bonded in its structure, and has an electron-deficient aromatic ring group. In the depolymerization method (i), the compound (1) has such properties, and is depolymerized (decomposed) by the compound (8) in the co-presence of a base. Then, the compound (121), compound (122) or compound (123) is obtained as a depolymerized substance (product), and the compound (18), a thioether compound, which depends on the structure of the compound (8), is obtained.

When Ar¹ described above is the group represented by the general formula (91) above, the compound (1) described above includes polysulfone (may be referred to as "PSU" as used herein) and its derivative.

When Ar¹ described above is the group represented by the general formula (92) above, the compound (1) described above includes polyether ether sulfone (may be referred to as "PEES" as used herein) and its derivative.

When Ar¹ described above is the group represented by the general formula (93) above, the compound (1) described above includes polyphenyl sulfone (may be referred to as "PPSU" as used herein and its derivative.

As used herein, when a structure in which one or more hydrogen atoms in a certain specific compound have been substituted with a group other than a hydrogen atom, is assumed, the compound having such a substituted structure is referred to as a "derivative" of the aforementioned specific compound.

The term "group" as used herein includes not only an atomic group formed by bonding a plurality of atoms, but also a single atom, unless otherwise specified.

In a case in which Ar¹ is the group represented by the general formula (91) above, the depolymerization method (i) comprises a depolymerization step (decomposition step) by reacting a compound represented by the following general formula (1-1): 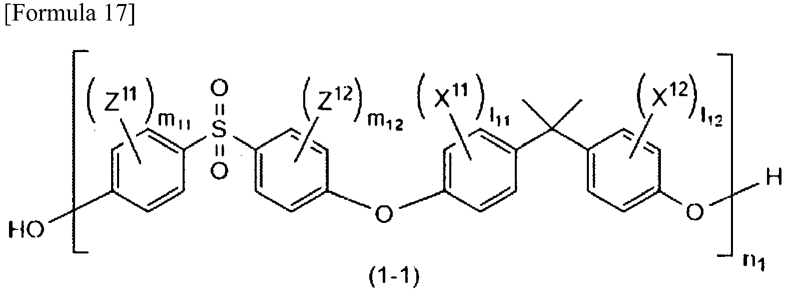
(wherein, n₁, Z¹¹, Z¹², m₁₁, m₁₂, X¹¹, X¹², l₁₁, and l₁₂ are the same as described above.),
   with a compound represented by the following general formula (8) (compound (8)):

   R⁸-SH (8)
(wherein R⁸ is an alkyl group, an aryl group, or an aralkyl group, and one or two or more hydrogen atoms in the alkyl group, aryl group, or aralkyl group may be substituted with a halogen atom, a hydroxyl group, a mercapto group, or a trialkoxysilyl group, and when the alkyl group, aryl group, or aralkyl group has one or two or more trimethylene groups therein, a central methylene group in the trimethylene group may be substituted with an oxycarbonyl group or a carbonyloxy group.) in the co-presence of a base to obtain a compound (compound (18)) represented by the following general formula (18): 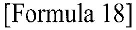 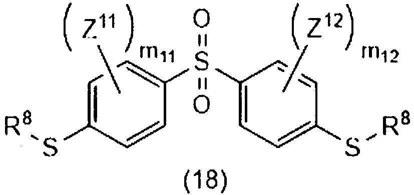
(wherein Z¹¹, Z¹², m₁₁, m₁₂, and R⁸ are the same as described above.) and a compound (compound (121)) represented by the following general formula (121): 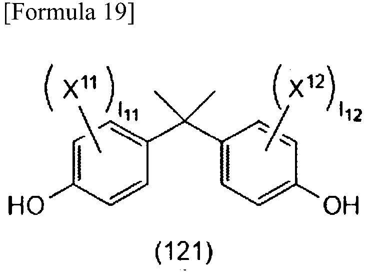
(wherein X¹¹, X¹², l₁₁ and l₁₂ are the same as described above.)

In a case in which Ar¹ is the group represented by the general formula (92) above, the depolymerization method (i) comprises a depolymerization step (decomposition step) by reacting a compound represented by the following general formula (1-2): [Formula 20] 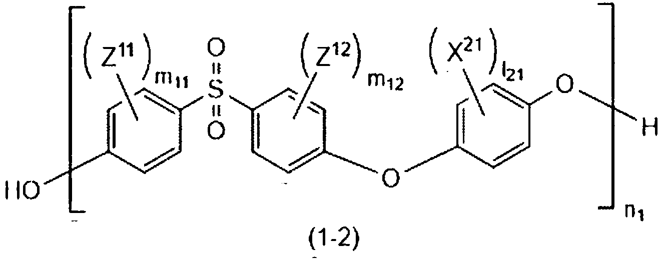
(wherein, n₁, Z¹¹, Z¹², m₁₁, m₁₂, X²¹ and l₂₁ are the same as described above.), with a compound represented by the following general formula (8) (compound (8)):

   R⁸-SH (8)
(wherein R⁸ is an alkyl group, an aryl group, or an aralkyl group, and one or two or more hydrogen atoms in the alkyl group, aryl group, or aralkyl group may be substituted with a halogen atom, a hydroxyl group, a mercapto group, or a trialkoxysilyl group, and when the alkyl group, aryl group, or aralkyl group has one or two or more trimethylene groups therein, a central methylene group in the trimethylene group may be substituted with an oxycarbonyl group or a carbonyloxy group.)
   in the co-presence of a base to obtain a compound (compound (18)) represented by the following general formula (18): 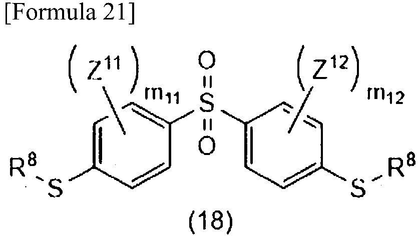
(wherein Z¹¹, Z¹², m₁₁, m₁₂, and R⁸ are the same as those described above.) and a compound (compound (122)) represented by the following general formula (121): 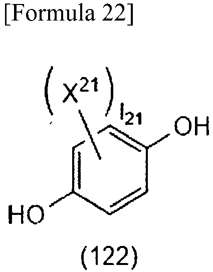
(wherein X²¹ and l₂₁ are the same as described above.)

In a case in which Ar¹ is the group represented by the general formula (93) above, the depolymerization method (i) comprises a depolymerization step (decomposition step) by reacting a compound represented by the following general formula (1-3): 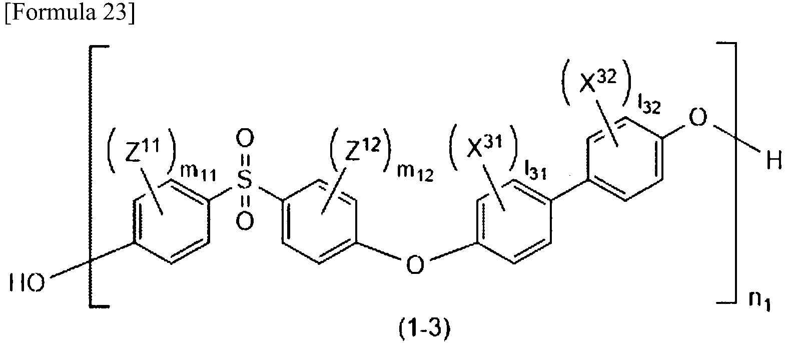
(wherein, n₁, Z¹¹, Z¹², m₁₁, m₁₂, X³¹, X³², l₃₁, and l₃₂ are the same as described above.)
   with a compound represented by the following general formula (8) (compound (8)):

   R⁸-SH (8)
(wherein R⁸ is an alkyl group, an aryl group, or an aralkyl group, and one or two or more hydrogen atoms in the alkyl group, aryl group, or aralkyl group may be substituted with a halogen atom, a hydroxyl group, a mercapto group, or a trialkoxysilyl group, and when the alkyl group, aryl group, or aralkyl group has one or two or more trimethylene groups therein, a central methylene group in the trimethylene group may be substituted with an oxycarbonyl group or a carbonyloxy group.) in the co-presence of a base to obtain a compound (compound (18)) represented by the following general formula (18): 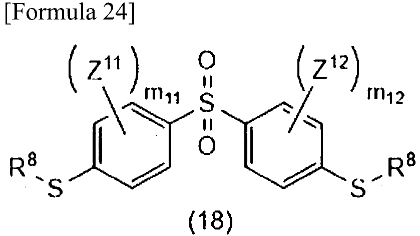
(wherein Z¹¹, Z¹², m₁₁, m₁₂, and R⁸ are the same as those described above.) and a compound (compound (123)) represented by the following general formula (123): 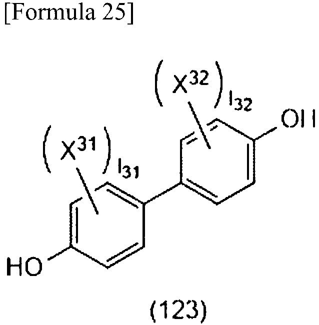
(wherein X³¹, X³², l₃₁ and l₃₂ are the same as described above.).

### <Compound (1)>

The compound (1) is an object of depolymerization in the depolymerization method (i).

In the general formula (1), n₁ is the number of repeating units, which defines the molecular size of the compound (1), and is an integer of 2 or greater.

The compound (1) in which n₁ is, for example, 10 to 200 in the formula is suitable as a high molecular weight super engineering plastic such as polysulfone (PSU), polyether ether sulfone (PEES) or polyphenyl sulfone (PPSU), which is difficult to be depolymerized by conventional methods and is particularly suitable as an applicable object for the depolymerization method (i).

In the general formula (1), Z¹¹ and Z¹² each independently represents a group other than a hydrogen atom (may be referred to as a "substituent" as used herein). That is, the substituents Z¹¹ and Z¹² may be the same or different from each other.

Examples of Z¹¹ and Z¹² (substituents) include an alkyl group, an alkylcarbonylamino group, a fluorinated alkyl group, and a fluorine atom.

The alkyl group in Z¹¹ and Z¹² may be any one of linear, branched-chained, and cyclic structures, and may have both a chain structure (a linear structure or a branched-chained structure) and a cyclic structure. The alkyl group having a cyclic structure as used herein is considered to be a cyclic alkyl group, regardless of whether or not it has an additional chain structure. A cyclic structure in a cyclic alkyl group (an alkyl group having a cyclic structure and no chain structure, as well as an alkyl group having both a cyclic structure and a chain structure) may be either monocyclic or polycyclic.

The alkyl group in Z¹¹ and Z¹² preferably has 1 to 15 carbon atoms.

Among the alkyl groups in Z¹¹ and Z¹², examples of the chained (linear or branched-chained) alkyl group include chained alkyl groups having 1 to 15 carbon atoms, such as a methyl group, an ethyl group, an n-propyl group, an isopropyl group, an n-butyl group, an isobutyl group, a sec-butyl group, a tert-butyl group, an n-pentyl group, an isopentyl group, a neopentyl group, a tert-pentyl group, a 1-methylbutyl group, an n-hexyl group, a 2-methylpentyl group, a 3-methylpentyl group, a 2,2-dimethylbutyl group, a 2,3-dimethylbutyl group, an n-heptyl group, a 2-methylhexyl group, a 3-methylhexyl group, a 2,2-dimethylpentyl group, a 2,3-dimethylpentyl group, a 2,4-dimethylpentyl group, a 3,3-dimethylpentyl group, a 3-ethylpentyl group, a 2,2,3-trimethylbutyl group, an n-octyl group, an isooctyl group, a 2-ethylhexyl group, a nonyl group, a decyl group, a 3,7-dimethyloctyl group, an undecyl group, a dodecyl group, a tridecyl group, a tetradecyl group, and a pentadecyl group.

Among the alkyl groups in Z¹¹ and Z¹², examples of the cyclic alkyl group (an alkyl group having a monocyclic or polycyclic structure) include cyclic alkyl groups having 3 to 15 carbon atoms, such as a cyclopropyl group, a cyclobutyl group, a cyclopentyl group, a cyclohexyl group, a cycloheptyl group, a cyclooctyl group, a cyclononyl group, a cyclodecyl group, a norbornyl group, an isobornyl group, a **1-**adamantyl group, a 2-adamantyl group, a tricyclodecyl group, and a cyclopropylmethyl group.

The alkyl group in Z¹¹ and Z¹² may be, for example, any one of an alkyl group having 1 to 10 carbon atoms (a linear alkyl group having 1 to 10 carbon atoms, or a cyclic alkyl group having 3 to 10 carbon atoms), an alkyl group having 1 to 8 carbon atoms (a linear alkyl group having 1 to 8 carbon atoms, or a cyclic alkyl group having 3 to 8 carbon atoms), an alkyl group having 1 to 6 carbon atoms (a linear alkyl group having 1 to 6 carbon atoms, or a cyclic alkyl group having 3 to 6 carbon atoms), and an alkyl group having 1 to 3 carbon atoms.

Examples of the alkylcarbonylamino group (a group represented by the general formula "-NH-C(=O)-R⁰¹ (wherein R⁰¹ is an alkyl group)") in Z¹¹ and Z¹² include monovalent groups having a structure in which a carbon atom having a free valence in the alkyl group in Z¹¹ and Z¹² described above is bonded to a carbon atom in a carbonylamino group (-NH-C(=O)-).

An example of the alkylcarbonylamino group includes, but is not limited to, a methylcarbonylamino group (-NH-C(=O)-CH₃).

The alkylcarbonylamino group in Z¹¹ and Z¹² preferably has 2 to 16 carbon atoms.

Examples of the fluorinated alkyl group in Z¹¹ and Z¹² include monovalent groups having a structure in which one or two or more hydrogen atoms (-H) in the alkyl group in Z¹¹ and Z¹² have been substituted with fluorine atoms (-F).

In the fluorinated alkyl group, the number of fluorine atoms depends on the number of carbon atoms in the fluorinated alkyl group, and is not particularly limited, and may be, for example, 1 to 3. The fluorinated alkyl group may be, for example, a perfluoroalkyl group (a monovalent group having a structure in which all hydrogen atoms in the alkyl group have been substituted with a fluorine atom) such as a trifluoromethyl group.

The fluorinated alkyl group in Z¹¹ and Z¹² preferably has 1 to 15 carbon atoms.

In the general formula (1), m₁₁ is the number of Z¹¹ bonded to one benzene ring backbone. m₁₂ is the number of Z¹² bonded to another benzene ring backbone different from the benzene ring backbone to which Z¹¹ is bonded. m₁₁ and m₁₂ each independently represents an integer of 0 to 4. That is, m₁₁ and m₁₂ may be the same as or different from each other.

The compound (1) has n₁ × m₁₁ Z¹¹ groups in one molecule, and when m₁₁ is an integer of 1 or greater (i.e., an integer of 1 to 4), the n₁ × m₁₁ Z¹¹ groups may be the same or different from each other. That is, when m₁₁ is an integer of 1 or more, the n₁ × m₁₁ Z¹¹ groups may all be the same, all be different, or may only partially be the same.

The same applies to Z¹², and compound (1) has n₁ × m₁₂ Z¹² groups in one molecule, and when m₁₂ is an integer of 1 or more (i.e., an integer of 1 to 4), the n₁ × m₁₂ Z¹² groups may be the same or different from each other. That is, when m₁₂ is an integer of 1 or more, the n₁ × m₁₂ Z¹² groups may all be the same, all be different, or may only partially be the same.

In the general formula (1), Ar¹ is the group represented by the general formula (91) above, the group represented by the general formula (92) above, or the group represented by the general formula (93) above.

The bond attached with the sign * of these groups is formed to one of the oxygen atoms that is not bonded to a sulfur atom (S) in the general formula (1), and the bond attached with the sign ** is formed to the other oxygen atom that is not bonded to the sulfur atom in the general formula (1).

In the general formula (91), X¹¹ and X¹² each independently represents a group (substituent) other than a hydrogen atom. That is, the substituents X¹¹ and X¹² may be the same or different from each other.

Examples of X¹¹ and X¹² (substituents) include the same as Z¹¹ and Z¹² above (for example, an alkyl group, an alkylcarbonylamino group, a fluorinated alkyl group, a fluorine atom, and the like).

In the general formula (91), l₁₁ is the number of X¹¹ bonded to one benzene ring backbone. l₁₂ is the number of X¹² bonded to another benzene ring backbone different from the benzene ring backbone to which X¹¹ is bonded. l₁₁ and l₁₂ each independently represents an integer of 0 to 4. That is, l₁₁ and l₁₂ may be the same as or different from each other.

The compound (1) has n₁ × l₁₁ X¹¹ groups in one molecule, and when l₁₁ is an integer of 1 or more (i.e., an integer of 1 to 4), the n₁ × l₁₁ X¹¹ groups may be the same or different from each other. That is, when l₁₁ is an integer of 1 or more, the n₁ × l₁₁ X¹¹ groups may all be the same, all be different, or may only partially be the same.

The same applies to X¹², and the compound (1) has n₁ × l₁₂ X¹² groups in one molecule, and when l₁₂ is an integer of 1 or more (i.e., an integer of 1 to 4), the n₁ × l₁₂ X¹² groups may be the same or different from each other. That is, when l₁₂ is an integer of 1 or more, the n₁ × l₁₂ X¹² groups may all be the same, all be different, or may only partially be the same.

In the general formula (91), l₁₁ and l₁₂ may each independently be, for example, any one of 0 to 3, 0 to 2, 0 to 1, and 0.

A preferred example of the group represented by the general formula (91) is a group in which l₁₁ and l₁₂ are 0 (that is, no X¹¹ or X¹² is present in the formula).

In the general formula (92) above, X²¹ is a group (substituent) other than a hydrogen atom.

Examples of X²¹ (substituent) include the same as Z¹¹ and Z¹² above (for example, an alkyl group, an alkylcarbonylamino group, a fluorinated alkyl group, a fluorine atom, and the like).

In formula (92), l₂₁ is the number of X²¹ bonded to one benzene ring backbone and is an integer of 0 to 4.

The compound (1) has n₁ × l₂₁ X²¹ groups in one molecule, and when l₂₁ is an integer of 1 or more (i.e., an integer of 1 to 4), the n₁ × l₂₁ X²¹ groups may be the same or different from each other. That is, when l₂₁ is an integer of 1 or more, the n₁ × l₂₁ X²¹ groups may all be the same, all be different, or may only partially be the same.

In general formula (92), l₂₁ may be, for example, any one of 0 to 3, 0 to 2, 0 or 1, and 0.

A preferred example of the group represented by the general formula (92) is a group in which l₂₁ is 0 (that is, X²¹ is not present in the formula.).

In the general formula (93), X³¹ and X³² each independently represents a group (substituent) other than a hydrogen atom. That is, the substituents X³¹ and X³² may be the same or different from each other.

Examples of X³¹ and X³² (substituents) include the same as Z¹¹ and Z¹² above (for example, an alkyl group, an alkylcarbonylamino group, a fluorinated alkyl group, a fluorine atom, and the like).

In the general formula (93), l₃₁ is the number of X³¹ bonded to one benzene ring backbone. l₃₂ is the number of X³² bonded to another benzene ring backbone different from the benzene ring backbone to which X³¹ is bonded. l₃₁ and l₃₂ each independently represents an integer of 0 to 4. That is, l₃₁ and l₃₂ may be the same as or different from each other.

The compound (1) has n₁ × l₃₁ X³¹ groups in one molecule, and when l₃₁ is an integer of 1 or greater (i.e., an integer of 1 to 4), the n₁ × l₃₁ X³¹ groups may be the same or different from each other. That is, when l₃₁ is an integer of 1 or more, the n₁ × l₃₁ X³¹ groups may all be the same, all be different, or may only partially be the same.

The same applies to X³², and the compound (1) has n₁ × l₃₂ X³² groups in one molecule, and when l₃₂ is an integer of 1 or greater (i.e., an integer of 1 to 4), the n₁ × l₃₂ X³² groups may be the same or different from each other. That is, when l₃₂ is an integer of 1 or more, the n₁ × l₃₂ X³² groups may all be the same, all be different, or may only partially be the same.

In the general formula (93), l₃₁ and l₃₂ may each independently be, for example, any one of 0 to 3, 0 to 2, 0 to 1, and 0.

A preferred example of the group represented by the general formula (93) is a group in which l₃₁ and l₃₂ are 0 (that is, no X³¹ or X³² is present in the formula).

A preferred example of the compound (1) includes a compound (1) in which m₁₁ and m₁₂ are 0 (i.e., the following formulae have no Z¹¹ and Z¹²) regardless of whether Ar¹ is any one of the group represented by the general formula (91), the group represented by the general formula (92), and the group represented by the general formula (93).

A more preferred example of the compound (1) includes a compound (1) in which Ar¹ is the group represented by the general formula (91), l₁₁ and l₁₂ are 0, and m₁₁ and m₁₂ are 0.

Another more preferred example of the compound (1) includes a compound (1) in which Ar¹ is the group represented by the general formula (92), l₂₁ is 0, and m₁₁ and m₁₂ are 0.

A further other more preferred example of the compound (1) includes a compound (1) in which Ar¹ is the group represented by the general formula (93), l₃₁ and l₃₂ are 0, and m₁₁ and m₁₂ are 0.

With the proviso that compound (1) is not limited thereto.

The compound (1) subjected to depolymerization may be reinforced with fibers. Namely, in the depolymerization method (i), the compound (1) reinforced with fibers can be depolymerized.

Examples of the fiber-reinforced compound (1) include a carbon fiber-reinforced compound (1) and a glass fiber-reinforced compound (1).

In the fiber-reinforced compound (1) (referred to as "fiber-reinforced resin" in this paragraph), the proportion of the content of the compound (1) relative to the total mass of the fiber-reinforced compound (1) (the mass of compound (1) contained in the fiber-reinforced resin/the total mass of the fiber-reinforced resin) × 100) is preferably 10 to 90% by mass, and may be, for example, any one of 10 to 70% by mass, 10 to 50% by mass, and 10 to 30% by mass, or any one of 30 to 90% by mass, 50 to 90% by mass, and 70 to 90% by mass, or may be 30 to 70% by mass. The proportion of the lower limit value or more further increases the yields of the compound (18) and compound (121), and compound (122) or compound (123). The proportion of the upper limit value or less enhances the versatility of fiber-reinforced compound (1).

A hydrogen atom in one or both (two) hydroxyl groups (-OHs, hydroxyl groups at one or both ends in the general formula (1)) in the compound (1) may be substituted with a group other than a hydrogen atom (hereinafter represented by the sign "M") to form a group represented by formula "-OM". Namely, the compound (1) may be a salt. The above-described formula "-OM" can also be represented as, for example, formula "-O⁻M⁺" .

In a case in which the hydrogen atoms in both hydroxyl groups in the compound (1) are substituted with the above-described M group, these two Ms may be the same or different.

An example of the above-described M group includes a counter cation, and it may be a metal atom (the salt of compound (1) may be an alkoxide). An example of the above-described M⁺ ion includes a counter cation, and it may be, for example, a phosphazenium cation.

### <Base>

The base accelerates the depolymerization of compound (1).

The base may be either an inorganic base (a basic inorganic compound) or an organic base (a basic organic compound).

Examples of the inorganic base include alkali metal hydroxides such as sodium hydroxide and potassium hydroxide; carbonates (alkali metal carbonates) such as potassium carbonate and cesium carbonate; and a phosphate (trialkali metal phosphate) such as tripotassium phosphate.

Examples of the organic base include alkali metal tert-butoxides such as lithium tert-butoxide, sodium tert-butoxide, and potassium tert-butoxide;
alkali metal bistrimethylsilylamides such as lithium bistrimethylsilylamide, sodium bistrimethylsilylamide, and potassium bistrimethylsilylamide;
diazabicycloundecene (DBU);
and phosphazene bases such as a phosphazene base P₄-t-Bu (alias: 1-tert-butyl-4,4,4-tris(dimethylamino)-2,2-bis[tris(dimethylamino)-phosphoranylideneamino]-2λ⁵,4λ⁵-catenadi(phosphazene)), a phosphazene base P₂-t-Bu (alias: 1-tert-butyl-2,2,4,4,4-pentakis(dimethylamino)-2λ⁵, 4λ⁵-catenadi(phosphazene)), a phosphazene base P₁-t-Bu (alias: tert-butylimino-tris(dimethylamino)phosphorane), a phosphazene base P₄-t-Oct(alias: 1-tert-octyl-4,4,4-tris(dimethylamino)-2,2-bis[tris(dimethylamino)-phosphoranylidencamino]-2λ⁵,4λ⁵-catenadi(phosphazene)), a phosphazene base P₂-Et (alias: 1-ethyl-2,2,4,4,4-pentakis(dimethylamino)-2λ⁵,4λ⁵-catenadi(phosphazene)), and a phosphazene base P₁-t-Bu-tris(tetramethylene) (alias: tert-butylimino-tris(pyrrolidino)phosphorane).

A structure of a representative phosphazene base is shown below.

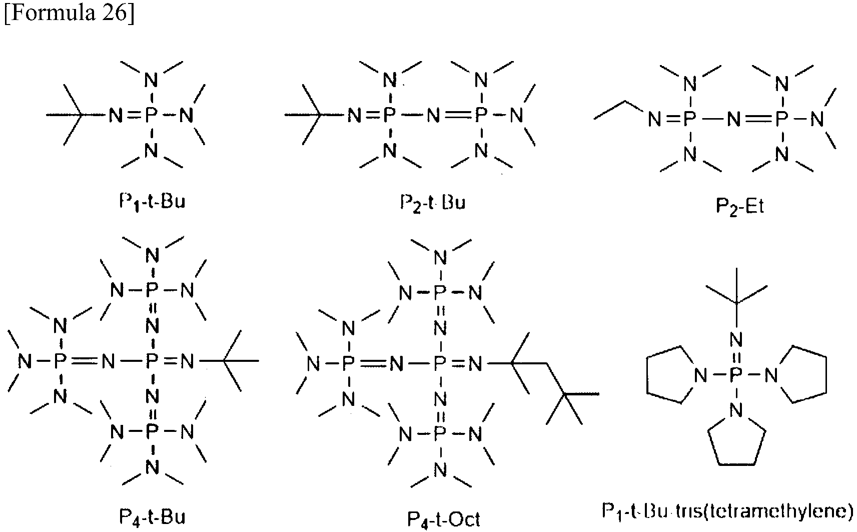

The base to be used in the depolymerization step may be used singly, or two or more thereof may be used, and in case of using the two or more, the combination and ratio thereof can be arbitrarily selected depending on the purpose.

For example, in case of using two or more bases, two or more inorganic bases may be used without using an organic base, two or more organic bases may be used without using an inorganic base, or one or more inorganic bases and one or more organic bases may be used together.

The base to be used in the depolymerization step is preferably one or two or more selected from the group consisting of a phosphazene base, a carbonate, and an alkali metal tert-butoxide, and more preferably one or two or more selected from the group consisting of a phosphazene base, cesium carbonate, sodium tert-butoxide, and potassium tert-butoxide. Using such a base facilitates the depolymerization of compound (1) to further proceed.

Among these, at least a phosphazene base or sodium tert-butoxide is preferably used as the base in the depolymerization step because they facilitate the depolymerization of compound (1) to particularly proceed, a phosphazene base in combination with an inorganic base, or sodium tert-butoxide is more preferably used, a phosphazene base in combination with a phosphate, or sodium tert-butoxide is still more preferably used, and a phosphazene base in combination with tripotassium phosphate, or sodium tert-butoxide, is particularly preferably used. In case of using sodium tert-butoxide, the sodium tert-butoxide may be used singly as the base or may be combined for use with another inorganic base.

In the depolymerization step, the amount (amount by mole) of base used is preferably 4 to 23 mol% relative to the amount (amount by mole) of repeating unit in the compound (1) (constituent unit attached with the sign n₁ in the general formula (1)), and may be, for example, any one of 4 to 13 mol%, 13 to 23 mol%, and 8 to 18 mol%. The amount of base used being the lower limit value or more facilitates the depolymerization of compound (1) to further proceed. The amount of base used being the upper limit value or less inhibits the base from being used excessively.

### <Compound (8)>

The compound (8) is a reaction object of the compound (1).

In the general formula (8), R⁸ is an alkyl group, an aryl group, or an aralkyl group, and one or two or more hydrogen atoms in the alkyl group, aryl group, or aralkyl group may be substituted with a halogen atom, a hydroxyl group (-OH), a mercapto group (-SH), or a trialkoxysilyl group (these groups may be collectively referred to as "monovalent substituents" as used herein), and in a case in which the alkyl group, aryl group, or aralkyl group has one or two or more trimethylene groups (-CH₂CH₂CH₂-) therein, the central methylene group in the trimethylene group may be substituted with an oxycarbonyl group (-O-C(=O)-) or a carbonyloxy group (-C(=O)-O-) (these groups may be collectively referred to as "divalent substituents" as used herein.).

An example of the alkyl group in R⁸ includes the same alkyl group as in Z¹¹ and Z¹². The alkyl group in R⁸ may be any one of linear, branched-chained, and cyclic groups, and may have both a chain structure (linear or branched-chained structure) and a cyclic structure. A cyclic structure in a cyclic alkyl group (an alkyl group having a cyclic structure and no chain structure, as well as an alkyl group having both a cyclic structure and a chain structure) may be either monocyclic or polycyclic.

The number of carbon atoms in the alkyl group in R⁸ is preferably 1 to 15. In other words, when R⁸ is an alkyl group, the compound (8) is preferably an alkyl mercaptan (alkanethiol) having 1 to 15 carbon atoms.

Examples of the compound (8) in which R⁸ is the chained (linear or branched-chained) alkyl group include alkyl mercaptans (alkanethiols) having 1 to 15 carbon atoms, such as methyl mercaptan, ethyl mercaptan, n-propyl mercaptan, isopropyl mercaptan, n-butyl mercaptan, isobutyl mercaptan, sec-butyl mercaptan, tert-butyl mercaptan, n-pentyl mercaptan, isopentyl mercaptan, neopentyl mercaptan, tert-pentyl mercaptan, 1-methylbutyl mercaptan, n-hexyl mercaptan, 2-methylpentyl mercaptan, 3-methylpentyl mercaptan, 2,2-dimethylbutyl mercaptan, 2,3-dimethylbutyl mercaptan, n-heptyl mercaptan, 2-methylhexyl mercaptan, 3-methylhexyl mercaptan, 2,2-dimethylpentyl mercaptan, 2,3-dimethylpentyl mercaptan, 2,4-dimethylpentyl mercaptan, 3,3-dimethylpentyl mercaptan, 3-ethylpentyl mercaptan, 2,2,3-trimethylbutyl mercaptan, n-octyl mercaptan, isooctyl mercaptan, 2-ethylhexyl mercaptan, nonyl mercaptan, decyl mercaptan, 3,7-dimethyloctyl mercaptan, undecyl mercaptan, dodecyl mercaptan, tridecyl mercaptan, tetradecyl mercaptan, and pentadecyl mercaptan.

Examples of the compound (8) in which R⁸ is the cyclic (monocyclic or polycyclic) alkyl group include cyclic alkyl mercaptans (alkanethiols) having 3 to 15 carbon atoms, such as cyclopropyl mercaptan, cyclobutyl mercaptan, cyclopentyl mercaptan, cyclohexyl mercaptan, cycloheptyl mercaptan, cyclooctyl mercaptan, cyclononyl mercaptan, cyclodecyl mercaptan, norbornyl mercaptan, isobornyl mercaptan, 1-adamantyl mercaptan, 2-adamantyl mercaptan, tricyclodecyl mercaptan, and cyclopropyl methyl mercaptan.

The alkyl group in R⁸ may be, for example, any one of an alkyl group having 1 to 15 carbon atoms (a chained alkyl group having 1 to 15 carbon atoms, or a cyclic alkyl group having 3 to 15 carbon atoms), an alkyl group having 1 to 10 carbon atoms (a chained alkyl group having 1 to 10 carbon atoms, or a cyclic alkyl group having 3 to 10 carbon atoms), an alkyl group having 1 to 8 carbon atoms (a chained alkyl group having 1 to 8 carbon atoms, or a cyclic alkyl group having 3 to 8 carbon atoms), an alkyl group having 1 to 6 carbon atoms (a chained alkyl group having 1 to 6 carbon atoms, or a cyclic alkyl group having 3 to 6 carbon atoms), and an alkyl group having 1 to 3 carbon atoms.

The aryl group in R⁸ may be either monocyclic or polycyclic.

The number of carbon atoms in the aryl group in R⁸ is preferably 6 to 15, and examples of the aryl group include a phenyl group, a 1-naphthyl group, a 2-naphthyl group, an o-tolyl group, a m-tolyl group, a p-tolyl group, a 4-tert-butylphenyl group, and a xylyl group (dimethylphenyl group), and further examples thereof also include groups having a structure in which one or two or more hydrogen atoms in these aryl groups have been substituted with the aryl group or the alkyl groups in Z¹¹ and Z¹². The number of carbon atoms of the aryl group having these substituents is preferably 6 to 15.

The number of carbon atoms of the aryl group in R⁸ is more preferably 6 to 12.

More specific examples of the compound (8) in which R⁸ is the aryl group include aryl mercaptans such as phenyl mercaptan, 1-naphthyl mercaptan, 2-naphthyl mercaptan, o-tolyl mercaptan (alias: o-toluenethiol), m-tolyl mercaptan (alias: m-toluenethiol), p-tolyl mercaptan (alias: p-toluenethiol), 4-tert-butylphenyl mercaptan (alias: 4-tert-butylbenzenethiol), and xylyl mercaptan (dimethylphenyl mercaptan). A further example thereof also includes mercaptan having a structure in which one or two or more hydrogen atoms in these aryl mercaptans have been substituted with the aryl group or the alkyl groups in Z¹¹ and Z¹².

The compound (8) in which R⁸ is the aryl group is preferably an aryl mercaptan having 6 to 12 carbon atoms.

An example of the aralkyl group in R⁸ include a monovalent group having a structure in which one hydrogen atom bonded to a carbon atom having no free valence in the alkyl group in Z¹¹ and Z¹² described above has been substituted with the aryl group in R⁸ described above.

The number of carbon atoms in the aralkyl group in R⁸ is preferably 7 to 17, and examples of the aralkyl group include a benzyl group (phenylmethyl group), a 4-tert-butylbenzyl group ((4-tert-butylphenyl)methyl group), a phenethyl group (phenylethyl group), a 4-tert-butylphenethyl group (2-(4-tert-butylphenyl)ethyl group), a 1-naphthylmethyl group, a 2-naphthylmethyl group, an o-tolylmethyl group, an m-tolylmethyl group, a p-tolylmethyl group, and a xylylmethyl group.

The number of carbon atoms of the aralkyl group in R⁸ is more preferably 7 to 14.

More specific examples of the compound (8) in which R⁸ is the aralkyl group include benzyl mercaptan (alias: phenyl methyl mercaptan), 4-tert-butyl benzyl mercaptan (alias: (4-tert-butylphenyl) methyl mercaptan or (4-tert-butylphenyl) methanethiol), phenethyl mercaptan (alias: 2-phenylethyl mercaptan), 4-tert-butyl phenethyl mercaptan (alias: 2-(4-tert-butylphenyl) ethyl mercaptan or 2-(4-tert-butylphenyl) ethanethiol), 1-naphthyl methyl mercaptan, 2-naphthyl methyl mercaptan, o-tolyl methyl mercaptan, m-tolyl methyl mercaptan, p-tolyl methyl mercaptan, and xylyl methyl mercaptan.

The compound (8) in which R⁸ is the aralkyl group is more preferably an aralkyl mercaptan having 7 to 14 carbon atoms.

Among the monovalent substituents in R⁸, examples of the halogen atom include a fluorine atom, a chlorine atom, a bromine atom, and an iodine atom.

In the trialkoxysilyl group which is the monovalent substituent in R⁸, an example of the alkoxy group constituting the group include a monovalent group having a structure in which a carbon atom having a free valence in the alkyl group in the above-described Z¹¹ and Z¹² has been bonded to an oxygen atom.

The alkoxy group may be linear, branched-chained, or cyclic, and may have both a chain structure (linear or branched-chained structure) and a cyclic structure. A cyclic structure in a cyclic alkoxy group (an alkoxy group that has a cyclic structure and no chain structure, and an alkoxy group that has both a cyclic structure and a chain structure) may be either monocyclic or polycyclic.

The number of carbon atoms of the alkoxy group is preferably 1 to 15.

Among the alkoxy groups, examples of the chained (linear or branched-chained) alkoxy group include chain alkoxy groups having 1 to 15 carbon atoms, such as a methoxy group, an ethoxy group, an n-propoxy group, an isopropoxy group, an n-butoxy group, an isobutoxy group, a sec-butoxy group, a tert-butoxy group, an n-pentyloxy group, an isopentyloxy group, a neopentyloxy group, a tert-pentyloxy group, a 1-methylbutyloxy group, an n-hexyloxy group, a 2-methylpentyloxy group, a 3-methylpentyloxy group, a 2,2-dimethylbutyloxy group, a 2,3-dimethylbutyloxy group, an n-heptyloxy group, a 2-methylhexyloxy group, 3-methylhexyloxy group, a 2,2-dimethylpentyloxy group, a 2,3-dimethylpentyloxy group, a 2,4-dimethylpentyloxy group, a 3,3-dimethylpentyloxy group, a 3-ethylpentyloxy group, a 2,2,3-trimethylbutyloxy group, an n-octyloxy group, an isooctyloxy group, a 2-ethylhexyloxy group, a nonyloxy group, a decyloxy group, a 3,7-dimethyloctyloxy group, an undecyloxy group, a dodecyloxy group, a tridecyloxy group, a tetradecyloxy group, and a pentadecyloxy group.

Among the alkoxy groups, examples of the cyclic (monocyclic or polycyclic) alkoxy groups include cyclic alkoxy groups having 3 to 15 carbon atoms, such as a cyclopropyloxy group, a cyclobutyloxy group, a cyclopentyloxy group, a cyclohexyloxy group, a cycloheptyloxy group, a cyclooctyloxy group, a cyclononyloxy group, a cyclodecyloxy group, a norbornyloxy group, an isobornyloxy group, a 1-adamantyloxy group, a 2-adamantyloxy group, a tricyclodecyloxy group, and a cyclopropylmethyloxy group.

The alkoxy group may be, for example, any one of an alkoxy group having 1 to 15 carbon atoms (a chained alkoxy group having 1 to 15 carbon atoms, or a cyclic alkoxy group having 3 to 15 carbon atoms), an alkoxy group having 1 to 10 carbon atoms (a chained alkoxy group having 1 to 10 carbon atoms, or a cyclic alkoxy group having 3 to 10 carbon atoms), an alkoxy group having 1 to 8 carbon atoms (a chained alkoxy group having 1 to 8 carbon atoms, or a cyclic alkoxy group having 3 to 8 carbon atoms), an alkoxy group having 1 to 6 carbon atoms (a chained alkoxy group having 1 to 6 carbon atoms, or a cyclic alkoxy group having 3 to 6 carbon atoms), and an alkoxy group having 1 to 3 carbon atoms.

In the trialkoxysilyl group which is the monovalent substituent in R⁸, the three alkoxy groups may be the same as or different from one another. That is, the three alkoxy groups may all be the same, all be different, or may only partially (two) be the same. When the two or three alkoxy groups are different from each other, a combination of these alkoxy groups is not particularly limited.

Of these, in terms of facilitation of availability or the production of the compound (8), the three alkoxy groups are all preferably the same.

Preferred examples of the trialkoxysilyl group which is the monovalent substituent in R⁸ include a trimethoxysilyl group and a triethoxysilyl group.

When one or two or more hydrogen atoms in the alkyl group, aryl group or aralkyl group in R⁸ are substituted with the monovalent substituent, the substituted position is not particularly limited.

The number of the hydrogen atoms substituted with the monovalent substituent is not particularly limited as long as it is the number of carbon atoms or less in the alkyl group, aryl group, or aralkyl group.

When the monovalent substituent is a hydroxyl group, a mercapto group, or a trialkoxysilyl group, the number of the hydrogen atoms substituted with these substituents is preferably 1 to 2 and more preferably 1.

When the monovalent substituent is a halogen atom, the number of the hydrogen atoms substituted with this substituent may be 1 to 2, or may be 3 or more, and all of the hydrogen atoms in the alkyl group, aryl group, or aralkyl group may have been substituted with halogen atoms. That is, the alkyl group, aryl group or aralkyl group in which one or two or more hydrogen atoms are substituted with halogen atoms may be a perhaloalkyl group, a perhaloaryl group or a perhaloaralkyl group.

When R⁸ is an alkyl group in which one or two or more hydrogen atoms are substituted with halogen atoms, an example of the compound (8) includes 3,3,4,4,5,5,6,6,7,7,8,8,9,9,10,10,10-heptadecafluorodecanethiol, and a halogenated alkyl mercaptan (halogenated alkanethiol) having 1 to 15 carbon atoms is preferable.

When R⁸ is an aryl group in which one or two or more hydrogen atoms are substituted with halogen atoms, an example of the compound (8) includes 4-chlorophenyl mercaptan (alias: 4-chlorobenzenethiol), and a halogenated aryl mercaptan having 6 to 12 carbon atoms is preferable.

When R⁸ is an alkyl group in which one or two or more hydrogen atoms are substituted with hydroxyl groups, an example of the compound (8) includes 2-hydroxyethyl mercaptan (alias: 2-hydroxyethanethiol), and a hydroxyalkyl mercaptan (hydroxyalkanethiol) having 1 to 15 carbon atoms is preferable.

When R⁸ is an alkyl group in which one or two or more hydrogen atoms are substituted with a mercapto group, an example of the compound (8) includes 1,2-dimercaptoethane (alias: 1,2-ethanedithiol), and a polymercaptoalkane having 1 to 15 carbon atoms (an alkane polythiol, an alkane having 2 or more mercapto groups) is preferable.

When R⁸ is an alkyl group in which one or two or more hydrogen atoms are substituted with a trialkoxysilyl group, an example of the compound (8) includes (3-mercaptopropyl)triethoxysilane (alias: 3-(triethoxysilyl)-1-propanethiol), and a (mercaptoalkyl)trialkoxysilanes having 4 to 15 carbon atoms is preferable.

The aryl group in R⁸ having one or two or more trimethylene groups corresponds to, for example, a group in which an alkyl group having 3 or more carbon atoms is bonded to a ring backbone of an aromatic hydrocarbon ring in the aryl group, and this alkyl group has a trimethylene group.

The aralkyl group in R⁸ having one or two or more trimethylene groups corresponds to, for example, either or both of the following groups: a group in which an alkylene group bonded to a ring backbone of an aromatic hydrocarbon ring in the aralkyl group has 3 or more carbon atoms and this alkylene group has a trimethylene group; and a group in which an alkyl group having 3 or more carbon atoms is bonded to a ring backbone of an aromatic hydrocarbon ring in the aralkyl group and this alkyl group has a trimethylene group.

In a case in which the alkyl group, aryl group or aralkyl group in R⁸ has one or two or more trimethylene groups (-CH₂CH₂CH₂-) therein, the central methylene group in the trimethylene group is a methylene group having two methylene groups (-CH₂-) bonded thereto.

The phrase "the central methylene group in the trimethylene group may be substituted with an oxycarbonyl group (-O-C(=O)-) or a carbonyloxy group (-C(=O)-O-) (divalent substituent)" means that in the substituted trimethylene group, the direction of a bond of the asymmetric group represented by formula "-C(=O)-O-" may be either one of the two directions.

As used herein, the carbon atom at one end of the trimethylene group may be bonded to a hydrogen atom; in other words, when the alkyl group, aryl group or aralkyl group is deemed to have a group represented by formula "CH₃CH₂CH₂-", this group may be a group represented by formula "CH₃-O-C(=O)-CH₂-" or a group represented by formula "CH₃-C(=O)-O-CH₂-" due to the divalent substituent.

When the central methylene group in one or two or more trimethylene groups, in the alkyl group, aryl group or aralkyl group in R⁸ is substituted with the divalent substituent, the substituted position is not particularly limited.

The number of the methylene groups substituted with the divalent substituent is not particularly limited as long as it is the number of trimethylene groups or less in the alkyl group, aryl group, or aralkyl group. Of these, the number of the methylene groups substituted with the divalent substituent is preferably 1 to 2 and more preferably 1.

When R⁸ is an alkyl group in which the central methylene group in one or two or more trimethylene groups are substituted with the above-described divalent substituent, examples of the compound (8) include 1-mercapto-2-methoxycarbonylethane (alias: 2-(methoxycarbonyl)ethanethiol, CH₃OC(=O)CH₂CH₂SH), and 1-mercapto-2-methylcarbonyloxyethane (alias: 2-(methylcarbonyloxy)ethanethiol, CH₃C(=O)OCH₂CH₂SH).

The compound (8) in which R⁸ is an alkyl group having the divalent substituent is more preferably an alkoxycarbonylalkanethiol having 3 to 15 carbon atoms.

Th compound (8) to be used in the depolymerization step may be used singly or two or more thereof may be used, and when the two or more are used, the combination and ratio thereof can be arbitrarily selected depending on the purpose. When the two or more compounds (8) are used, two or more of compounds (18) can be produced.

In terms of facilitating the depolymerization of compound (1) to further proceed, the compound (8) is preferably one or two or more selected from the group consisting of an alkyl mercaptan having 1 to 15 carbon atoms, an aryl mercaptan having 6 to 12 carbon atoms, an aralkyl mercaptan having 7 to 14 carbon atoms, a halogenated alkyl mercaptan having 1 to 15 carbon atoms, a halogenated aryl mercaptan having 6 to 12 carbon atoms, a hydroxyalkyl mercaptan having 1 to 15 carbon atoms, a polymercaptoalkane having 1 to 15 carbon atoms, a (mercaptoalkyl)trialkoxysilane having 4 to 15 carbon atoms, and an alkoxycarbonylalkanethiol having 3 to 15 carbon atoms.

In the depolymerization step, the amount (amount by mole) of compound (8) used may be, for example, 1.5 to 6 times the amount (amount by mole) of repeating unit in the compound (1) (constituent unit attached with the sign n₁ in the general formula (1)), but is preferably 2 to 6 times the amount by mole of repeating unit in the compound (1), more preferably 2 to 4 times the amount by mole thereof, and still more preferably 2 to 3 times the amount by mole thereof. The above-described amount of compound (8) used of the above-described lower limit value or more facilitates the depolymerization of compound (1) to further proceed. The above-described amount of compound (8) used of the above-described upper limit value or less inhibits the compound (8) from being used excessively.

### <Compound (18)>

The compound (18) is one of products by the depolymerization method (i).

The compound (18) is a derivative of bisphenol S (may be referred to as "BPS" as used herein).

Z¹¹, Z¹², m₁₁, and m₁₂ in the general formula (18) are the same as Z¹¹, Z¹², m₁₁, and m₁₂ in the general formula (1), respectively.

R⁸ in the general formula (18) is the same as R⁸ in the general formula (8).

In case of using the two or more compounds (8), the two R⁸s in the general formula (18) may be the same or different from each other.

### <Compound (121)>

The compound (121) is the other product by the depolymerization method (i) when Ar¹ is the group represented by the general formula (91).

The compound (121) is bisphenol A (may be referred to as "BPA" as used herein) and its derivative.

X¹¹, X¹², l₁₁, and l₁₂ in the general formula (121) are each the same as X¹¹, X¹², l₁₁, and l₁₂ in the general formula (91).

### <Compound (122)>

The compound (122) is the other product by the depolymerization method (i) when Ar¹ is the group represented by the general formula (92).

The compound (122) is hydroquinone (may be referred to as "HQ" as used herein) and its derivative.

X²¹ and l₂₁ in the general formula (122) are each the same as X²¹ and l₂₁ in the general formula (92).

### <Compound (123)>

The compound (123) is the other product by the depolymerization method (i) when Ar¹ is the group represented by the general formula (93).

The compound (123) is 4,4'-dihydroxybiphenyl (alias: 4,4'-biphenol) (may be referred to as "4,4'-DHBP" as used herein) and its derivative.

X³¹, X³², l₃₁, and l₃₂ in the general formula (123) are each the same as X³¹, X³², l₃₁, and l₃₂ in the general formula (93).

### <Solvent>

In the depolymerization step, it is preferable to further use a solvent. The use of solvent particularly allows the compound (1) to be dissolved in the solvent thereby to carry out the depolymerization step, facilitating the depolymerization of compound (1) to further proceed.

The term "solvent" as used herein encompasses both a component that is liquid at room temperature for dissolving a solute, and a component that is liquid at room temperature and functions as a dispersion medium for dispersing a dispersoid, unless otherwise specified. Also, the phrase "room temperature" means a temperature that is not particularly cooled or heated, that is, an ordinary temperature, and examples thereof include temperatures of 15 to 25°C.

The solvent is preferably an organic solvent.

Examples of the organic solvent include amides such as 1,3-dimethyl-2-imidazolidinone (DMI), N,N-dimethylacetamide (DMAc), N,N-dimethylformamide (DMF), and N-methyl-2-pyrrolidone (NMP); a nitrile such as benzonitrile; and an ether (cyclic ether) such as 1,4-dioxane.

The solvent may be used singly in the depolymerization step or two or more thereof may be used therein, and in the case of the two or more, the combination and ratio of the solvents may be arbitrarily selected depending on the purpose.

In case of using the solvent in the depolymerization step, the amount of solvent used is preferably 0.2 to 1.5 L relative to 100 g of compound (1), and may be, for example, any of 0.2 to 1 L and 0.2 to 0.6 L, or any of 0.4 to 1.5 L and 0.6 to 1.5 L, or 0.4 to 1 L. The above-described amount of solvent used that is the above-described lower limit value or more facilitates the depolymerization of compound (1) to further proceed. The above-described amount of solvent used being the above-described upper limit value or less inhibits the solvent from being used excessively.

### <Other component>

In the depolymerization step, the compound (1) may be depolymerized using other components that do not fall under any of the compound (1), a base, the compound (8), and a solvent, if necessary, as long as the effects of the present invention are not impaired, or the compound (1) may be depolymerized without using the other components.

The other components can be arbitrarily selected depending on the purpose, and are not particularly limited.

The other components to be used in the depolymerization step may be the single component or two or more thereof, and in the case of the two or more, the combination and ratio thereof can be arbitrarily selected depending on the purpose.

When the compound (1) undergoes depolymerization in the depolymerization step, the proportion of the total amount used (parts by mass) of a base, the compound (8), and a solvent to the total amount used (parts by mass) of the base, the compound (8), the solvent, and the other components (([amount (parts by mass) of base used] + [amount (parts by mass) of compound (8) used] + [amount (parts by mass) of solvent used])/([amount (parts by mass) of base used] + [amount (parts by mass) of compound (8) used] + [amount (parts by mass) of solvent used] + [amount (parts by mass) of other components used]) × 100) is preferably 80% by mass or more, more preferably 90% by mass or more, and still more preferably 95% by mass or more, and may be, for example, any one of 97% by mass or more and 99% by mass or more. The proportion of the lower limit value or more facilitates the depolymerization of compound (1) to further proceed.

The proportion is, on the other hand, 100% by mass or less.

When no optional component (i.e., a solvent or the other components) is used upon the depolymerization of compound (1), the amount of optional component used when calculating the proportion is 0 parts by mass.

### <Other conditions>

In the depolymerization step, the compound (1) can be depolymerized by mixing the compound (1), a base, the compound (8), a solvent, if necessary, and the other components, if necessary, and heating and stirring the resulting mixture.

In the depolymerization step, a temperature (reaction temperature) at which the mixture is heated and stirred may be room temperature or higher, and is preferably 90°C or higher, and may be, for example, either 110°C or higher and 140°C or higher. The higher the reaction temperature, the more easily the depolymerization of compound (1) proceeds.

The reaction temperature is, on the other hand, in terms of inhibiting by-production of impurities, preferably 200°C or lower and more preferably 160°C or lower, and may be, for example, 120°C or lower.

In the depolymerization step, a time (reaction time) for heating and stirring the mixture is preferably 1 hour or more, more preferably 8 hours or more, and still more preferably 15 hours or more. The reaction time of the lower limit value or more results in the higher yield of the compound (18), compound (121), compound (122), or compound (123).

The reaction time is, on the other hand, preferably 70 hours or less, more preferably 40 hours or less, and still more preferably 20 hours or less, in terms of avoiding an excessively long reaction time.

The above-described reaction time is particularly suitable when the reaction temperature is within the above range.

In the depolymerization step, the depolymerization of compound (1) may be carried out in an atmosphere of inert gas such as argon gas, helium gas, or nitrogen gas, or in an air atmosphere.

In the depolymerization method (i), after completion of the depolymerization step, the reaction mixture obtained may be subjected to post-treatment by a publicly known method, if necessary, to be able to take out the target substance (product). In other words, post-treatment operations such as filtration, cleaning, extraction, pH adjustment, dehydration, and concentration may be appropriately carried out, if necessary, in a single manner among them or in combination of two or more thereof, and the target substance can be taken out by concentration, crystallization, reprecipitation, column chromatography, or the like. Also, the taken out target substance may be further purified, if necessary, by carrying out one or more times any one of operations such as crystallization, reprecipitation, column chromatography, extraction, and stirring and washing of crystals with a solvent, in a single manner among these operations or in combination of two or more thereof. Alternatively, after completion of the depolymerization step, the resulting reaction mixture may be subjected to post-treatment and then used for the next intended application without taking out the target substance. For example, the target substance may be subjected to the next intended reaction without being taken out.

The structure of the product by the depolymerization method (i) can be confirmed by publicly known techniques such as nuclear magnetic resonance (NMR) spectroscopy, mass spectrometry (MS), and infrared spectroscopy (IR).

### <<Depolymerization method (ii)>>

The method for depolymerizing a compound according to one embodiment of the present invention comprises the depolymerization step (decomposition step) of reacting a compound represented by the following general formula (2) (may be referred to as "compound (2)" as used herein): 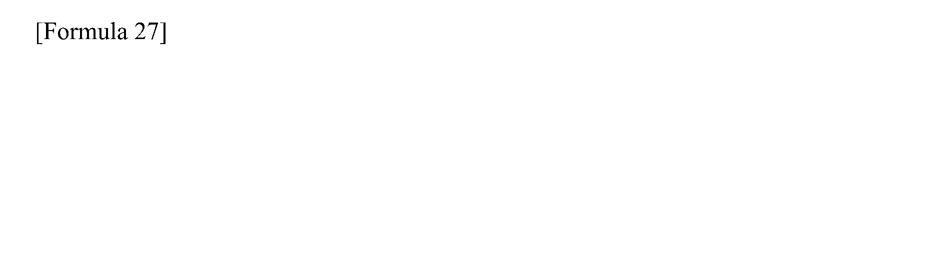 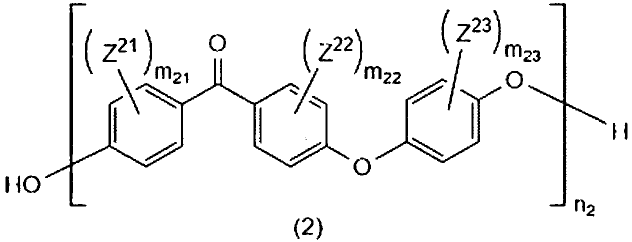
(wherein n₂ is an integer of 2 or greater (preferably 10 to 200); Z²¹, Z²², and Z²³ are each independently a group other than a hydrogen atom; m₂₁, m₂₂, and m₂₃ are each independently an integer of 0 to 4, and when m₂₁ is an integer of 1 or greater, n₂ × m₂₁ Z²¹ groups may be the same or different from each other, when m₂₂ is an integer of 1 or greater, n₂ × m₂₂ Z²² groups may be the same or different from each other, and when m₂₃ is an integer of 1 or greater, n₂ × m₂₃ Z²³ groups may be the same or different from each other.) with a compound (compound (8)) represented by the following general formula (8):

   R⁸-SH (8)
(wherein R⁸ is an alkyl group, an aryl group, or an aralkyl group, and one or two or more hydrogen atoms in the alkyl group, aryl group, or aralkyl group may be substituted with a halogen atom, a hydroxyl group, a mercapto group, or a trialkoxysilyl group, and when the above-described alkyl group, aryl group, or aralkyl group has one or two or more trimethylene groups therein, a central methylene group in the above-described trimethylene group may be substituted with an oxycarbonyl group or a carbonyloxy group.)
   in the co-presence of a base to obtain a compound represented by the following general formula (28) (may be referred to as "compound (28)" as used herein): 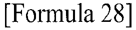 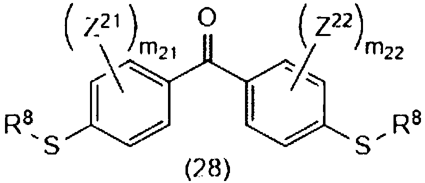
(wherein Z²¹, Z²², m₂₁, m₂₂, and R⁸ are the same as described above.) and a compound represented by the following general formula (22) (may be referred to as "compound (22)" as used herein): 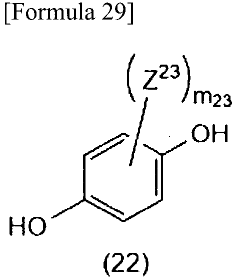
(wherein Z²³ and m₂₃ are the same as described above.). As used herein, the depolymerization method of a compound of the present embodiment may be referred to as "depolymerization method (ii)".

The depolymerization method of the present embodiment (depolymerization method (ii)) is a novel method for depolymerizing the compound (2), which includes super engineering plastics.

The compound (2) has a benzene ring backbone to which an electron-withdrawing carbonyl group (-CO-) is bonded in its structure, and has an electron-deficient aromatic ring group. In the depolymerization method (ii), the compound (2) is depolymerized by the compound (8) in the co-presence of a base due to such characteristics. Then, the compound (22) is obtained as a depolymerized substance (product), as well as the compound (28), a thioether compound, which depends on the structure of the compound (8), is obtained.

The depolymerization method (ii) is the same as the depolymerization method (i), except that the compound (2) is used instead of the compound (1), that is, an object for depolymerization is different.

The compound (2) includes polyether ether ketone (may be referred to as "PEEK" as used herein) and its derivative.

### <Compound (2)>

The compound (2) is an object for depolymerization in the depolymerization method (ii).

In the general formula (2), n₂ is the number of repeating units, which defines the molecular size of the compound (2), and is an integer of 2 or greater.

The compound (2) in which n₂, is, for example, 20 to 200 in the formula, is suitable as polyether ether ketone (PEEK), a high molecular weight super engineering plastic, which is difficult to be depolymerized using conventional methods and is particularly suitable as an applicable object of the depolymerization method (ii).

In the general formula (2), Z²¹, Z²², and Z²³ are each independently a group (substituent) other than a hydrogen atom. In other words, the substituents Z²¹, Z²², and Z²³ may all be the same, all be different, or may only partially (any two thereof) be the same.

Examples of Z²¹, Z²², and Z²³ (substituents) include the same as Z¹¹ and Z¹² above (for example, an alkyl group, an alkylcarbonylamino group, a fluorinated alkyl group, a fluorine atom, and the like).

In the general formula (2), m₂₁ is the number of Z²¹ bonded to one benzene ring backbone. m₂₂ is the number of Z²² bonded to another benzene ring backbone different from the benzene ring backbone to which Z²¹ is bonded. m₂₃ is the number of Z²³ bonded to another benzene ring backbone different from both the benzene ring backbone to which Z²¹ is bonded and the benzene ring backbone to which Z²² is bonded.

m₂₁, m₂₂, and m₂₃ are each independently an integer of 0 to 4. That is, m₂₁, m₂₂, and m₂₃ may all be the same, all be different, or may only partially (any two thereof) be the same.

The compound (2) has n₂ × m₂₁ Z²¹ groups in one molecule, and when m₂₁ is an integer of 1 or more (i.e., an integer of 1 to 4), the n₂ × m₂₁ Z²¹ groups may be the same or different from each other. That is, when m₂₁ is an integer of 1 or greater, the n₂ × m₂₁ Z²¹ groups may all be the same, all be different, or may only partially be the same.

The same applies to Z²², and the compound (2) has n₂ × m₂₂ Z²² groups in one molecule, and when m₂₂ is an integer of 1 or greater (i.e., an integer of 1 to 4), the n₂ × m₂₂ Z²² groups may be the same or different from each other. That is, when m₂₂ is an integer of 1 or greater, the n₂ × m₂₂ Z²² groups may all be the same, all be different, or may only partially be the same.

The same applies to Z²³, and the compound (2) has n₂ × m₂₃ Z²³ groups in one molecule, and when m₂₃ is an integer of 1 or greater (i.e., an integer of 1 to 4), the n₂ × m₂₃ Z²³ groups may be the same or different from each other. That is, when m₂₃ is an integer of 1 or greater, the n₂ × m₂₃ Z²³ groups may all be the same, all be different, or may only partially be the same.

A preferred example of the compound (2) includes a compound (2) in which m₂₁, m₂₂, and m₂₃ are 0 (i.e., Z²¹, Z²², and Z²³ are not present in the formula.).

With the proviso that the compound (2) is not limited thereto.

The compound (2) subjected to depolymerization may be reinforced with fibers. That is, in the depolymerization method (ii), the fiber-reinforced compound (2) can be depolymerized.

Examples of the fiber-reinforced compound (2) include a carbon fiber-reinforced compound (2) and a glass fiber-reinforced compound (2).

In the fiber-reinforced compound (2), the proportion of the content of the compound (2) to the total mass of fiber-reinforced compound (2) ([content of the compound (2) in the fiber-reinforced compound (2) (parts by mass)]/[total mass of fiber-reinforced compound (2) (parts by mass)] × 100) is preferably 10 to 90% by mass, and may be, for example, any one of 10 to 70% by mass, 10 to 50% by mass, and 10 to 30% by mass, or any one of 30 to 90% by mass, 50 to 90% by mass, and 70 to 90% by mass, or 30 to 70% by mass. The proportion of the lower limit value or more results in the higher yields of the compound (28) and compound (22). The proportion of the upper limit or less enhances the versatility of the fiber-reinforced compound (2).

The hydrogen atom in one or both hydroxyl groups in the compound (2) (-OHs, hydroxyl groups at one or both ends in the general formula (2)) may be substituted with a group (M) other than a hydrogen atom to form a group represented by formula "-OM" or a group represented by formula "-O⁻M⁺" as in the case of the compound (1). Namely, the compound (2) may be a salt.

The manner whereby the compound (2) forms a salt is the same as the manner whereby the compound (1) forms a salt. That is, the salt of compound (2) is also formed in the same manner as the salt of compound (1), and is the same as the salt of compound (1) except that the group represented by formula "-OM" is bonded to a different group.

### <Base>

The base in the depolymerization method (ii) is the same as the base in the depolymerization method (i).

The base to be used in the depolymerization step of the depolymerization method (ii) may be the single base or two or more thereof, and in the case of the two or more, the combination and ratio thereof can be arbitrarily selected according to the purpose.

For example, in case of using two or more bases, two or more inorganic bases may be used without using an organic base, two or more organic bases may be used without using an inorganic base, or one or more inorganic bases and one or more organic bases may be used together.

The base to be used in the depolymerization step of the depolymerization method (ii) is preferably one or two or more selected from the group consisting of a phosphazene base, a carbonate, and an alkali metal tert-butoxide and more preferably one or two or more selected from the group consisting of a phosphazene base, cesium carbonate, sodium tert-butoxide, and potassium tert-butoxide. Using such a base facilitates the depolymerization of compound (2) to further proceed.

Among these, at least a phosphazene base or sodium tert-butoxide is preferably used as the base in the depolymerization step because they facilitate the depolymerization of compound (2) to particularly proceed, a phosphazene base in combination with an inorganic base, or sodium tert-butoxide is more preferably used, a phosphazene base in combination with a phosphate, or sodium tert-butoxide is still more preferably used, and a phosphazene base in combination with tripotassium phosphate, or sodium tert-butoxide, is particularly preferably used. In case of using sodium tert-butoxide, the sodium tert-butoxide may be used singly as the base or may be combined for use with another inorganic base.

In the depolymerization step of the depolymerization method (ii), the amount (amount by mole) of base used is preferably 4 to 23 mol% relative to the amount (amount by mole) of repeating unit in the compound (2) (a constituent unit attached with the sign n₂ in the general formula (2)) and may be, for example, any one of 4 to 13 mol%, 13 to 23 mol%, and 8 to 18 mol%. The amount of base used being the lower limit value or more facilitates the depolymerization of compound (2) to further proceed. The amount of base used being the upper limit value or less inhibits the base from being used excessively.

### <Compound (8)>

The compound (8) in the depolymerization method (ii) is the same as the compound (8) in the depolymerization method (i).

The compound (8) to be used in the depolymerization step of the depolymerization method (ii) may be the single compound or two or more thereof, and in the case of the two or more, the combination and ratio thereof can be arbitrarily selected according to the purpose. When the two or more compounds (8) are used, two or more of compounds (28) can be produced.

A preferred example of the compound (8) in the depolymerization method (ii) includes the same as the preferable compound (8) in the depolymerization method (i).

In the depolymerization step of the depolymerization method (ii), the amount (amount by mole) of compound (8) used may be, for example, 1.5 to 6 times the amount (amount by mole) of repeating unit (a constituent unit attached with the sign n₂ in the general formula (2)) in the compound (2), but is preferably 2 to 6 times the amount, more preferably 2 to 4 times the amount, and still more preferably 2 to 3 times the amount. The amount of compound (8) used of the lower limit value or more facilitates the depolymerization of compound (2) to further proceed. The amount of compound (8) used of the upper limit value or less inhibits the compound (8) from being used excessively.

### <Compound (28)>

The compound (28) is one of products by the depolymerization method (ii).

The compound (28) is a derivative of 4,4'-dihydroxybenzophenone.

Z²¹, Z²², m₂₁, and m₂₂ in the general formula (28) are the same as Z²¹, Z²², m₂₁, and m₂₂ in the general formula (2), respectively.

R⁸ in the general formula (28) is the same as R⁸ in the general formula (8).

In case of using the two or more compounds (8), the two R⁸s in the general formula (28) may be the same or different from each other.

### <Compound (22)>

The compound (22) is the other product by the depolymerization method (ii).

The compound (22) is 1,4-dihydroxybenzene (alias: hydroquinone) and its derivative.

Z²³ and m₂₃ in the general formula (22) are the same as Z²³ and m₂₃ in the general formula (2), respectively.

### <Solvent>

In the depolymerization step of the depolymerization method (ii), it is preferable to further use a solvent. The use of solvent particularly allows the compound (2) to be dissolved in the solvent to carry out the depolymerization step, facilitating the depolymerization of compound (2) to further proceed.

The solvent in the depolymerization method (ii) is the same as the solvent in the depolymerization method (i).

The solvent to be used in the depolymerization step of the depolymerization method (ii) may be the single solvent, or two or more thereof, and in the case of the two or more, the combination and ratio thereof can be arbitrarily selected according to the purpose.

When a solvent is used in the depolymerization step of the depolymerization method (ii), the amount of solvent used is preferably 0.2 to 1.5 L relative to 100 g of compound (2) used, and may be, for example, any one of 0.2 to 1 L and 0.2 to 0.6 L, or any one of 0.4 to 1.5 L and 0.6 to 1.5 L, or 0.4 to 1 L. The amount of solvent used that is the lower limit value or more facilitates the depolymerization of compound (2) to further proceed. The amount of solvent used being the upper limit value or less inhibits the solvent from being used excessively.

### <Other component>

In the depolymerization step of the depolymerization method (ii), the compound (2) may be depolymerized using other components that do not fall under any of the compound (2), a base, the compound (8), and a solvent, if necessary, as long as the effects of the present invention are not impaired, or the compound (2) may be depolymerized without using the other components.

The other components in the depolymerization method (ii) are the same as the other components in the depolymerization method (i).

The other components to be used in the depolymerization step of the depolymerization method (ii) may be the single component or two or more thereof, and when the two or more are used, the combination and ratio thereof can be selected arbitrarily according to the purpose.

In depolymerizing the compound (2) in the depolymerization step of the depolymerization method (ii), the proportion of the total amount used (parts by mass) of a base, the compound (8), and a solvent to the total amount used (parts by mass) of the base, the compound (8), the solvent, and other components (([amount (parts by mass) of base used] + [amount (parts by mass) of compound (8) used] + [amount (parts by mass) of solvent used])/([amount (parts by mass) of base used] + [amount (parts by mass) of compound (8) used] + [amount (parts by mass) of solvent used] + [amount (parts by mass) of other components used]) × 100) is preferably 80% by mass or more, more preferably 90% by mass or more, and still more preferably 95% by mass or more, and may be, for example, any one of 97% by mass or more and 99% by mass or more. The proportion of the lower limit value or more facilitates the depolymerization of compound (2) to further proceed.

The proportion is, on the other hand, 100% by mass or less.

When no optional component (i.e., a solvent or the other components) is used upon the depolymerization of compound (2), the amount of optional component used when calculating the proportion is 0 parts by mass.

### <Other conditions>

In the depolymerization step of the depolymerization method (ii), the compound (2), a base, the compound (8), a solvent, if necessary, and the other components, if necessary, are mixed, and the resulting mixture is heated and stirred, whereby the compound (2) can be depolymerized.

In the depolymerization step of the depolymerization method (ii), a temperature (reaction temperature) when the mixture is heated and stirred, a time (reaction time) when the mixture is heated and stirred, and an atmosphere upon the depolymerization of compound (2) are the same as the temperature (reaction temperature) when the mixture is heated and stirred, the time (reaction time) when the mixture is heated and stirred, and the atmosphere upon the depolymerization of compound (1) in the depolymerization step of the depolymerization method (i), respectively.

In the depolymerization method (ii), after completion of the depolymerization step, the obtained reaction mixture may be post-treated, if necessary in the same manner as in the depolymerization method (i) to be able to take out the target substance (product), and the target substance taken out may be further purified, if necessary. Alternatively, after completion of the depolymerization step, the obtained reaction mixture may undergo post-treatment, if necessary in the same manner as in the depolymerization method (i), and then used for the next intended purpose without taking out the target substance.

The structure of the product by the depolymerization method (ii) can be confirmed by publicly known techniques, such as nuclear magnetic resonance (NMR) spectroscopy, mass spectrometry (MS), and infrared spectroscopy (IR).

### <<Depolymerization (iii)>>

The method for depolymerizing a compound according to one embodiment of the present invention comprises the depolymerization step (decomposition step) of reacting a compound represented by the following general formula (3) (may be referred to as "compound (3)" as used herein): 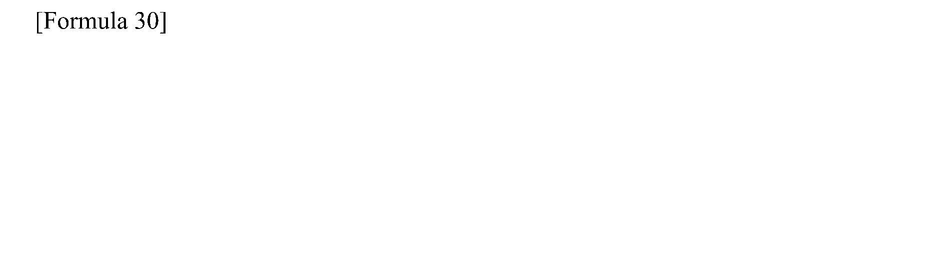 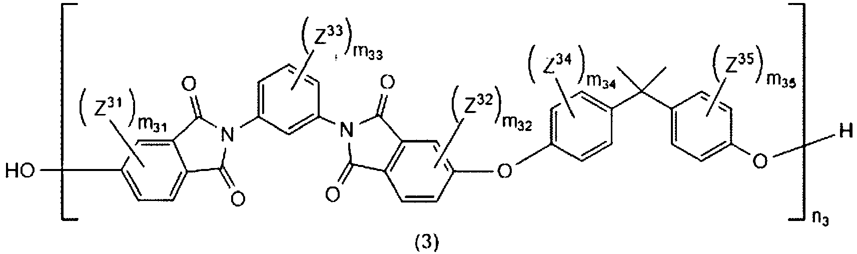
(wherein, n₃ is an integer of 2 or greater (preferably 10 to 200); Z³¹, Z³², Z³³, Z³⁴, and Z³⁵ are each independently a group other than a hydrogen atom; m₃₁ and m₃₂ are each independently an integer of 0 to 3, and m₃₃, m₃₄, and m₃₅ are each independently an integer of 0 to 4, and when m₃₁ is an integer of 1 or greater, n₃ × m₃₁ Z³¹ groups may be the same or different from each other, when m₃₂ is an integer of 1 or greater, n₃ × m₃₂ Z³² groups may be the same or different from each other, when m₃₃ is an integer of 1 or greater, n₃ × m₃₃ Z³³ groups may be the same or different from each other, when m₃₄ is an integer of 1 or greater, n₃ × m₃₄ Z³⁴ groups may be the same or different from each other, and when m₃₅ is an integer of 1 or greater, n₃ × m₃₅ Z³⁵ groups may be the same or different from each other.)
   with a compound (8) represented by the following general formula (8) (compound (8)):

   R⁸-SH (8)
(wherein R⁸ is an alkyl group, an aryl group, or an aralkyl group, and one or two or more hydrogen atoms in the alkyl group, aryl group, or aralkyl group may be substituted with a halogen atom, a hydroxyl group, a mercapto group, or a trialkoxysilyl group, and when the alkyl group, aryl group, or aralkyl group has one or two or more trimethylene groups therein, a central methylene group in the trimethylene group may be substituted with an oxycarbonyl group or a carbonyloxy group.)
   in the co-presence of a base to obtain a compound represented by the following general formula (38) (may be referred to as "compound (38)" as used herein): 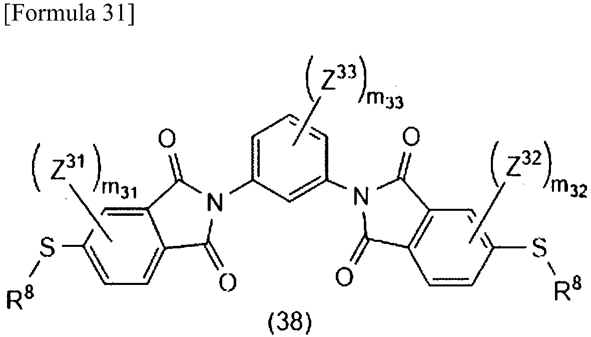
(wherein, Z³¹, Z³², Z³³, m₃₁, m₃₂, m₃₃ and R⁸ are the same as described above.) and a compound represented by the following general formula (32) (may be referred to referred to as "compound (32)" as used herein): 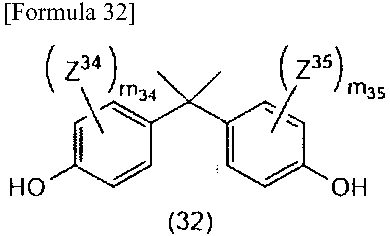
(wherein Z³⁴, Z³⁵, m₃₄, and m₃₅ are the same as described above.). As used herein, the method for depolymerizing a compound of the present embodiment may be referred to as "depolymerization method (iii)".

The depolymerization method of the present embodiment (depolymerization method (iii)) is a novel method for depolymerizing the compound (3), which includes super engineering plastics.

The compound (3) has a benzene ring backbone to which an electron-withdrawing carbonyl group (-CO-) is bonded in its structure, and has an electron-deficient aromatic ring group. In the depolymerization method (iii), the compound (3) is depolymerized by the compound (8) in the presence of a base, due to the characteristics of the compound (3). Then, the compound (32) is obtained as a depolymerized substance (product), as well as the compound (38), a thioether compound, which depends on the structure of the compound (8), is obtained.

The depolymerization method (iii) is the same as the depolymerization method (i), except that the compound (3) is used instead of the compound (1), that is, an object of depolymerization is different.

The compound (3) includes polyetherimide (may be referred to as "PEI" as used herein) and its derivative.

### <Compound (3)>

The compound (3) is an object of depolymerization in the depolymerization method (iii).

In the general formula (3), n₃ is the number of repeating units, which defines the molecular size of the compound (3), and is an integer of 2 or greater.

The compound (3) in which n₃, is, for example, 10 to 100 in the formula is suitable as polyetherimide (PEI) which is a high molecular weight super engineering plastic, is difficult to be depolymerized by conventional methods and is particularly suitable as an applicable object of the depolymerization method (iii).

In the general formula (3), Z³¹, Z³², Z³³, Z³⁴, and Z³⁵ are each independently a group (substituent) other than a hydrogen atom. That is, the substituents Z³¹, Z³², Z³³, Z³⁴, and Z³⁵ may all be the same, all be different, or may only partially (any two, three or four among them) be the same.

Examples of Z³¹, Z³², Z³³, Z³⁴, and Z³⁵ (substituents) include the same as Z¹¹ and Z¹² above (for example, an alkyl group, an alkylcarbonylamino group, a fluorinated alkyl group, a fluorine atom, and the like).

In the general formula (3), m₃₁ is the number of Z³¹ bonded to one benzene ring backbone. m₃₂ is the number of Z³² bonded to another benzene ring backbone different from the benzene ring backbone to which Z³¹ is bonded. m₃₃ is the number of Z³³ bonded to another benzene ring backbone different from both the benzene ring backbone to which Z³¹ is bonded and the benzene ring backbone to which Z³² is bonded. m₃₄ is the number of Z³⁴ bonded to another benzene ring backbone different from any of the benzene ring backbone to which Z³¹ is bonded, the benzene ring backbone to which Z³² is bonded, and the benzene ring backbone to which Z³³ is bonded. m₃₅ is the number of Z³⁵ bonded to another benzene ring backbone different from any one of the benzene ring backbone to which Z³¹ is bonded, the benzene ring backbone to which Z³² is bonded, the benzene ring backbone to which Z³³ is bonded, and the benzene ring backbone to which Z³⁴ is bonded.

m₃₁ and m₃₂ each independently represents an integer of 0 to 3. That is, m₃₁ and m₃₂ may be the same as or different from each other.

m₃₃, m₃₄, and m₃₅ are each independently an integer of 0 to 4. That is, m₃₃, m₃₄, and m₃₅ may all be the same, all be different, or may only partially (any two thereof) be the same.

The compound (3) has n₃ × m₃₁ Z³¹ groups in one molecule, and when m₃₁ is an integer of 1 or more (i.e., an integer of 1 to 3), the n₃ × m₃₁ Z³¹ groups may be the same or different from each other. That is, when m₃₁ is an integer of 1 or greater, the n₃ × m₃₁ Z³¹ groups may all be the same, all be different, or may only partially be the same.

The same applies to Z³², and the compound (3) has n₃ × m₃₂ Z³² groups in one molecule, and when m₃₂ is an integer of 1 or greater (i.e., an integer of 1 to 3), the n₃ × m₃₂ Z³² groups may be the same or different from each other. That is, when m₃₂ is an integer of 1 or greater, the n₃ × m₃₂ Z³² groups may all be the same, all be different, or may only partially be the same.

The same applies to Z³³, and the compound (3) has n₃ × m₃₃ Z³³ groups in one molecule, and when m₃₃ is an integer of 1 or greater (i.e., an integer of 1 to 4), the n₃ × m₃₃ Z³³ groups may be the same or different from each other. That is, when m₃₃ is an integer of 1 or greater, the n₃ × m₃₃ Z³³ groups may all be the same, all be different, or may only partially be the same.

The same applies to Z³⁴, and the compound (3) has n₃ × m₃₄ Z³⁴ groups in one molecule, and when m₃₄ is an integer of 1 or greater (i.e., an integer of 1 to 4), the n₃ × m₃₄ Z³⁴ groups may be the same or different from each other. That is, when m₃₄ is an integer of 1 or greater, the n₃ × m₃₄ Z³⁴ groups may all be the same, all be different, or may only partially be the same.

The same applies to Z³⁵, and the compound (3) has n₃ × m₃₅ Z³⁵ groups in one molecule, and when m₃₅ is an integer of 1 or greater (i.e., an integer of 1 to 4), the n₃ × m₃₅ Z³⁵ groups may be the same or different from each other. That is, when m₃₅ is an integer of 1 or greater, the n₃ × m₃₅ Z³⁵ groups may all be the same, all be different, or may only partially be the same.

A preferred example of the compound (3) is a compound (3) in which m₃₁, m₃₂, m₃₃, m₃₄, and m₃₅ are 0 (i.e., no Z³¹, Z³², Z³³, Z³⁴, and Z³⁵ are present in the formula.).

With the proviso that the compound (3) is not limited thereto.

The compound (3) subjected to depolymerization may be reinforced with fibers. In other words, in the depolymerization method (iii), the fiber-reinforced compound (3) can be depolymerized.

Examples of the fiber-reinforced compound (3) include a carbon fiber-reinforced compound (3) and a glass fiber-reinforced compound (3).

In the fiber-reinforced compound (3), the proportion of the content of the compound (3) to the total mass of fiber-reinforced compound (3) ([content of the compound (3) in the fiber-reinforced compound (3) (parts by mass)]/[total mass of fiber-reinforced compound (3) (parts by mass)] × 100) is preferably 10 to 90% by mass, and may be, for example, any one of 10 to 70% by mass, 10 to 50% by mass, and 10 to 30% by mass, or any one of 30 to 90% by mass, 50 to 90% by mass, and 70 to 90% by mass, or 30 to 70% by mass. The proportion of the lower limit value or more results in the higher yields of the compound (38) and compound (32). The proportion of the upper limit value or less enhances the versatility of fiber-reinforced compound (3).

The hydrogen atom in one or both hydroxyl groups (-OHs, hydroxyl groups at one or both ends in the general formula (3)) in the compound (3) may be substituted with a group (M) other than a hydrogen atom to form a group represented by formula "-OM" or a group represented by formula "-O⁻M⁺" as in the case of the compound (1). Namely, the compound (3) may be a salt.

The manner whereby the compound (3) forms a salt is the same as the manner whereby the compound (1) forms a salt. That is, the salt of compound (3) is also formed in the same manner as the salt of compound (1), and is the same as the salt of compound (1) except that the group represented by formula "-OM" is bonded to a different group.

### <Base>

The base in the depolymerization method (iii) is the same as the base in the depolymerization method (i).

The base to be used in the depolymerization step of the depolymerization method (iii) may be the single base or two or more thereof, and in the case of the two or more, the combination and ratio thereof can be arbitrarily selected according to the purpose.

For example, in case of using two or more bases, two or more inorganic bases may be used without using an organic base, two or more organic bases may be used without using an inorganic base, or one or more inorganic bases and one or more organic bases may be used together.

The base to be used in the depolymerization step of the depolymerization method (iii) is preferably one or two or more selected from the group consisting of a phosphazene base, a carbonate, and an alkali metal tert-butoxide, and more preferably one or two or more selected from the group consisting of a phosphazene base, cesium carbonate, sodium tert-butoxide, and potassium tert-butoxide. Using such a base facilitates the depolymerization of compound (3) to further proceed.

Among these, at least a phosphazene base or sodium tert-butoxide is preferably used as the base in the depolymerization step because they facilitate the depolymerization of compound (3) to particularly proceed, a phosphazene base in combination with an inorganic base, or sodium tert-butoxide is more preferably used, a phosphazene base in combination with a phosphate, or sodium tert-butoxide is still more preferably used, and a phosphazene base in combination with tripotassium phosphate, or sodium tert-butoxide, is particularly preferably used. In case of using sodium tert-butoxide, the sodium tert-butoxide may be used singly as the base or may be combined for use with another inorganic base.

In the depolymerization step of the depolymerization method (iii), the amount (amount by mole) of base used is preferably 4 to 23 mol% relative to the amount (amount by mole) of repeating unit (the constituent unit attached with the sign n₂ in the general formula (3)) in the compound (3), and may be, for example, any one of 4 to 13 mol%, 13 to 23 mol%, and 8 to 18 mol%. The amount of base used being the lower limit value or more facilitates the depolymerization of compound (3) to further proceed. The amount of base used being the upper limit value or less inhibits the base from being used excessively.

### <Compound (8)>

The compound (8) in the depolymerization method (iii) is the same as the compound (8) in the depolymerization method (i).

The compound (8) to be used in the depolymerization step of the depolymerization method (iii) may be the single compound or two or more thereof, and in the case of the two or more, the combination and ratio thereof can be arbitrarily selected according to the purpose. When the two or more compounds (8) are used, two or more of compounds (38) can be produced.

Preferred examples of the compound (8) in the depolymerization method (iii) include the same as the preferred compound (8) in the depolymerization method (i).

In the depolymerization step of the depolymerization method (iii), the amount (amount by mole) of compound (8) used may be, for example, 1.5 to 6 times the amount (amount by mole) of repeating unit (the constituent unit attached with the sign n₃ in the general formula (3)) in the compound (3), but is preferably 2 to 6 times the amount, more preferably 2 to 4 times the amount, and still more preferably 2 to 3 times the amount. The amount of compound (8) used of the lower limit value or more facilitates the depolymerization of compound (3) to further proceed. The amount of compound (8) used of the upper limit value or less inhibits the compound (8) from being used excessively.

### <Compound (38)>

The compound (38) is one of products by the depolymerization method (iii).

The compound (38) is a derivative of N,N'-(1,3-phenylene)bis(5-hydroxyphthalimide).

Z³¹, Z³², Z³³, m₃₁, m₃₂ and m₃₃ in the general formula (38) are the same as Z³¹, Z³², Z³³, m₃₁, m₃₂ and m₃₃ in the general formula (3).

R⁸ in the general formula (38) is the same as R⁸ in the general formula (8).

In case of using the two or more compounds (8), the two R⁸s in the general formula (38) may be the same or different from each other.

### <Compound (32)>

The compound (32) is the other product by the depolymerization method (iii).

The compound (32) is bisphenol A (BPA) and its derivative.

Z³⁴, Z³⁵, m₃₄, and m₃₅ in the general formula (32) are the same as Z³⁴, Z³⁵, m₃₄, and m₃₅ in the general formula (3), respectively.

### <Solvent>

In the depolymerization step of the depolymerization method (iii), it is preferable to further use a solvent. The use of solvent particularly allows the compound (3) to be dissolved in the solvent to carry out the depolymerization step, facilitating the depolymerization of compound (3) to further proceed.

The solvent in the depolymerization method (iii) is the same as the solvent in the depolymerization method (i).

The solvent to be used in the depolymerization step of the depolymerization method (iii) may be the single solvent or two or more thereof, and in the case of the two or more, the combination and ratio thereof can be arbitrarily selected according to the purpose.

When a solvent is used in the depolymerization step of the depolymerization method (ii), the amount of solvent used is preferably 0.2 to 1.5 L relative to 100 g of compound (3) used, and may be, for example, any one of 0.2 to 1 L and 0.2 to 0.6 L, or any one of 0.4 to 1.5 L and 0.6 to 1.5 L, or 0.4 to 1 L. The amount of solvent used that is the lower limit value or more facilitates the depolymerization of compound (3) to further proceed. The amount of solvent used being the upper limit value or less inhibits the solvent from being used excessively.

### <Other component>

In the depolymerization step of the depolymerization method (iii), the compound (3) may be depolymerized using other components that do not fall under any of the compound (3), a base, the compound (8), and a solvent, if necessary, as long as the effects of the present invention are not impaired, or the compound (3) may be depolymerized without using the other components.

The other components in the depolymerization method (iii) are the same as the other components in the depolymerization method (i).

The other components to be used in the depolymerization step of the depolymerization method (iii) may be used singly, or two or more thereof may be used, and in the case of the two or more, the combination and ratio thereof can be selected arbitrarily according to the purpose.

In depolymerizing the compound (3) in the depolymerization step of the depolymerization method (iii), the proportion of the total amount used (parts by mass) of a base, the compound (8), and a solvent to the total amount used (parts by mass) of the base, the compound (8), the solvent, and the other components (([amount (parts by mass) of base used] + [amount (parts by mass) of compound (8) used] + [amount (parts by mass) of solvent used])/([amount (parts by mass) of base used] + [amount (parts by mass) of compound (8) used] + [amount (parts by mass) of solvent used] + [amount (parts by mass) of other components used]) × 100) is preferably 80% by mass or more, more preferably 90% by mass or more, and still more preferably 95% by mass or more, and may be, for example, any one of 97% by mass or more and 99% by mass or more. The proportion of the lower limit value or more facilitates the depolymerization of compound (3) to further proceed.

The proportion is, on the other hand, 100% by mass or less.

When no optional component (i.e., a solvent or the other components) is used upon the depolymerization of compound (3), the amount of optional component used when calculating the proportion is 0 parts by mass.

### <Other conditions>

In the depolymerization step of the depolymerization method (iii), the compound (3) can be depolymerized by mixing the compound (3), a base, the compound (8), and a solvent, if necessary, and the other components, if necessary, and heating and stirring the resulting mixture.

In the depolymerization step of the depolymerization method (iii), a temperature (reaction temperature) when the mixture is heated and stirred, a time (reaction time) when the mixture is heated and stirred, and an atmosphere upon the depolymerization of compound (3) are the same as the temperature (reaction temperature) when the mixture is heated and stirred, the time (reaction time) when the mixture is heated and stirred, and the atmosphere upon the depolymerization of compound (1) in the depolymerization step of the depolymerization method (i), respectively.

In the depolymerization method (iii), after completion of the depolymerization step, the obtained reaction mixture may be subjected to post-treatment, if necessary, in the same manner as in the depolymerization method (i) to take out the target substance (product), and the target substance taken out may be further purified, if necessary. Alternatively, after completion of the depolymerization step, the obtained reaction mixture may undergo post-treatment, if necessary in the same manner as in the depolymerization method (i), and then used for the next intended purpose without taking out the target substance.

The structure of the product by the depolymerization method (iii) can be confirmed by publicly known techniques, such as nuclear magnetic resonance (NMR) spectroscopy, mass spectrometry (MS), and infrared spectroscopy (IR).

### <<Depolymerization method (iv)>>

The method for depolymerizing (decomposing) a compound according to one embodiment of the present invention comprises the depolymerization step (decomposition step) of reacting a compound represented by the following general formula (4) (may be referred to as "compound (4)" as used herein): 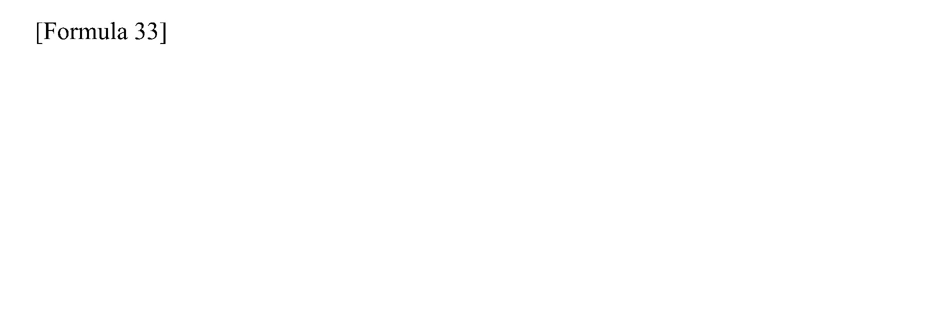 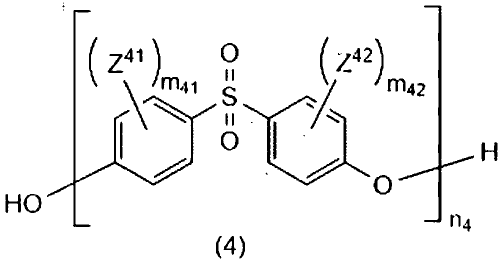
(wherein n₄ is an integer of 2 or greater (preferably 10 to 200); Z⁴¹ and Z⁴² are each independently a group other than a hydrogen atom; m₄₁ and m₄₂ are each independently an integer of 0 to 4, and when m₄₁ is an integer of 1 or greater, n₄ × m₄₁ Z⁴¹ groups may be the same or different from each other, and when m₄₂ is an integer of 1 or greater, n₄ × m₄₂ Z⁴² groups may be the same or different from each other.) with a compound represented by the following general formula (8):

   R⁸-SH (8)
(wherein R⁸ is an alkyl group, an aryl group, or an aralkyl group, and one or two or more hydrogen atoms in the alkyl group, aryl group, or aralkyl group may be substituted with a halogen atom, a hydroxyl group, a mercapto group, or a trialkoxysilyl group, and when the alkyl group, aryl group, or aralkyl group has one or two or more trimethylene groups therein, a central methylene group in the trimethylene group may be substituted with an oxycarbonyl group or a carbonyloxy group.)
   in the co-presence of a base to obtain a compound represented by the following general formula (48) (may be referred to referred to as "compound (48)" as used herein): 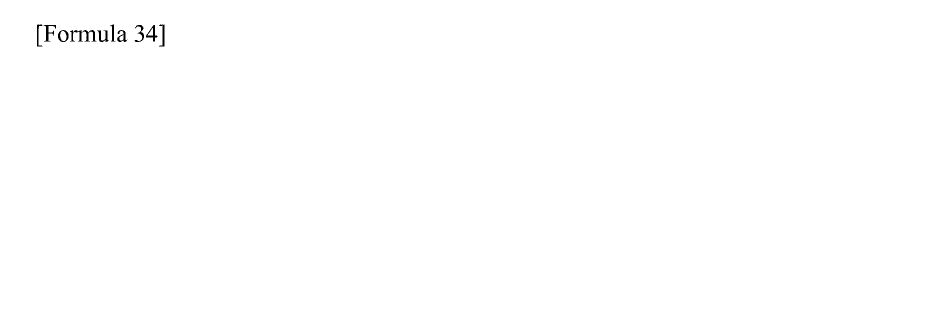 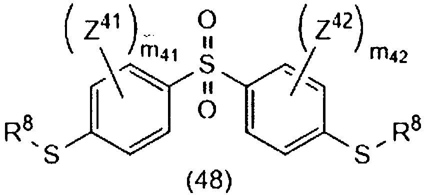
(wherein Z⁴¹, Z⁴², m₄₁, m₄₂, and R⁸ are the same as described above.). As used herein, the method for depolymerizing a compound of the present embodiment may be referred to as "depolymerization method (iv)".

The depolymerization method of the present embodiment (depolymerization method (iv) is a novel method for depolymerizing the compound (4), which includes super engineering plastics.

The compound (4) has a benzene ring backbone to which an electron-withdrawing sulfonyl group (-SO₂-) is bonded in its structure, and has an electron-deficient aromatic ring group. In the depolymerization method (iv), the compound (4) is depolymerized by the compound (8) in the co-presence of a base, due to the characteristics of the compound (4). Then, the compound (48) which is a thioether compound, is obtained, depending on the structure of the compound (8), as the depolymerized substance (product).

The depolymerization method (iv) is the same as the depolymerization method (i), except that the compound (4) is used instead of the compound (1), that is, an object of depolymerization is different.

Examples of the compound (4) include polyether sulfone (may be referred to as "PESU" as used herein) and its derivative.

### <Compound (4)>

The compound (4) is an object of depolymerization in the depolymerization method (iv).

In the general formula (4), n₄ is the number of repeating units, which defines the molecular size of the compound (4), and is an integer of 2 or greater.

The compound (4) in which n₄ is, for example, 30 to 350 in the formula is suitable as polyether sulfone (PESU) which is a high molecular weight super engineering plastic, is difficult to be depolymerized by conventional methods and is particularly suitable as an applicable object for the depolymerization method (iv).

In the general formula (4), Z⁴¹ and Z⁴² are each independently a group (substituent) other than a hydrogen atom. That is, the substituents Z⁴¹ and Z⁴² may be the same as each other or different from each other.

Examples of Z⁴¹ and Z⁴² (substituents) include the same as Z¹¹ and Z¹² above (for example, an alkyl group, an alkylcarbonylamino group, a fluorinated alkyl group, a fluorine atom, and the like.).

In the general formula (4), m₄₁ is the number of Z⁴¹ bonded to one benzene ring backbone. m₄₂ is the number of Z⁴² bonded to another benzene ring backbone different from the benzene ring backbone to which Z⁴¹ is bonded.

m₄₁ and m₄₂ each independently represents an integer of 0 to 4. That is, m₄₁ and m₄₂ may be the same as or different from each other.

The compound (4) has n₄ × m₄₁ Z⁴¹ groups in one molecule, and when m₄₁ is an integer of 1 or more (i.e., an integer of 1 to 4), the n₄ × m₄₁ Z⁴¹ groups may be the same or different from each other. That is, when m₄₁ is an integer of 1 or greater, the n₄ × m₄₁ Z⁴¹ groups may all be the same, all be different, or may only partially be the same.

The same applies to Z⁴², and the compound (4) has n₄ × m₄₂ Z⁴² groups in one molecule, and when m₄₂ is an integer of 1 or greater (i.e., an integer of 1 to 4), the n₄ × m₄₂ Z⁴² groups may be the same or different from each other. That is, when m₄₂ is an integer of 1 or greater, the n₄ × m₄₂ Z⁴² groups may all be the same, all be different, or may only partially be the same.

A preferred example of the compound (4) includes a compound (4) in which m₄₁ and m₄₂ are 0 (i.e., no Z⁴¹ and Z⁴² is present in the formula.).

With the proviso that the compound (4) is not limited thereto.

The compound (4) subjected to depolymerization may be reinforced with fibers. That is, in the depolymerization method (iv), the fiber-reinforced compound (4) can be depolymerized.

Examples of the fiber-reinforced compound (4) include a carbon fiber-reinforced compound (4) and a glass fiber-reinforced compound (4).

In the fiber-reinforced compound (4), the proportion of the content of the compound (4) to the total mass of fiber-reinforced compound (4) ([content of the compound (4) in the fiber-reinforced compound (4) (parts by mass)]/[total mass of fiber-reinforced compound (4) (parts by mass)] × 100) is preferably 10 to 90% by mass, and may be, for example, any one of 10 to 70% by mass, 10 to 50% by mass, and 10 to 30% by mass, or any one of 30 to 90% by mass, 50 to 90% by mass, and 70 to 90% by mass, or 30 to 70% by mass. The proportion of the lower limit value or more results in the higher yield of the compound (48). The proportion of the upper limit value or less enhances the versatility of fiber-reinforced compound (4).

The hydrogen atom in one or both hydroxyl groups (-OHs, hydroxyl groups at one or both ends in the general formula (4)) in the compound (4) may be substituted with a group (M) other than a hydrogen atom to form a group represented by formula "-OM" or a group represented by formula "-O⁻M⁺" as in the case of the compound (1). Namely, the compound (4) may be a salt.

The manner whereby the compound (4) forms a salt is the same as the manner whereby the compound (1) forms a salt. That is, the salt of compound (4) is also formed in the same manner as the salt of compound (1), and is the same as the salt of compound (1) except that the group represented by formula "-OM" is bonded to a different group.

### <Base>

The base in the depolymerization method (iv) is the same as the base in the depolymerization method (i).

The base to be used in the depolymerization step of the depolymerization method (iv) may be the single base or two or more thereof, and in the case of the two or more, the combination and ratio thereof can be arbitrarily selected according to the purpose.

For example, in case of using two or more bases, two or more inorganic bases may be used without using an organic base, two or more organic bases may be used without using an inorganic base, or one or more inorganic bases and one or more organic bases may be used together.

The base to be used in the depolymerization step of the depolymerization method (iv) is preferably one or two or more selected from the group consisting of a phosphazene base, a carbonate, and an alkali metal tert-butoxide, and more preferably one or two or more selected from the group consisting of a phosphazene base, cesium carbonate, sodium tert-butoxide, and potassium tert-butoxide.
Using such a base facilitates the depolymerization of compound (4) to further proceed.

Among these, at least a phosphazene base or sodium tert-butoxide is preferably used as the base in the depolymerization step because they facilitates the depolymerization of compound (4) to particularly proceed, a phosphazene base in combination with an inorganic base, or sodium tert-butoxide is more preferably used, a phosphazene base in combination with a phosphate, or sodium tert-butoxide is still more preferably used, and a phosphazene base in combination with tripotassium phosphate, or sodium tert-butoxide, is particularly preferably used. In case of using sodium tert-butoxide, sodium tert-butoxide may be used singly as the base or may be combined for use with another inorganic base.

In the depolymerization step of the depolymerization method (iv), the amount (amount by mole) of base used is preferably 4 to 23 mol% relative to the amount (amount by mole) of repeating unit in compound (4) (the constituent unit attached with the sign n₄ in the general formula (4)) and may be, for example, any one of 4 to 13 mol%, 13 to 23 mol%, and 8 to 18 mol%. The amount of base used being the lower limit value or more facilitates the depolymerization of compound (4) to further proceed. The amount of base used being the upper limit value or less inhibits the base from being used excessively.

### <Compound (8)>

The compound (8) in the depolymerization method (iv) is the same as the compound (8) in the depolymerization method (i).

The compound (8) to be used in the depolymerization step of the depolymerization method (iv) may be the single compound or two or more thereof, and in the case of the two or more, the combination and ratio thereof can be arbitrarily selected according to the purpose. When the two or more compounds (8) are used, two or more of compounds (38) can be produced.

A preferred example of the compound (8) in the depolymerization method (iv) includes the same as the preferred compound (8) in the depolymerization method (i).

In the depolymerization step of the depolymerization method (iv), the amount (amount by mole) of compound (8) used may be, for example, 1.5 to 6 times the amount (amount by mole) of repeating unit (the constituent unit attached with the sign n₄ in the general formula (4)) in the compound (4), but is preferably 2 to 6 times the amount, more preferably 2 to 4 times the amount, and still more preferably 2 to 3 times the amount. The amount of compound (8) used of the lower limit value or more facilitates the depolymerization of compound (4) to further proceed. The amount of compound (8) used of the upper limit value or less inhibits the compound (8) from being used excessively.

### <Compound (48)>

The compound (48) is a product by the depolymerization method (iv).

The compound (48) is a derivative of bisphenol S (BPS).

Z⁴¹, Z⁴², m₄₁, and m₄₂ in the general formula (48) are the same as Z⁴¹, Z⁴², m₄₁, and m₄₂ in the general formula (4), respectively.

R⁸ in the general formula (48) is the same as R⁸ in the general formula (8).

In case of using the two or more compounds (8), the two R⁸s in the general formula (48) may be the same or different from each other.

### <Solvent>

In the depolymerization step of the depolymerization method (iv), it is preferable to further use a solvent. The use of solvent particularly allows the compound (4) to be dissolved in the solvent to carry out the depolymerization step, facilitating the depolymerization of compound (4) to further proceed.

The solvent in the depolymerization method (iv) is the same as the solvent in the depolymerization method (i).

The solvent to be used in the depolymerization step of the depolymerization method (iv) may be the single solvent or two or more thereof, and in the case of the two or more, the combination and ratio thereof can be selected arbitrarily according to the purpose.

When a solvent is used in the depolymerization step of the depolymerization method (iv), the amount of solvent used is preferably 0.2 to 1.5 L relative to 100 g of compound (4) used, and may be, for example, any one of 0.2 to 1 L and 0.2 to 0.6 L, any one of 0.4 to 1.5 L and 0.6 to 1.5 L, or 0.4 to 1 L. The amount of solvent used that is the lower limit value or more facilitates the depolymerization of compound (4) to further proceed. The amount of solvent used being the upper limit value or less inhibits the solvent from being used excessively.

### <Other component>

In the depolymerization step of the depolymerization method (iv), the compound (4) may be depolymerized using other components that do not fall under any of the compound (4), a base, the compound (8), and a solvent, if necessary, as long as the effects of the present invention are not impaired, or the compound (4) may be depolymerized without using the other components.

The other components in the depolymerization method (iv) are the same as the other components in the depolymerization method (i).

The other components to be used in the depolymerization step of the depolymerization method (iv) may be the single component or two or more thereof, and when the two or more are used, the combination and ratio thereof can be selected arbitrarily according to the purpose.

In depolymerizing the compound (4) in the depolymerization step of the depolymerization method (iv), the proportion of the total amount used (parts by mass) of a base, the compound (8), and a solvent to the total amount used (parts by mass) of the base, compound (8), solvent, and the other components (([amount (parts by mass) of base used] + [amount (parts by mass) of compound (8) used] + [amount (parts by mass) of solvent used])/([amount (parts by mass) of base used] + [amount (parts by mass) of compound (8) used] + [amount (parts by mass) of solvent used] + [amount (parts by mass) of other components used]) × 100) is preferably 80% by mass or more, more preferably 90% by mass or more, and still more preferably 95% by mass or more, and may be, for example, any one of 97% by mass or more and 99% by mass or more. The proportion of the lower limit value or more facilitates the depolymerization of compound (4) to further proceed.

The proportion is, on the other hand, 100% by mass or less.

When no optional components (i.e., a solvent or the other components) are used upon the depolymerization of compound (4), the amount of optional component used when calculating the proportion is 0 parts by mass.

### <Other conditions>

In the depolymerization step of the depolymerization method (iv), the compound (4), a base, the compound (8), and a solvent, if necessary, and the other components, if necessary, are mixed, and the resulting mixture is heated and stirred, whereby the compound (4) can be depolymerized.

In the depolymerization step of the depolymerization method (iv), a temperature (reaction temperature) when the mixture is heated and stirred, a time (reaction time) when the mixture is heated and stirred, and an atmosphere upon the depolymerization of compound (4) are each the same as the temperature (reaction temperature) when the mixture is heated and stirred, the time (reaction time) when the mixture is heated and stirred, and the atmosphere upon the depolymerization of compound (1) in the depolymerization step of the depolymerization method (i).

In the depolymerization method (iv), after completion of the depolymerization step, the obtained reaction mixture may be post-treated, if necessary in the same manner as in the depolymerization method (i) to be able to take out the target substance (product), and the target substance taken out may be further purified, if necessary. Alternatively, after completion of the depolymerization step, the obtained reaction mixture may undergo post-treatment, if necessary in the same manner as in the depolymerization method (i), and then used for the next intended purpose without taking out the target substance.

The structure of the product by the depolymerization method (iv) can be confirmed by publicly known techniques, such as nuclear magnetic resonance (NMR) spectroscopy, mass spectrometry (MS), and infrared spectroscopy (IR).

### Examples

The present invention will be specifically described in detail below by way of Examples. However, the present invention is not limited thereto.

### <<Depolymerization of polysulfone (depolymerization method (i))>>

### [Example 1]

Under an argon atmosphere, to a colorless and transparent pellet-shaped polysulfone (44.7 mg; the amount of repeating unit in the general formula (1) of 0.101 mmol; weight-average molecular weight 35,000; and number-average molecular weight 16,000, manufactured by Sigma-Aldrich Co. LLC, Cat. No. 428302) were added a 0.8 M concentration n-hexane solution of a phosphazene base P₄-t-Bu (manufactured by Sigma-Aldrich Co. LLC, Cat. No. 79421) (12.5 µL, 0.01 mmol as the phosphazene base P₄-t-Bu, 10 mol% relative to a repeating unit in the general formula (1)), tripotassium phosphate (K₃PO₄) (1.1 mg, 0.005 mmol, 5 mol% relative to a repeating unit in the general formula (1)), N,N-dimethylacetamide (DMAc) (0.2 mL), and 2-ethylhexyl mercaptan (alias: 2-ethylhexanethiol, corresponding to the compound (8)) (36.5 mg, 0.250 mmol, 2.5 times the amount by mole (2.5 equivalents) of repeating unit in the general formula (1)) in sequence to dissolve the polysulfone, and the resulting mixture was then stirred at 150°C for 16 hours.

Next, the temperature of the resulting yellow reaction mixture was returned to room temperature, and 1,4-dioxane (5.2 mg, 5.0 µL, 0.059 mmol) was added as an internal standard substance, and a small amount of the resulting mixture was taken as a sample, dissolved in deuterated chloroform (CDCl₃), and analyzed by ¹H NMR. As a result, it was confirmed that target substances (depolymerized products), bis(4-(2-ethylhexylthio)phenyl)sulfone (BEHTPS, corresponding to the compound (18)) and bisphenol A (BPA, corresponding to the compound (121)), were obtained quantitatively (with a yield of over 95%).

The sample used in the ¹H NMR analysis described above was returned to the yellow reaction mixture, and a crude product was obtained by distillation under reduced pressure.

The obtained crude product underwent silica gel column chromatography (eluent: hexane/ethyl acetate 96/4 → 70/30), yielding the target substances bis(4-(2-ethylhexylthio)phenyl)sulfone (BEHTPS) (yield: 45.3 mg, 89%) and bisphenol A (BPA) (yield: 21.3 mg, 94%).

The results of NMR analysis of the obtained bis(4-(2-ethylhexylthio)phenyl)sulfone and bisphenol A are shown below.

### Bis(4-(2-ethylhexylthio)phenyl)sulfone (BEHTPS):

¹H NMR (600 MHz, CDCl₃) δ 0.87-0.90 (m, 12H, methyl), 1.24-1.31 (m, 8H, methylene), 1.36-1.49 (m, 8H, methylene), 1.61 (sept, J = 6.3 Hz, 2H, methyne), 2.92 (d, J = 6.4 Hz, 4H, SCH₂), 7.30 (AA'BB', 4H, aromatic), 7.77 (AA'BB', 4H, aromatic).

¹³C NMR (151 MHz, CDCl₃) δ 10.7, 14.1, 22.9, 25.7, 28.7, 32.4, 36.3, 38.6, 126.7, 127.7, 137.6, 146.2.

### Bisphenol A (BPA)

¹H NMR (600 MHz, acetone-d₆) δ 1.58 (s, 6H, methyl), 6.72 (AA'BB', 4H, aromatic), 7.05 (AA'BB', 4H, aromatic), 8.07 (s, 2H, OH). 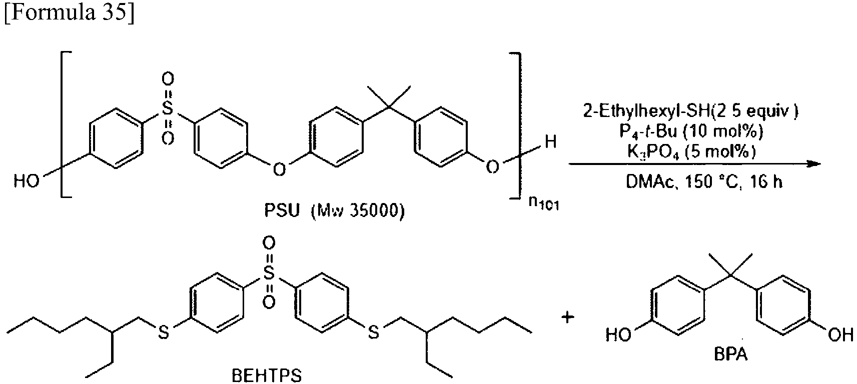 (wherein n₁₀₁ is an integer (preferably 10 to 200)).

### [Example 2]

Under an argon atmosphere, to a colorless and transparent pellet-shaped polysulfone (45.3 mg; the amount of repeating unit in the general formula (1) of 0.102 mmol; weight-average molecular weight 60,000, manufactured by NACALAI TESQUE, INC., Cat. No. 178910050) were added a 0.8 M concentration n-hexane solution of a phosphazene base P₄-t-Bu (manufactured by Sigma-Aldrich Co. LLC, Cat. No. 79421) (12.5 µL, 0.01 mmol as the phosphazene base P₄-t-Bu, 10 mol% relative to a repeating unit in the general formula (1)), tripotassium phosphate (K₃PO₄) (1.1 mg, 0.005 mmol, 5 mol% relative to a repeating unit in the general formula (1)), N,N-dimethylacetamide (DMAc) (0.2 mL), and 2-ethylhexyl mercaptan (36.5 mg, 0.250 mmol, 2.5 times the amount by mole (2.5 equivalents) of repeating unit in the general formula (1)) in sequence to dissolve the polysulfone, and the resulting mixture was then stirred at 150°C for 16 hours.

Next, the temperature of the resulting yellow reaction mixture was returned to room temperature, and 1,4-dioxane (5.2 mg, 5.0 µL, 0.059 mmol) was added as an internal standard substance, and a small amount of the resulting mixture was taken as a sample, dissolved in deuterated chloroform and analyzed by ¹H NMR. As a result, it was confirmed that target substances (depolymerized products), bis(4-(2-ethylhexylthio)phenyl)sulfone (BEHTPS, corresponding to the compound (18)) and bisphenol A (BPA, corresponding to the compound (121)), were obtained quantitatively (with a yield of over 95%).

The sample used in the ¹H NMR analysis described above was returned to the yellow reaction mixture, and a crude product was obtained by distillation under reduced pressure.

The obtained crude product underwent silica gel column chromatography (eluent: hexane/ethyl acetate 96/4 → 70/30), yielding the target substances bis(4-(2-ethylhexylthio)phenyl)sulfone (BEHTPS) (yield: 51 mg, 99%) and bisphenol A (BPA) (yield: 25.3 mg, 99%). In the present Example, the same results as in Example 1 were obtained by ¹H NMR and ¹³C NMR analyses. 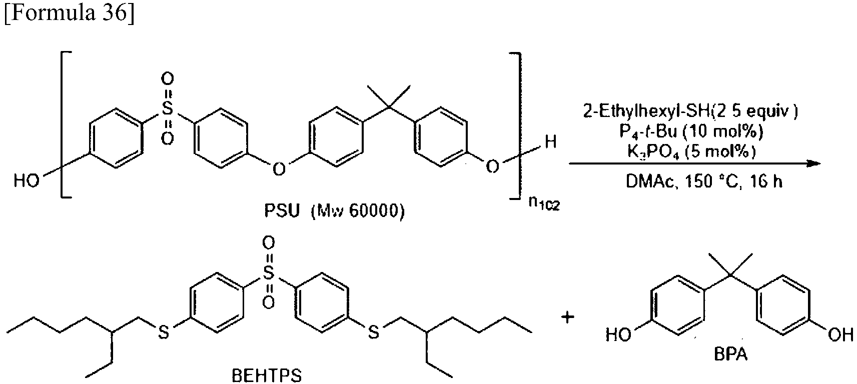 (wherein n₁₀₂ is an integer (preferably 10 to 200)).

As is clarified by the results of Examples 1 and 2, even when polysulfones with different degrees of polymerization were used, the depolymerization proceeded favorably, and the target substances, bis(4-(2-ethylhexylthio)phenyl)sulfone and bisphenol A, were obtained in high yields.

In the course of the depolymerization step, the carbon-sulfur bond in the diphenylsulfone moiety in the polysulfone was hardly cleaved, confirming that the carbon-oxygen bond, which has a stronger bonding strength than a general carbon-sulfur bond, was selectively cleaved.

### <<Depolymerization of polysulfone (conventional method)>>

### [Comparative Example 1]

According to the method described in Non Patent Literature 6, polysulfone was depolymerized by a conventional method.

That is, under an argon atmosphere, to a colorless and transparent pellet-shaped polysulfone (43.8 mg; the amount of repeating unit in the general formula (1) of 0.099 mmol; weight-average molecular weight 35,000; and number-average molecular weight 16,000, manufactured by Sigma-Aldrich Co. LLC, Cat. No. 428302) were sequentially added sodium tert-butoxide (28.8 mg, 0.3 mmol, 3 times the amount by mole (3 equivalents) of repeating unit in the general formula (1)), N,N-dimethylacetamide (DMAc) (0.2 mL), and phenethyl mercaptan (C₆H₅CH₂CH₂SH) (corresponding to the compound (8)) (55.6 mg, 0.4 mmol, 4 times the amount by mole (4 equivalents) of repeating unit in the general formula (1)) to dissolve the polysulfone, and the resulting mixture was then stirred at 150°C for 16 hours.

Then, the temperature of the obtained reaction mixture was returned to room temperature, 41.4 mg (0.3 mmol) of methyl iodide was added, and the mixture was stirred at 100°C for 1 hour. To the obtained reaction mixture was added ethyl acetate (1.5 mL), and the organic layer was washed successively with water and a saturated sodium chloride aqueous solution. The obtained organic layer was dried over anhydrous magnesium sulfate, and then distilled under reduced pressure to obtain a crude product.

Next, to the crude product was added 1,4-dioxane (5.2 mg, 5.0 µL, 0.059 mmol) as an internal standard substance, and a small amount of the obtained product was taken out as a sample, dissolved in deuterated acetone ((CD₃)₂CO), and analyzed by ¹H NMR and ¹³C NMR. As a result, it was confirmed that the target substances (depolymerized products), bis(4-methylthiophenyl)sulfone (BMTPS, corresponding to the compound (18)) (yield 84%) and bisphenol A (yield 80%) were obtained. The yields of these target substances were lower than those in Examples 1 and 2.

The analytical results in this case are shown below.

### Bis(4-methylthiophenyl)sulfone (BMTPS):

¹H NMR (600 MHz, acetone-d₆) δ 2.48 (s, 6H, SCH₃), 7.27 (AA'BB', 4H, aromatic), 7.79 (AA'BB', 4H, aromatic).

¹³C NMR (151 MHz, acetone-d₆) δ 14.8, 125.5, 127.8, 137.5, 146.5. 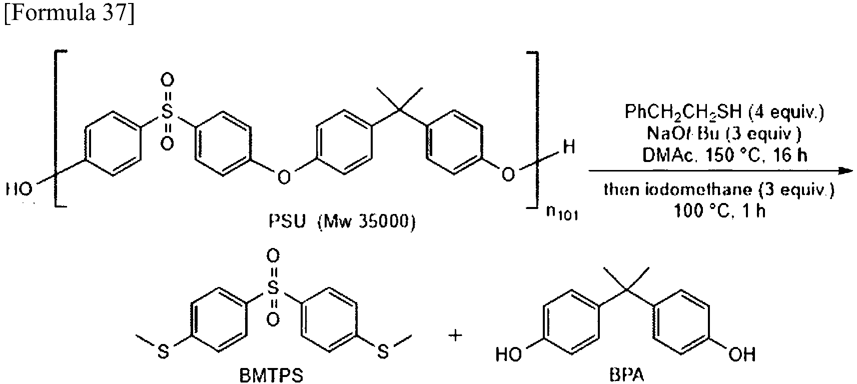 (wherein n₁₀₁ is an integer (preferably 10 to 200)).

### <<Depolymerization of polyether ether sulfone (depolymerization method (i)>>

### [Example 3]

Under an argon atmosphere, to a brown pellet-shaped polysulfone (34.1 mg; the amount of repeating unit in the general formula (1) of 0.105 mmol; manufactured by Sigma-Aldrich Co., LLC, Cat. No. 440965) were added a 0.8 M concentration n-hexane solution of a phosphazene base P₄-t-Bu (manufactured by Sigma-Aldrich Co. LLC, Cat. No. 79421) (13.2 µL, 0.0105 mmol as the phosphazene base P₄-t-Bu, 10 mol% relative to a repeating unit in the general formula (1)), tripotassium phosphate (K₃PO₄) (1.1 mg, 0.005 mmol, 5 mol% relative to a repeating unit in the general formula (1)), N,N-dimethylacetamide (DMAc) (0.2 mL), and 2-ethylhexyl mercaptan (corresponding to the compound (8)) (38.7 mg, 0.263 mmol, 2.5 times the amount by mole (2.5 equivalents) of repeating unit in the general formula (1)) in sequence to dissolve the polysulfone, and the resulting mixture was then stirred at 150°C for 16 hours.

Next, the temperature of the resulting yellow reaction mixture was returned to room temperature, and 1,4-dioxane (5.2 mg, 5.0 µL, 0.059 mmol) was added as an internal standard substance, and a small amount of the resulting mixture was taken out as a sample, dissolved in deuterated chloroform and analyzed by ¹H NMR. As a result, it was confirmed that the target substances (depolymerized products), bis(4-(2-ethylhexylthio)phenyl)sulfone (BEHTPS, corresponding to the compound (18)) and hydroquinone (HQ, corresponding to the compound (122)), were obtained quantitatively (with a yield of over 95%).

The sample used in the ¹H NMR analysis described above was returned to the yellow reaction mixture, and a crude product was obtained by distillation under reduced pressure.

The obtained crude product underwent silica gel column chromatography (eluent: hexane/ethyl acetate 96/4 → 70/30), yielding the target substances bis(4-(2-ethylhexylthio)phenyl)sulfone (BEHTPS) (yield: 57.6 mg, 99%) and hydroquinone (HQ (yield: 9.3 mg, 80%).

The results of ¹H NMR and ¹³C NMR analyses of the obtained bis(4-(2-ethylhexylthio)phenyl)sulfone were the same as in Example 1.

The results of ¹H NMR analysis of the obtained hydroquinone are shown below.

### Hydroquinone (HQ):

¹H NMR (600 MHz, acetone-d₆) δ 6.66 (s, 4H). 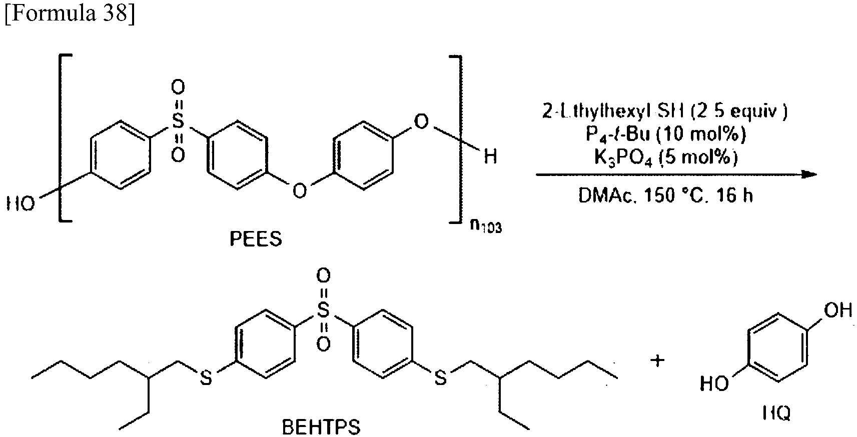 (wherein n₁₀₃ is an integer (preferably 10 to 200)).

### <<Depolymerization of polyphenylsulfone (depolymerization method (i))>>

### [Example 4]

A sheet of polyphenylsulfone (manufactured by Standard-Testpiece K. K., Cat. No. RMOLDED0050) was processed into a powder form.

Under an argon atmosphere, to the colorless powdered polyphenylsulfone obtained above (40.1 mg; the amount of repeating unit in the general formula (1) of 0.1 mmol)) were added a 0.8 M concentration n-hexane solution of a phosphazene base P₄-t-Bu (manufactured by Sigma-Aldrich Co. LLC, Cat. No. 79421) (12.5 µL, 0.01 mmol as the phosphazene base P₄-t-Bu, 10 mol% relative to a repeating unit in the general formula (1)), tripotassium phosphate (K₃PO₄) (1.3 mg, 0.006 mmol, 6 mol% relative to a repeating unit in the general formula (1)), N,N-dimethylacetamide (DMAc) (0.2 mL), and 2-ethylhexyl mercaptan (corresponding to the compound (8)) (36.7 mg, 0.251 mmol, 2.5 times the amount by mole (2.5 equivalents) of repeating unit in the general formula (1)) in sequence to dissolve the polysulfone, and the resulting mixture was then stirred at 150°C for 16 hours.

Next, the temperature of the resulting yellow reaction mixture was returned to room temperature, and 1,4-dioxane (5.2 mg, 5.0 µL, 0.059 mmol) was added as an internal standard substance, and a small amount of the resulting mixture was taken out as a sample, dissolved in deuterated chloroform and analyzed by ¹H NMR. As a result, it was confirmed that the target substances (depolymerized products), bis(4-(2-ethylhexylthio)phenyl)sulfone (BEHTPS, corresponding to the compound (18)) and 4,4'-dihydroxybiphenyl (4,4'-DHBP, corresponding to the compound (123)), were obtained quantitatively (with a yield of over 95%).

The sample used in the ¹H NMR analysis described above was returned to the yellow reaction mixture, and a crude product was obtained by distillation under reduced pressure.

The obtained crude product underwent silica gel column chromatography (eluent: hexane/ethyl acetate 96/4 → 70/30), yielding the target substances bis(4-(2-ethylhexylthio)phenyl)sulfone (BEHTPS) (yield: 49 mg, 95%) and 4,4'-dihydroxybiphenyl (4,4'-DHBP) (yield: 19 mg, 99%).

The results of ¹H NMR and ¹³C NMR analyses of the obtained bis(4-(2-ethylhexylthio)phenyl)sulfone were the same as in Example 1.

The results of ¹H NMR analysis of the obtained 4,4'-dihydroxybiphenyl are shown below.

### 4,4'-Dihydroxybiphenyl (4,4'-DHBP):

¹HNMR (600 MHz, acetone-d₆) δ 6.87 (AA'BB', 4H, aromatic), 7.40 (AA'BB', 4H, aromatic), 8.32 (s, 2H, OH). 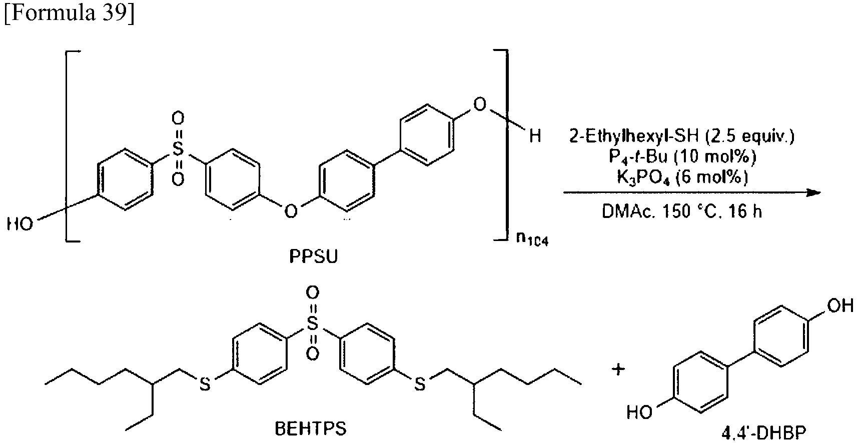 (wherein n₁₀₄ is an integer (preferably 10 to 200)).

### <<Depolymerization of polyether sulfone (depolymerization method (iv))>>

### [Example 5]

Under an argon atmosphere, to a yellow and transparent pellet-shaped polysulfone (23.2 mg; the amount of repeating unit in the general formula (4) of 0.1 mmol, manufactured by Sigma-Aldrich Co. LLC, Cat No. 191094) were added a 0.8 M concentration n-hexane solution of a phosphazene base P₄-t-Bu (manufactured by Sigma-Aldrich Co. LLC, Cat. No. 79421) (12.5 µL, 0.01 mmol as the phosphazene base P₄-t-Bu, 10 mol% relative to a repeating unit in the general formula (4)), tripotassium phosphate (K₃PO₄) (1.2 mg, 0.0057 mmol, 6 mol% relative to a repeating unit in the general formula (4)), N,N-dimethylacetamide (DMAc) (0.2 mL), and 2-ethylhexyl mercaptan (corresponding to the compound (8)) (22.1 mg, 0.151 mmol, 1.5 times the amount by mole (1.5 equivalents) of repeating unit in the general formula (4)) in sequence to dissolve the polyether sulfone, and the resulting mixture was then stirred at 150°C for 16 hours.

Next, the temperature of the resulting yellow reaction mixture was returned to room temperature, and a crude product was obtained by distillation under reduced pressure.

The resulting crude product was subjected to silica gel column chromatography (eluent: hexane/ethyl acetate 96/4 → 70/30), yielding the target substances, bis(4-(2-ethylhexylthio)phenyl)sulfone (BEHTPS) (yield: 10.9 mg, 22%), 4-(2-ethylhexylthio)-4'-hydroxydiphenylsulfone (compound (a)) (yield: 7.8 mg, 21%), bisphenol S (BPS) (yield: 3.3 mg, 13%), and 4-((4-(4-((4-((2-ethylhexyl)thio)phenyl)-sulfoyl)phenoxy)phenyl)sulfonyl)phenol (compound (b)) (yield: 4.7 mg, 15%) .

The results of ¹H NMR and ¹³C NMR analyses of the obtained bis(4-(2-ethylhexylthio)phenyl)sulfone were the same as in Example 1.

The results of NMR analysis of the other compounds obtained above are shown below.

### Compound (a):

¹H NMR (600 MHz, CDCl₃) δ 0.88 (t, J = 7.0 Hz, 3H, methyl), 0.89 (t, J = 7.5 Hz, 3H, methyl), 1.25-1.29 (m, 4H, methylene), 1.37-1.48 (m, 4H, methylene), 1.61 (sept, J = 6.3 Hz, 1H, methyne), 2.92 (d, J = 6.2 Hz, 4H, SCH₂), 6.89 (AA'BB', 2H, aromatic), 7.30 (AA'BB', 2H, aromatic), 7.76 (AA'BB', 2H, aromatic), 7.80 (AA'BB', 2H, aromatic).

¹³C NMR (151 MHz, CDCl₃) δ 10.7, 14.1, 22.9, 25.7, 28.7, 32.4, 36.3, 38.6, 116.0, 126.7, 127.6, 130.0, 133.6, 137.9, 146.0, 159.8.

### Bisphenol S (BPS):

¹H NMR (600 MHz, acetone-d₆) δ 6.97 (AA'BB', 4H, aromatic), 7.77 (AA'BB', 4H, aromatic), 9.44 (br, 2H, OH).

### Compound (b):

¹H NMR (600 MHz, acetone-d₆) δ 0.87 (t, J = 7.1 Hz, 3H, methyl), 0.91 (t, J = 7.5 Hz, 3H, methyl), 1.27-1.33 (m, 4H, methylene), 1.40-1.51 (m, 4H, methylene), 1.65 (sept, J = 6.2 Hz, 1H, methyne), 3.07 (d, J = 6.3 Hz, 4H, SCH₂), 7.01 (AA'BB', 2H, aromatic), 7.238 (AA'BB', 2H, aromatic), 7.242 (AA'BB', 2H, aromatic), 7.49 (AA'BB', 2H, aromatic), 7.83 (AA'BB', 2H, aromatic), 7.86 (AA'BB', 2H, aromatic), 7.98 (AA'BB', 2H, aromatic), 8.00 (AA'BB', 2H, aromatic), 9.59 (br, 1H, OH).

¹³C NMR (151 MHz, acetone-d₆) δ 11.0, 14.3, 23.6, 26.3, 29.4, 33.1, 36.5, 39.4, 117.0, 120.5, 120.6, 127.7, 128.8, 130.7, 130.9, 131.0, 133.4, 138.6, 138.9, 139.7, 147.1, 160.3, 160.9, 162.8. 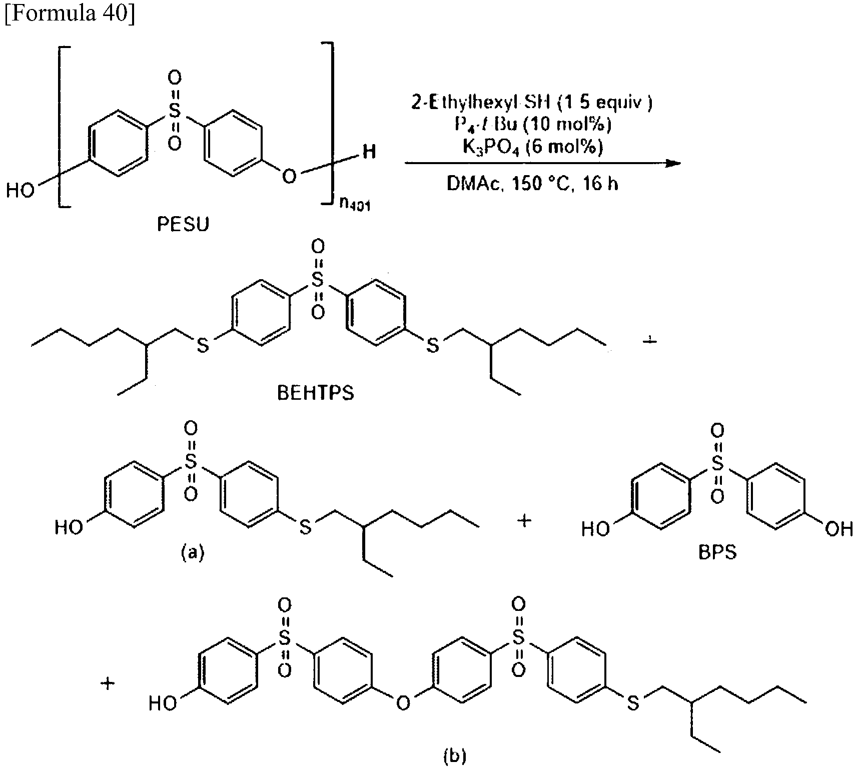 (wherein n₄₀₁ is an integer (preferably 10 to 200)).

### <<Depolymerization of polyetherimide (depolymerization method (iii))>>

### [Example 6]

Under an argon atmosphere, to a yellow and transparent pellet-shaped polyethereimide (59.2 mg; the amount of repeating unit in the general formula (3) of 0.1 mmol, manufactured by Sigma-Aldrich Co. LLC, Cat No. 700193) were added a 0.8 M concentration n-hexane solution of a phosphazene base P₄-t-Bu (manufactured by Sigma-Aldrich Co. LLC, Cat. No. 79421) (13.2 µL, 0.0105 mmol as the phosphazene base P₄-t-Bu, 11 mol% relative to a repeating unit in the general formula (3)), tripotassium phosphate (K₃PO₄) (1.1 mg, 0.005 mmol, 5 mol% relative to a repeating unit in the general formula (3)), N,N-dimethylacetamide (DMAc) (0.2 mL), and 2-ethylhexyl mercaptan (38.7 mg, 0.263 mmol, 2.6 times the amount by mole (2.6 equivalents) of repeating unit in the general formula (3)) in sequence to dissolve the polyether sulfone, and the resulting mixture was then stirred at 150°C for 16 hours.

Next, the temperature of the resulting yellow reaction mixture was returned to room temperature, and 1,4-dioxane (5.2 mg, 5.0 µL, 0.059 mmol) was added as an internal standard substance, and a small amount of the resulting mixture was taken as a sample, dissolved in deuterated chloroform and analyzed by ¹H NMR. As a result, it was confirmed that the target substances (depolymerized products), a compound (c) (corresponding to the compound (38)) and bisphenol A (BPA, corresponding to the compound (31)), were obtained quantitatively (with a yield of more than 95%).

The sample used in the ¹H NMR analysis described above was returned to the yellow reaction mixture, and a crude product was obtained by distillation under reduced pressure.

The obtained crude product underwent silica gel column chromatography (eluent: hexane/ethyl acetate 96/4 → 70/30), yielding the target substances the compound (c) (yield: 49.2 mg, 75%) and bisphenol A (BPA) (yield: 24.9 mg, over 99%).

The results of ¹H NMR analysis of the obtained bisphenol A were the same as those in Example 1.

The results of ¹H NMR and ¹³C NMR analyses of the compound (c) are shown below.

### Compound (c):

¹H NMR (600 MHz, CDCl₃) δ 0.90-0.95 (m, 12H, methyl), 1.30-1.34 (m, 8H, methylene), 1.43-1.53 (m, 8H, methylene), 1.68 (sept, J = 6.4 Hz, 2H, methyne), 3.04 (d, J = 6.4 Hz, 4H, SCH₂), 7.52 (dd, J = 2.0, 8.0 Hz, 2H, aromatic), 7.59 (dd, J = 1.7, 7.9 Hz, 2H, aromatic), 7.62-7.63 (m, 1H, aromatic), 7.67 (t, J = 2.0 Hz, 1H, aromatic), 7.76 (d, J = 1.3 Hz, 2H, aromatic), 7.79 (dd, J = 0.26, 7.9 Hz, 2H, aromatic).

¹³C NMR (151 MHz, CDCl₃) δ 10.8, 14.1, 22.9, 25.7, 28.8, 32.5, 36.7, 38.6, 120.7, 123.78, 123.85, 125.4, 127.2, 129.4, 131.8, 132.4 (two signals), 148.7, 166.6, 166.7. 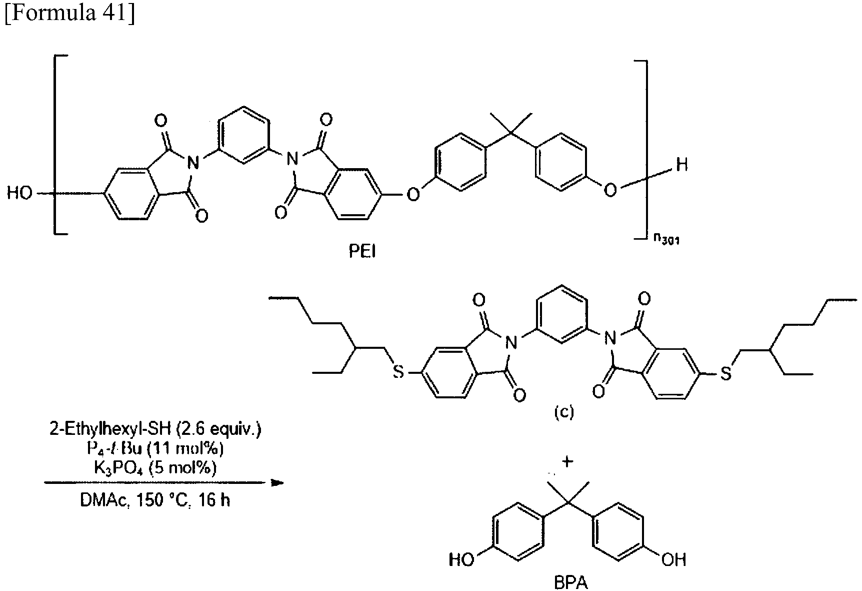 (wherein n₃₀₁ is an integer (preferably 10 to 200)).

### <<Depolymerization of polyether ether ketone (depolymerization method (ii))>>

### [Example 7]

In an argon atmosphere, to powdered polyether ether ketone (28.8 mg; the amount of repeating unit in the general formula (2) of 0.1 mmol; weight-average molecular weight 20,800; and number-average molecular weight 10,300, manufactured by Sigma-Aldrich Co., LLC, Cat. No. 456640) were added potassium tert-butoxide (KOt-Bu) (1.1 mg, 0.01 mmol, manufactured by Sigma-Aldrich Co., LLC, Cat. No. 156671-25G, 10 mol% relative to a repeating unit in the general formula (2)), 1,3-dimethyl-2-imidazolidinone (DMI) (0.2 mL), and 2-ethylhexyl mercaptan (31 mg, 0.2 mmol, twice the amount by mole (2 equivalents) of repeating unit in the general formula (2)) in sequence to dissolve the polyether ether ketone, and the resulting mixture was then stirred at 150°C for 16 hours.

Next, the temperature of the resulting reaction mixture was returned to room temperature, and 1,4-dioxane (5.2 mg, 5.0 µL, 0.059 mmol) was added as an internal standard substance, and a small amount of the resulting mixture was taken as a sample, dissolved in deuterated chloroform, and analyzed by ¹H NMR. Then, the yields of the target substances (depolymerized products), bis(4-(2-ethylhexylthio)phenyl)methanone (BEHTPM, corresponding to the compound (28)) and hydroquinone (HQ, corresponding to the compound (22)), and the intermediate compound (d) were calculated. The results are shown in Table 1.

The analytical results by ¹H NMR of the obtained hydroquinone were similar to those in Example 3.

The analytical results by NMR of BEHTPM and compound (d) are shown below.

### BEHTPM:

¹H NMR (600 MHz, CDCl₃) δ 0.89-0.93 (m, 12H, methyl), 1.29-1.31 (m, 8H, methylene), 1.39-1.53 (m, 8H, methylene), 1.64 (sept, J = 6.2 Hz, 2H, methyne), 2.98 (d, J = 6.4 Hz, 4H, SCH₂), 7.30 (d, 8.4 Hz, 4H, aromatic), 7.69 (d, J = 8.4 Hz, 4H, aromatic).

¹³C NMR (151 MHz, CDCl₃) δ 10.8, 14.1, 23.0, 25.7, 28.8, 32.5, 36.5, 38.7, 126.3, 130.4, 134.1, 144.6, 195.0.

### Compound (d):

¹H NMR (600 MHz, CDCl₃) δ 0.89 (t, J = 7.0 Hz, 3H, methyl), 0.91 (t, J = 7.5 Hz, 3H, methyl), 1.30-1.51 (m, 8H, methyl), 1.64 (sept, J = 6.2 Hz, 1H, methyne), 2.97 (d, J = 6.4 Hz, 2H, SCH₂), 4.80 (s, 1H, OH), 6.87 (AA'BB', 2H, aromatic), 6.97 (AA'BB', 2H, aromatic), 6.99 (AA'BB', 2H, aromatic), 7.32 (AA'BB', 2H, aromatic), 7.69 (AA'BB', 2H, aromatic), 7.76 (AA'BB', 2H, aromatic).

In Table 1, the phrase "base (amount used (mol %))" refers to, as described above, the proportion of the amount of base used (amount by mole) to the amount of repeating unit (amount by mole) in the general formula (2).

### [Examples 8 to 13]

Depolymerization of polyether ether ketone was carried out in the same manner as in Example 7, except that the base shown in Table 1 (0.01 mmol, 10 mol% relative to a repeating unit in the general formula (2)) was used instead of potassium tert-butoxide (0.01 mmol). That is, the base used was potassium hydroxide (KOH) in Example 8, tripotassium phosphate (K₃PO₄) in Example 9, cesium carbonate (Cs₂CO₃) in Example 10, a phosphazene base P₄-t-Bu in Example 11, a phosphazene base P₂-t-Bu in Example 12, and a phosphazene base P₂-Et in Example 13. The results are shown in Table 1.

In Table 1, the terms "phosphazene base P₄-t-Bu" (Example 11), "phosphazene base P₂-t-Bu" (Example 12), and "phosphazene base P₂-Et" (Example 13) are abbreviated as "P₄-t-Bu," "P₂-t-Bu," and "P₂-Et," respectively.

### [Example 14]

Depolymerization of polyether ether ketone was carried out in the same manner as in Example 11, except that the amount of phosphazene base P₄-t-Bu used was 0.005 mmol (5 mol% relative to a repeating unit in the general formula (2)) instead of 0.01 mmol (10 mol% relative to a repeating unit in the general formula (2)). The results are shown in Table 1.

### [Example 15]

Depolymerization of polyether ether ketone was carried out in the same manner as in Example 11, except that the amount of 2-ethylhexyl mercaptan used was 0.25 mmol (2.5 times the amount by mole (2.5 equivalents) of repeating unit in the general formula (2)) instead of 0.2 mmol (twice the amount by mole (2 equivalents) of repeating unit in the general formula (2)). The results are shown in Table 1.

### [Example 16]

Depolymerization of polyether ether ketone was carried out in the same manner as in Example 11, except that tripotassium phosphate (K₃PO₄) (0.005 mmol, 5 mol% relative to a repeating units in the general formula (2)) was used as the base in addition to the phosphazene base P₄-t-Bu (0.01 mmol, 10 mol% relative to a repeating units in the general formula (2)), and that N,N-dimethylacetamide (DMAc) (0.2 mL) was used instead of 1,3-dimethyl-2-imidazolidinone (0.2 mL), and that the amount of 2-ethylhexyl mercaptan used was 0.25 mmol (2.5 times the amount by mole (2.5 equivalents) of repeating unit in the general formula (2)) instead of 0.2 mmol (2 times the amount by mole (2 equivalents) of repeating unit in the general formula (2)). The results are shown in Table 1.

### [Example 17]

Depolymerization of polyether ether ketone was carried out in the same manner as in Example 11, except that the reaction temperature upon the depolymerization was changed to 120°C instead of 150°C, and the amount of 2-ethylhexyl mercaptan used was changed to 0.25 mmol (2.5 times the amount by mole (2.5 equivalents) of repeating unit in the general formula (2)) instead of 0.2 mmol (twice the amount by mole (2 equivalents) of repeating unit in the general formula (2)). The results are shown in Table 1.

### [Example 18]

Depolymerization of polyether ether ketone was carried out in the same manner as in Example 11, except that the reaction temperature upon the depolymerization was changed to 100°C instead of 150°C, and the amount of 2-ethylhexyl mercaptan used was changed to 0.25 mmol (2.5 times the amount by mole (2.5 equivalents) of repeating unit in the general formula (2)) instead of 0.2 mmol (twice the amount by mole (2 equivalents) of repeating unit in the general formula (2)). The results are shown in Table 1.

### [Example 19]

Depolymerization of polyether ether ketone was carried out in the same manner as in Example 11, except that benzonitrile (C₆H₅CN) (0.2 mL) was used instead of 1,3-dimethyl-2-imidazolidinone (0.2 mL), and the amount of 2-ethylhexyl mercaptan used was 0.25 mmol (2.5 times the amount by mole (2.5 equivalents) of repeating unit in the general formula (2)) instead of 0.2 mmol (twice the amount by mole (2 equivalents) of repeating unit in the general formula (2). The results are shown in Table 1.

### [Example 20]

Depolymerization of polyether ether ketone was carried out in the same manner as in Example 11, except that xylene (C₆H₄(CH₃)₂) (0.2 mL) was used instead of 1,3-dimethyl-2-imidazolidinone (0.2 mL), and the amount of 2-ethylhexyl mercaptan used was 0.25 mmol (2.5 times the amount by mole (2.5 equivalents) of repeating unit in the general formula (2)) instead of 0.2 mmol (twice the amount by mole (2 equivalents) of repeating unit in the general formula (2)). The results are shown in Table 1.

It is to be noted that the results of NMR analysis of the obtained BEHTPM, hydroquinone, and the compound (d) in Examples 8 to 20 as well were the same as in Example 7. 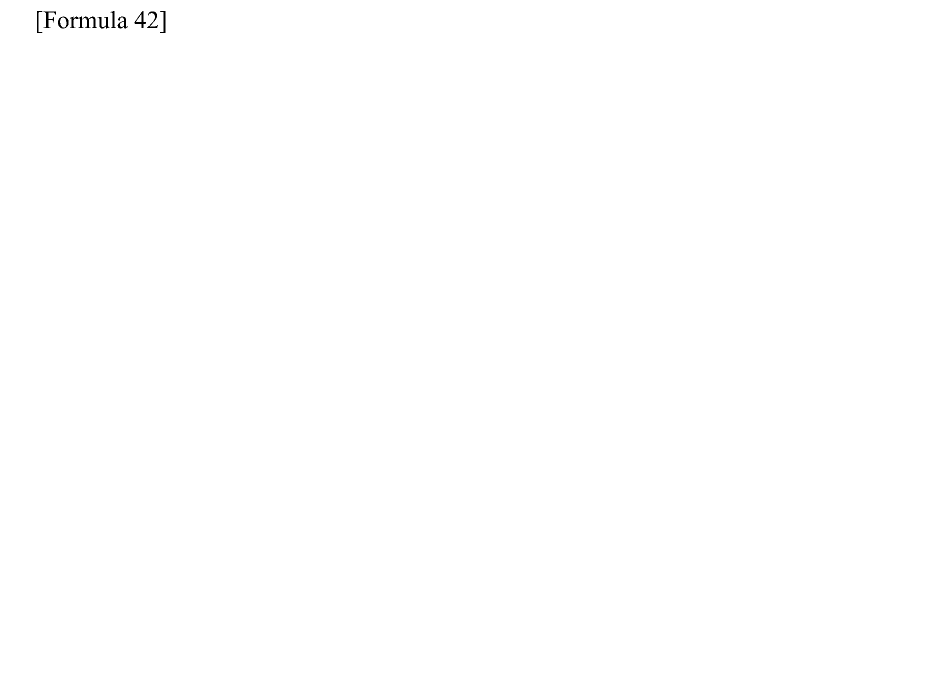 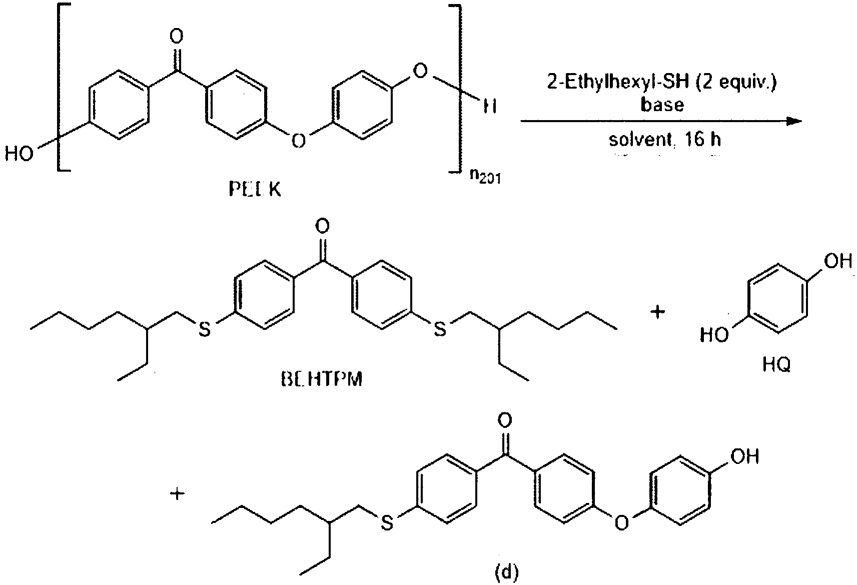 (wherein n₂₀₁ is an integer (preferably 10 to 200)).

**[Table 1]**

| | Conditions of depolymerization | | | | Yield | | |
|---|---|---|---|---|---|---|---|
| | Base (amount used (mol%) | Amount of compound (8) used | Solvent | Reaction temperature (°C) | BEHTPM | HQ | Compound (d) |
| Example 7 | KOt-Bu(10) | 0.2 | DMI | 150 | 76 | 68 | 12 |
| Example 8 | KOH(10) | 0.2 | DMI | 150 | 51 | 45 | 17 |
| Example 9 | K₃PO₄(10) | 0.2 | DMI | 150 | 66 | 61 | 12 |
| Example 10 | Cs₂CO₃(10) | 0.2 | DMI | 150 | 83 | 73 | 13 |
| Example 11 | P₄-t-Bu(10) | 0.2 | DMI | 150 | 83 | 78 | 16 |
| Example 12 | P₂-t-Bu(10) | 0.2 | DMI | 150 | 75 | 72 | 14 |
| Example 13 | P₂-Et(10) | 0.2 | DMI | 150 | 64 | 56 | 19 |
| Example 14 | P₄-t-Bu(5) | 0.2 | DMI | 150 | 71 | 63 | 12 |
| Example 15 | P₄-t-Bu(10) | 0.25 | DMI | 150 | 91 | 80 | 7 |
| Example 16 | P₄-t-Bu(10)/ K₃PO₄(5) | 0.25 | DMAc | 150 | > 99 | > 99 | - |
| Example 17 | P₄-t-Bu(10) | 0.25 | DMI | 120 | 62 | 49 | 11 |
| Example 18 | P₄-t-Bu(10) | 0.25 | DMI | 100 | 46 | 34 | 8 |
| Example 19 | P₄-t-Bu(10) | 0.25 | C₆H₅CN | 150 | 39 | 13 | 28 |
| Example 20 | P₄-t-Bu(10) | 0.25 | C₆H₄(CH₃)₂ | 150 | 14 | 2 | 19 |

As is clarified from the above-described results, it was confirmed that BEHTPM and hydroquinone could be obtained in favorable yields even though the type of base was changed, for example, whether the base was an organic base or an inorganic base.

Among them, in case of using the phosphazene base P₄-t-Bu (Examples 11, 14 to 16), the phosphazene base P₂-t-Bu (Example 12), cesium carbonate (Example 10) or potassium tert-butoxide (Example 7) as the base, the yields of BEHTPM and hydroquinone were higher, and in case of using the phosphazene base P₄-t-Bu and tripotassium phosphate in combination thereof as the bases (Example 16), the yields of BEHTPM and hydroquinone were particularly high.

The comparison between Examples 15, 17 and 18 confirmed that the higher the reaction temperature upon depolymerization (for example, 140 to 180°C), the more the yields of BEHTPM and hydroquinone tend to be.

The comparison between Examples 11 and 14 confirmed that the more the amount of base used (for example, 0.1 to 0.3 equivalents), the more the yields of BEHTPM and hydroquinone tended to be.

The comparison of Examples 11 and 15 confirmed that the more the amount of compound (8) used (for example, 2.2 to 4 equivalents, preferably 2.2 to 4 equivalents), the more the yields of BEHTPM and hydroquinone tended to be.

The comparison between Examples 17, 19, and 20 confirmed that the yields of BEHTPM and hydroquinone were higher by using 1,3-dimethyl-2-imidazolidinone as a solvent.

### <<Depolymerization of polyether ether ketone (depolymerization method (ii))>>

### [Example 21]

Depolymerization of polyether ether ketone was carried out in the same manner as in Example 16.

In an argon atmosphere, to powdered polyether ether ketone (28.8 mg; the amount of repeating unit in the general formula (2) of 0.1 mmol; weight-average molecular weight 20,800; and number-average molecular weight 10,300, manufactured by Sigma-Aldrich Co., LLC, Cat. No. 456640) were added a 0.8 M concentration n-hexane solution of a phosphazene base P₄-t-Bu (manufactured by Sigma-Aldrich Co. LLC, Cat. No. 79421) (12.5 µL, 0.01 mmol as the phosphazene base P₄-t-Bu, 10 mol% relative to a repeating unit in the general formula (2)), tripotassium phosphate (K₃PO₄) (1.1 mg, 0.005 mmol, 5 mol% relative to a repeating unit in the general formula (2)), N,N-dimethylacetamide (DMAc) (0.2 mL), and 2-ethylhexyl mercaptan (corresponding to the compound (8)) (31 mg, 0.2 mmol, twice the amount by mole (2 equivalents) of repeating unit in the general formula (2)) in sequence to dissolve the polyether ether ketone, and the resulting mixture was then stirred at 150°C for 16 hours.

Next, the temperature of the resulting yellow reaction mixture was returned to room temperature, and a crude product was obtained by distillation under reduced pressure.

The obtained crude product was subjected to silica gel column chromatography (eluent: hexane/ethyl acetate 96/4 → 70/30), yielding the target substances, bis(4-(2-ethylhexylthio)phenyl)methanone (BEHTPM) (yield: 39.9 mg, 85% yield) and hydroquinone (HQ) (yield: 6.6 mg, 61% yield).

The results of NMR analysis of the resulting BEHTPM and hydroquinone were the same as those in Example 7. 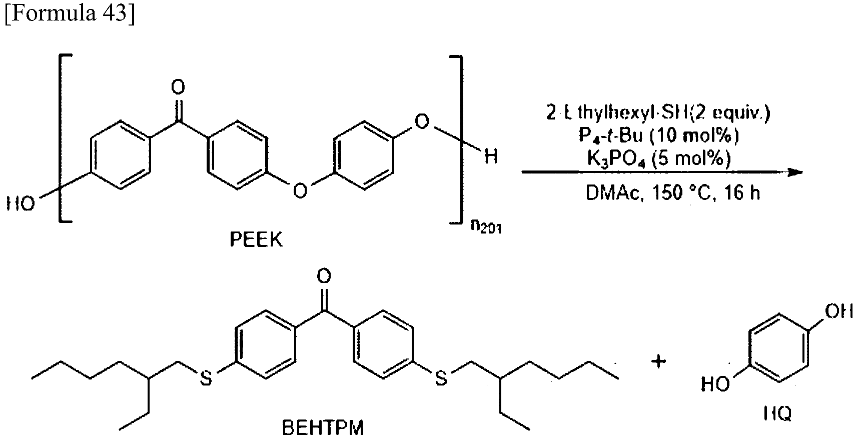 (wherein n₂₀₁ is an integer (preferably 10 to 200)).

### <<Depolymerization of polyether ether ketone reinforced with carbon fiber (depolymerization method (ii))>>

### [Example 22]

A polyether ether ketone reinforced with carbon fibers (TECAPEEK CF30, Cat. No. 3-3094-02, manufactured by Ensinger GmbH) was prepared. In this carbon fiber-reinforced polyether ether ketone, the proportion of the content of polyether ether ketone to the total mass of carbon fiber-reinforced polyether ether ketone was 70% by mass.

This carbon fiber-reinforced polyether ether ketone was processed into powder.

The polyether ether ketone was depolymerized to isolate the target substance in the same manner as in Example 21, except that the powder of the carbon fiber-reinforced polyether ether ketone obtained above (the amount of repeating unit in the general formula (2) of 0.1 mmol) was used instead of the powdered polyether ether ketone (the amount of repeating unit in the general formula (2) of 0.1 mmol). As a result, the target substances bis(4-(2-ethylhexylthio)phenyl)methanone (BEHTPM) (yield: 86%) and hydroquinone (HQ) (yield: 70%) were obtained.

The results of NMR analysis of the resulting BEHTPM and hydroquinone were the same as those in Example 7.

### <<Depolymerization of glass fiber-reinforced polyether ether ketone (depolymerization method (ii))>>

### [Example 23]

Glass fiber-reinforced polyether ether ketone (TECAPEEK GF30, Cat. No. 3-3095-01, manufactured by Ensinger GmbH) was prepared. In this glass fiber-reinforced polyether ether ketone, the proportion of the content of polyether ether ketone to the total mass of the glass fiber-reinforced polyether ether ketone was 30% by mass.

This glass fiber-reinforced polyether ether ketone was processed into powder.

The polyether ether ketone was depolymerized to isolate the target substance in the same manner as in Example 21, except that the powder of the glass fiber-reinforced polyether ether ketone obtained above (the amount of repeating unit in the general formula (2) of 0.1 mmol) was used instead of the powdered polyether ether ketone (the amount of repeating unit in the general formula (2) of 0.1 mmol). As a result, the target substances bis(4-(2-ethylhexylthio)phenyl)methanone (BEHTPM) (yield: 76%) and hydroquinone (HQ) (yield: 33%) were obtained.

The results of NMR analysis of the resulting BEHTPM and hydroquinone were the same as those in Example 7.

### <<Depolymerization of polysulfone (depolymerization method (i))>>

### [Example 24]

Under an argon atmosphere, to a colorless and transparent pellet-shaped polysulfone (44.6 mg; the amount of repeating unit in the general formula (1) of 0.100 mmol; weight-average molecular weight 35,000; and number-average molecular weight 16,000, manufactured by Sigma-Aldrich Co. LLC, Cat. No. 428302) were added a 0.8 M concentration n-hexane solution of a phosphazene base P₄-t-Bu (manufactured by Sigma-Aldrich Co. LLC, Cat. No. 79421) (12.6 µL, 0.01 mmol as the phosphazene base P₄-t-Bu, 10 mol% relative to a repeating unit in the general formula (1)), tripotassium phosphate (K₃PO₄) (1.1 mg, 0.005 mmol, 5 mol% relative to a repeating unit in the general formula (1)), N,N-dimethylacetamide (DMAc) (0.2 mL), and 2-phenylethyl mercaptan (alias: 2-phenylethanethiol, corresponding to the compound (8)) (35.0 mg, 0.253 mmol, 2.5 times the amount by mole (2.5 equivalents) of repeating unit in the general formula (1)) in sequence to dissolve the polysulfone, and the resulting mixture was then stirred at 100°C for 64 hours.

Next, the temperature of the resulting yellow reaction mixture was returned to room temperature, and 1,4-dioxane (5.2 mg, 5.0 µL, 0.059 mmol) was added as an internal standard substance, and a small amount of the resulting mixture was taken as a sample, dissolved in deuterated chloroform, and analyzed by ¹H NMR. As a result, it was confirmed that target substances (depolymerized products), bis(4-(2-phenylethylthio)phenyl)sulfone (BPETPS, corresponding to the compound (18)) and bisphenol A (BPA, corresponding to the compound (121)), were obtained.

The sample used in the ¹H NMR analysis described above was returned to the yellow reaction mixture, and a crude product was obtained by distillation under reduced pressure.

The obtained crude product was subjected to silica gel column chromatography (eluent: hexane/ethyl acetate 96/4 → 70/30), yielding the target substances, bis(4-(2-phenylethylthio)phenyl)sulfone (BPETPS) (yield:46.9 mg, 95%) and bisphenol A (BPA) (yield: 24.7 mg, 95%).

The results of ¹H NMR and ¹³C NMR analyses of the obtained BPETPS are shown below. The results of ¹H NMR analysis of bisphenol A were the same as those in Example 1.

### BPETPS:

¹H NMR (600 MHz, CDCl₃) δ 2.96 (t, *J =* 7.9 Hz, 4H, methyne), 3.22 (d, *J* = 7.9 Hz, 4H, SCH₂), 7.20 (d, *J =* 7.0 Hz, 4H, aromatic), 7.22-7.24 (m, 2H, aromatic), 7.30-7.33 (m, 8H, aromatic), 7.79 (AA'BB', 4H, aromatic).

¹³C NMR (151 MHz, CDCl₃) δ 33.5, 35.0, 1268, 126.9, 127.9, 128.5, 128.7, 138.0, 139.4, 145.0. 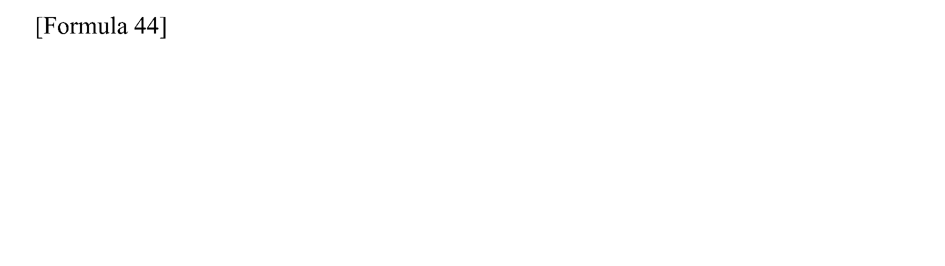 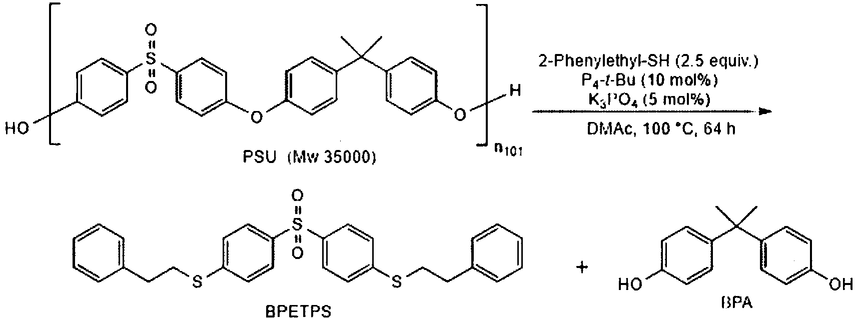 (wherein n₁₀₁ is an integer (preferably 10 to 200)).

### <<Depolymerization of polysulfone (depolymerization method (i))>>

### [Example 25]

Under an argon atmosphere, to a colorless and transparent pellet-shaped polysulfone (44.3 mg; the amount of repeating unit in the general formula (1) of 0.10 mmol; weight-average molecular weight 35,000; and number-average molecular weight 16,000, manufactured by Sigma-Aldrich Co. LLC, Cat. No. 428302) were added a 0.8 M concentration n-hexane solution of a phosphazene base P₄-t-Bu (manufactured by Sigma-Aldrich Co. LLC, Cat. No. 79421) (12.5 µL, 0.01 mmol as the phosphazene base P₄-t-Bu, 10 mol% relative to a repeating unit in the general formula (1)), tripotassium phosphate (K₃PO₄) (1.1 mg, 0.005 mmol, 5 mol% relative to a repeating unit in the general formula (1)), N,N-dimethylacetamide (DMAc) (0.2 mL), and 2-mercaptoethanol (corresponding to the compound (8)) (19.6 mg, 0.25 mmol, 2.5 times the amount by mole (2.5 equivalents) of repeating unit in the general formula (1)) in sequence to dissolve the polysulfone, and the resulting mixture was then stirred at 100°C for 64 hours.

Next, the temperature of the resulting yellow reaction mixture was returned to room temperature, and 1,4-dioxane (5.2 mg, 5.0 µL, 0.059 mmol) was added as an internal standard substance, and a small amount of the resulting mixture was taken as a sample, dissolved in deuterated chloroform, and analyzed by ¹H NMR. As a result, it was confirmed that target substances (depolymerized products), bis(4-(2-hydroxyethylthio)phenyl)sulfone (BHETPS, corresponding to the compound (18)) and bisphenol A (BPA, corresponding to the compound (121)) were obtained.

The sample used in the ¹H NMR analysis described above was returned to the yellow reaction mixture, and a crude product was obtained by distillation under reduced pressure.

The obtained crude product was subjected to silica gel column chromatography (eluent: hexane/ethyl acetate 96/4 → 70/30), yielding the target substances, bis(4-(2-hydroxyethylthio)phenyl)sulfone (BHETPS) (yield: 29.1 mg, 79%) and bisphenol A (BPA) (yield: 17.0 mg, 71%).

The results of ¹H NMR and ¹³C NMR analyses of the obtained BHETPS are shown below. The results of ¹H NMR analysis of bisphenol A were the same as those in Example 1.

### BHETPS:

¹H NMR (600 MHz, CDCl₃) δ 3.21 (t, *J =* 6.6 Hz, 4H, SCH₂), 3.76-3.78 (m, 4H, HOC*H₂*), 4.12 (br, 2H, OH), 7.50 (AA'BB', 4H, aromatic), 7.84 (AA'BB', 4H, aromatic). ¹³C NMR (151 MHz, CDCl₃) δ 35.0, 61.1, 127.7, 128.7, 139.2, 146.3. 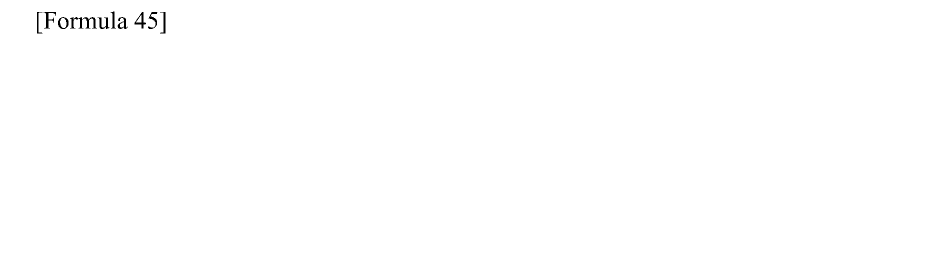 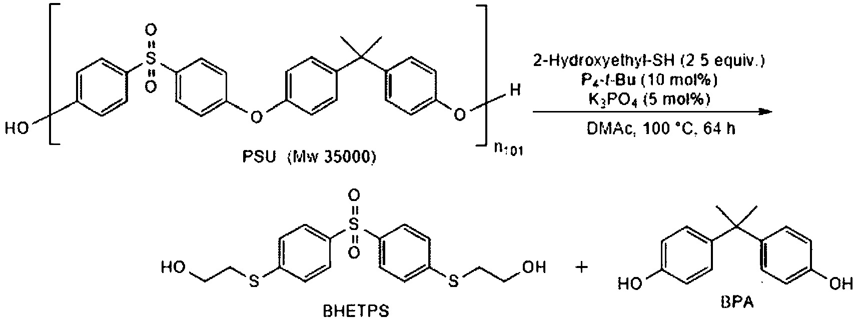 (wherein n₁₀₁ is an integer (preferably 10 to 200)).

### <<Depolymerization of polysulfone (depolymerization method (i))>>

### [Example 26]

Under an argon atmosphere, to a colorless and transparent pellet-shaped polysulfone (44.5 mg; the amount of repeating unit in the general formula (1) of 0.101 mmol; weight-average molecular weight 35,000; and number-average molecular weight 16,000, manufactured by Sigma-Aldrich Co. LLC, Cat. No. 428302) were added a 0.8 M concentration n-hexane solution of a phosphazene base P₄-t-Bu (manufactured by Sigma-Aldrich Co. LLC, Cat. No. 79421) (12.5 µL, 0.01 mmol as the phosphazene base P₄-t-Bu, 10 mol% relative to a repeating unit in the general formula (1)), tripotassium phosphate (K₃PO₄) (1.3 mg, 0.006 mmol, 5 mol% relative to a repeating unit in the general formula (1)), N,N-dimethylacetamide (DMAc) (0.2 mL), and 3-(triethoxysilyl)propanethiol (corresponding to the compound (8)) (59.4 mg, 0.25 mmol, 2.5 times the amount by mole (2.5 equivalents) of repeating unit in the general formula (1)) in sequence to dissolve the polysulfone, and the resulting mixture was then stirred at 150°C for 16 hours.

Next, the temperature of the resulting orange reaction mixture was returned to room temperature, and 1,4-dioxane (5.2 mg, 5.0 µL, 0.059 mmol) was added as an internal standard substance, and a small amount of the resulting mixture was taken as a sample, dissolved in deuterated chloroform and analyzed by ¹H NMR. As a result, it was confirmed that target substances (depolymerized products), bis(4-(3-(triethoxysilyl)propylthio)phenyl)sulfone (BTEOSPTPS, corresponding to the compound (18)) and bisphenol A (BPA, corresponding to the compound (121)) were obtained.

The sample used in the ¹H NMR analysis described above was returned to the orange reaction mixture, and a crude product was obtained by distillation under reduced pressure.

The obtained crude product was subjected to silica gel column chromatography (eluent: hexane/ethyl acetate 96/4 → 70/30), yielding the target substances, bis(4-(3-(triethoxysilyl)propylthio)phenyl)sulfone (BTEOSPTPS) (yield: 18.5 mg, 26%) and bisphenol A (BPA) (yield: 24.9 mg, slightly more than 99%).

The results of ¹H NMR and ¹³C NMR analyses of the obtained BTEOSPTPS are shown below. The results of ¹H NMR analysis of bisphenol A were the same as those in Example 1.

### BPETPS:

¹H NMR (600 MHz, CDCl₃) δ 0.75-80 (m, 4H, methylene), 1.19 (t, *J =* 7.0 Hz, 18H, methyl), 1.79 (tt, *J* = 7.7 Hz, 4H, methylene), 2.99 (t, *J =* 7.5 Hz, 4H, SCH₂), 3.79 (q, *J* = 7.0 Hz, 4H, OC*H*₂), 7.30 (AA'BB', 4H, aromatic), 7.76 (AA'BB', 4H, aromatic). ¹³C NMR (151 MHz, CDCl₃) δ 9.93, 18.3, 22.4, 34.6, 58.5, 126.7, 127.8, 137.7, 145.5. 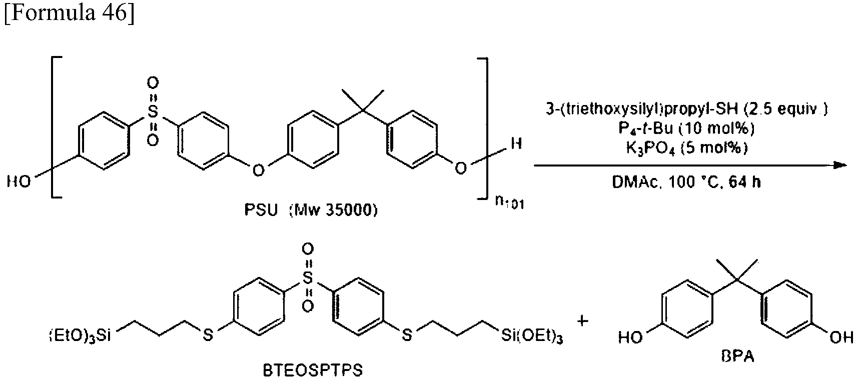 (wherein n₁₀₁ is an integer (preferably 10 to 200)).

### <<Depolymerization of polysulfone (depolymerization method (i))>>

### [Example 27]

Under an argon atmosphere, to a colorless and transparent pellet-shaped polysulfone (44.1 mg; the amount of repeating unit in the general formula (1) of 0.100 mmol; weight-average molecular weight 35,000; and number-average molecular weight 16,000, manufactured by Sigma-Aldrich Co. LLC, Cat. No. 428302) were added a 0.8 M concentration n-hexane solution of a phosphazene base P₄-t-Bu (manufactured by Sigma-Aldrich Co. LLC, Cat. No. 79421) (12.5 µL, 0.01 mmol as the phosphazene base P₄-t-Bu, 10 mol% relative to a repeating unit in the general formula (1)), tripotassium phosphate (K₃PO₄) (1.1 mg, 0.005 mmol, 5 mol% relative to a repeating unit in the general formula (1)), N,N-dimethylacetamide (DMAc) (0.2 mL), and cyclopentanethiol (corresponding to the compound (8)) (25.4 mg, 0.25 mmol, 2.5 times the amount by mole (2.5 equivalents) of repeating unit in the general formula (1)) in sequence to dissolve the polysulfone, and the resulting mixture was then stirred at 150°C for 16 hours.

Next, the temperature of the resulting yellow reaction mixture was returned to room temperature, and 1,4-dioxane (5.2 mg, 5.0 µL, 0.059 mmol) was added as an internal standard substance, and a small amount of the resulting mixture was taken as a sample, dissolved in deuterated chloroform, and analyzed by ¹H NMR. As a result, it was confirmed that the target substances (depolymerized products), bis(4-(cyclopentylthio)phenyl)sulfone (BcyPenTPS, corresponding to the compound (18)) and bisphenol A (BPA, corresponding to the compound (121) were obtained.

The sample used in the ¹H NMR analysis described above was returned to the yellow reaction mixture, and a crude product was obtained by distillation under reduced pressure.

The obtained crude product was subjected to silica gel column chromatography (eluent: hexane/ethyl acetate 96/4 → 70/30), yielding the target substances, bis(4-(cyclopentylthio)phenyl)sulfone (BcyPenTPS) (yield: 39.3 mg, 94%) and bisphenol A (BPA) (yield: 26.3 mg, slightly more than 99%).

The results of ¹H NMR and ¹³C NMR analyses of the obtained BcyPenTPS shown below. The results of ¹H NMR analysis of bisphenol A were the same as those in Example 1.

### BPETPS:

¹ H NMR (600 MHz, CDCl₃) δ 1.59-1.67 (m, 8H, methylene), 1.75-1.81 (m, 4H, methylene), 2.09-2.16 (m, 4H, methylene), 3.65-3.70 (m, 2H, SC*H*), 7.32 (AA'BB', 4H, aromatic), 7.77 (AA'BB', 4H, aromatic). ¹³C NMR (151 MHz, CDCl₃) δ 24.9, 33.4, 44.1, 127.2, 127.7, 137.6, 146.2. 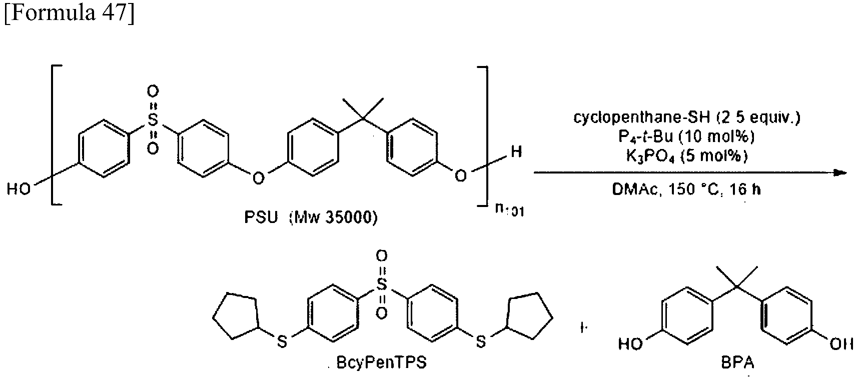 (wherein n₁₀₁ is an integer (preferably 10 to 200)).

### <<Depolymerization of polysulfone (depolymerization method (i))>>

### [Example 28]

Under an argon atmosphere, to a colorless and transparent pellet-shaped polysulfone (44.1 mg; the amount of repeating unit in the general formula (1) of 0.100 mmol; weight-average molecular weight 35,000; and number-average molecular weight 16,000, manufactured by Sigma-Aldrich Co. LLC, Cat. No. 428302) were added a 0.8 M concentration n-hexane solution of a phosphazene base P₄-t-Bu (manufactured by Sigma-Aldrich Co. LLC, Cat. No. 79421) (12.5 µL, 0.01 mmol as the phosphazene base P₄-t-Bu, 10 mol% relative to a repeating unit in the general formula (1)), tripotassium phosphate (K₃PO₄) (1.1 mg, 0.005 mmol, 5 mol% relative to a repeating unit in the general formula (1)), N,N-dimethylacetamide (DMAc) (0.2 mL), and trimethylsilylmethylthiol (corresponding to the compound (8)) (30.2 mg, 0.25 mmol, 2.5 times the amount by mole (2.5 equivalents) of repeating unit in the general formula (1)) in sequence to dissolve the polysulfone, and the resulting mixture was then stirred at 150°C for 64 hours.

Next, the temperature of the resulting brown reaction mixture was returned to room temperature, and 1,4-dioxane (5.2 mg, 5.0 µL, 0.059 mmol) was added as an internal standard substance, and a small amount of the resulting mixture was taken as a sample, dissolved in deuterated chloroform and analyzed by ¹H NMR. As a result, it was confirmed that bis(4-methylthiophenyl)sulfone (BMTPS, corresponding to compound (18)) and bisphenol A (BPA, corresponding to compound (121)), which were depolymerized products, were obtained.

The sample used in the ¹H NMR analysis described above was returned to the brown reaction mixture, and a crude product was obtained by distillation under reduced pressure.

The obtained crude product was subjected to silica gel column chromatography (eluent: hexane/ethyl acetate 96/4 → 70/30), yielding bis(4-methylthiophenyl)sulfone (BMTPS) (yield: 23.0 mg, 74%) and bisphenol A (BPA) (yield: 23.3 mg, slightly more than 99%).

When the obtained BMTPS and bisphenol A were measured by ¹H NMR, the results thereof were the same as those in Comparative Example 1. 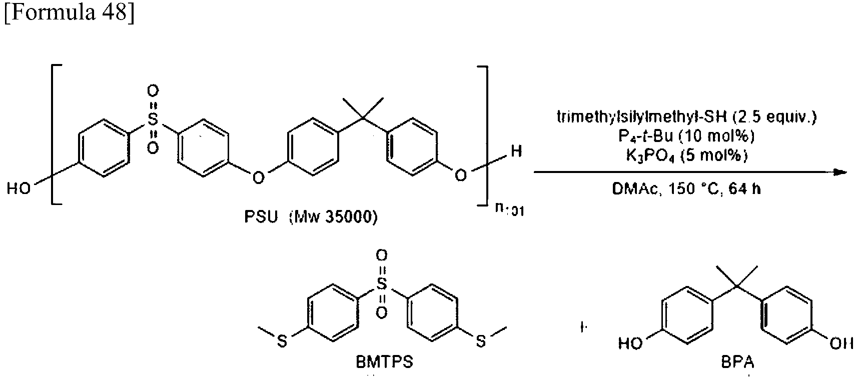 (wherein n₁₀₁ is an integer (preferably 10 to 200)).

### <<Depolymerization of polysulfone (depolymerization method (i))>>

### [Example 29]

Under an argon atmosphere, to a colorless and transparent pellet-shaped polysulfone (44.3 mg; the amount of repeating unit in the general formula (1) of 0.100 mmol; weight-average molecular weight 35,000; and number-average molecular weight 16,000, manufactured by Sigma-Aldrich Co. LLC, Cat. No. 428302) were added sodium tert-butoxide (2.0 mg, 0.02 mmol, 20 mol% relative to a repeating unit in the general formula (1)), N,N-dimethylacetamide (DMAc) (0.2 mL), and 4-tert-butylphenyl mercaptan (alias: 4-tert-butylbenzenethiol) (41.4 mg, 0.25 mmol, 2.5 times the amount by mole (2.5 equivalents) of repeating unit in the general formula (1)) in sequence to dissolve the polysulfone, and the resulting mixture was then stirred at 150°C for 16 hours.

Next, the temperature of the resulting yellow reaction mixture was returned to room temperature, and 1,4-dioxane (5.2 mg, 5.0 µL, 0.059 mmol) was added as an internal standard substance, and a small amount of the resulting mixture was taken as a sample, dissolved in deuterated chloroform, and analyzed by ¹H NMR. As a result, it was confirmed that the target substances (depolymerized products), bis(4-(4-tert-butylphenylthio)phenyl)sulfone (BBPTPS, corresponding to the compound (18)) and bisphenol A (BPA, corresponding to the compound (121)) were obtained.

The sample used in the ¹H NMR analysis described above was returned to the yellow reaction mixture, and a crude product was obtained by distillation under reduced pressure.

The obtained crude product was subjected to silica gel column chromatography (eluent: hexane/ethyl acetate 96/4 → 70/30), yielding the target substances, bis(4-(4-tert-butylphenylthio)phenyl)sulfone (BBPTPS) (yield: 53.8 mg, 98%) and bisphenol A (BPA) (yield: 23.2 mg, over 99%).

The results of ¹H NMR and ¹³C NMR analyses of the obtained BBPTPS shown below. The results of ¹H NMR analysis of bisphenol A were the same as those in Example 1.

### BBPTPS:

¹H NMR (600 MHz, CDCl₃) δ 1.34 (s, 18H, tBu), 7.19 (AA'BB', 4H, aromatic), 7.42-7.46 (m, 8H, aromatic), 7.64 (AA'BB', 4H, aromatic). ¹³C NMR (151 MHz, CDCl₃) δ 31.3, 34.8, 126.7, 126.8, 128.1, 130.6, 134.0, 134.5, 144.9, 152.4, 194.9. 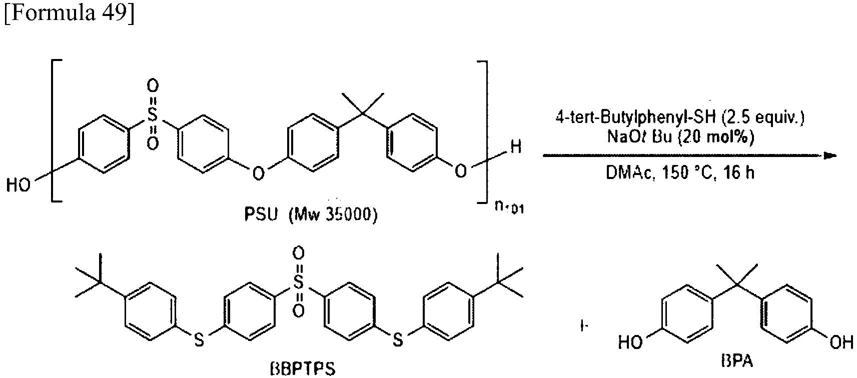 (wherein n₁₀₁ is an integer (preferably 10 to 200)).

### <<Depolymerization of polysulfone (depolymerization method (i))>>

### [Example 30]

In an argon atmosphere, to powdered polyether ether ketone (29.0 mg; the amount of repeating unit in the general formula (2) of 0.1 mmol; weight-average molecular weight 20,800; and number-average molecular weight 10,300, manufactured by Sigma-Aldrich Co., LLC, Cat. No. 456640) were added a 0.8 M concentration n-hexane solution of a phosphazene base P₄-t-Bu (manufactured by Sigma-Aldrich Co. LLC, Cat. No. 79421) (12.5 µL, 0.01 mmol as the phosphazene base P₄-t-Bu, 10 mol% relative to a repeating unit in the general formula (1)), cesium carbonate (Cs₃CO₃) (4.3 mg, 0.01 mmol, 10 mol% relative to a repeating unit in the general formula (1)), 1,3-dimethyl-2-imidazolidinone (DMI) (1.0 mL), and 4-tert-butylphenyl mercaptan (alias: 4-tert-butylbenzenethiol) (corresponding to the compound (8)) (40.5 mg, 0.24 mmol, 2.4 times the amount by mole (2.4 equivalents) of repeating unit in the general formula (1)) in sequence to dissolve the polysulfone, and the resulting mixture was then stirred at 150°C for 109 hours.

Next, the temperature of the resulting yellow reaction mixture was returned to room temperature, and 1,4-dioxane (5.2 mg, 5.0 µL, 0.059 mmol) was added as an internal standard substance, and a small amount of the resulting mixture was taken as a sample, dissolved in deuterated chloroform, and analyzed by ¹H NMR. As a result, it was confirmed that bis(4-(4-tert-butylphenylthio)phenyl)ketone (BBPTPK, corresponding to the compound (18)) and hydroquinone (HQ, corresponding to the compound (121), yield: 75%), which were depolymerized products, were obtained.

The sample used in the ¹H NMR analysis described above was returned to the yellow reaction mixture, and a crude product was obtained by distillation under reduced pressure.

The obtained crude product was subjected to silica gel column chromatography (eluent: hexane/ethyl acetate 96/4 → 70/30), yielding bis(4-(4-tert-butylphenylthio)phenyl)ketone (BBPTPK) (yield: 44.1 mg, 86%).

The results of ¹H NMR and ¹³C NMR analyses of the obtained BBPTPK shown below. The results of ¹H NMR analysis of hydroquinone were the same as those in Comparative Example 7.

### BMTPK:

¹ H NMR (600 MHz, CDCl₃) δ 1.34 (s, 18H, tBu), 7.19 (AA'BB', 4H, aromatic), 7.42-7.46 (m, 8H, aromatic), 7.64 (AA'BB', 4H, aromatic). ¹³C NMR (151 MHz, CDCl₃) δ 31.3, 34.8, 126.7, 126.8, 128.1, 130.6, 134.0, 134.5, 144.9, 152.4, 194.9. 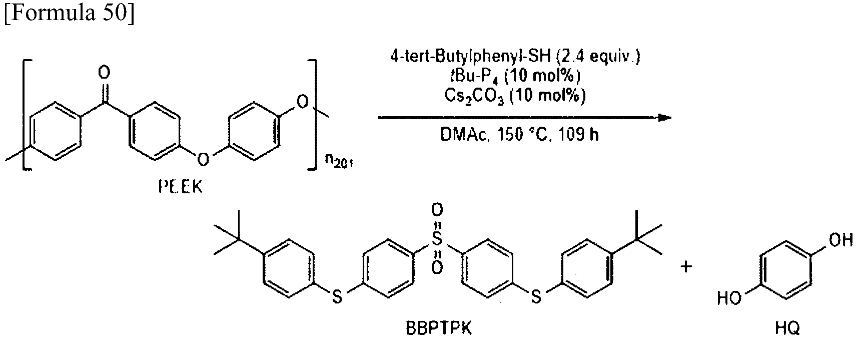 (wherein n₂₀₁ is an integer (preferably 10 to 200)).

### <<Depolymerization of polysulfone (depolymerization method (i))>>

### [Example 31]

A polyphenylsulfone (PPSU) baby feeding bottle (manufactured by CHU CHU HEALTHCARE LIMITED) was cut into 5 mm pieces.

In an argon atmosphere, to the pale yellow polyphenylsulfone pieces obtained above (81.4 mg; the amount of repeating unit in the general formula (1) of 0.2 mmol) were added a 0.8 M concentration n-hexane solution of a phosphazene base P₄-t-Bu (manufactured by Sigma-Aldrich Co. LLC, Cat. No. 79421) (25.0 µL, 0.01 mmol as the phosphazene base P₄-t-Bu, 10 mol% relative to a repeating unit in the general formula (1)), tripotassium phosphate (K₃PO₄) (2.1 mg, 0.001 mmol, 5 mol% relative to a repeating unit in the general formula (1)), N,N-dimethylacetamide (DMAc) (0.4 mL), and 2-ethylhexyl mercaptan (corresponding to the compound (8)) (72.5 mg, 0.50 mmol, 2.5 times the amount by mole (2.5 equivalents) of repeating unit in the general formula (1)) in sequence to dissolve the polyphenylsulfone, and the resulting mixture was then stirred at 150°C for 20 hours.

Next, the temperature of the resulting yellow reaction mixture was returned to room temperature, and 1,4-dioxane (5.2 mg, 5.0 µL, 0.059 mmol) was added as an internal standard substance, and a small amount of the resulting mixture was taken as a sample, dissolved in deuterated chloroform and analyzed by ¹H NMR. As a result, it was confirmed that target substances that were depolymerized products, bis(4-(2-ethylhexylthio)phenyl)sulfone (BEHTPS, corresponding to the compound (18)) and 4,4'-dihydroxybiphenyl (4,4'-DHBP, corresponding to the compound (123)) were produced.

The sample used in the ¹H NMR analysis described above was returned to the yellow reaction mixture, and a crude product was obtained by distillation under reduced pressure.

The obtained crude product was subjected to silica gel column chromatography (eluent: hexane/ethyl acetate 96/4 → 70/30), yielding the target substances, bis(4-(2-ethylhexylthio)phenyl)sulfone (BEHTPS) (yield: 87 mg, 84%) and 4,4'-dihydroxybiphenyl (4,4'-DHBP) (yield: 34 mg, 91%).

The results of ¹H NMR and ¹³C NMR analyses of the obtained bis(4-(2-ethylhexylthio)phenyl)sulfone were the same as in Example 1.

The results of ¹H NMR analysis of the obtained 4,4'-dihydroxybiphenyl were the same as those of Example 4. 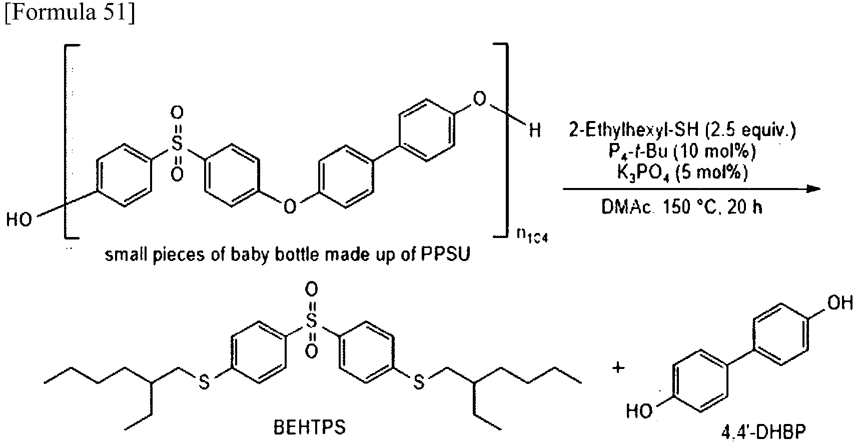 (wherein n₁₀₄ is an integer (preferably 10 to 200).)

### (Embodiment 2)

The second embodiment according to the present invention will be described below, but even though the terms used in the aforementioned embodiment are the same, they may have different meanings. Moreover, Examples 1 to 23 to be described below are different from Examples in the first embodiment described above.

### ⊚Method for depolymerizing compound

### <<Depolymerization method (1)>>

The method for depolymerizing (decomposing) a compound according to one embodiment of the present invention comprises the depolymerization step (decomposition step) of reacting a compound represented by the following general formula (1) (compound (1)): 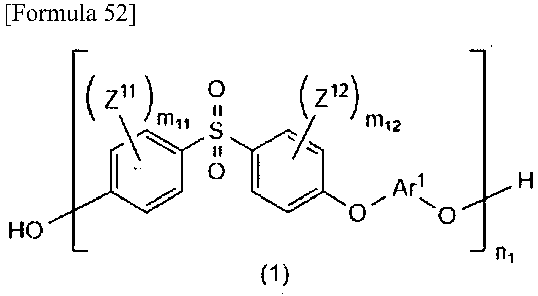
(wherein n₁ is an integer of 2 or greater (preferably 10 to 200); Z¹¹ and Z¹² are each independently a group other than a hydrogen atom; m₁₁ and m₁₂ are each independently an integer of 0 to 4, and when m₁₁ is an integer of 1 or greater, n₁ × m₁₁ Z¹¹ groups may be the same or different from each other, and when m₁₂ is an integer of 1 or greater, n₁ × m₁₂ Z¹² groups may be the same or different from each other; Ar¹ is represented by the following general formula (91), (92) or (93): 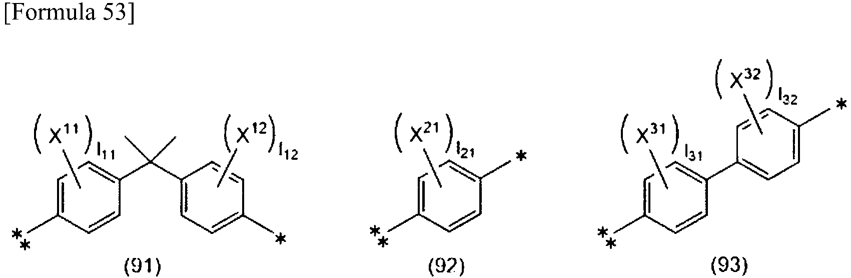
(wherein X¹¹, X¹², X²¹, X³¹, and X³² are each independently a group other than a hydrogen atom; l₁₁, l₁₂, l₂₁, l₃₁, and l₃₂ are each independently an integer of 0 to 4, and when l₁₁ is an integer of 1 or greater, n₁ × l₁₁ X¹¹ groups may be the same or different from each other, when l₁₂ is an integer of 1 or greater, n₁ × l₁₂ X¹² groups may be the same or different from each other, when l₂₁ is an integer of 1 or greater, n₁ × l₂₁ X²¹ groups may be the same or different from each other, when l₃₁ is an integer of 1 or greater, n₁ × l₃₁ X³¹ groups may be the same or different from each other, and when l₃₂ is an integer of 1 or greater, n₁ × l₃₂ X³² groups may be the same or different from each other.), and the bond attached with the sign * and the bond attached with the sign ** in the general formulae (91), (92) and (93) are each formed to an oxygen atom in the general formula (1).)
   in the co-presence of a hydroxide, to obtain a compound represented by the following general formula (11) (may be referred to as "compound (11)" as used herein) 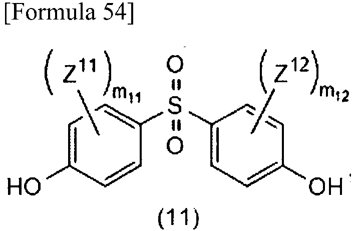
(wherein Z¹¹, Z¹², m₁₁, and m₁₂ are the same as described above.) or a salt thereof as well as a compound represented by the following general formula (121), (122) or (123) (may be each referred to as "compound (121)", "compound (122)", and "compound (123)", as used herein): 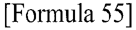 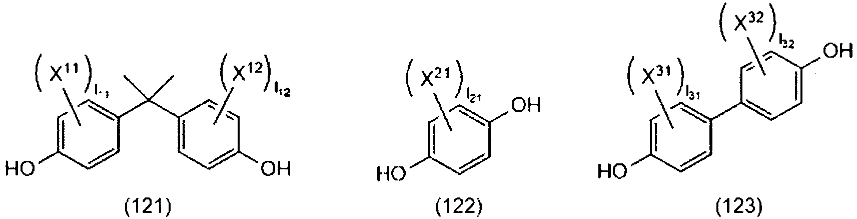
(wherein, X¹¹, X¹², X²¹ X³¹, X³², l₁₁, l₁₂, l₂₁, l₃₁ and l₃₂ are the same as described above.) or a salt thereof. As used herein, the method for depolymerizing a compound of the present embodiment may be referred to as "depolymerization method (1)".

The depolymerization method (depolymerization method (1)) of the present embodiment is a novel depolymerization method (decomposition method) of the compound (1) including super engineering plastics.

When Ar¹ described above is the group represented by the general formula (91) above, the compound (1) described above includes polysulfone (may be referred to as "PSU" as used herein) and its derivative.

When Ar¹ described above is the group represented by the general formula (92) above, the compound (1) described above includes polyether ether sulfone (may be referred to as "PEES" as used herein) and its derivative.

When Ar¹ described above is the group represented by the general formula (93) above, the compound (1) described above includes polyphenyl sulfone (may be referred to as "PPSU" as used herein and its derivative.

As used herein, when a structure in which one or more hydrogen atoms in a certain specific compound have been substituted with a group other than a hydrogen atom, is assumed, the compound having such a substituted structure is referred to as a "derivative" of the aforementioned specific compound.

The term "group" as used herein includes not only an atomic group formed by bonding a plurality of atoms, but also a single atom, unless otherwise specified.

In a case in which Ar¹ is a group represented by the general formula (91) above, the depolymerization method (1) comprises the depolymerization step (decomposition step) of depolymerizing a compound represented by the following general formula (1-1): 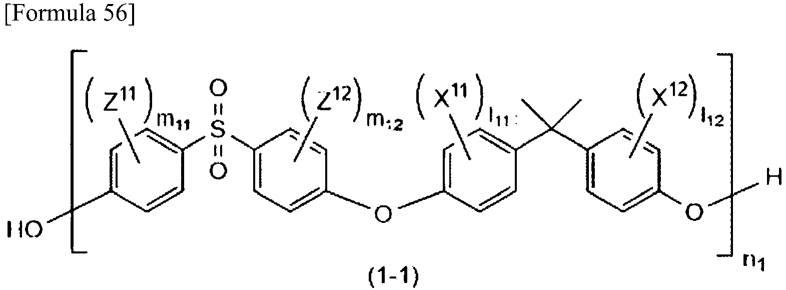
(wherein, n₁, Z¹¹, Z¹², m₁₁, m₁₂, X¹¹, X¹², l₁₁, and l₁₂ are the same as described above.)
   in the co-presence of a hydroxide, to obtain a compound represented by the following general formula (11) (compound (11)): 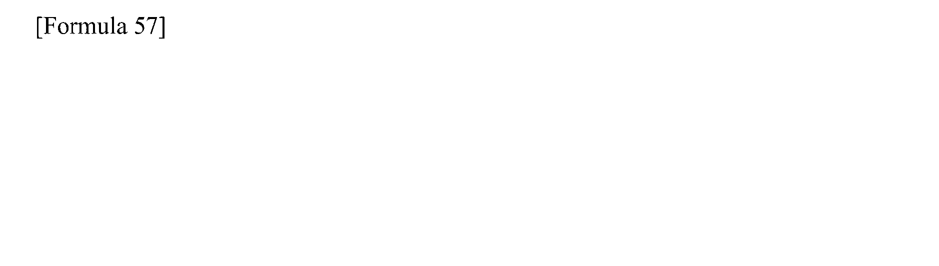 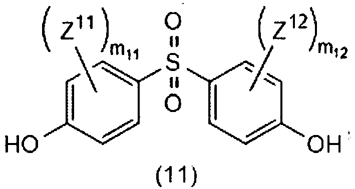
(wherein Z¹¹, Z¹², m₁₁, and m₁₂ are the same as described above.) or its derivative, and a compound represented by the following general formula (121) (compound (121)) 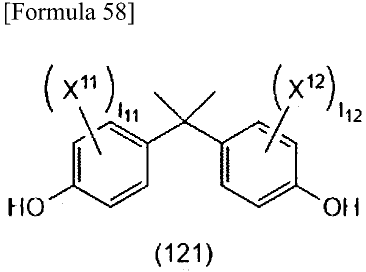
(wherein X¹¹, X¹², l₁₁ and l₁₂ are the same as described above.) or its derivative.

In a case in which Ar¹ is a group represented by the general formula (92) above, the depolymerization method (1) comprises the depolymerization step (decomposition step) of depolymerizing a compound represented by the following general formula (1-2): 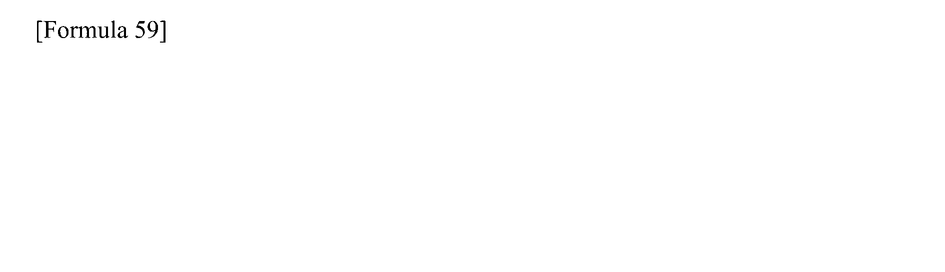 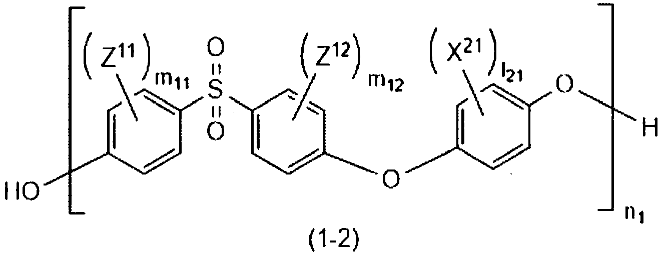
(wherein, n₁, Z¹¹, Z¹², m₁₁, m₁₂, X²¹ and l₂₁ are the same as described above.), in the co-presence of a hydroxide, to obtain a compound represented by the following general formula (11) (compound (11)): 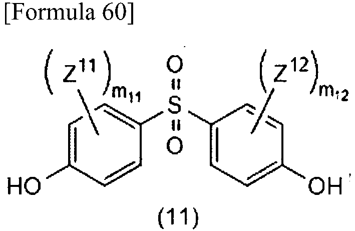
(wherein Z¹¹, Z¹², m₁₁, and m₁₂ are the same as described above.) or its derivative, and a compound represented by the following general formula (122) (compound (122)) 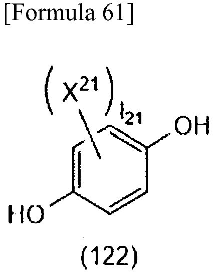
(wherein X²¹ and l₂₁ are the same as described above.) or its derivative.

In a case in which Ar¹ is a group represented by the general formula (93) above, the depolymerization method (1) comprises the depolymerization step (decomposition step) of depolymerizing a compound represented by the following general formula (1-3): 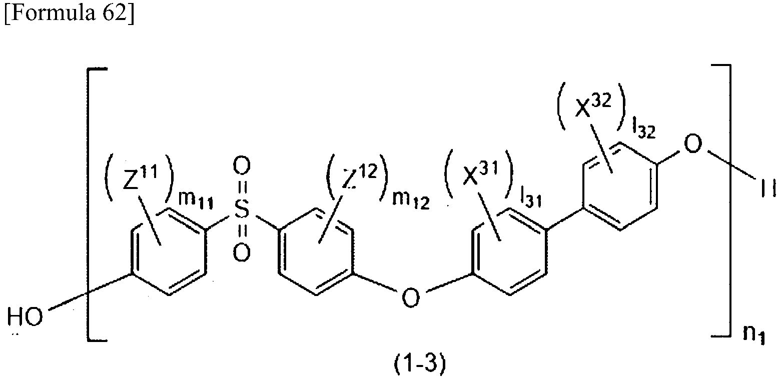
(wherein, n₁, Z¹¹, Z¹², m₁₁, m₁₂, X³¹, X³², l₃₁, and l₃₂ are the same as described above.)
   in the co-presence of a hydroxide, to obtain a compound represented by the following general formula (11) (compound (11)): 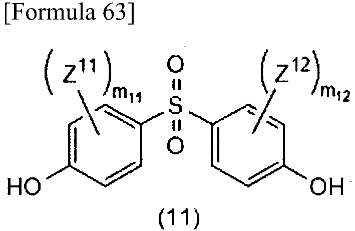
(wherein Z¹¹, Z¹², m₁₁, and m₁₂ are the same as described above.)
   or its derivative, and a compound represented by the following general formula (123) (compound (122)) 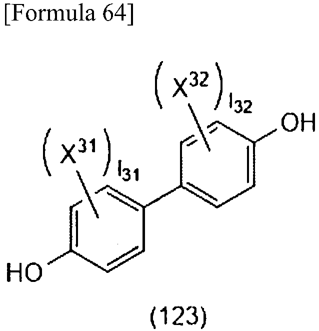
(wherein X³¹, X³², l₃₁ and l₃₂ are the same as described above.)
   or its derivative.

### <Compound (1)>

The compound (1) is an object of depolymerization in the depolymerization method (1).

In the general formula (1), n₁ is the number of repeating units, which defines the molecular size of the compound (1), and is an integer of 2 or greater.

The compound (1) in which n₁ is, for example, 35 to 150 in the formula is suitable as a high molecular weight super engineering plastic such as polysulfone (PSU), polyether ether sulfone (PEES) or polyphenyl sulfone (PPSU), which is difficult to be depolymerized by conventional methods and is particularly suitable as an applicable object for depolymerization method (1).

In the general formula (1), Z¹¹ and Z¹² each independently represents a group other than a hydrogen atom (may be referred to as a "substituent" as used herein). That is, the substituents Z¹¹ and Z¹² may be the same or different from each other.

Examples of Z¹¹ and Z¹² (substituents) include an alkyl group, an alkylcarbonylamino group, a fluorinated alkyl group, and a fluorine atom.

The alkyl group in Z¹¹ and Z¹² may be any one of linear, branched-chained, and cyclic structures, and may have both a chain structure (a linear structure or a branched-chained structure) and a cyclic structure. The alkyl group having a cyclic structure as used herein is considered to be a cyclic alkyl group, regardless of whether or not it has an additional chain structure. A cyclic structure in a cyclic alkyl group (an alkyl group having a cyclic structure and no chain structure, as well as an alkyl group having both a cyclic structure and a chain structure) may be either monocyclic or polycyclic.

The alkyl group in Z¹¹ and Z¹² preferably has 1 to 15 carbon atoms.

Among the alkyl groups in Z¹¹ and Z¹², examples of the chained (linear or branched-chain) alkyl group include chained alkyl groups having 1 to 15 carbon atoms, such as a methyl group, an ethyl group, an n-propyl group, an isopropyl group, an n-butyl group, an isobutyl group, a sec-butyl group, a tert-butyl group, an n-pentyl group, an isopentyl group, a neopentyl group, a tert-pentyl group, a 1-methylbutyl group, an n-hexyl group, a 2-methylpentyl group, a 3-methylpentyl group, a 2,2-dimethylbutyl group, a 2,3-dimethylbutyl group, an n-heptyl group, a 2-methylhexyl group, a 3-methylhexyl group, a 2,2-dimethylpentyl group, a 2,3-dimethylpentyl group, a 2,4-dimethylpentyl group, a 3,3-dimethylpentyl group, a 3-ethylpentyl group, a 2,2,3-trimethylbutyl group, an n-octyl group, an isooctyl group, a 2-ethylhexyl group, a nonyl group, a decyl group, a 3,7-dimethyloctyl group, an undecyl group, a dodecyl group, a tridecyl group, a tetradecyl group, and a pentadecyl group.

Among the alkyl groups in Z¹¹ and Z¹², examples of a cyclic alkyl group (an alkyl group having a monocyclic or polycyclic structure) include cyclic alkyl groups having 3 to 15 carbon atoms, such as a cyclopropyl group, a cyclobutyl group, a cyclopentyl group, a cyclohexyl group, a cycloheptyl group, a cyclooctyl group, a cyclononyl group, a cyclodecyl group, a norbornyl group, an isobornyl group, a 1-adamantyl group, a 2-adamantyl group, a tricyclodecyl group, and a cyclopropylmethyl group.

The alkyl group in Z¹¹ and Z¹² may be, for example, any one of an alkyl group having 1 to 10 carbon atoms (a chained alkyl group having 1 to 10 carbon atoms, or a cyclic alkyl group having 3 to 10 carbon atoms), an alkyl group having 1 to 8 carbon atoms (a chained alkyl group having 1 to 8 carbon atoms, or a cyclic alkyl group having 3 to 8 carbon atoms), an alkyl group having 1 to 6 carbon atoms (a chained alkyl group having 1 to 6 carbon atoms, or a cyclic alkyl group having 3 to 6 carbon atoms), and an alkyl group having 1 to 3 carbon atoms.

Examples of the alkylcarbonylamino group (a group represented by the general formula "-NH-C(=O)-R⁰¹ (wherein R⁰¹ is an alkyl group)") in Z¹¹ and Z¹² include monovalent groups having a structure in which a carbon atom having a free valence in the alkyl group in Z¹¹ and Z¹² described above is bonded to a carbon atom in a carbonylamino group (-NH-C(=O)-).

An example of the alkylcarbonylamino group includes, but is not limited to, a methylcarbonylamino group (-NH-C(=O)-CH₃).

The alkylcarbonylamino group in Z¹¹ and Z¹² preferably has 2 to 16 carbon atoms.

Examples of the fluorinated alkyl group in Z¹¹ and Z¹² include monovalent groups having a structure in which one or two or more hydrogen atoms (-H) in the alkyl group in Z¹¹ and Z¹² have been substituted with fluorine atoms (-F).

In the fluorinated alkyl group, the number of fluorine atoms depends on the number of carbon atoms in the fluorinated alkyl group, and is not particularly limited, and may be, for example, 1 to 3. The fluorinated alkyl group may be, for example, a perfluoroalkyl group (a monovalent group having a structure in which all hydrogen atoms in the alkyl group have been substituted with a fluorine atom) such as a trifluoromethyl group.

The fluorinated alkyl group in Z¹¹ and Z¹² preferably has 1 to 15 carbon atoms.

In the general formula (1), m₁₁ is the number of Z¹¹ bonded to one benzene ring backbone. m₁₂ is the number of Z¹² bonded to another benzene ring backbone different from the benzene ring backbone to which Z¹¹ is bonded. m₁₁ and m₁₂ each independently represents an integer of 0 to 4. That is, m₁₁ and m₁₂ may be the same as or different from each other.

The compound (1) has n₁ × m₁₁ Z¹¹ groups in one molecule, and when m₁₁ is an integer of 1 or more (i.e., an integer of 1 to 4), the n₁ × m₁₁ Z¹¹ groups may be the same or different from each other. That is, when m₁₁ is an integer of 1 or greater, the n₁ × m₁₁ Z¹¹ groups may all be the same, all be different, or may only partially be the same.

The same applies to Z¹², and the compound (1) has n₁ × m₁₂ Z¹² groups in one molecule, and when m₁₂ is an integer of 1 or greater (i.e., an integer of 1 to 4), the n₁ × m₁₂ Z¹² groups may be the same or different from each other. That is, when m₁₂ is an integer of 1 or greater, the n₁ × m₁₂ Z¹² groups may all be the same, all be different, or may only partially be the same.

In the general formula (1), Ar¹ is the group represented by the general formula (91) above, the group represented by the general formula (92) above, or the group represented by the general formula (93) above.

The bond attached with the sign * of these groups is formed to one of the oxygen atoms that is not bonded to a sulfur atom (S) in the general formula (1), and the bond attached with the sign ** is formed to the other oxygen atom that is not bonded to the sulfur atom in the general formula (1).

In the general formula (91), X¹¹ and X¹² each independently represents a group (substituent) other than a hydrogen atom. That is, the substituents X¹¹ and X¹² may be the same or different from each other.

Examples of X¹¹ and X¹² (substituents) include the same as Z¹¹ and Z¹² above (for example, an alkyl group, an alkylcarbonylamino group, a fluorinated alkyl group, a fluorine atom, and the like).

In the general formula (91), l₁₁ is the number of X¹¹ bonded to one benzene ring backbone. l₁₂ is the number of X¹² bonded to another benzene ring backbone different from the benzene ring backbone to which X¹¹ is bonded. l₁₁ and l₁₂ each independently represents an integer of 0 to 4. That is, l₁₁ and l₁₂ may be the same as or different from each other.

The compound (1) has n₁ × l₁₁ X¹¹ groups in one molecule, and when l₁₁ is an integer of 1 or more (i.e., an integer of 1 to 4), the n₁ × l₁₁ X¹¹ groups may be the same or different from each other. That is, when l₁₁ is an integer of 1 or more, the n₁ × l₁₁ X¹¹ groups may all be the same, all be different, or may only partially be the same.

The same applies to X¹², and the compound (1) has n₁ × l₁₂ X¹² groups in one molecule, and when l₁₂ is an integer of 1 or more (i.e., an integer of 1 to 4), the n₁ × l₁₂ X¹² groups may be the same or different from each other. That is, when l₁₂ is an integer of 1 or more, the n₁ × l₁₂ X¹² groups may all be the same, all be different, or may only partially be the same.

In the general formula (91), l₁₁ and l₁₂ may each independently be, for example, any one of 0 to 3, 0 to 2, 0 to 1, and 0.

A preferred example of the group represented by the general formula (91) is a group in which l₁₁ and l₁₂ are 0 (i.e., no X¹¹ or X¹² is present in the formula).

In the general formula (92) above, X²¹ is a group (substituent) other than a hydrogen atom.

Examples of X²¹ (substituent) include the same as Z¹¹ and Z¹² above (for example, an alkyl group, an alkylcarbonylamino group, a fluorinated alkyl group, a fluorine atom, and the like).

In formula (92), l₂₁ is the number of X²¹ bonded to one benzene ring backbone and is an integer of 0 to 4.

The compound (1) has n₁ × l₂₁ X²¹ groups in one molecule, and when l₂₁ is an integer of 1 or more (i.e., an integer of 1 to 4), the n₁ × l₂₁ X²¹ groups may be the same or different from each other. That is, when l₂₁ is an integer of 1 or more, the n₁ × l₂₁ X²¹ groups may all be the same, all be different, or may only partially be the same.

In general formula (92), l₂₁ may be, for example, any one of 0 to 3, 0 to 2, 0 or 1, and 0.

A preferred example of the group represented by the general formula (92) is a group in which l₂₁ is 0 (that is, X²¹ is not present in the formula.).

In the general formula (93), X³¹ and X³² each independently represents a group (substituent) other than a hydrogen atom. That is, the substituents X³¹ and X³² may be the same or different from each other.

Examples of X³¹ and X³² (substituents) include the same as Z¹¹ and Z¹² above (for example, an alkyl group, an alkylcarbonylamino group, a fluorinated alkyl group, a fluorine atom, and the like).

In the general formula (93), l₃₁ is the number of X³¹ bonded to one benzene ring backbone. l₃₂ is the number of X³² bonded to another benzene ring backbone different from the benzene ring backbone to which X³¹ is bonded. l₃₁ and l₃₂ each independently represents an integer of 0 to 4. That is, l₃₁ and l₃₂ may be the same as or different from each other.

The compound (1) has n₁ × l₃₁ X³¹ groups in one molecule, and when l₃₁ is an integer of 1 or more (i.e., an integer of 1 to 4), the n₁ × l₃₁ X³¹ groups may be the same or different from each other. That is, when l₃₁ is an integer of 1 or more, the n₁ × l₃₁ X³¹ groups may all be the same, all be different, or may only partially be the same.

The same applies to X³², and the compound (1) has n₁ × l₃₂ X³² groups in one molecule, and when l₃₂ is an integer of 1 or more (i.e., an integer of 1 to 4), the n₁ × l₃₂ X³² groups may be the same or different from each other. That is, when l₃₂ is an integer of 1 or more, the n₁ × l₃₂ X³² groups may all be the same, all be different, or may only partially be the same.

In the general formula (93), l₃₁ and l₃₂ may each independently be, for example, any one of 0 to 3, 0 to 2, 0 to 1, and 0.

A preferred example of the group represented by the general formula (93) is a group in which l₃₁ and l₃₂ are 0 (that is, no X³¹ or X³² is present in the formula).

A preferred example of the compound (1) includes a compound (1) in which m₁₁ and m₁₂ are 0 (i.e., no Z¹¹ and Z¹² are present in the following the formulae) regardless of whether Ar¹ is any one of the group represented by the general formula (91), the group represented by the general formula (92), and the group represented by the general formula (93).

A more preferred example of the compound (1) includes a compound (1) in which Ar¹ is the group represented by the general formula (91), l₁₁ and l₁₂ are 0, and m₁₁ and m₁₂ are 0.

Another more preferred example of the compound (1) includes a compound (1) in which Ar¹ is the group represented by the general formula (92), l₂₁ are 0, and m₁₁ and m₁₂ are 0.

Furthermore, the other more preferred example of the compound (1) includes a compound (1) in which Ar¹ is the group represented by the general formula (93), l₃₁ and l₃₂ are 0, and m₁₁ and m₁₂ are 0.

With the proviso that the compound (1) is not limited thereto.

The hydrogen atom in one or both (two) hydroxyl groups (-OHs, hydroxyl groups at one or both ends in the general formula (1)) in the compound (1) may be substituted with a group (M) other than a hydrogen atom to form a group represented by formula "-OM", as in the case of the compound (11) described later. Namely, the compound (1) may be a salt.

### <Compound (11)>

The compound (11) is one of products by the depolymerization method (1).

The compound (11) is bisphenol S (may be referred to as "BPS" as used herein) and its derivative.

Z¹¹, Z¹², m₁₁, and m₁₂ in the general formula (11) are the same as Z¹¹, Z¹², m₁₁, and m₁₂ in the general formula (1), respectively.

### <Compound (121)>

The compound (121) is the other product by the depolymerization method (1) when Ar¹ is represented by the general formula (91).

The compound (121) is bisphenol A (may be referred to as "BPA" as used herein) and its derivative.

X¹¹, X¹², l₁₁, and l₁₂ in the general formula (121) are each the same as X¹¹, X¹², l₁₁, and l₁₂ in the general formula (91).

### <Compound (122)>

The compound (122) is the other product by the depolymerization method (1) when Ar¹ is represented by the general formula (92).

The compound (122) is hydroquinone and its derivative.

X²¹ and l₂₁ in the general formula (122) are each the same as X²¹ and l₂₁ in the general formula (92).

### <Compound (123)>

The compound (123) is the other product by the depolymerization method (1) when Ar¹ is represented by the general formula (93).

The compound (123) is 4,4'-biphenol (alias: 4,4'-dihydroxybiphenyl) and its derivative.

X³¹, X³², l₃₁, and l₃₂ in the general formula (123) are each the same as X³¹, X³², l₃₁, and l₃₂ in the general formula (93).

### <Salt of compound (11), salt of compound (121), salt of compound (122), and salt of compound (123)>

The salt of compound (11) is a compound in which a hydrogen atom in one or both hydroxyl groups (-OHs) in the compound (11) are substituted with a group other than a hydrogen atom (hereinafter represented by the sign "M") to form a group represented by formula "-OM". The above-described formula "-OM" can also be represented as, for example, formula "-O⁻M⁺".

In a case in which hydrogen atoms in both hydroxyl groups in the compound (11) are substituted with the above-described M group, these two Ms may be the same or different.

The presence or absence of substitution of a hydrogen atom by the above-described M, and the type of M when substituted, are determined, for example, by the conditions for post-treatment of a reaction liquid after the depolymerization of compound (1). For example, when the reaction liquid is treated with an acid after the depolymerization of compound (1), the compound (11) is obtained as the main component, rather than a salt of compound (11).

An example of the above-described M includes a group derived from a hydroxide described later (group that can be a counter cation), and is preferably a metal atom (the salt of compound (11) is an alkoxide).

The manner whereby the compound (121), compound (122), and compound (123) form their salts is the same as the manner whereby the compound (11) forms a salt. That is, salts of compound (121), compound (122), and compound (123) are also formed in the same manner as the salt of compound (11), and are the same as the salt of compound (11) except that the group represented by formula "-OM" is bonded to a different group.

### <Hydroxide>

The hydroxide is not particularly limited as long as it is a compound having a hydroxide ion (OH⁻) (a compound that generates a hydroxide ion when dissolved in water), and may be either an organic hydroxide or an inorganic hydroxide.

Examples of the organic hydroxide include an ammonium hydroxide salt and a phosphonium hydroxide salt.

Examples of the ammonium hydroxide salts include tetraalkylammonium hydroxides such as tetramethylammonium hydroxide and tetrabutylammonium hydroxide.

An example of the phosphonium hydroxide salt includes a tetraalkylphosphonium hydroxide such as tetrabutylphosphonium hydroxide.

Examples of the inorganic hydroxide include alkali metal hydroxides such as lithium hydroxide, sodium hydroxide, potassium hydroxide, cesium hydroxide monohydrate, a hydrate other than cesium hydroxide monohydrate, and cesium hydroxide anhydride.

The above-mentioned hydrate other than cesium hydroxide monohydrate refers to cesium hydroxide hydrate having two or more water molecules in one molecule (for example, cesium hydroxide dihydrate).

The hydroxide to be used in the depolymerization step is preferably an alkali metal hydroxide.

In the depolymerization step, it is preferable to use a hydroxide that has been heated and dried under reduced pressure. Doing such a manner enhances an effect obtained by using the hydroxide, and facilitates the depolymerization of compound (1) to particularly proceed. This is presumably because the presence of water makes it difficult for the depolymerization of compound (1) to proceed, whereas the hydroxide can reduce the water content by heating and drying water under reduced pressure.

The hydroxide is preferably heated and dried under a pressure of, for example, 1333.22 Pa (10 mmHg) or less, more preferably 666.61 Pa (5 mmHg) or less, and still more preferably 399.966 Pa (3 mmHg) or less. The reduced pressure of the upper limit value or less further improves the degree of dryness of the hydroxide.

The hydroxide is, on the other hand, preferably heated and dried under a pressure of, for example, 133.322 Pa (1 mmHg) or more. Such a pressure under reduced pressure can be achieved more easily.

A drying temperature (heating temperature) when the hydroxide is dried by heating under reduced pressure is preferably 100°C or higher, more preferably 120°C or higher, and still more preferably 140°C or higher. By setting the drying temperature to be the lower limit value or more, the degree of dryness of the hydroxide is further improved.

The drying temperature is, on the other hand, preferably 180°C or lower. Setting the drying temperature in this manner inhibits excessive heating.

A drying time (heating time) when the hydroxide is dried by heating under reduced pressure can be appropriately adjusted according to a pressure upon reduced pressure and a drying temperature. For example, when both the pressure upon reduced pressure and the drying temperature fall within the numerical ranges described above, the drying time is preferably 2 to 8 hours. The drying time of the lower limit value or more further improves the degree of dryness of the hydroxide. The drying time of the upper limit value or less inhibits excessive heating.

In case of using a hydrate such as cesium hydroxide monohydrate as the hydroxide, the hydrate can be anhydrified by heating and drying under reduced pressure (for example, the hydrate is anhydrified to produce anhydrous cesium hydroxide), which can be used in the depolymerization step. Also, a hydrate that has not been anhydrified by heating and drying under reduced pressure (still in the hydrated state) can be used in the depolymerization step. For example, a hydrate other than the above-described cesium hydroxide monohydrate can be partially or totally converted to cesium hydroxide monohydrate by heating and drying under reduced pressure, and this cesium hydroxide monohydrate can also be used in the depolymerization step.

The hydroxide to be used in the depolymerization step may be used singly or two or more thereof can be used, and in the case of the two or more, the combination and ratio of the hydroxides may be arbitrarily selected depending on the purpose.

For example, when a hydrate such as cesium hydroxide monohydrate is heated and dried under reduced pressure as the hydroxide, a mixture of a hydroxide hydrate and a hydroxide anhydride may be obtained. In the depolymerization step, a mixture of such a hydroxide hydrate and anhydride may be used as the hydroxide. When a hydrate other than cesium hydroxide monohydrate is heated and dried under reduced pressure, a mixture of cesium hydroxide monohydrate and cesium hydroxide anhydride may be obtained. In the depolymerization step, such a mixture may be used as the hydroxide.

In the depolymerization step, the amount of hydroxide used is preferably 2 to 8 times the amount by mole of repeating unit in the compound (1) (the constituent unit attached with the sign n₁ in the general formula (1)), and may be, for example, any one of 3 to 6 times the amount by mole and 3.5 to 4.5 times the amount by mole. The amount of hydroxide used of the lower limit value or more facilitates the depolymerization of compound (1) to further proceed. The amount of hydroxide used that is the upper limit value or less inhibits the hydroxide from been excessively used.

### <Solvent>

In the depolymerization step, it is preferable to further use a solvent. The use of solvent particularly allows the compound (1) to be dissolved in the solvent to carry out the depolymerization step, facilitating the depolymerization of compound (1) to further proceed.

The term "solvent" as used herein encompasses both a component that is liquid at room temperature for dissolving a solute, and a component that is liquid at room temperature and functions as a dispersion medium for dispersing a dispersoid, unless otherwise specified. Also, the phrase "room temperature" means a temperature that is not particularly cooled or heated, that is, an ordinary temperature, and examples thereof include temperatures of 15 to 25°C.

The solvent is preferably an organic solvent.

Examples of the organic solvent include amides such as 1,3-dimethyl-2-imidazolidinone (DMI), N,N-dimethylacetamide (DMAc), N,N-dimethylformamide (DMF), and N-methyl-2-pyrrolidone (NMP); a nitrile such as benzonitrile; and an ether (cyclic ether) such as 1,4-dioxane.

The solvent may be used singly in the depolymerization step or two or more thereof may be used therein. In the case of the two or more, the combination and ratio of the solvents may be arbitrarily selected depending on the purpose.

When a solvent is used in the depolymerization step, the amount of the solvent used is preferably 0.2 to 1.5 L relative to 100 g of compound (1), and may be, for example, either of 0.2 to 1 L and 0.2 to 0.6 L, or either of 0.5 to 1.5 L or 0.7 to 1.5 L. The amount of solvent used that is the lower limit value or more facilitates the depolymerization of compound (1) to further proceed. The amount of solvent used being the upper limit value or less inhibits the solvent from being used excessively.

### <Dehydrating agent>

In the depolymerization step, it is preferable to further use a dehydrating agent. In the depolymerization step, the presence of water makes it difficult for the depolymerization of compound (1) to proceed. In other words, using the dehydrating agent in the depolymerization step, facilitates the depolymerization of compound (1) to further proceed, and increases the yield of the compound (11) or its salt and the yield of compound (121), compound (122), or compound (123) or its salt.

Examples of the dehydrating agent include a water-reactive component that reacts with water to form a different component, and a hygroscopic component that does not react with water but adsorbs water (in other words, absorbs moisture).

Examples of the water-reactive component include sodium hydride (NaH), potassium hydride (KH), calcium hydride (CaH₂), calcium oxide (CaO), cesium chloride (CsCl₂), calcium chloride (CaCl₂), and magnesium sulfate (MgSO₄).

An example of the hygroscopic component includes zeolite. The zeolite that is a commercially available product under the trade name of molecular sieve can be used.

The dehydrating agent to be used in the depolymerization step may be the single dehydrating agent or two or more thereof, and in the case of the two or more, the combination and ratio thereof can be arbitrarily selected according to the purpose.

The dehydrating agent to be used in the depolymerization step is preferably one or two or more selected from the group consisting of sodium hydride, potassium hydride, calcium hydride, calcium oxide, cesium chloride, calcium chloride, magnesium sulfate, and zeolite.

In the depolymerization step, when using a dehydrating agent, the amount of dehydrating agent used is preferably 2 to 8 times the amount by mole of repeating unit in the compound (1) (the constituent unit attached with the sign n₁ in the general formula (1)), and may be, for example, any one of 4 to 6 times the amount by mole and 3.5 to 4.5 times the amount by mole. The amount of dehydrating agent used, which is the lower limit value or more facilitates the depolymerization of compound (1) to further proceed. The amount of dehydrating agent used, which is the upper limit value or less inhibits the dehydrating agent from being excessively used.

### <Other component>

In the depolymerization step, the compound (1) may be depolymerized using other components that do not fall under any of the compound (1), a hydroxide, a solvent, if necessary, and a dehydrating agent, if necessary, as long as the effects of the present invention are not impaired, or the compound (1) may be depolymerized without using the other components.

The other components can be arbitrarily selected depending on the purpose, and are not particularly limited.

The other components to be used in the depolymerization step may be the single component or two or more thereof, and in the case of the two or more, the combination and ratio thereof can be arbitrarily selected depending on the purpose.

In depolymerizing the compound (1) in the depolymerization step, the proportion of the total amount used (parts by mass) of a hydroxide, a solvent, and a dehydrating agent to the total amount used (parts by mass) of the hydroxide, solvent, dehydrating agent, and the other components (([amount (parts by mass) of hydroxide used] + [amount (parts by mass) of solvent used]) + [amount (parts by mass) of dehydrating agent used]/([amount (parts by mass) of hydroxide used] + [amount (parts by mass) of solvent used] + [amount (parts by mass) of dehydrating agent used] + [amount (parts by mass) of other components used]) × 100) is preferably 80% by mass or more, more preferably 90% by mass or more, and still more preferably 95% by mass or more, and may be, for example, any one of 97% by mass or more or 99% by mass or more. The proportion of the lower limit value or more facilitates the depolymerization of compound (1) to further proceed.

The proportion is, on the other hand, 100% by mass or less.

When no optional components (i.e., a solvent, a dehydrating agent or the other components) are not used upon the depolymerization of compound (1), the amount of optional component used when calculating the proportion is 0 parts by mass.

### <Other conditions>

In the depolymerization step, the compound (1) can be depolymerized by mixing the compound (1), a hydroxide, a solvent, if necessary, a dehydrating agent, if necessary, and the other components, if necessary, and heating and stirring the resulting mixture.

In the depolymerization step, a temperature (reaction temperature) at which the mixture is heated and stirred is preferably 100°C or higher, and more preferably 115°C or higher, and may be, for example, either of 130°C or higher and 145°C or higher. The reaction temperature, which is the lower limit vale or higher facilitates the depolymerization of compound (1) to further proceed.

On the other hand, the reaction temperature is, in terms of inhibiting a byproduct from being formed as impurities, preferably 170°C or lower.

In the depolymerization step, a time (reaction time) for heating and stirring the mixture is preferably 10 to 40 hours, and may be either of 10 to 24 hours or 24 to 40 hours.

The above-described reaction time is particularly suitable when the reaction temperature is within the above range.

In the depolymerization step, the depolymerization of compound (1) may be carried out in an atmosphere of inert gas such as argon gas, helium gas, or nitrogen gas, or in an air atmosphere. Doing such a manner enables the amount of water in the mixture to be reduced, and the same effect as when a dehydrating agent is used can be obtained. That is, the depolymerization of compound (1) is facilitated to further proceed, and the yield of the compound (11) or its salt, and the yield of the compound (121), compound (122), or compound (123) or its salt increase.

In the depolymerization step, it is more preferable to carry out the depolymerization of compound (1) using a dehydrating agent in the inert gas atmosphere, it is more preferable to carry out the depolymerization of compound (1) using a hydroxide that has been dried by heating under reduced pressure in the inert gas atmosphere, and it is still more preferable to carry out the depolymerization of compound (1) using a dehydrating agent in the inert gas atmosphere and a hydroxide that has been dried by heating under reduced pressure. Doing such a manner further increases the yield of the objective substances (the compound (11) or its salt and the compound (121), compound (122), or compound (123) or its salt).

In the depolymerization method (1), after completion of the depolymerization step, the obtained reaction mixture may be post-treated, if necessary by a publicly known method to be able to take out the target substance (product). In other words, post-treatment operations such as filtration, cleaning, extraction, pH adjustment, dehydration, and concentration may be appropriately carried out, if necessary, in a single manner among them or in combination of two or more thereof, and the target substance can be taken out by concentration, crystallization, reprecipitation, column chromatography, or the like. Also, the taken out target substance may be further purified, if necessary, by carrying out one or more times any one of operations such as crystallization, reprecipitation, column chromatography, extraction, and stirring and washing of crystals with a solvent, in a single manner among these operations or in a combined manner of two or more thereof. Alternatively, after completion of the depolymerization step, the resulting reaction mixture may be subjected to post-treatment and then used for the next intended application without taking out the target substance. For example, the target substance may be subjected to the next intended reaction without being taken out.

The structure of the product obtained by the depolymerization method (1) can be confirmed by publicly known techniques, such as nuclear magnetic resonance (NMR) spectroscopy, mass spectrometry (MS), and infrared spectroscopy (IR).

### <<Depolymerization method (2)>>

The method for depolymerizing a compound according to one embodiment of the present invention comprises the depolymerization step (decomposition step) of depolymerizing a compound represented by the following general formula (2) (may be referred to as "compound (2)" as used herein): 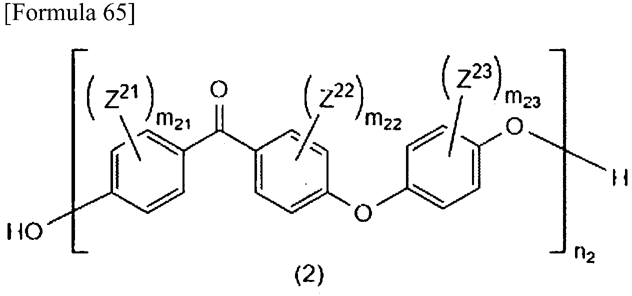
(wherein n₂ is an integer of 2 or greater (preferably 10 to 200); Z²¹, Z²², and Z²³ are each independently a group other than a hydrogen atom; m₂₁, m₂₂, and m₂₃ are each independently an integer of 0 to 4, and when m₂₁ is an integer of 1 or greater, n₂ × m₂₁ Z²¹ groups may be the same or different from each other, when m₂₂ is an integer of 1 or greater, n₂ × m₂₂ Z²² groups may be the same or different from each other, and when m₂₃ is an integer of 1 or greater, n₂ × m₂₃ Z²³ groups may be the same or different from each other.)
   in the co-presence of a hydroxide to obtain a compound represented by the following general formula (21) (may be referred to "compound (21)" as used herein): 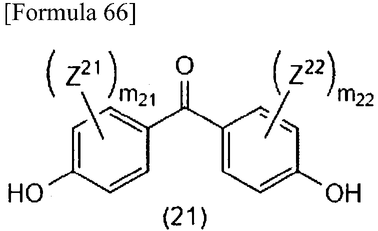
(wherein Z²¹, Z²², m₂₁, and m₂₂ are the same as described above.) or it derivative and a compound represented by the following general formula (22) (may be referred to "compound (22)" as used herein): 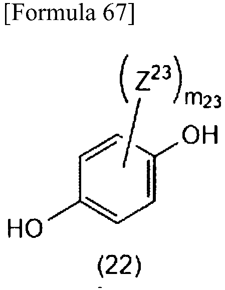
(wherein Z²³ and m₂₃ are the same as described above.) or it derivative. As used herein, the method for depolymerizing a compound of the present embodiment may be referred to as "depolymerization method (2)".

The depolymerization method of the present embodiment (the depolymerization method (2)) is a novel method for depolymerizing the compound (2), which includes super engineering plastics.

The depolymerization method (2) is the same as the depolymerization method (1), except that the compound (2) is used instead of the compound (1), that is, an object of depolymerization is different.

The compound (2) includes polyether ether ketone (may be referred to as "PEEK" as used herein) and its derivative.

### <Compound (2)>

The compound (2) is an object of depolymerization in the depolymerization method (2).

In the general formula (2), n₂ is the number of repeating units, which defines the molecular size of the compound (2), and is an integer of 2 or greater.

The compound (2) in which n₂ is, for example, 20 to 100 in the formula, is suitable as polyether ether ketone (PEEK), a high molecular weight super engineering plastic, which is difficult to be depolymerized using conventional methods and is particularly suitable as an applicable object of the depolymerization method (2).

In the general formula (2), Z²¹, Z²², and Z²³ are each independently a group (substituent) other than a hydrogen atom. In other words, the substituents Z²¹, Z²², and Z²³ may all be the same, all be different, or may only partially (any two among them) be the same.

Examples of Z²¹, Z²², and Z²³ (substituents) include the same as Z¹¹ and Z¹² above (for example, an alkyl group, an alkylcarbonylamino group, a fluorinated alkyl group, a fluorine atom, and the like).

In the general formula (2), m₂₁ is the number of Z²¹ bonded to one benzene ring backbone. m₂₂ is the number of Z²² bonded to another benzene ring backbone different from the benzene ring backbone to which Z²¹ is bonded. m₂₃ is the number of Z²³ bonded to another benzene ring backbone different from both the benzene ring backbone to which Z²¹ is bonded and the benzene ring backbone to which Z²² is bonded.

m₂₁, m₂₂, and m₂₃ are each independently an integer of 0 to 4. That is, m₂₁, m₂₂, and m₂₃ may all be the same, all be different, or may only partially (any two among them) be the same.

The compound (2) has n₂ × m₂₁ Z²¹ groups in one molecule, and when m₂₁ is an integer of 1 or more (i.e., an integer of 1 to 4), the n₂ × m₂₁ Z²¹ groups may be the same or different from each other. That is, when m₂₁ is an integer of 1 or greater, the n₂ × m₂₁ Z²¹ groups may all be the same, all be different, or may only partially be the same.

The same applies to Z²², and the compound (2) has n₂ × m₂₂ Z²² groups in one molecule, and when m₂₂ is an integer of 1 or greater (i.e., an integer of 1 to 4), the n₂ × m₂₂ Z²² groups may be the same or different from each other. That is, when m₂₂ is an integer of 1 or greater, the n₂ × m₂₂ Z²² groups may all be the same, all be different, or may only partially be the same.

The same applies to Z²³, and the compound (2) has n₂ × m₂₃ Z²³ groups in one molecule, and when m₂₃ is an integer of 1 or greater (i.e., an integer of 1 to 4), the n₂ × m₂₃ Z²³ groups may be the same or different from each other. That is, when m₂₃ is an integer of 1 or greater, the n₂ × m₂₃ Z²³ groups may all be the same, all be different, or may only partially be the same.

A preferred example of the compound (2) includes a compound (2) in which m₂₁, m₂₂, and m₂₃ are 0 (i.e., Z²¹, Z²², and *Z²³* are not present in the formula.).

With the proviso that the compound (2) is not limited thereto.

The hydrogen atom in one or both hydroxyl groups (-OHs, hydroxyl groups at one or both ends in the general formula (2)) in the compound (2) may be substituted with a group (M) other than a hydrogen atom to form a group represented by formula "-OM", as in the case of the compound (21) described later. Namely, the compound (2) may be a salt.

### <Compound (21)>

The compound (21) is one of products obtained by the depolymerization method (2).

The compound (21) is 4,4'-dihydroxybenzophenone and its derivative.

Z²¹, Z²², m₂₁, and m₂₂ in the general formula (21) are the same as Z²¹, Z²², m₂₁, and m₂₂ in the general formula (2), respectively.

### <Compound (22)>

The compound (22) is the other product obtained by the depolymerization method (2).

The compound (22) is 1,3-dihydroxybenzene (alias: resorcinol) and its derivative.

Z²³ and m₂₃ in the general formula (22) are the same as Z²³ and m₂₃ in the general formula (2), respectively.

### <Salt of compound (21) and salt of compound (22)>

As is the case with the above-described compound (11), the salt of compound (21) is a compound in which a hydrogen atom in one or both hydroxyl groups (-OHs) in the compound (21) is substituted with a group (M) other than a hydrogen atom to form a group represented by formula "-OM" (formula "-O⁻M⁺").

In a case in which the hydrogen atoms in both hydroxyl groups in the compound (21) are substituted with the above-described M, these two Ms may be the same or different.

The presence or absence of substitution of a hydrogen atom by the above-described M, and the type of M when substituted, are determined, for example, by the conditions for post-treatment of a reaction liquid after the depolymerization of compound (2). For example, when the reaction liquid is treated with an acid after the depolymerization of compound (2), the compound (21) is obtained as the main component, rather than a salt of compound (21).

The above-described M is the same as in the case of the salt of compound (11) above.

The manner whereby the compound (22) forms a salt is the same as the manner whereby the compound (21) forms a salt. That is, the salt of compound (22) is also formed in the same manner as the salt of compound (21), and is the same as the salt of compound (21) except that the group represented by formula "-OM" is bonded to a different group.

### <Hydroxide>

The hydroxide in the depolymerization method (2) is the same as the hydroxide in the depolymerization method (1).

The hydroxide to be used in the depolymerization step of the depolymerization method (2) may be the single hydroxide or two or more thereof, and in the case of the two or more, the combination and ratio thereof can be arbitrarily selected according to the purpose.

The hydroxide to be used in the depolymerization step of the depolymerization method (2) is preferably an alkali metal hydroxide.

In the depolymerization step of the depolymerization method (2), it is preferable to use a hydroxide that has been heated and dried under reduced pressure. Doing such a manner enhances an effect obtained by using the hydroxide, and facilitates the depolymerization of compound (2) to particularly proceed.

The hydroxide that has been heated and dried under reduced pressure in the depolymerization method (2) is the same as the hydroxide that has been heated and dried under reduced pressure in the depolymerization method (1), and is obtained by the same method as in the case of the depolymerization method (1).

In the depolymerization step of the depolymerization method (2), the amount of hydroxide used is preferably 2 to 8 times the amount by mole of repeating unit in the compound (2) (the constituent unit attached with the sign n₂ in the general formula (2)), and may be, for example, any one of 3 to 6 times the amount by mole and 3.5 to 4.5 times the amount by mole. The amount of hydroxide used of the lower limit value or more facilitates the depolymerization of compound (2) to further proceed. The amount of hydroxide used that is the upper limit value or less inhibits the hydroxide from been excessively used.

### <Solvent>

In the depolymerization step of the depolymerization method (2), it is preferable to further use a solvent. The use of solvent particularly allows the compound (2) to be dissolved in the solvent to carry out the depolymerization step, facilitating the depolymerization of compound (2) to further proceed.

The solvent in the depolymerization method (2) is the same as the solvent in the depolymerization method (1).

The solvent in the depolymerization step of the depolymerization method (2) may be used singly or two or more thereof may be used therein, and in the case of two or more thereof, the combination and ratio of the solvents may be arbitrarily selected depending on the purpose.

In case of using the solvent in the depolymerization step of the depolymerization method (2), the amount of solvent used is preferably 0.2 to 1.5 L relative to 100 g of compound (2), and may be, for example, any of 0.2 to 1 L and 0.2 to 0.6 L, or any of 0.5 to 1.5 L and 0.7 to 1.5 L. The amount of solvent used that is the lower limit value or more facilitates the depolymerization of compound (2) to further proceed. The amount of solvent used being the upper limit value or less inhibits the solvent from being used excessively.

### <Dehydrating agent>

In the depolymerization step of the depolymerization method (2), it is preferable to further use a dehydrating agent. In the depolymerization step, the presence of water makes it difficult for the depolymerization of compound (2) to proceed. In other words, using the dehydrating agent in the depolymerization step, facilitates the depolymerization of compound (2) to further proceed, and increases the yield of the compound (21) or its salt and the yield of the compound (22) or its salt.

The dehydrating agent in the depolymerization method (2) is the same as the dehydrating agent in the depolymerization method (1).

The dehydrating agent in the depolymerization step of the depolymerization method (2) may be used singly, two or more thereof may be used, and in the case of the two or more, the combination and ratio thereof can be arbitrarily selected according to the purpose.

The dehydrating agent to be used in the depolymerization step of the depolymerization method (2) is preferably one or more selected from the group consisting of sodium hydride, potassium hydride, calcium hydride, calcium oxide, cesium chloride, calcium chloride, magnesium sulfate, and zeolite.

In the depolymerization step of the depolymerization method (2), when using the dehydrating agent, the amount of the dehydrating agent used is preferably 2 to 8 times the amount by mole of repeating unit in the compound (2) (the constituent unit attached with the sign n₂ in the general formula (2)), and may be, for example, either of 4 to 6 times the amount by mole and 3.5 to 4.5 times the amount by mole. The amount of dehydrating agent used, which is the lower limit value or more facilitates the depolymerization of compound (2) to further proceed. The amount of dehydrating agent used, which is the upper limit value or less inhibits the dehydrating agent from being excessively used.

### <Other component>

In the depolymerization step of the depolymerization method (2), the compound (2) may be depolymerized using other components that do not fall under any of the compound (2), a hydroxide, a solvent, if necessary, and a dehydrating agent, if necessary, as long as the effects of the present invention are not impaired, or the compound (2) may be depolymerized without using the other components.

The other components in the depolymerization method (2) are the same as the other components in the depolymerization method (1).

The other components in the depolymerization step of the depolymerization method (2) may be used singly, or two or more thereof may be used in combination thereof, and in the case of the two or more, the combination and ratio thereof can be selected arbitrarily depending on the purpose.

In depolymerizing the compound (2) in the depolymerization step of the depolymerization method (2), the proportion of the total amount used (parts by mass) of a hydroxide, a solvent, and a dehydrating agent to the total amount used (parts by mass) of the hydroxide, solvent, dehydrating agent, and the other components (([amount (parts by mass) of hydroxide used] + [amount (parts by mass) of solvent used]) + [amount (parts by mass) of dehydrating agent used]/([amount (parts by mass) of hydroxide used] + [amount (parts by mass) of solvent used] + [amount (parts by mass) of dehydrating agent used] + [amount (parts by mass) of other components used]) × 100) is preferably 80% by mass or more, more preferably 90% by mass or more, and still more preferably 95% by mass or more, and may be, for example, any one of 97% by mass or more or 99% by mass or more. The proportion of the lower limit value or more facilitates the depolymerization of compound (2) to further proceed.

The proportion is, on the other hand, 100% by mass or less.

When no optional components (i.e., a solvent, a dehydrating agent or the other components) are not used upon the depolymerization of compound (2), the amount of optional component used when calculating the proportion is 0 parts by mass.

### <Other conditions>

In the depolymerization step of the depolymerization method (2), the compound (2) can be depolymerized by mixing the compound (2), a hydroxide, a solvent, if necessary, a dehydrating agent, if necessary, and the other components, if necessary, and heating and stirring the resulting mixture.

In the depolymerization step of the depolymerization method (2), a temperature (reaction temperature) when the mixture is heated and stirred, a time (reaction time) when the mixture is heated and stirred, and an atmosphere upon the depolymerization of compound (2) are each the same as the temperature (reaction temperature) when the mixture is heated and stirred, the time (reaction time) when the mixture is heated and stirred, and the atmosphere upon the depolymerization of compound (1) in the depolymerization step of the depolymerization method (1).

In the depolymerization step of the depolymerization method (2), it is more preferable to carry out the depolymerization of compound (2) using a dehydrating agent in the inert gas atmosphere, it is more preferable to carry out the depolymerization of compound (2) using a hydroxide that has been dried by heating under reduced pressure, in the inert gas atmosphere, and it is still more preferable to carry out the depolymerization of compound (2) using a dehydrating agent in the inert gas atmosphere and a hydroxide that has been dried by heating under reduced pressure. Doing such a manner further increases the yields of the target substances (the compound (21) or its salt and the compound (22) or its salt).

In the depolymerization method (2), after completion of the depolymerization step, the obtained reaction mixture may be post-treated, if necessary in the same manner as in the depolymerization method (1) to be able to take out a target substance (product), and the obtained target substance may be further purified, if necessary. Alternatively, after completion of the depolymerization step, the resulting reaction mixture may be subjected to post-treatment by the same method as in the depolymerization method (1) and then used for the next intended application without taking out the target substance.

The structure of the product obtained by the depolymerization method (2) can be confirmed by publicly known techniques, such as nuclear magnetic resonance (NMR) spectroscopy, mass spectrometry (MS), and infrared spectroscopy (IR).

### <<Depolymerization method (4)>>

The method for depolymerizing a compound according to one embodiment of the present invention comprises the depolymerization step (decomposition step) of depolymerizing a compound represented by the following general formula (4) (may be referred to as "compound (4)" as used herein): 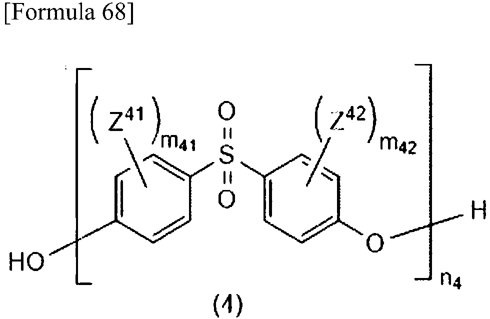
(wherein n₄ is an integer of 2 or greater (preferably 10 to 200); Z⁴¹ and Z⁴² are each independently a group other than a hydrogen atom; m₄₁ and m₄₂ are each independently an integer of 0 to 4, and when m₄₁ is an integer of 1 or greater, n₄ × m₄₁ Z⁴¹ groups may be the same or different from each other, and when m₄₂ is an integer of 1 or greater, n₄ × m₄₂ Z⁴² may be the same or different from each other.)
   in the co-presence of a hydroxide to obtain a compound represented by the following general formula (41) (may be referred to "compound (41)" as used herein): 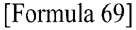 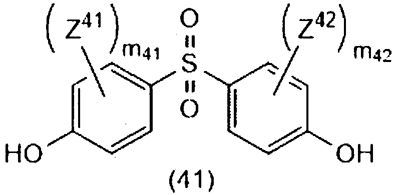
(wherein Z⁴¹, Z⁴², m₄₁, and m₄₂ are the same as described above.)
   or its derivative. As used herein, the method for depolymerizing a compound of the present embodiment may be referred to as "depolymerization method (4)".

The depolymerization method of the present embodiment (the depolymerization method (4)) is a novel method for depolymerizing the compound (4), which includes super engineering plastics.

The depolymerization method (4) is the same as the depolymerization method (1), except that the compound (4) is used instead of the compound (1), that is, an object of depolymerization is different.

Examples of the compound (4) include polyether sulfone (may be referred to as "PESU" as used herein) and its derivative.

### <Compound (4)>

The compound (4) is an object of depolymerization in the depolymerization method (4).

In the general formula (4), n₄ is the number of repeating units, which defines the molecular size of the compound (4), and is an integer of 2 or greater.

The compound (4) in which n₄ is, for example, 30 to 350 in the formula, is suitable as polyether sulfone (PESU), a high molecular weight super engineering plastic, which is difficult to be depolymerized using conventional methods and is particularly suitable as an applicable object of the depolymerization method (4).

In the general formula (4), Z⁴¹ and Z⁴² are each independently a group (substituent) other than a hydrogen atom. That is, the substituents Z⁴¹ and Z⁴² may be the same as each other or different from each other.

Examples of Z⁴¹ and Z⁴² (substituents) include the same as Z¹¹ and Z¹² above (for example, an alkyl group, an alkylcarbonylamino group, a fluorinated alkyl group, a fluorine atom, and the like.).

In the general formula (4), m₄₁ is the number of Z⁴¹ bonded to one benzene ring backbone. m₄₂ is the number of Z⁴² bonded to another benzene ring backbone different from the benzene ring backbone to which Z⁴¹ is bonded.

m₄₁ and m₄₂ each independently represents an integer of 0 to 4. That is, m₄₁ and m₄₂ may be the same as or different from each other.

The compound (4) has n₄ × m₄₁ Z⁴¹ groups in one molecule, and when m₄₁ is an integer of 1 or more (i.e., an integer of 1 to 4), the n₄ × m₄₁ Z⁴¹ groups may be the same or different from each other. That is, when m₄₁ is an integer of 1 or greater, the n₄ × m₄₁ Z⁴¹ groups may all be the same, all be different, or may only partially be the same.

The same applies to Z⁴², and the compound (4) has n₄ × m₄₂ Z⁴² groups in one molecule, and when m₄₂ is an integer of 1 or greater (i.e., an integer of 1 to 4), the n₄ × m₄₂ Z⁴² groups may be the same or different from each other. That is, when m₄₂ is an integer of 1 or greater, the n₄ × m₄₂ Z⁴² groups may all be the same, all be different, or may only partially be the same.

A preferred example of the compound (4) includes a compound (4) in which m₄₁ and m₄₂ are 0 (i.e., no Z⁴¹ and Z⁴² is present in the formula.).

With the proviso that the compound (4) is not limited thereto.

The hydrogen atom in one or both hydroxyl groups (-OHs, hydroxyl groups at one or both ends in the general formula (4)) in the compound (4) may be substituted with a group (M) other than a hydrogen atom to form a group represented by formula "-OM", as in the case of the compound (41) described later. Namely, the compound (4) may be a salt.

### <Compound (41)>

The compound (41) is a product by the depolymerization method (4).

The compound (41) is bisphenol S (BPS) and its derivative.

Z⁴¹, Z⁴², m₄₁, and m₄₂ in the general formula (41) are the same as Z⁴¹, Z⁴², m₄₁, and m₄₂ in the general formula (4), respectively.

### <Salt of compound (41)>

As is the case with the above-described compound (11), the salt of compound (41) is a compound in which a hydrogen atom in one or both hydroxyl groups (-OHs) in the compound (41) is substituted with a group (M) other than a hydrogen atom to form a group represented by formula "-OM" (formula "-O⁻M⁺").

In a case in which the hydrogen atoms in both hydroxyl groups in the compound (41) are substituted with the above-described M, these two Ms may be the same or different.

The presence or absence of substitution of a hydrogen atom by the above-described M, and the type of M when substituted, are determined, for example, by the conditions for post-treatment of a reaction liquid after the depolymerization of compound (4). For example, when the reaction liquid is treated with an acid after the depolymerization of compound (4), the compound (41) is obtained as the main component, rather than a salt of compound (41).

The above-described M is the same as in the case of the salt of compound (41) above.

### <Hydroxide>

The hydroxide in the depolymerization method (4) is the same as the hydroxide in the depolymerization method (1).

The hydroxide to be used in the depolymerization step of the depolymerization method (4) may be the single hydroxide or two or more thereof, and in the case of the two or more, the combination and ratio thereof can be arbitrarily selected according to the purpose.

The hydroxide to be used in the depolymerization step of the depolymerization method (4) is preferably an alkali metal hydroxide.

In the depolymerization step of the depolymerization method (4), it is preferable to use a hydroxide that has been heated and dried under reduced pressure. Doing such a manner enhances an effect obtained by using the hydroxide, and facilitates the depolymerization of compound (4) to particularly proceed.

The hydroxide that has been heated and dried under reduced pressure in the depolymerization method (4) is the same as the hydroxide that has been heated and dried under reduced pressure in the depolymerization method (1), and is obtained by the same method as in the case of the depolymerization method (1).

In the depolymerization step of depolymerization method (4), the amount of hydroxide used is preferably 2 to 8 times the amount by mole of repeating unit in the compound (4) (the constituent unit attached with the sign n₄ in the general formula (4)), and may be, for example, either 2 to 6 times the amount by mole or 2 to 4.5 times the amount by mole. The amount of hydroxide used of the lower limit value or more facilitates the depolymerization of compound (4) to further proceed. The amount of hydroxide used that is the upper limit value or less inhibits the hydroxide from been excessively used.

### <Solvent>

In the depolymerization step of the depolymerization method (4), it is preferable to further use a solvent. The use of solvent particularly allows the compound (4) to be dissolved in the solvent to carry out the depolymerization step, facilitating the depolymerization of compound (4) to further proceed.

The solvent in the depolymerization method (4) is the same as the solvent in the depolymerization method (1).

The solvent in the depolymerization step of the depolymerization method (4) may be used singly in the depolymerization step or two or more thereof may be used therein, and in the case of two or more thereof, the combination and ratio of the solvents may be arbitrarily selected depending on the purpose.

In case of using the solvent in the depolymerization step of the depolymerization method (4), the amount of solvent used is preferably 0.2 to 1.5 L relative to 100 g of compound (4), and may be, for example, any of 0.2 to 1 L and 0.2 to 0.6 L, or any of 0.5 to 1.5 L and 0.7 to 1.5 L. The amount of solvent used that is the lower limit value or more facilitates the depolymerization of compound (4) to further proceed. The amount of solvent used being the upper limit value or less inhibits the solvent from being used excessively.

### <Dehydrating agent>

In the depolymerization step of the depolymerization method (4), it is preferable to further use a dehydrating agent. In the depolymerization step, the presence of water makes it difficult for the depolymerization of compound (4) to proceed. In other words, using the dehydrating agent in the depolymerization step, facilitates the depolymerization of compound (4) to further proceed, and further increases the yield of the compound (41).

The dehydrating agent in the depolymerization method (4) is the same as the dehydrating agent in the depolymerization method (1).

The dehydrating agent in the depolymerization step of the depolymerization method (4) may be used singly, two or more thereof may be used, and in the case of the two or more, the combination and ratio thereof can be arbitrarily selected according to the purpose.

The dehydrating agent to be used in the depolymerization step of the depolymerization method (4) is preferably one or two or more selected from the group consisting of sodium hydride, potassium hydride, calcium hydride, calcium oxide, cesium chloride, calcium chloride, magnesium sulfate, and zeolite.

In the depolymerization step of depolymerization method (4), when using the dehydrating agent, the amount of the dehydrating agent used is preferably 2 to 8 times the amount by mole of repeating unit in the compound (4) (the constituent unit attached with the sign n₄ in the general formula (4)), and may be, for example, either of 2 to 6 times the amount by mole and 2 to 4.5 times the amount by mole. The amount of dehydrating agent used, which is the lower limit value or more facilitates the depolymerization of compound (4) to further proceed. The amount of dehydrating agent used, which is the upper limit value or less inhibits the dehydrating agent from being excessively used.

### <Other component>

In the depolymerization step of the depolymerization method (4), the compound (4) may be depolymerized using other components that do not fall under any of the compound (4), a hydroxide, a solvent, if necessary, and a dehydrating agent, if necessary, as long as the effects of the present invention are not impaired, or the compound (4) may be depolymerized without using the other components.

The other components in the depolymerization method (4) are the same as the other components in the depolymerization method (1).

The other components in the depolymerization step of the depolymerization method (4) may be used singly, or two or more thereof may be used in combination thereof, and in the case of the two or more, the combination and ratio thereof can be selected arbitrarily depending on the purpose.

In depolymerizing the compound (4) in the depolymerization step of the depolymerization method (4), the proportion of the total amount used (parts by mass) of a hydroxide, a solvent, and a dehydrating agent to the total amount used (parts by mass) of the hydroxide, solvent, dehydrating agent, and the other components (([amount (parts by mass) of hydroxide used] + [amount (parts by mass) of solvent used]) + [amount (parts by mass) of dehydrating agent used]/([amount (parts by mass) of hydroxide used] + [amount (parts by mass) of solvent used] + [amount (parts by mass) of dehydrating agent used] + [amount (parts by mass) of other components used]) × 100) is preferably 80% by mass or more, more preferably 90% by mass or more, and still more preferably 95% by mass or more, and may be, for example, any one of 97% by mass or more or 99% by mass or more. The proportion of the lower limit value or more facilitates the depolymerization of compound (4) to further proceed.

The proportion is, on the other hand, 100% by mass or less.

When no optional components (i.e., a solvent, a dehydrating agent or the other components) are not used upon the depolymerization of compound (4), the amount of optional component used when calculating the proportion is 0 parts by mass.

### <Other conditions>

In the depolymerization step of the depolymerization method (4), the compound (4) can be depolymerized by mixing the compound (4), a hydroxide, a solvent, if necessary, a dehydrating agent, if necessary, and the other components, if necessary, and heating and stirring the resulting mixture.

In the depolymerization step of the depolymerization method (4), a temperature (reaction temperature) when the mixture is heated and stirred, a time (reaction time) when the mixture is heated and stirred, and an atmosphere upon the depolymerization of compound (4) are each the same as the temperature (reaction temperature) when the mixture is heated and stirred, the time (reaction time) when the mixture is heated and stirred, and the atmosphere upon the depolymerization of compound (1) in the depolymerization step of the depolymerization method (1).

In the depolymerization step of the depolymerization method (4), it is more preferable to carry out the depolymerization of compound (4) using a dehydrating agent in the inert gas atmosphere, it is more preferable to carry out the depolymerization of compound (4) using a hydroxide that has been dried by heating under reduced pressure, in the inert gas atmosphere, and it is still more preferable to carry out the depolymerization of compound (4) using a dehydrating agent in the inert gas atmosphere and a hydroxide that has been dried by heating under reduced pressure. Doing such a manner further increases the yield of the target substance (compound (41)).

In the depolymerization method (4), after completion of the depolymerization step, the obtained reaction mixture may be post-treated, if necessary in the same manner as in the depolymerization method (1) to be able to take out a target substance (product), and the obtained target substance may be further purified, if necessary. Alternatively, after completion of the depolymerization step, the resulting reaction mixture may be subjected to post-treatment by the same method as in the depolymerization method (1) and then used for the next intended application without taking out the target substance.

The structure of the product obtained by the depolymerization method (4) can be confirmed by publicly known techniques, such as nuclear magnetic resonance (NMR) spectroscopy, mass spectrometry (MS), and infrared spectroscopy (IR).

### ⊚ Method for producing ether compound

The method for producing an ether compound according to one embodiment of the present invention includes the step (may be referred to as "etherification step" as used herein) of etherifying one or more phenolic hydroxyl groups or one or more groups in which the phenolic hydroxyl group has formed a salt, in one or two or more selected from the group consisting of the compound represented by the general formula (11) (compound (11)) and its salt, the compound represented by the general formula (121) (compound (121)) and its salt, the compound represented by the general formula (122) (compound (122)) and its salt, and the compound represented by the general formula (123) (compound (123)) and its salt,
one or two or more selected from the group consisting of the compound represented by the general formula (21) (compound (21)) and its salt, and the compound represented by the general formula (22) (compound (22)) and its salt,
or one or two or more selected from the group consisting of the compound represented by the general formula (41) (compound (41)) and its salt, which have been obtained by the aforementioned methods for depolymerizing a compound according to one embodiment of the present invention,
to obtain an ether compound.

As used herein, the compound (11) and the like obtained by the depolymerization method may be collectively referred to as "monomer".

According to the method of production of the present invention, the monomer that is the product obtained by the depolymerization method (depolymerization method (1), depolymerization method (2), or depolymerization method (4)), can be easily etherified at their hydroxyl groups or groups in which the hydroxyl group forms a salt. In particular, the monomer can be etherified without being taken out, which is because the depolymerization of compound (1), compound (2), or compound (4) in the depolymerization method proceeds as expected with side reactions being inhibited.

Among the monomers, the salt of compound (11), the salt of compound (121), the salt of compound (122), the salt of compound (123), the salt of compound (21), the salt of compound (22), and the salt of compound (41) are the target substances in the depolymerization method (1), (2), or (4) described above.

The ether compound has a structure in which one or two hydrogen atoms (Hs) in a phenolic hydroxyl group (-OH) contained in the monomer, or the M in the group represented by formula "-OM" above, have been substituted with a monovalent hydrocarbon group. The group represented by formula "-OM" above is a group in which the phenolic hydroxyl group forms a salt.

An example of the ether compound obtained from the compound (11) or its salt includes a compound represented by the following general formula (110).

An example of the ether compound obtained from the compound (121) or its salt includes a compound represented by the following general formula (1210).

An example of the ether compound obtained from the compound (122) or its salt includes a compound represented by the following general formula (1220).

An example of the ether compound obtained from the compound (123) or its salt includes a compound represented by the following general formula (1230). 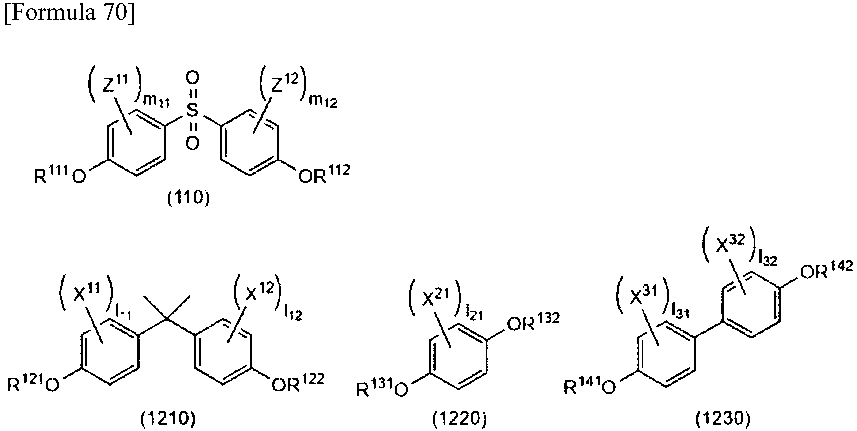 (wherein Z¹¹, Z¹², X¹¹, X¹², X²¹, X³¹, X³², m₁₁, m₁₂, l₁₁, l₁₂, l₂₁, l₃₁ and l₃₂ are the same as above; R¹¹¹ and R¹¹² are each independently a hydrogen atom or a hydrocarbon group, provided that R¹¹¹ and R¹¹² are not both hydrogen atoms, and when R¹¹¹ or R¹¹² is a hydrogen atom, the group represented by formula "-OR¹¹¹" or the group represented by formula "-OR¹¹²" may form the group represented by formula "-OM" above; R¹²¹ and R¹²² are each independently a hydrogen atom or a hydrocarbon group, provided that R¹²¹ and R¹²² are not both hydrogen atoms, and when R¹²¹ or R¹²² is a hydrogen atom, the group represented by the formula "-OR¹²¹" or the group represented by formula "-OR¹²² may form the group represented by formula "-OM" above; R¹³¹ and R¹³² are each independently a hydrogen atom or a hydrocarbon group, provided that R¹³¹ and R¹³² are not both hydrogen atoms, and when R¹³¹ or R¹³² is a hydrogen atom, a group represented by formula "-OR¹³¹" or a group represented by formula "-OR¹³²" may form the group represented by formula "-OM" above; R¹⁴¹ and R¹⁴² are each independently a hydrogen atom or a hydrocarbon group, provided that R¹⁴¹ and R¹⁴² are not both hydrogen atoms, and when R¹⁴¹ or R¹⁴² is a hydrogen atom, a group represented by formula "-OR¹⁴¹" or a group represented by formula "-OR¹⁴²" may form the group represented by formula "-OM" above.

In the compound represented by the general formula (110), the sentence "when R¹¹¹ or R¹¹² is a hydrogen atom, the group represented by formula "-OR¹¹¹" or the group represented by formula "-OR¹¹²" forms the group represented by formula "-OM" above", means that when the compound represented by the general formula (110) has a hydroxyl group (-OH), this compound forms a salt at the hydroxyl group.

This also applies to the compound represented by the general formula (110), the compound represented by the general formula (1210), the compound represented by the general formula (1220), and the compound represented by the general formula (1230).

An example of the ether compound obtained from the compound (21) or its salt includes a compound represented by the following general formula (210).

An example of the ether compound obtained from the compound (22) or its salt includes a compound represented by the following general formula (220). 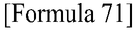 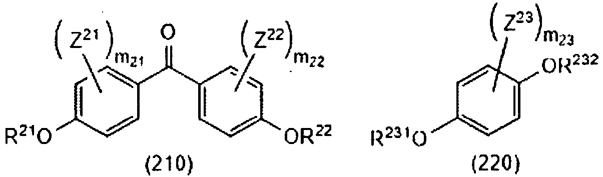 (wherein Z²¹, Z²², Z²³, m₂₁, m₂₂ and m₂₃ are the same as above; R²¹ and R²² are each independently a hydrogen atom or a hydrocarbon group, provided that R²¹ and R²² are not both hydrogen atoms, and when R²¹ or R²² is a hydrogen atom, the group represented by formula "-OR²¹" or the group represented by formula "-OR²²" may form the group represented by formula "-OM" above; and R²³¹ and R²³² are each independently a hydrogen atom or a hydrocarbon group, provided that R²³¹ and R²³² are not both hydrogen atoms, and when R²³¹ or R²³² is a hydrogen atom, the group represented by formula "-OR²³¹" or the group represented by formula "-OR²³²" may form the group represented by formula "-OM" above;

In the compound represented by the general formula (210), the sentence "when R²¹ or R²² is a hydrogen atom, the group represented by formula "-OR²¹" or the group represented by formula "-OR²²" forms the group represented by formula "-OM" above", means that when the compound represented by the general formula (210) has a hydroxyl group (-OH), this compound forms a salt at the hydroxyl group.

This also applies to the compound represented by the general formula (220).

An example of the ether compound obtained from the compound (41) or its salt includes a compound represented by the following general formula (410). 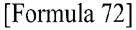 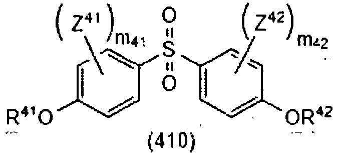 (wherein Z⁴¹, Z⁴², m₄₁, and m₄₂ are the same as above; R⁴¹ and R⁴² are each independently a hydrogen atom or a hydrocarbon group, provided that R⁴¹ and R⁴² are not both hydrogen atoms, and when R⁴¹ or R⁴² is a hydrogen atom, the group represented by formula "-OR⁴¹" or the group represented by formula "-OR⁴²" may form the group represented by formula "-OM" above.

In the compound represented by the general formula (410) above, the sentence "when R⁴¹ or R⁴² is a hydrogen atom, the group represented by formula "-OR⁴¹" or the group represented by formula "-OR⁴²" forms the group represented by formula "-OM" above", means that when the compound represented by the general formula (410) above has a hydroxyl group (-OH), this compound forms a salt at the hydroxyl group.

Examples of the hydrocarbon groups (the hydrocarbon groups in R¹¹¹, R¹¹², R¹²¹, R¹²², R¹³¹, R¹³², R¹⁴¹, R¹⁴², R²¹, R²², R²³¹, R²³², R⁴¹, and R⁴²) include an alkyl group and an aralkyl group (alias: an arylalkyl group).

Examples of the alkyl group with which a hydrogen atom is substituted include the same as the alkyl groups in Z¹¹ and Z¹². The alkyl group with which a hydrogen atom is substituted may be linear, branched-chained, or cyclic, and may have both a chain structure (linear or branched-chained structure) and a cyclic structure. A cyclic structure in a cyclic alkyl group (an alkyl group having a cyclic structure and no chain structure, as well as an alkyl group having both a cyclic structure and a chain structure) may be either monocyclic or polycyclic.

The alkyl group with which a hydrogen atom is substituted is preferably an alkyl group having 1 to 15 carbon atoms (a chained alkyl group having 1 to 15 carbon atoms, or a cyclic alkyl group having 3 to 15 carbon atoms), and may be, for example, any one of an alkyl group having 1 to 10 carbon atoms (a chained alkyl group having 1 to 10 carbon atoms, or a cyclic alkyl group having 3 to 10 carbon atoms), an alkyl group having 1 to 8 carbon atoms (a chained alkyl group having 1 to 8 carbon atoms, or a cyclic alkyl group having 3 to 8 carbon atoms), an alkyl group having 1 to 6 carbon atoms (a chained alkyl group having 1 to 6 carbon atoms, or a cyclic alkyl group having 3 to 6 carbon atoms), and an alkyl group having 1 to 3 carbon atoms.

Examples of the aralkyl group with which a hydrogen atom is substituted include monovalent groups having a structure in which one hydrogen atom bonded to a carbon atom having no free valence in the alkyl group in Z¹¹ and Z¹² described above has been substituted with an aryl group.

The aryl group may be either monocyclic or polycyclic.

The number of carbon atoms in the aryl group is preferably 6 to 15, and examples of the aryl group include a phenyl group, a 1-naphthyl group, a 2-naphthyl group, an o-tolyl group, a m-tolyl group, a p-tolyl group, and a xylyl group (dimethylphenyl group), and further examples thereof also include groups having a structure in which one or two or more hydrogen atoms in these aryl groups have been substituted with the aryl group or the alkyl group in Z¹¹ and Z¹². The number of carbon atoms of the aryl group having these substituents is preferably 6 to 15.

The number of carbon atoms in the aryl group is more preferably 6 to 12.

The number of carbon atoms of the aralkyl group with which a hydrogen atom is substituted is preferably 7 to 17, and examples of the aralkyl group include a benzyl group (phenylmethyl group), a phenethyl group (phenylethyl group), a 1-naphthylmethyl group, a 2-naphthylmethyl group, an o-tolylmethyl group, a m-tolylmethyl group, a p-tolylmethyl group, and a xylylmethyl group.

The aralkyl group more preferably has 7 to 14 carbon atoms.

In the etherification step, an etherifying agent capable of substituting a hydrogen atom in a hydroxyl group or M in the group represented by formula "-OM" described above with the hydrocarbon group is reacted with the monomer, to be able to etherify one or more (more specifically, one or two) phenolic hydroxyl groups in the monomer or one or more groups in which the phenolic hydroxyl group forms a salt.

The etherifying agent may be a publicly known agent.

When the etherification is alkyl etherification, examples of the etherifying agent include an alkyl halide and an acid alkyl ester.

Examples of the alkyl halide include an alkyl iodide such as methyl iodide (CH₃I); alkyl bromides such as (bromomethyl)cyclopropane (c-(C₃H₅)CH₂Br) and 1-bromo-3,7-dimethyloctane (CH₃CH(CH₃)CH₂CH₂CH₂CH(CH₃)CH₂CH₂Br).

Examples of the acid alkyl ester include methyl trifluoromethanesulfonate (CF₃SO₃CH₃) and dimethyl sulfate ((CH₃)₂SO₄).

When the etherification is aralkyl etherification, examples of the etherifying agent include an aralkyl halide (for example, an aralkyl bromide) such as benzyl bromide (C₆H₅CH₂Br).

When the ether compound is a compound in which two of phenolic hydroxyl groups in the monomer or groups of salts formed of the phenolic hydroxyl group are etherified, the amount of the etherifying agent used in the etherification step is preferably 2 to 5 times the amount by mole of the monomer, regardless of the type of monomer, and may be, for example, either 2 to 4 times the amount by mole and 2 to 3 times the amount by mole. The amount of the etherifying agent used, which is the lower limit value or more increases the yield of the target ether compound. The amount of the etherifying agent used, which is the upper limit value or less inhibits the etherifying agent from being excessively used.

When the ether compound is a compound in which one phenolic hydroxyl group in the monomer or a group in which the phenolic hydroxyl group forms a salt is etherified, the amount of the etherifying agent used in the etherification step is preferably 1 to 2.5 times the amount by mole of the monomer, regardless of the type of monomer, and may be, for example, either 1 to 2 times the amount by mole and 1 to 1.5 times the amount by mole. The amount of the etherifying agent used, which is the lower limit value or more increases the yield of the target ether compound. The amount of the etherifying agent used, which is the upper limit value or less inhibits the etherifying agent from being excessively used.

When the etherification step is carried out without taking out the monomer obtained by the depolymerization method (1) and the monomer is one or two or more selected from the group consisting of the compound (11) and its salt, the compound (121) and its salt, the compound (122) and its salt, and the compound (123) and its salt, and the ether compound is a compound in which two of phenolic hydroxyl groups in the monomer or groups in which the phenolic hydroxyl group forms a salt are etherified, the amount of etherifying agent used in the etherification step may be, for example, any one of 4 to 10 times the amount by mole of repeating unit in the compound (1) (the constituent unit attached with the sign n₁ in the general formula (1)), 4 to 8 times the amount by mole, and 4 to 6 times the amount by mole. The amount of the etherifying agent used, which is the lower limit value or more increases the yield of the target ether compound. The amount of etherifying agent used as described above, which is the upper limit value or less inhibits the etherifying agent from being excessively used.

When the ether compound is a compound in which one phenolic hydroxyl group in the monomer or a group in which the phenolic hydroxyl group has formed a salt has been etherified, the amount of the etherifying agent used in the etherification step may be, for the same reason as above, any one of 2 to 5 times the amount by mole of repeating unit in the compound (1), 2 to 4 times the amount by mole, and 2 to 3 times the amount by mole.

The same applies when adopting the depolymerization method (2).

That is, when the etherification step is carried out without taking out the monomer obtained by the depolymerization method (2) and the monomer is one or two or more selected from the group consisting of the compound (21) and its salt and the compound (22) and its salt, and the ether compound is a compound in which two of phenolic hydroxyl groups in the monomer or groups of salts formed of the phenolic hydroxyl group, are etherified, the amount of the etherifying agent used in the etherification step may be, for example, any one of 4 to 10 times the amount by mole of repeating unit in the compound (2) (the constituent unit attached with the sign n₂ in the general formula (2)), 4 to 8 times the amount by mole, and 4 to 6 times the amount by mole. The amount of the etherifying agent used, which is the lower limit value or more increases the yield of the target ether compound. The amount of etherifying agent used as described above, which is the upper limit value or less inhibits the etherifying agent from being excessively used.

When the ether compound is a compound in which one phenolic hydroxyl group in the monomer or a group in which the phenolic hydroxyl group has formed a salt has been etherified, the amount of the etherifying agent used in the etherification step may be, for the same reason as above, any one of 2 to 5 times the amount by mole of repeating unit in the compound (2), 2 to 4 times the amount by mole, and 2 to 3 times the amount by mole.

As will be described later, when the etherification step is carried out without taking out the monomer obtained by the depolymerization method (4) and the monomer is one or two or more selected from the group consisting of the compound (41) and its salt, and the ether compound is a compound in which two of phenolic hydroxyl groups in the monomer or groups in which the phenolic hydroxyl group has formed a salt have been etherified, the amount of the etherifying agent used in the etherification step may be, for example, any one of 2 to 5 times the amount by mole of repeating unit in the compound (4) (the constituent unit attached with the sign n₄ in the general formula (4)), 2 to 4 times the amount by mole, and 2 to 3 times the amount by mole. The amount of the etherifying agent used, which is the lower limit value or more increases the yield of the target ether compound. The amount of etherifying agent used as described above, which is the upper limit value or less inhibits the etherifying agent from being excessively used.

When the ether compound is a compound in which one phenolic hydroxyl group in the monomer or a group in which the phenolic hydroxyl group has formed a salt has been etherified, the amount of the etherifying agent used in the etherification step may be, for the same reason as above, any one of 1 to 2.5 times the amount by mole of repeating unit in the compound (4), 1 to 2 times the amount by mole, and 1 to 1.5 times the amount by mole.

The etherification in the etherification step is preferably carried out using a solvent.

The solvent may be, for example, the same as the solvent used in the depolymerization step in the depolymerization method (1), (2) or (4).

In case of carrying out the etherification step without taking out the monomer obtained by the depolymerization method (1), (2) or (4), after completion of the depolymerization step in the depolymerization method (1), (2) or (4), as described above, a solution obtained by subjecting the obtained reaction mixture to post-treatment, if necessary, can be used as it is to etherify the monomer.

In the above-mentioned production method, after completion of the etherification step, the obtained reaction mixture may be post-treated, if necessary, by a publicly known method to take out the target substance (ether compound). In other words, post-treatment operations such as filtration, cleaning, extraction, pH adjustment, dehydration, and concentration may be appropriately carried out, if necessary, in a single manner among them or in combination of two or more thereof, and the target substance can be taken out by concentration, crystallization, reprecipitation, column chromatography, or the like. Also, the taken out target substance may be further purified, if necessary, by carrying out one or more times any one of operations such as crystallization, reprecipitation, column chromatography, extraction, and stirring and washing of crystals with a solvent, in a single manner among these operations or in a combined manner of two or more thereof. Alternatively, after completion of the etherization step, the resulting reaction mixture may be subjected to post-treatment and then used for the next intended application without taking out the target substance. For example, the target substance may be subjected to the next intended reaction without being taken out.

The structure of the product by the depolymerization method can be confirmed by publicly known techniques, such as nuclear magnetic resonance (NMR) spectroscopy, mass spectrometry (MS), and infrared spectroscopy (IR).

### Examples

The present invention will be specifically described in detail below by way of Examples. However, the present invention is not limited thereto.

### <<Depolymerization of polysulfone (depolymerization method (1))>>

### [Example 1]

Under an argon atmosphere, to pellet-shaped polysulfone (89.2 mg; the amount of repeating unit in the general formula (1) of 0.20 mmol; weight-average molecular weight 35,000; and number-average molecular weight 16,000, manufactured by Sigma-Aldrich Co. LLC, Cat. No. 428302) were added sodium hydroxide (NaOH) (0.8 mmol, 4 times the amount by mole (4 equivalents) of repeating unit in the general formula (1)) and 1,3-dimethyl-2-imidazolidinone (DMI) (0.4 mL) in sequence, and the mixture was heated at 150°C to dissolve the polysulfone. Thereafter, the resulting mixture was stirred at 150°C for 18 hours. Sodium hydroxide was dried by heating under reduced pressure (266.644 Pa (2 mmHg)) at 150°C for 5 hours before use.

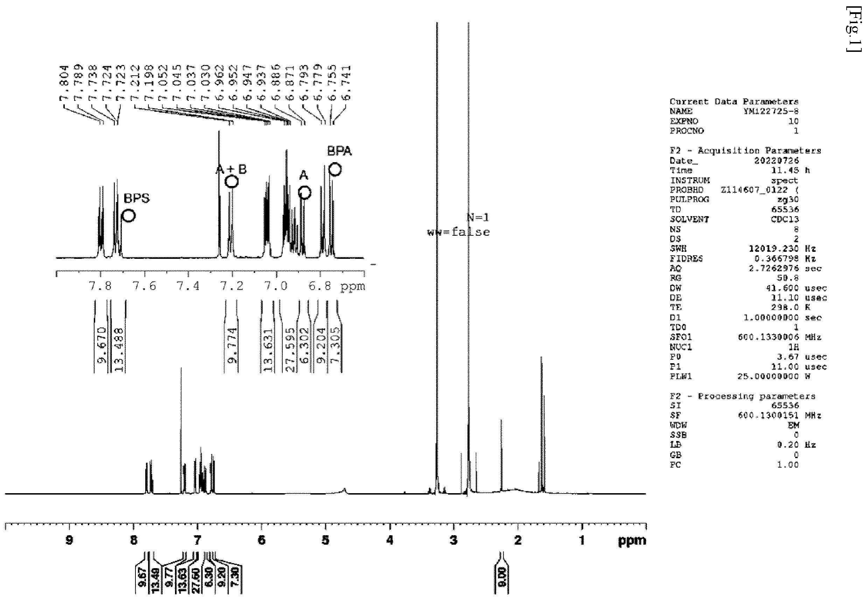

Then, the temperature of the resulting reaction mixture was returned to room temperature, and 1M concentration hydrochloric acid (1.0 mL) was added and stirred. Thereafter, mesitylene (5.0 µL, 0.036 mmol) and deuterated chloroform (CDCl₃) (0.5 mL) were added, and the organic layer was taken out, and ¹H NMR analysis confirmed that the target substances (depolymerized products) bisphenol S (BPS, corresponding to the compound (11)) and bisphenol A (BPA, corresponding to the compound (121)) were obtained, and the yields of these target substances and the compound (A) represented by the following formula (A) and the compound (B) represented by the following formula (B), which were intermediate products were calculated. The results are shown in Table 1. The results of ¹H NMR analysis are also shown in Fig. 1.

### [Example 2]

Under an argon atmosphere, to pellet-shaped polysulfone (222 mg; the amount of repeating unit in the general formula (1) of 0.50 mmol; weight-average molecular weight 35,000; and number-average molecular weight 16,000, manufactured by Sigma-Aldrich Co. LLC, Cat. No. 428302) were added potassium hydroxide (KOH) (2.0 mmol, 4 times the amount by mole (4 equivalents) of repeating unit in the general formula (1)) and 1,3-dimethyl-2-imidazolidinone (DMI) (1.0 mL) in sequence, and the mixture was heated at 150°C to dissolve the polysulfone. Thereafter, the resulting mixture was stirred at 150°C for 20 hours. Potassium hydroxide was dried by heating under reduced pressure (266.644 Pa (2 mmHg)) at 150°C for 5 hours before use.

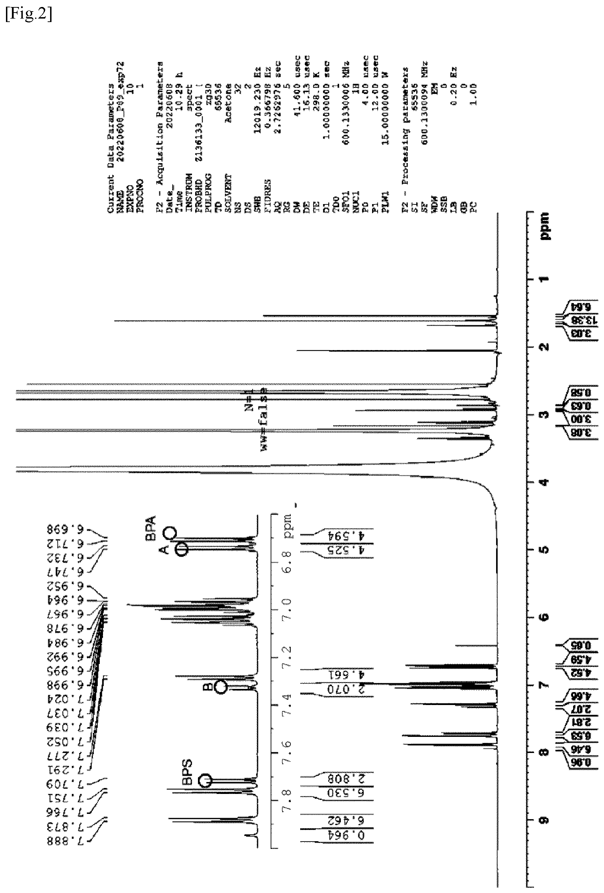

Then, the temperature of the resulting reaction mixture was returned to room temperature, and 2M concentration hydrochloric acid (1.0 mL) N,N-dimethylformamide (10 µL, 0.13 mmol) were added. A small amount of the solution was taken out, dissolved in deuterated acetone ((CD₃)₂CO) (0.50 mL), and analyzed by ¹H NMR. The results are shown in Table 1. The results of the ¹H NMR analysis are also shown in Fig. 2.

### [Example 3]

Depolymerization of polysulfone was carried out in the same manner as in Example 2, except that cesium hydroxide monohydrate (CsOH·H₂O) (2.0 mmol, four times the amount by mole (4 equivalents) of repeating unit in the general formula (1)) was used instead of potassium hydroxide (2.0 mmol). Cesium hydroxide monohydrate was dried by heating at 150°C for 5 hours under reduced pressure (266.644 Pa (2 mmHg)) before use. Polysulfone was dissolved upon depolymerization.

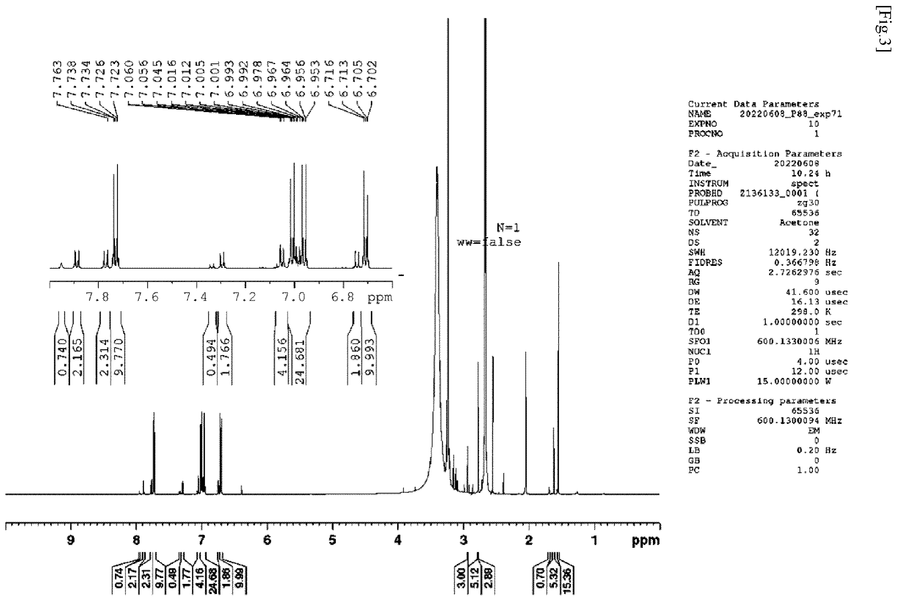

The reaction mixture thus obtained was analyzed in the same manner as in Example 2, and it was confirmed that bisphenol S (BPS) and bisphenol A (BPA) were obtained, and furthermore, the yields of these target substances, the compound (A), and the compound (B) were calculated. The results are shown in Table 1. The results of the ¹H NMR analysis are also shown in Fig. 3.

### [Example 4]

Depolymerization of polysulfone was carried out in the same manner as in Example 3, except that the reaction temperature was changed to 120°C instead of 150°C and the reaction time was changed to 18 hours instead of 20 hours. Polysulfone was dissolved upon depolymerization.

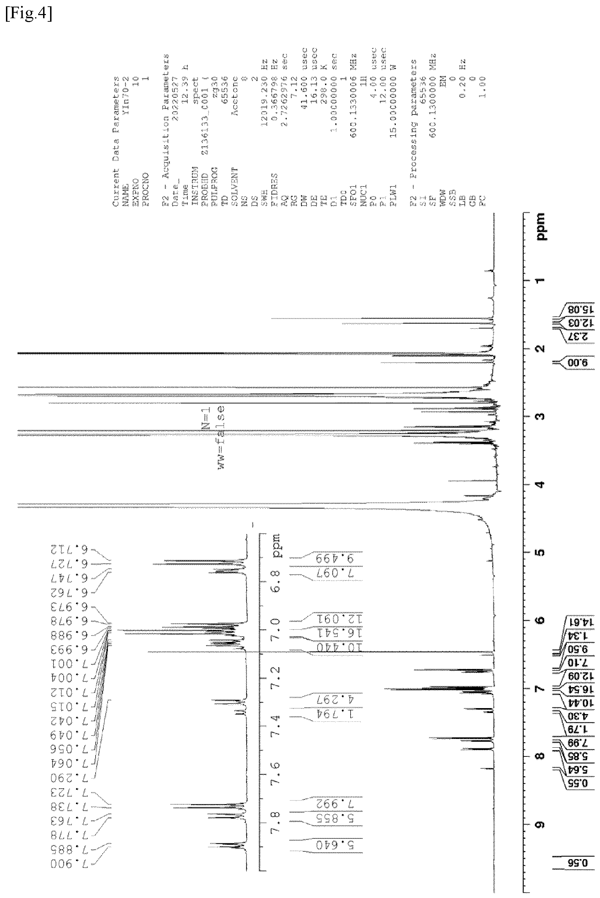

The reaction mixture thus obtained was analyzed in the same manner as in Example 2, and it was confirmed that bisphenol S (BPS) and bisphenol A (BPA) were obtained, and furthermore, the yields of these target substances, the compound (A), and the compound (B) were calculated. The results are shown in Table 1. The results of the ¹H NMR analysis are also shown in Fig. 4.

### [Example 5]

Depolymerization of polysulfone was carried out in the same manner as in Example 1, except that the amount of sodium hydroxide used was changed from 0.8 mmol to 1.2 mmol (6 times the amount by mole (6 equivalents) of repeating unit in the general formula (1)). Polysulfone was dissolved upon depolymerization.

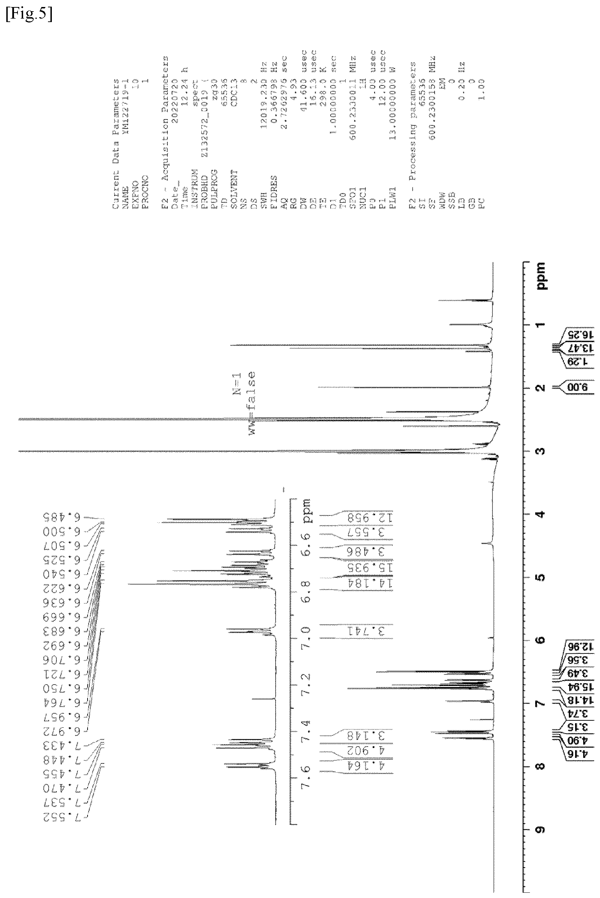

The reaction mixture thus obtained was analyzed in the same manner as in Example 1, and it was confirmed that bisphenol S (BPS) and bisphenol A (BPA) were obtained, and furthermore, the yields of these target substances, the compound (A), and the compound (B) were calculated. The results are shown in Table 1. The results of the ¹H NMR analysis are also shown in Fig. 5.

### [Example 6]

Depolymerization of polysulfone was carried out in the same manner as in Example 5, except that potassium hydroxide (1.2 mmol) was used instead of sodium hydroxide (1.2 mmol), and the reaction time was 19 hours instead of 18 hours. Polysulfone was dissolved upon depolymerization.

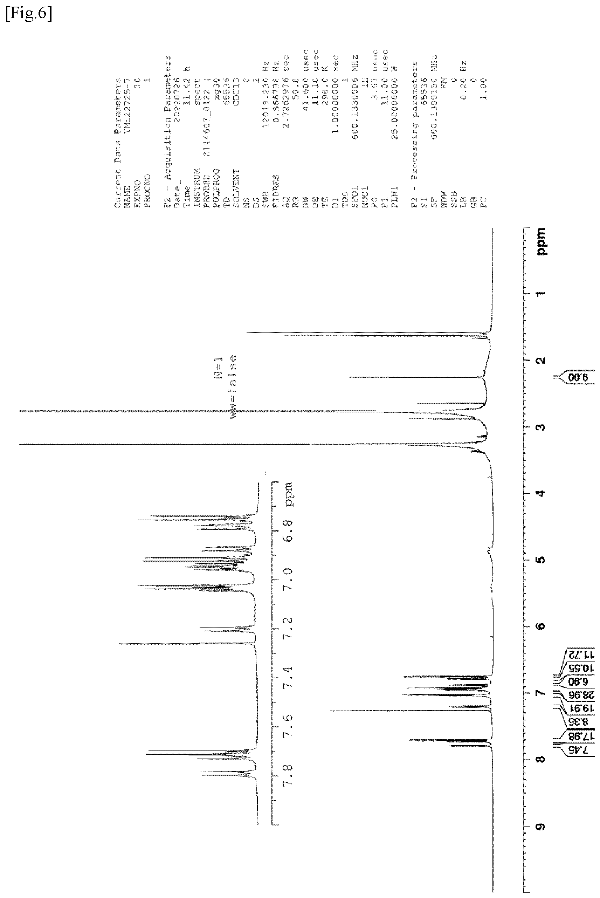

The reaction mixture thus obtained was analyzed in the same manner as in Example 1, and it was confirmed that bisphenol S (BPS) and bisphenol A (BPA) were obtained, and furthermore, the yields of these target substances, the compound (A), and the compound (B) were calculated. The results are shown in Table 1. The results of the ¹H NMR analysis are also shown in Fig. 6.

### [Example 7]

Depolymerization of polysulfone was carried out in the same manner as in Example 3, except that the amount of cesium hydroxide monohydrate used was changed from 2.0 mmol to 3.0 mmol (6 times the amount by mole (6 equivalents) of repeating unit in the general formula (1)). Polysulfone was dissolved upon depolymerization.

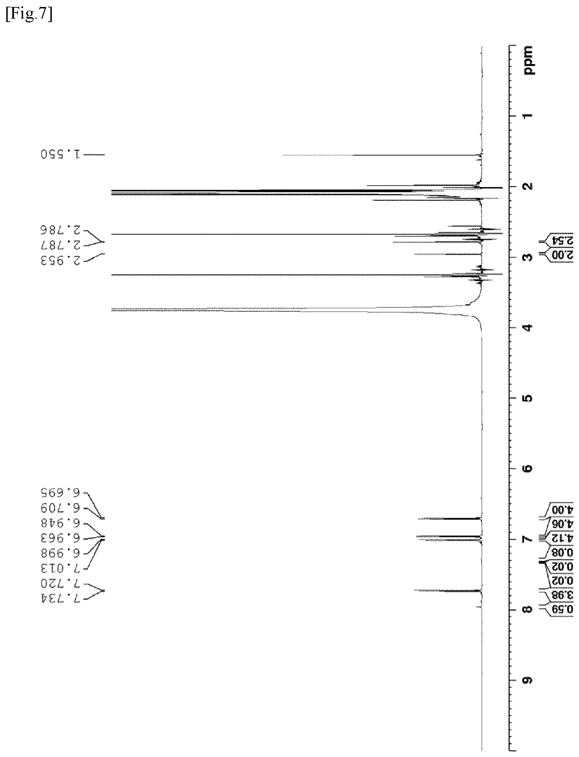

The reaction mixture thus obtained was analyzed in the same manner as in Example 1, and it was confirmed that bisphenol S (BPS) and bisphenol A (BPA) were obtained, and furthermore, the yields of these target substances, the compound (A), and the compound (B) were calculated. The results are shown in Table 1. The results of the ¹H NMR analysis are also shown in Fig. 7. 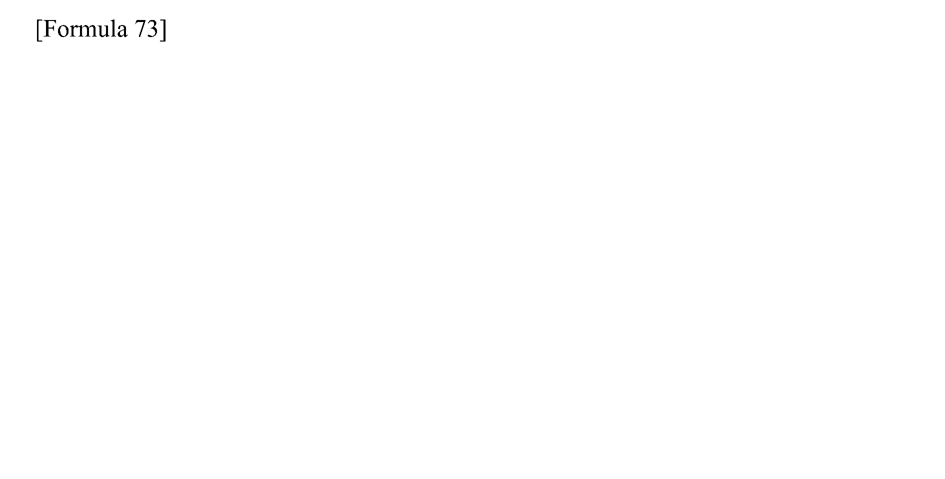 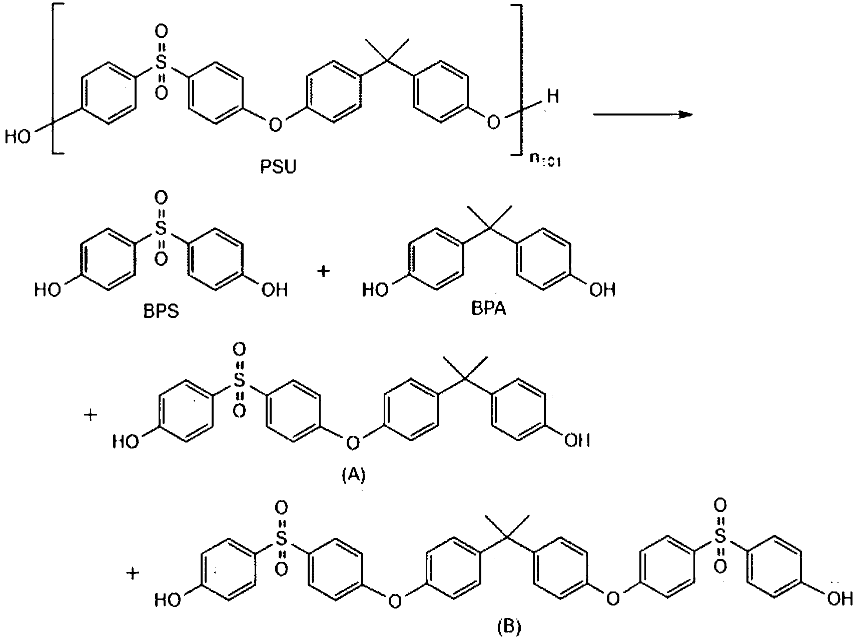 (wherein n₁₀₁ is an integer (preferably 10 to 200)).

**[Table 2]**

| | Hydroxide | Reaction conditions | | Product yield | | | |
|---|---|---|---|---|---|---|---|
| | Component name (amount used (times the amount by mole of compound (1)) | Temperature (°C) | Time (h) | BPS | BPA | Compound (A) | Compound (B) |
| Example 1 | NaOH(4) | 150 | 18 | 16 | 31 | 54 | 15 |
| Example 2 | KOH(4) | 150 | 20 | 17 | 29 | 59 | 12 |
| Example 3 | CsOH·H₂O(4) | 150 | 20 | 72 | 72 | 24 | 4 |
| Example 4 | CsOH·H₂O(4) | 120 | 18 | 32 | 32 | 57 | 11 |
| Example 5 | NaOH(6) | 150 | 18 | 15 | 46 | 32 | 12 |
| Example 6 | KOH(6) | 150 | 19 | 40 | 45 | 53 | 2 |
| Example 7 | CsOH·H₂O(6) | 150 | 20 | 93 | 93 | 4 | 1 |

As is clarified from the above results, it was confirmed that bisphenol S and bisphenol A could be obtained in favorable yields even though the type of hydroxide was changed. Of these, when cesium hydroxide monohydrate was used as the hydroxide, the yields of bisphenol S and bisphenol A were particularly high.

Comparing Examples 1 to 3 with Examples 5 to 7, it was confirmed that increasing the amount of hydroxide used tends to increase the yields of bisphenol S and bisphenol A.

In comparison between Examples 3 and Example 4, the increase in reaction temperature was confirmed to increase the yields of bisphenol S and bisphenol A.

It is to be noted that depolymerization of polysulfone was performed in the same manner as in Example 3, except that cesium hydroxide monohydrate that had not been heated and dried at 150°C under reduced pressure for 5 hours was used instead of cesium hydroxide monohydrate that had been heated and dried in this manner. As a result, the yields of bisphenol S (BPS) and bisphenol A (BPA) were lower than those of Example 3. These results specifically confirmed that depolymerization of compound (1) was preferably carried out under dehydrating conditions.

### <<Depolymerization of polysulfone (depolymerization method (1))>>

### [Example 8]

Under an argon atmosphere, to pellet-shaped polysulfone (44.3 mg; the amount of repeating unit in the general formula (1) is 0.10 mmol; weight-average molecular weight 35,000; and number-average molecular weight 16,000, manufactured by Sigma-Aldrich Co. LLC, Cat. No. 428302) were added calcium hydride (CaH₂, dehydrating agent) (0.4 mmol, 4 times the amount by mole (4 equivalents) of repeating unit in the general formula (1)), cesium hydroxide monohydrate (CsOH·H₂O) (0.4 mmol, 4 times the amount by mole (4 equivalents) of repeating unit in the general formula (1)), and 1,3-dimethyl-2-imidazolidinone (DMI) (0.2 mL) in sequence, and the resulting mixture was heated at 150°C to dissolve the polysulfone. Thereafter, the resulting mixture was stirred at 150°C for 18 hours. Cesium hydroxide monohydrate was dried by heating at 150°C for 5 hours under reduced pressure (266.644 Pa (2 mmHg)) before use.

Then, the temperature of the reaction mixture was returned to room temperature, 1M concentration hydrochloric acid (1.0 mL) and ethyl acetate were added, and the organic layer was washed successively with water and a saturated sodium chloride aqueous solution. The obtained organic layer was dried over anhydrous magnesium sulfate, and then distilled under reduced pressure to obtain a crude product.

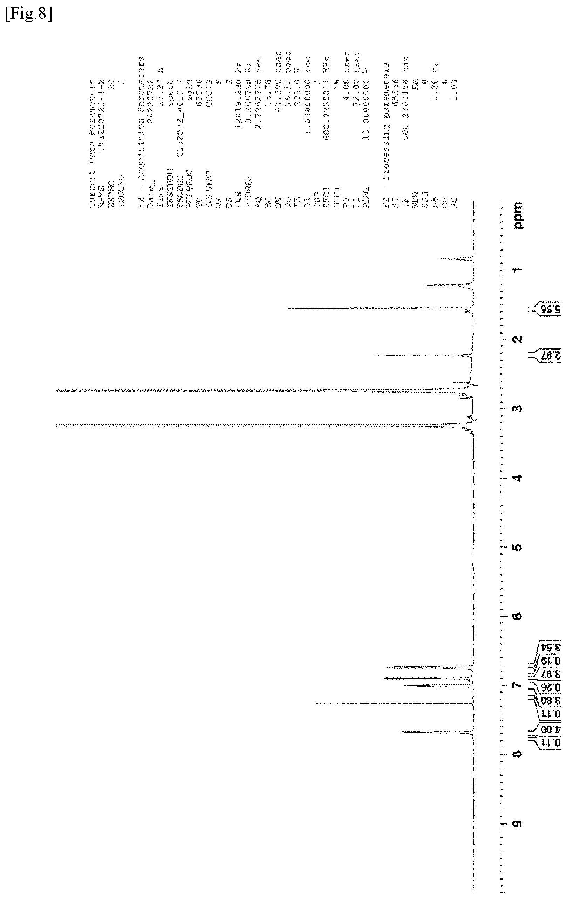

The crude product obtained was analyzed in the same manner as in Example 1, and bisphenol S (BPS) and bisphenol A (BPA) were confirmedly obtained, and furthermore, the yields of these target substances, the compound (A), and the compound (B) were calculated. The results are shown in Table 2. The results of the ¹H NMR analysis are also shown in Fig. 8.

### [Example 9]

Depolymerization of polysulfone was carried out in the same manner as in Example 8, except that the amount of calcium hydride used was changed from 0.4 mmol to 0.2 mmol (twice the amount by mole (2 equivalents) of repeating unit in the general formula (1)). Polysulfone was dissolved upon depolymerization.

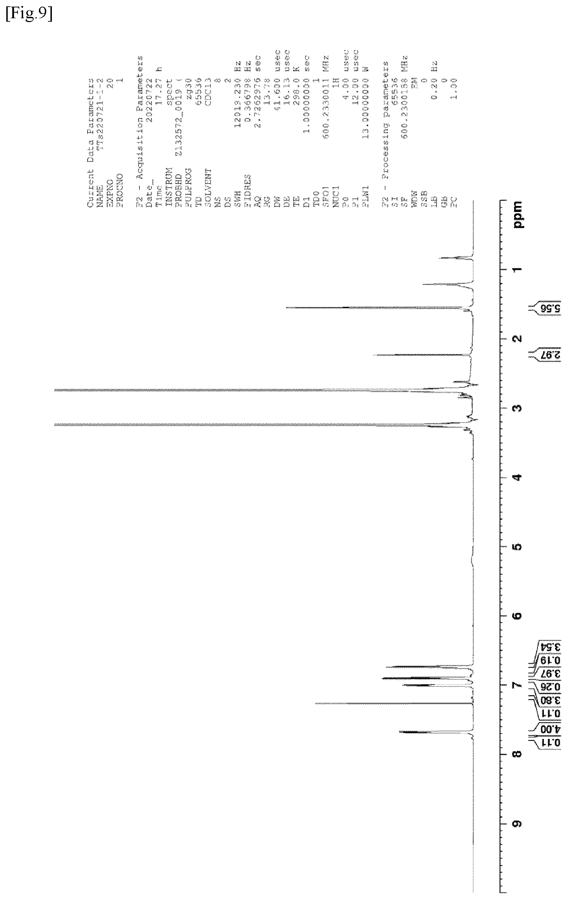

The crude product thus obtained was analyzed in the same manner as in Example 1, and bisphenol S (BPS) and bisphenol A (BPA) were confirmedly obtained, and furthermore, the yields of these target substances, the compound (A), and the compound (B) were calculated. The results are shown in Table 2. The results of the ¹H NMR analysis are also shown in Fig. 9.

### [Example 10]

Depolymerization of polysulfone was carried out in the same manner as in Example 8, except that instead of calcium hydride (0.4 mmol), calcium oxide (CaO) (0.4 mmol, 4 times the amount by mole (4 equivalents) of repeating unit in the general formula (1)) was used. Polysulfone was dissolved upon depolymerization.

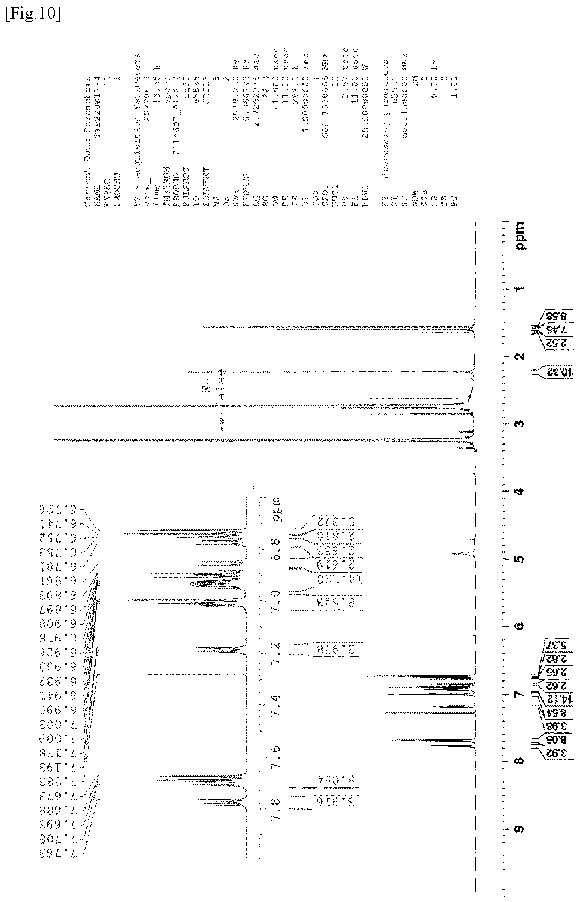

The crude product thus obtained was analyzed in the same manner as in Example 1, and bisphenol S (BPS) and bisphenol A (BPA) were confirmedly obtained, and furthermore, the yields of these target substances, the compound (A), and the compound (B) were calculated. The results are shown in Table 2. The results of the ¹H NMR analysis are also shown in Fig. 10.

### [Example 11]

Under an argon atmosphere, to pellet-shaped polysulfone (45.6 mg; the amount of repeating unit in the general formula (1) is 0.10 mmol; weight-average molecular weight 35,000; and number-average molecular weight 16,000, manufactured by Sigma-Aldrich Co. LLC, Cat. No. 428302) were added cesium hydroxide monohydrate (CsOH·H₂O) (0.4 mmol, 4 times the amount by mole (4 equivalents) of repeating unit in the general formula (1)), cesium chloride (CsCl₂) (0.3 mmol, 3 times the amount by mole (3 equivalents) of repeating unit in the general formula (1)), and 1,3-dimethyl-2-imidazolidinone (DMI) (0.2 mL) in sequence, and the mixture was heated at 150°C to dissolve the polysulfone. Thereafter, the resulting mixture was stirred at 150°C for 20 hours. Cesium hydroxide monohydrate and cesium chloride was dried by heating at 150°C for 5 hours under reduced pressure (266.644 Pa (2 mmHg)) before use.

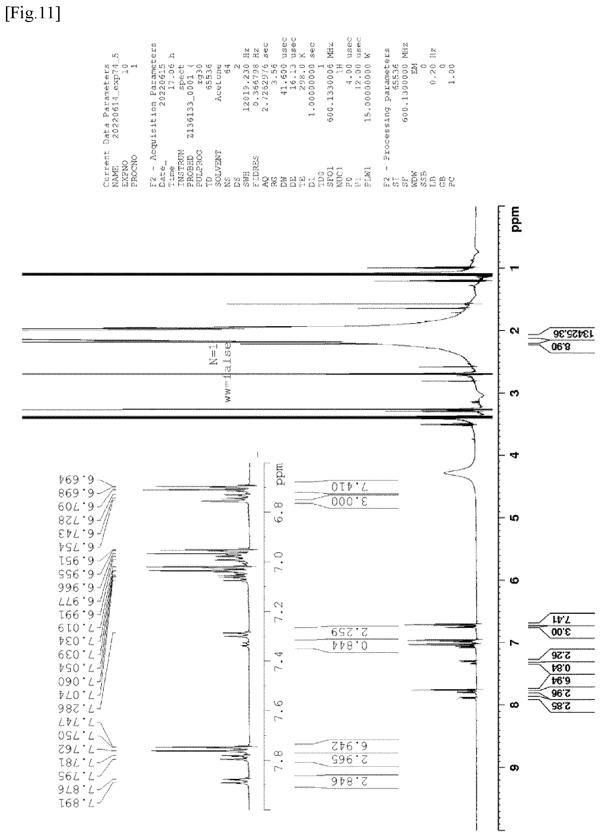

The reaction mixture thus obtained was analyzed in the same manner as in Example 1, and it was confirmed that bisphenol S (BPS) and bisphenol A (BPA) were obtained, and furthermore, the yields of these target substances, the compound (A), and the compound (B) were calculated. The results are shown in Table 2. The results of the ¹H NMR analysis are also shown in Fig. 11.

### [Example 12]

Depolymerization of polysulfone was carried out in the same manner as in Example 11, except that zeolite ("Molecular Sieve 4A" manufactured by FUJIFILM Wako Pure Chemical Corporation) (3 grains) was used instead of cesium chloride (0.3 mmol), and the reaction time was changed to 18 hours instead of 20 hours. Polysulfone was dissolved upon depolymerization.

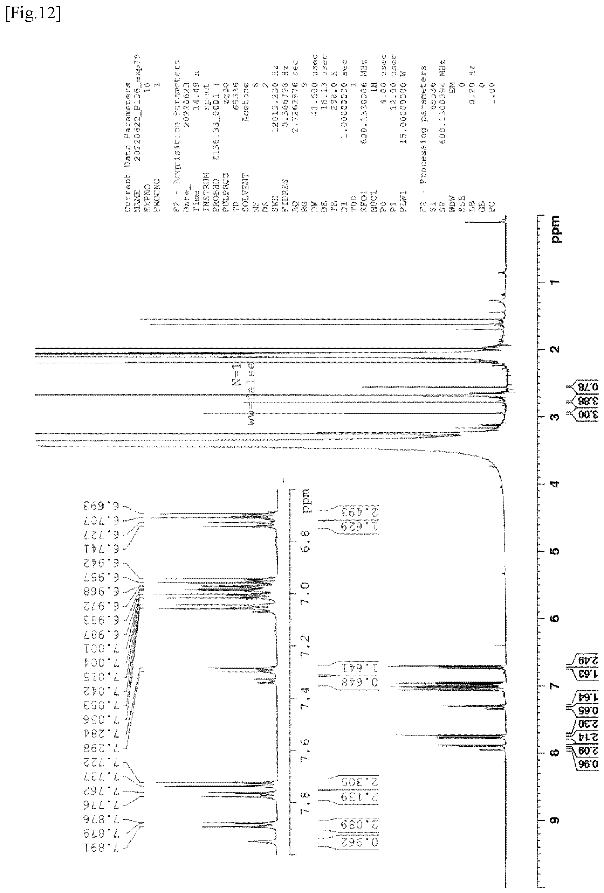

The reaction mixture thus obtained was analyzed in the same manner as in Example 1, and it was confirmed that bisphenol S (BPS) and bisphenol A (BPA) were obtained, and furthermore, the yields of these target substances, the compound (A), and the compound (B) were calculated. The results are shown in Table 2. The results of the ¹H NMR analysis are also shown in Fig. 12. 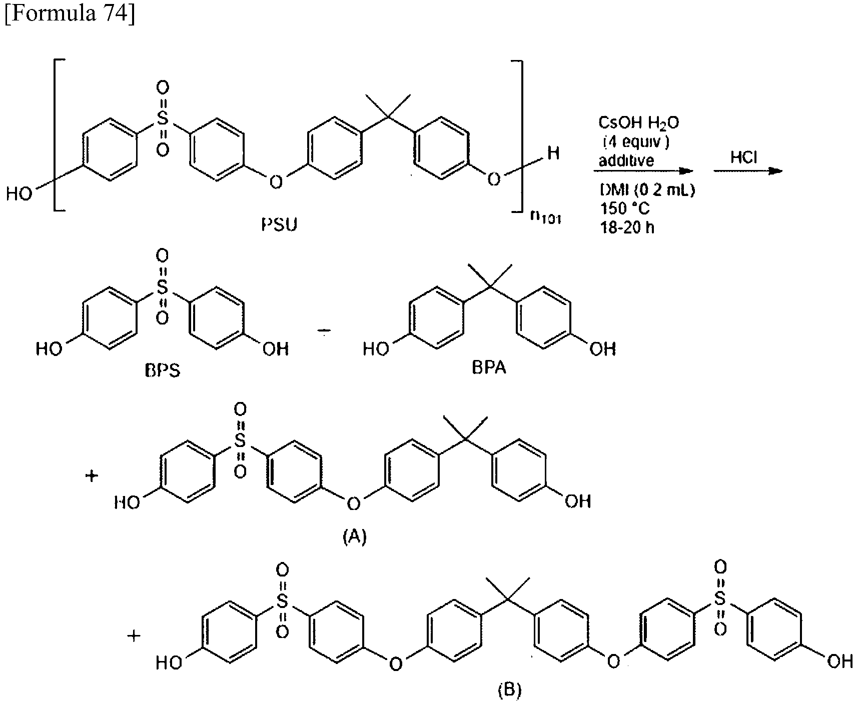 (wherein n₁₀₁ is an integer (preferably 10 to 200)).

**[Table 3]**

| | Hydroxide | Dehydrating agent | Reaction conditions | | Product yield | | | |
|---|---|---|---|---|---|---|---|---|
| | Component name (amount used (times the amount by mole of compound (1)) | Component name (amount used (times the amount by mole of compound (1)) | Temperature (°C) | Time (h) | BPS | BPA | Compound (A) | Compound (B) |
| Example 8 | CsOH·H₂O(4) | CaH₂(4) | 150 | 18 | 96 | 88 | 4 | nd |
| Example 9 | CsOH·H₂O(4) | CaH₂(2) | 150 | 18 | 77 | 77 | 23 | nd |
| Example 10 | CsOH·H₂O(4) | CaO(4) | 150 | 18 | 35 | 45 | 44 | 11 |
| Example 11 | CsOH·H₂O(4) | CsCl₂(3) | 150 | 20 | 55 | 58 | 35 | 6 |
| Example 12 | CsOH·H₂O(4) | MS4A* | 150 | 18 | 36 | 39 | 51 | 10 |

| | | | | | | | | |
|---|---|---|---|---|---|---|---|---|
| * The amount used is 3 grains. | | | | | | | | |

Comparing Example 3 with Examples 8 and 9, it was confirmed that using calcium hydride as the dehydrating agent allows the depolymerization to proceed further efficiently. In Examples 8 and 9, compound (B) was not detected.

In Examples 10 to 12, the use of a dehydrating agent reduced the yield of bisphenol S and bisphenol A, however, this was presumed to be due to the dehydrating agent partially reacting or interacting with the hydroxide.

Comparing Examples 8 and 9, it was confirmed that the increase in an amount of dehydrating agent used increased the yields of bisphenol S and bisphenol A.

### <<Depolymerization of polysulfone on a gram scale (depolymerization method upon scale-up (1))>>

### [Example 13]

Under an argon atmosphere, to pellet-shaped polysulfone (2.21g; the amount of repeating unit in the general formula (1) of 5.0 mmol; weight-average molecular weight 35,000; and number-average molecular weight 16,000, manufactured by Sigma-Aldrich Co. LLC, Cat. No. 428302) were added cesium hydroxide monohydrate (CsOH·H₂O) (20 mmol, 4 times the amount by mole (4 equivalents) of repeating unit in the general formula (1)), calcium hydride (CaH₂) (20 mmol, 4 times the amount by mole (4 equivalents) of repeating unit in the general formula (1)), and 1,3-dimethyl-2-imidazolidinone (DMI) (10 mL) in sequence, and the resulting mixture was heated at 150°C to dissolve the polysulfone. Thereafter, the resulting mixture was stirred at 150°C for 18 hours. Cesium hydroxide monohydrate was dried by heating at 150°C for 5 hours under reduced pressure (266.644 Pa (2 mmHg)) before use.

Next, the temperature of the reaction mixture obtained was returned to room temperature, and 0.5 M concentration hydrochloric acid (20 mL) and ethyl acetate (30 mL) were added and stirred. Thereafter, the reaction mixture was filtered using Celite (manufactured by FUJIFULM Wako Pure Chemical CORPORATION), and ethyl acetate (30 mL) and water (30 mL) were added and stirred to separate the organic layer and the aqueous layer. The organic layer was washed with water (50 mL × 3), and the obtained organic layer was dried over anhydrous magnesium sulfate, and then distilled under reduced pressure to obtain a crude product (C1).

Next, to the aqueous layer obtained above was added 2 M concentration hydrochloric acid (10 mL), and the organic layer was extracted using ethyl acetate (50 mL), washed with water (50 mL × 3), and the obtained organic layer was dried over anhydrous magnesium sulfate, and then distilled under reduced pressure to obtain a crude product (C2).

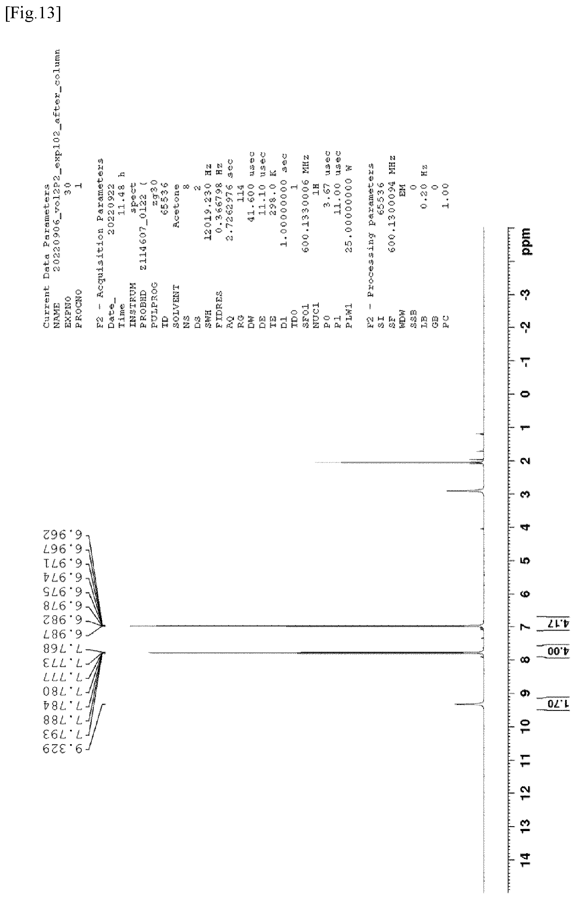

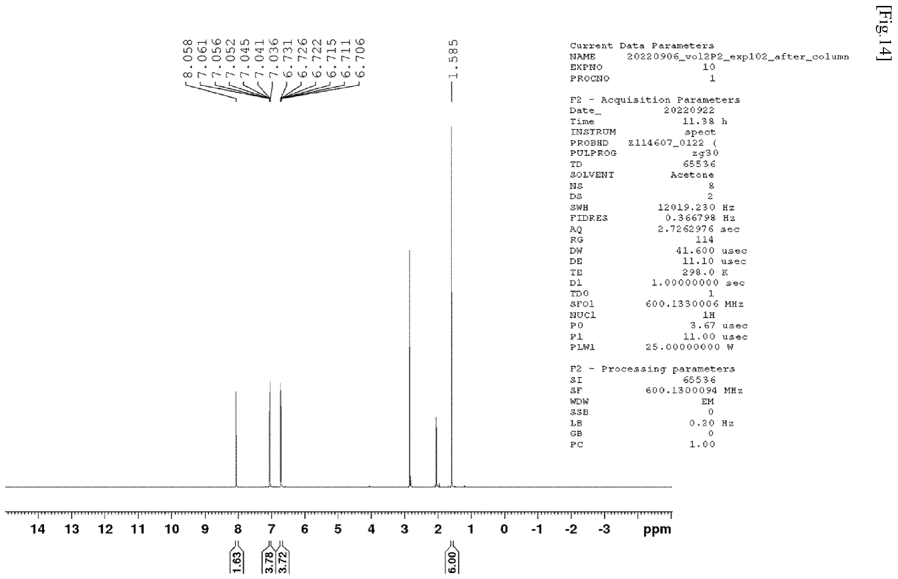

The crude product (C1) was then recrystallized using acetone to isolate bisphenol A (BPA) (yield: 532 mg, 47%). The crude product (C2) was, on the other hand, purified by silica gel column chromatography to separate bisphenol S (BPS) (yield: 897 mg, 72%) as well as isolate bisphenol A (BPA) (yield: 127 mg, 11%). The results of ¹H NMR analysis of the obtained bisphenol S are shown in Fig. 13, and the results of ¹H NMR analysis of bisphenol A are shown in Fig. 14.

### <<Depolymerization of polysulfone (depolymerization method (1)), production of ether compounds>>

### [Example 14]

### <Depolymerization of polysulfone>

Under an argon atmosphere, to pellet-shaped polysulfone (131.2 mg; the amount of repeating unit in the general formula (1) of 0.296 mmol; weight-average molecular weight 35,000; and number-average molecular weight 16,000, manufactured by Sigma-Aldrich Co. LLC, Cat. No. 428302) were added calcium hydride (CaH₂, dehydrating agent) (50.5 mg, 1.2 mmol, 4 times the amount by mole (4 equivalents) of repeating unit in the general formula (1)), cesium hydroxide monohydrate (CsOH·H₂O) (201 mg, 1.2 mmol, 4 times the amount by mole (4 equivalents) of repeating unit in the general formula (1)), and 1,3-dimethyl-2-imidazolidinone (DMI)

(0.6 mL) in sequence, and the resulting mixture was heated at 150°C to dissolve the polysulfone. Thereafter, the resulting mixture was stirred at 150°C for 19 hours. Cesium hydroxide monohydrate was dried by heating at 150°C for 5 hours under reduced pressure (266.644 Pa (2 mmHg)) before use.

### <Production of ether compound>

Then, the temperature of the reaction mixture obtained above was returned to room temperature, and benzyl bromide (C₆H₅CH₂Br) (1.2 mmol, 4 times the amount by mole (4 equivalents) of repeating unit in the general formula (1)) was added, and the mixture was stirred at 100°C for 3 hours. The temperature of the reaction mixture obtained was returned to room temperature, and 1 M concentration hydrochloric acid (1.0 mL) and ethyl acetate (2.0 mL) were added in sequence, and the organic layer was sequentially washed with water and saturated sodium chloride aqueous solution. The obtained organic layer was dried over anhydrous magnesium sulfate, and then distilled under reduced pressure to obtain a crude product.

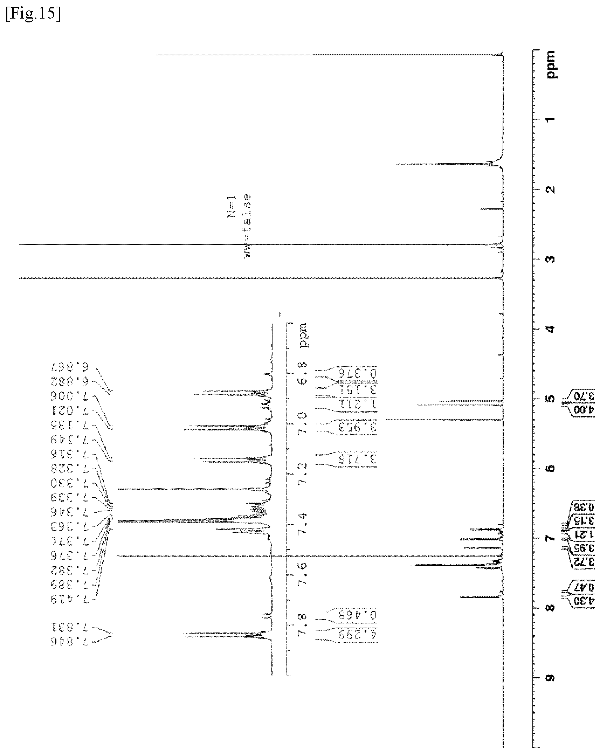

To the obtained crude product was added mesitylene (5.0 µL, 0.036 mmol), and the resulting mixture was analyzed by ¹H NMR, confirming that the target substances (ether compounds) bisphenol S dibenzyl ether (corresponding to the compound (110)) and bisphenol A dibenzyl ether (corresponding to the compound (1210)) were obtained. Furthermore, the yields of these target substances were calculated, resulting in that the yield of bisphenol S dibenzyl ether was 92%, and the yield of bisphenol A dibenzyl ether was 89%. The results of the ¹H NMR analysis are shown in Figure 15. 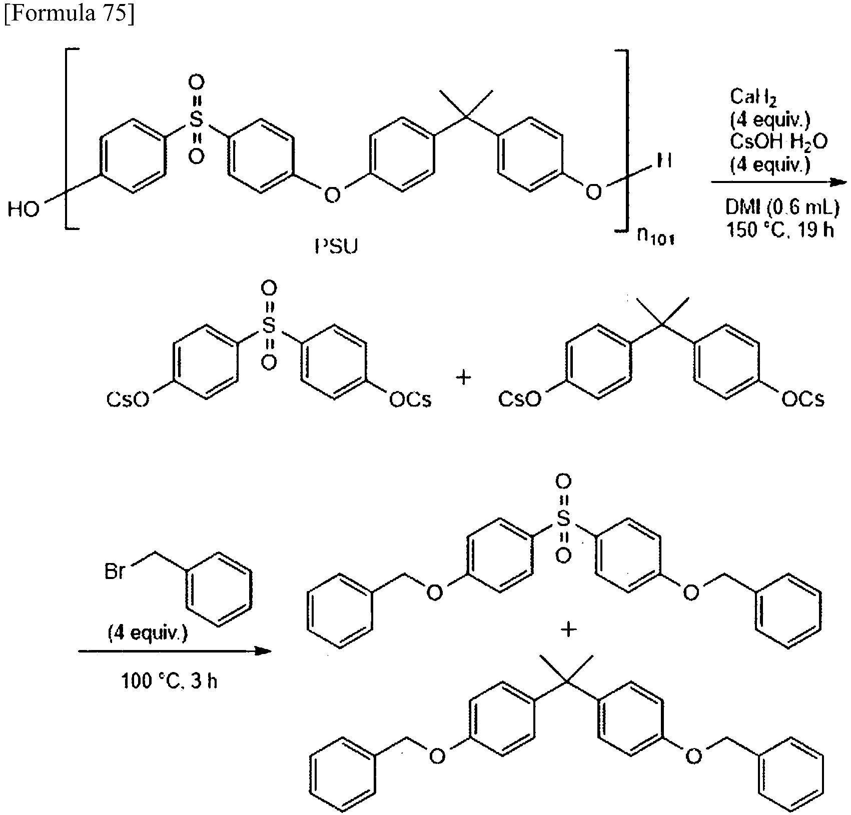 (wherein n₁₀₁ is an integer (preferably 10 to 200)).

### <<Depolymerization of polysulfone (depolymerization method (1)), production of ether compounds>>

### [Example 15]

### <Depolymerization of polysulfone>

Under an argon atmosphere, to pellet-shaped polysulfone (132.1 mg; the amount of repeating unit in the general formula (1) of 0.3 mmol; weight-average molecular weight 35,000; and number-average molecular weight 16,000, manufactured by Sigma-Aldrich Co. LLC, Cat. No. 428302) were added calcium hydride (CaH₂, dehydrating agent) (50.5 mg, 1.2 mmol, 4 times the amount by mole (4 equivalents) of repeating unit in the general formula (1)), cesium hydroxide monohydrate (CsOH·H₂O) (201 mg, 1.2 mmol, 4 times the amount by mole (4 equivalents) of repeating unit in the general formula (1)), and 1,3-dimethyl-2-imidazolidinone (DMI) (0.6 mL) in sequence, and the resulting mixture was heated at 150°C to dissolve the polysulfone. Thereafter, the resulting mixture was stirred at 150°C for 19 hours. Cesium hydroxide monohydrate was dried by heating at 150°C for 5 hours under reduced pressure (266.644 Pa (2 mmHg)) before use.

### <Production of ether compound>

Then, the temperature of the reaction mixture obtained above was returned to room temperature, and 1,3-dimethyl-2-imidazolidinone (DMI) (0.6 mL) and (bromomethyl)cyclopropane (c-(C₃H₅)CH₂Br) (1.2 mmol, 4 times the amount by mole (equivalents) of repeating unit in the general formula (1), and the mixture was stirred at 100°C for 3 hours.

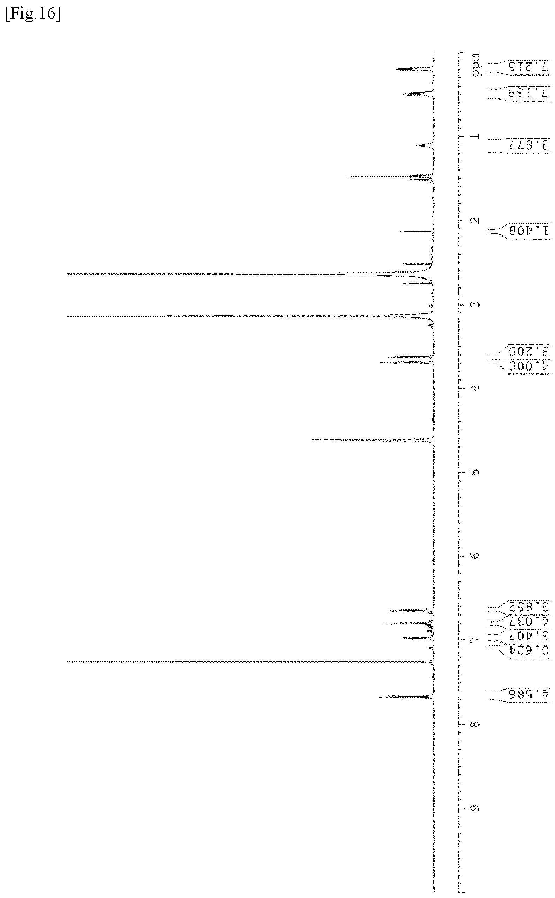

The temperature of the reaction mixture was returned to room temperature, mesitylene (5.0 µL, 0.036 mmol) and deuterated chloroform (CDCl₃) (0.5 mL) were added, and the organic layer was taken out, and the analysis by ¹H NMR confirmed that the target substances (ether compounds) bisphenol S di(cyclopropylmethyl)ether (corresponding to the compound (110)) and bisphenol A di(cyclopropylmethyl)ether (corresponding to the compound (1210)) were obtained. Furthermore, the yields of these target substances were calculated, and the yield of bisphenol S di(cyclopropylmethyl)ether was 71%, and the yield of bisphenol A di(cyclopropylmethyl)ether was 56%. The results of the ¹H NMR analysis are shown in Fig. 16. 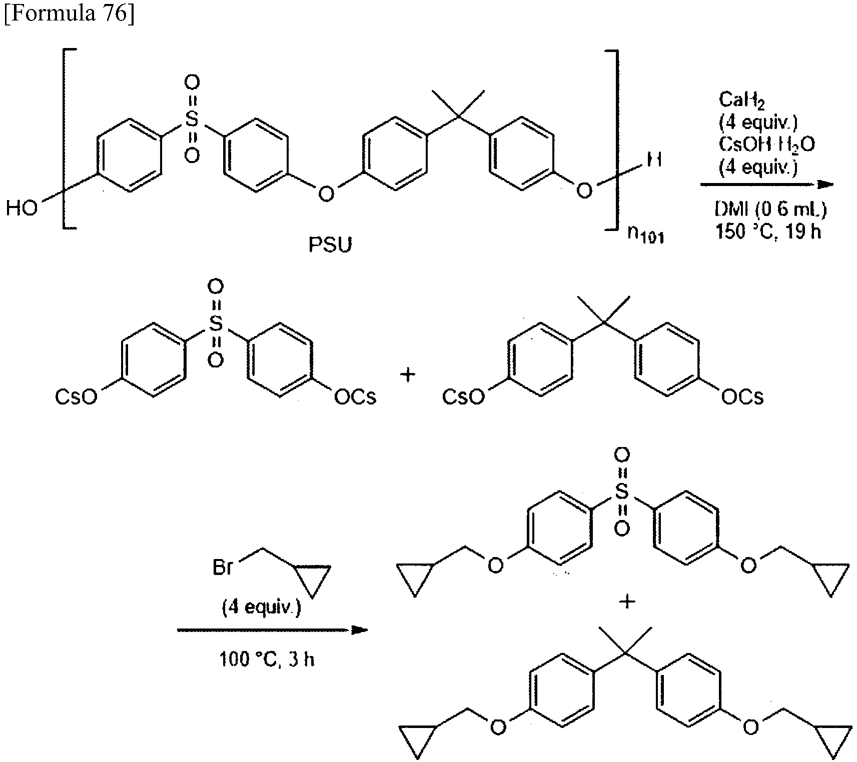 (wherein n₁₀₁ is an integer (preferably 10 to 200)).

### <<Depolymerization of polysulfone (depolymerization method (1)), production of ether compounds>>

### [Example 16]

### <Depolymerization of polysulfone>

Under an argon atmosphere, to pellet-shaped polysulfone (133.6 mg; the amount of repeating unit in the general formula (1) of 0.3 mmol; weight-average molecular weight 35,000; and number-average molecular weight 16,000, manufactured by Sigma-Aldrich Co. LLC, Cat. No. 428302) were added calcium hydride (CaH₂, dehydrating agent) (50.7 mg, 1.2 mmol, 4 times the amount by mole (4 equivalents) of repeating unit in the general formula (1)), cesium hydroxide monohydrate (CsOH·H₂O) (201 mg, 1.2 mmol, 4 times the amount by mole (4 equivalents) of repeating unit in the general formula (1)), and 1,3-dimethyl-2-imidazolidinone (DMI) (0.6 mL) in sequence, and the resulting mixture was heated at 150°C to dissolve the polysulfone. Thereafter, the resulting mixture was stirred at 150°C for 19 hours. Cesium hydroxide monohydrate was dried by heating at 150°C for 5 hours under reduced pressure (266.644 Pa (2 mmHg)) before use.

### <Production of ether compound>

Then, the temperature of the reaction mixture obtained above was returned to room temperature, and 1,3-dimethyl-2-imidazolidinone (DMI) (0.6 mL) and 1-bromo-3,7-dimethyloctane (CH₃CH(CH₃)CH₂CH₂CH₂CH(CH₃)CH₂CH₂Br) (1.2 mmol, 4 times the amount by mole (4 equivalents) of repeating unit in the general formula (1)), and the mixture was stirred at 100°C for 3 hours.

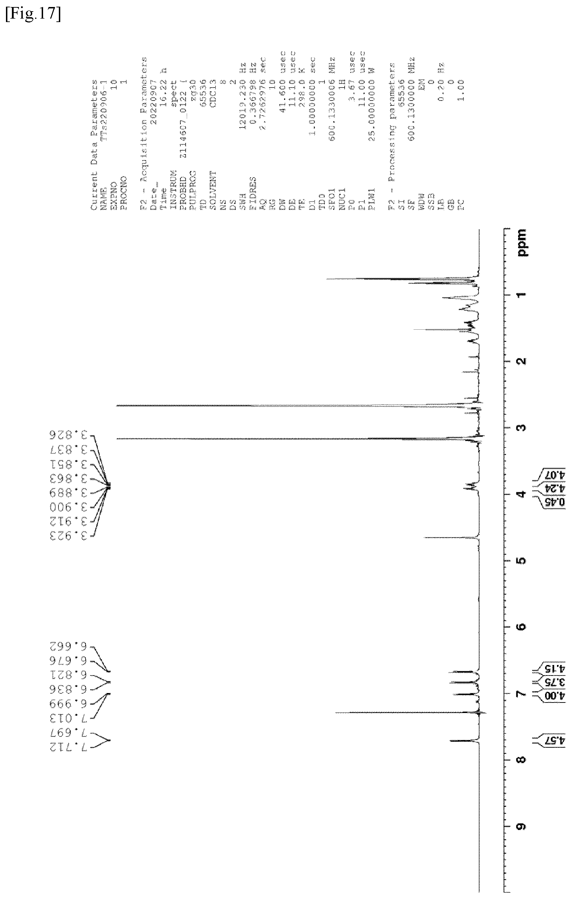

The temperature of the resulting reaction mixture was returned to room temperature, and mesitylene (5.0 µL, 0.036 mmol) and deuterated chloroform (CDCl₃) (0.5 mL) were added to extract the organic layer, which was analyzed by ¹H NMR, confirming that the target substances (ether compounds), bisphenol S di(3,7-dimethyloctyl) ether (corresponding to the compound (110)) and bisphenol A di(3,7-dimethyloctyl) ether (corresponding to the compound (1210)), were obtained. Furthermore, the yields of these target compounds were calculated, and the yield of bisphenol S di(3,7-dimethyloctyl) ether was 92%, and the yield of bisphenol A di(3,7-dimethyloctyl) ether was 89%. The results of the ¹H NMR analysis are shown in Fig. 17. 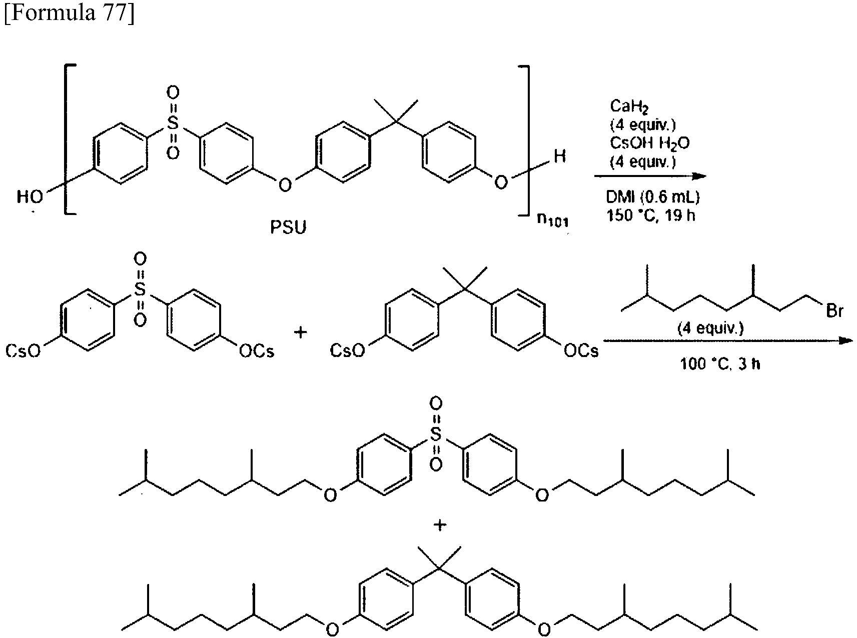 (wherein n₁₀₁ is an integer (preferably 10 to 200)).

As is clarified from the results of Examples 14 to 16, the reaction of etherifying agent (here, an alkyl halide or an aralkyl halide) with the product (monomer) of depolymerization of polysulfone facilitated the target ether compounds to be obtained. Also, an ether compound was obtained in favorable yield even though the type of etherifying agent was changed.

### <<Depolymerization of polyether sulfone (depolymerization method (4))>>

### [Example 17]

Under an argon atmosphere, to pellet-shaped polysulfone (23.2 mg; the amount of repeating unit in the general formula (4) of 0.100 mmol, manufactured by Sigma-Aldrich Co. LLC, Cat. No. 191094) were added cesium hydroxide monohydrate (CsOH·H₂O) (51.2 mg, 0.3 mmol, 3 times the amount by mole (3 equivalents) of repeating unit in the general formula (1)) and 1,3-dimethyl-2-imidazolidinone (DMI) (0.2 mL) successively, and the resulting mixture was heated at 150°C to dissolve the polyphenylsulfone. Thereafter, the resulting mixture was stirred at 150°C for 19 hours. Cesium hydroxide monohydrate was dried by heating at 150°C for 5 hours under reduced pressure (266.644 Pa (2 mmHg)) before use.

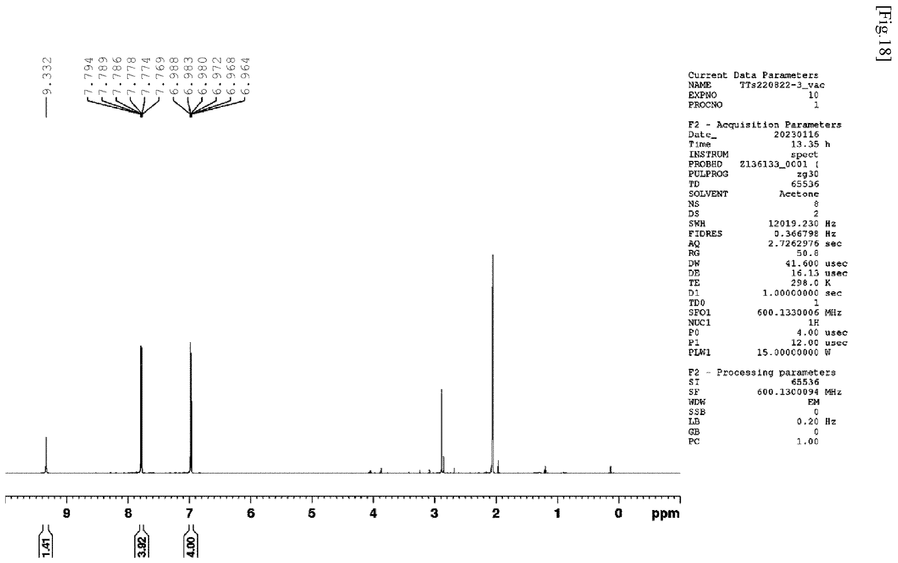

Next, the temperature of the reaction mixture was returned to room temperature, and 1M concentration hydrochloric acid (2.0 mL) and methylene chloride (3 mL) were added to extract the organic layer. The organic layer was then washed successively with water and a saturated sodium chloride aqueous solution. The organic layer was dried over anhydrous magnesium sulfate, and the precipitated solid was cleaned with hexane and distilled under reduced pressure to obtain the target substance, bisphenol S (BPS) (corresponding to the compound (41)) (yield: 20.7 mg, 83%). The results of the ¹H NMR analysis of the obtained bisphenol S are shown in Fig. 18. 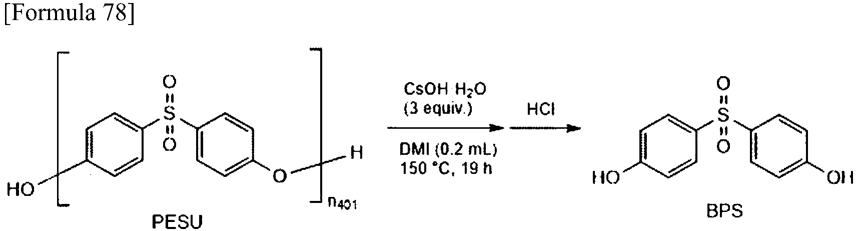 (wherein n₄₀₁ is an integer (preferably 10 to 200)).

### <<Depolymerization of polyether sulfone (depolymerization method (4)), Production of ether compound >>

### [Example 18]

### <Depolymerization of polyether sulfone>

Under an argon atmosphere, to pellet-shaped polysulfone (23.8 mg; the amount of repeating unit in the general formula (4) of 0.103 mmol, manufactured by Sigma-Aldrich Co. LLC, Cat. No. 191094) were added cesium hydroxide monohydrate (CsOH·H₂O) (50.4 mg, 0.3 mmol, 3 times the amount by mole (3 equivalents) of repeating unit in the general formula (4)) and 1,3-dimethyl-2-imidazolidinone (DMI)

(0.2 mL) successively, and the resulting mixture was heated at 150°C to dissolve the polyphenylsulfone. Thereafter, the resulting mixture was stirred at 150°C for 19 hours. Cesium hydroxide monohydrate was dried by heating at 150°C for 5 hours under reduced pressure (266.644 Pa (2 mmHg)) before use.

### <Production of ether compound>

Then, the temperature of the reaction mixture obtained above was returned to room temperature, and benzyl bromide (C₆H₅CH₂Br) (0.3 mmol, 3 times the amount by mole (3 equivalents) of repeating unit in the general formula (4)) and 1,3-dimethyl-2-imidazolidinone (DMI) (0.2 mL), and the mixture was stirred at 100°C for 2 hours.

The temperature of the resulting reaction mixture was returned to room temperature, and water (1.0 mL) and methylene chloride (2.0 mL) were added in that order, and the organic layer was then sequentially washed with water and a saturated aqueous solution of sodium chloride. The obtained organic layer was dried over anhydrous magnesium sulfate, and then distilled under reduced pressure to obtain a crude product.

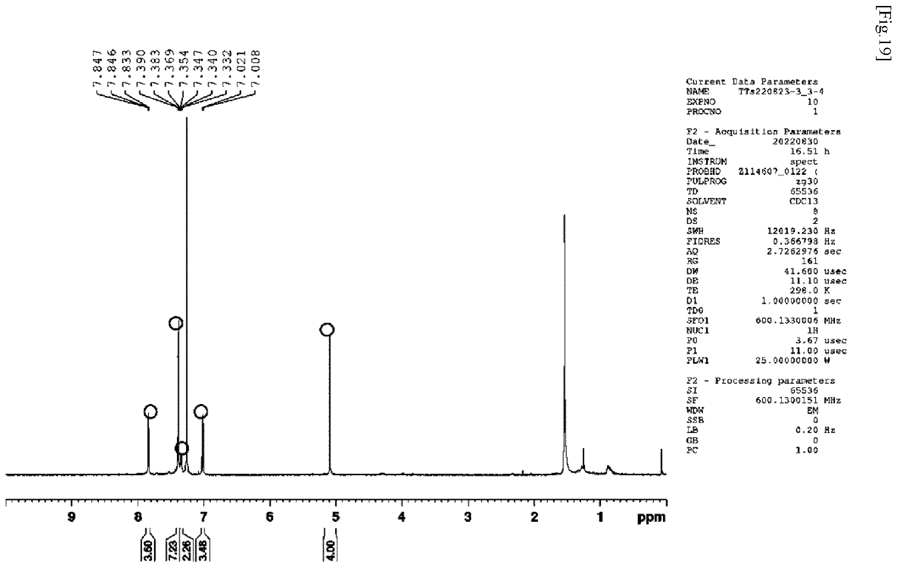

The resulting crude product was purified by silica gel column chromatography to isolate bisphenol S dibenzyl ether (corresponding to the compound (410)) (yield: 38.4 mg, 87%). The results of the ¹H NMR analysis of the obtained bisphenol S dibenzyl ether are shown in Fig. 19. 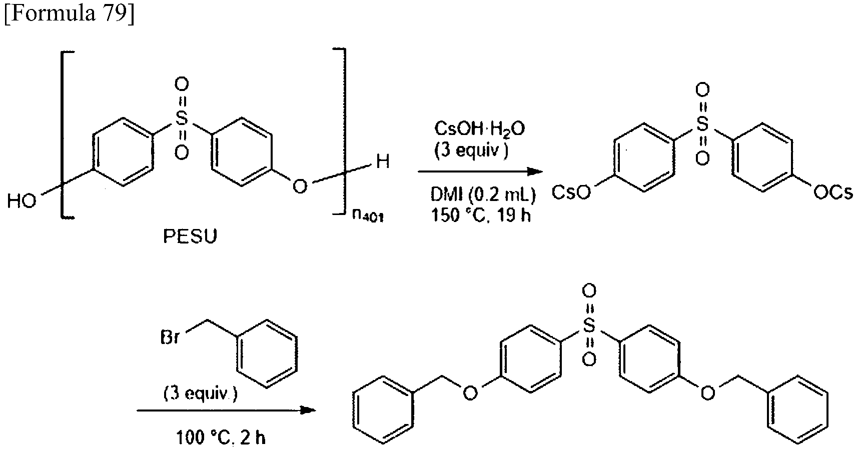 (wherein n₄₀₁ is an integer (preferably 10 to 200)).

### <Depolymerization of polyphenylsulfone (depolymerization method (1))>>

### [Example 19]

Under an argon atmosphere, to powdered polyphenylsulfone (120 mg; the amount of repeating unit in the general formula (1) of 0.3 mmol, manufactured by Standard-Testpiece K. K., Cat. No. RMOLDED0050) were added cesium hydroxide monohydrate (CsOH·H₂O) (202 mg, 1.2 mmol, 4 times the amount by mole (4 equivalents) of repeating unit in the general formula (1)), calcium hydride (CaH₂, dehydrating agent) (50.7 mg, 1.2 mmol, 4 times the amount by mole (4 equivalents) of repeating unit in the general formula (1)), and 1,3-dimethyl-2-imidazolidinone (DMI) (0.6 mL) in sequence, and the resulting mixture was heated at 150°C to dissolve the polyphenylsulfone. Thereafter, the resulting mixture was stirred at 150°C for 19 hours. Cesium hydroxide monohydrate was dried by heating at 150°C for 5 hours under reduced pressure (266.644 Pa (2 mmHg)) before use.

Next, the temperature of the reaction mixture was returned to room temperature, 1M concentration hydrochloric acid (3.6 mL) was added followed y addition of water (10 mL) and ethyl acetate (20 mL) to extract the organic layer. The organic layer was then washed successively with water and a saturated sodium chloride aqueous solution. The obtained organic layer was dried over anhydrous magnesium sulfate, and then distilled under reduced pressure to obtain a crude product.

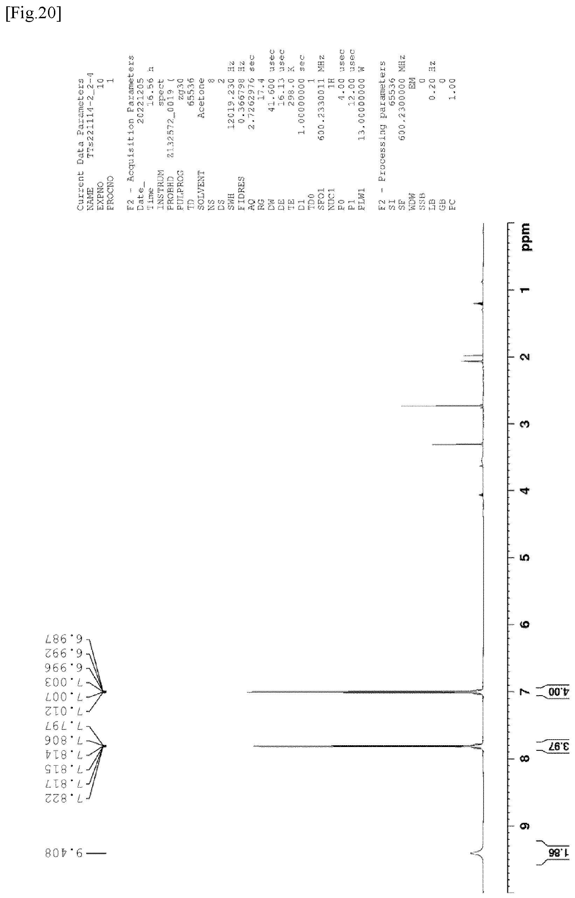

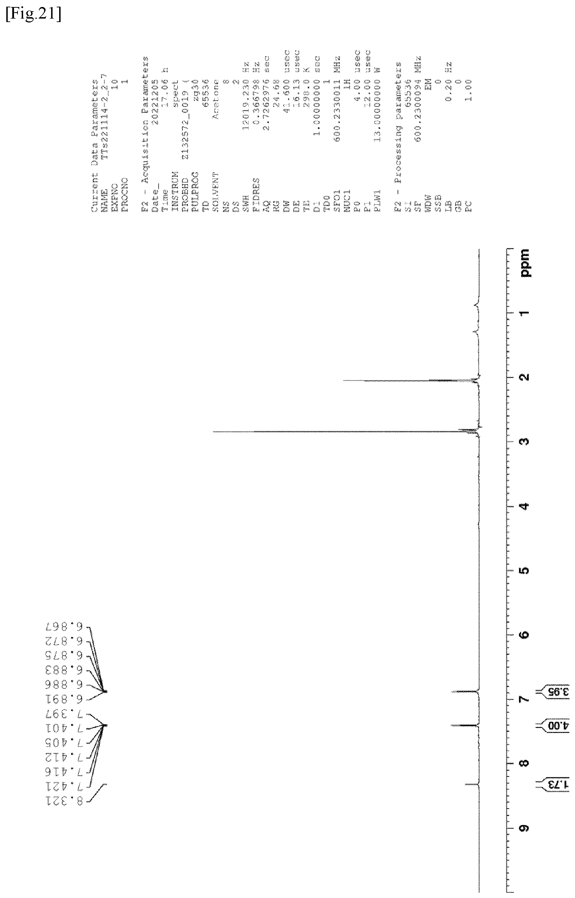

The obtained crude product was purified by silica gel column chromatography to isolate bisphenol S (corresponding to the compound (110)) (yield: 65.2 mg, 87%) and 4,4'-dihydroxybiphenyl (corresponding to the compound (123)) (yield: 44.7 mg, 80%). The results of the ¹H NMR analysis of the bisphenol S obtained are shown in Fig. 20, and the results of the ¹H NMR analysis of 4,4'-dihydroxybiphenyl are shown in Fig. 21. 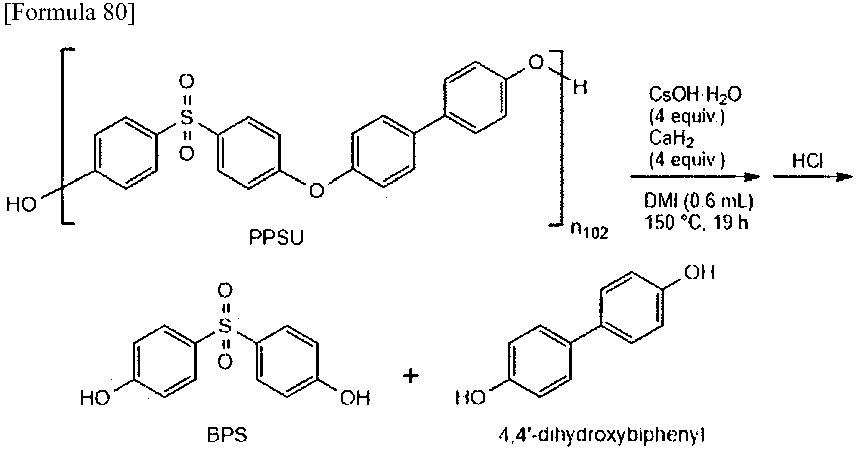 (wherein n₁₀₂ is an integer (preferably 10 to 200)).

### <<Depolymerization of polyether ether sulfone (depolymerization method (1))>>

### [Example 20]

Under an argon atmosphere, to pellet-shaped polyether ether sulfone (95.4 mg; the amount of repeating unit in the general formula (1) of 0.30 mmol, manufactured by Sigma-Aldrich Co. LLC, Cat. No.79421) were added cesium hydroxide monohydrate (CsOH·H₂O) (1.2 mmol, 4 times the amount by mole (4 equivalents) of repeating unit in the general formula (1)), calcium hydride (CaH₂) (1.2 mmol, 4 times the amount by mole (4 equivalents) of repeating unit in the general formula (1)), and 1,3-dimethyl-2-imidazolidinone (DMI) (0.6 mL) in sequence, and the resulting mixture was heated at 150°C for 18 hours to dissolve the polyether ether sulfone. Thereafter, the resulting mixture was stirred at 150°C for 22 hours. Cesium hydroxide monohydrate was dried by heating at 150°C for 5 hours under reduced pressure (266.644 Pa (2 mmHg)) before use.

Next, the temperature of the reaction mixture was returned to room temperature, and 2M concentration hydrochloric acid (1 mL) was added. Thereto was further added N,N-dimethylformamide (DMF) (5.0 µL, 0.065 mmol), and the analysis by¹H NMR confirmed that the target substances, bisphenol S (corresponding to the compound (11)) and hydroquinone (corresponding to the compound (122)), were obtained. Furthermore, the yields of these target substances were calculated, and the yield of bisphenol S was 75% and the yield of hydroquinone was 68%.

To the reaction mixture obtained above were then added ethyl acetate (5 mL) and water (5 mL), and the mixture was stirred to extract the organic layer. The obtained organic layer was dried over anhydrous magnesium sulfate, and then distilled under reduced pressure to obtain a crude product.

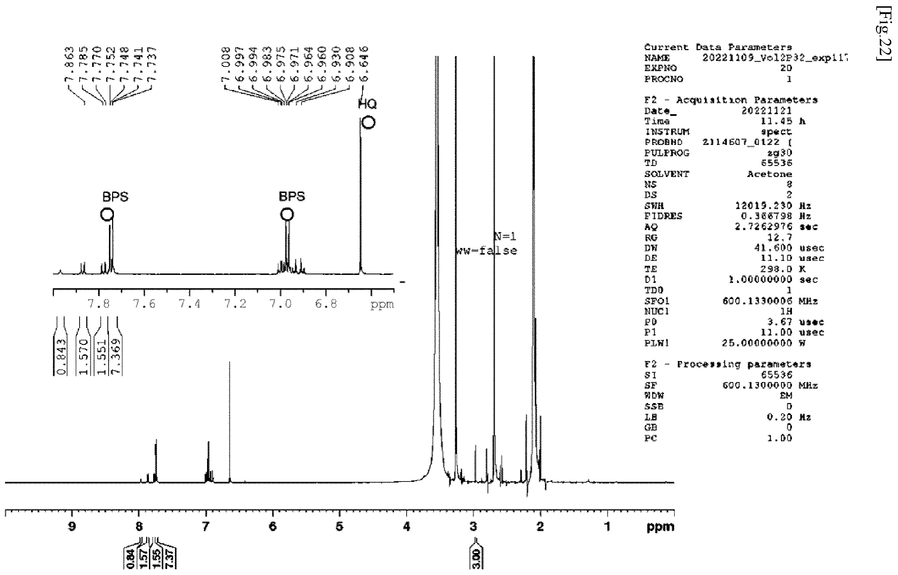

The resulting crude product was purified by silica gel column chromatography to isolate bisphenol S (BPS) (yield: 40.6 mg, 55%). The results of the ¹H NMR analysis of the obtained bisphenol S are shown in Fig. 22. 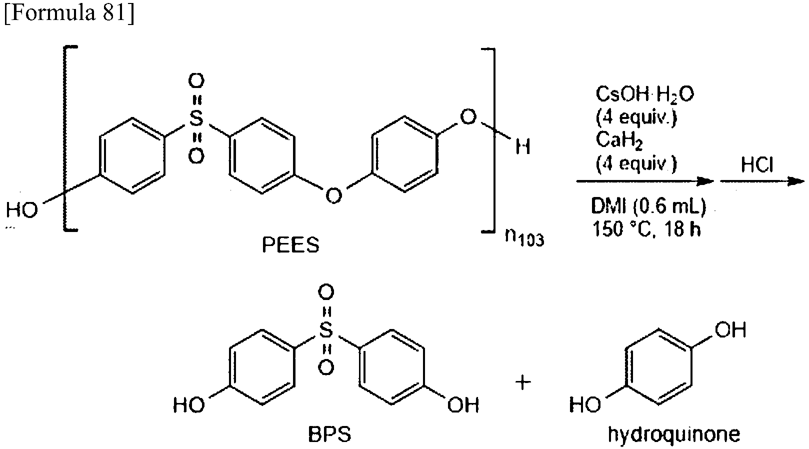 (wherein n₁₀₃ is an integer (preferably 10 to 200)).

### <<Depolymerization of polyether ether ketone (depolymerization method (2))>>

### [Example 21]

Under an argon atmosphere, to pellet-shaped polyether ether ketone (86.6 mg; the amount of repeating unit in the general formula (2) of 0.30 mmol, manufactured by Sigma-Aldrich Co. LLC, Cat. No.79421) were added cesium hydroxide monohydrate (CsOH·H₂O) (1.2 mmol, 4 times the amount by mole (4 equivalents) of repeating unit in the general formula (2)), calcium hydride (CaH₂) (1.2 mmol, 4 times the amount by mole (4 equivalents) of repeating unit in the general formula (2)), and 1,3-dimethyl-2-imidazolidinone (DMI) (0.6 mL) in sequence, and the resulting mixture was heated at 150°C for 18 hours to dissolve the polyether ether ketone. Thereafter, the resulting mixture was stirred at 150°C for 22 hours. Cesium hydroxide monohydrate was dried by heating at 150°C for 5 hours under reduced pressure (266.644 Pa (2 mmHg)) before use.

Next, the temperature of the reaction mixture was returned to room temperature, and 2M concentration hydrochloric acid (1 mL) was added. Thereto were further added ethyl acetate (5 mL) and water (5 mL) to extract the organic layer. The organic layer obtained was dried over anhydrous magnesium sulfate, and then distilled under reduced pressure to obtain a crude product.

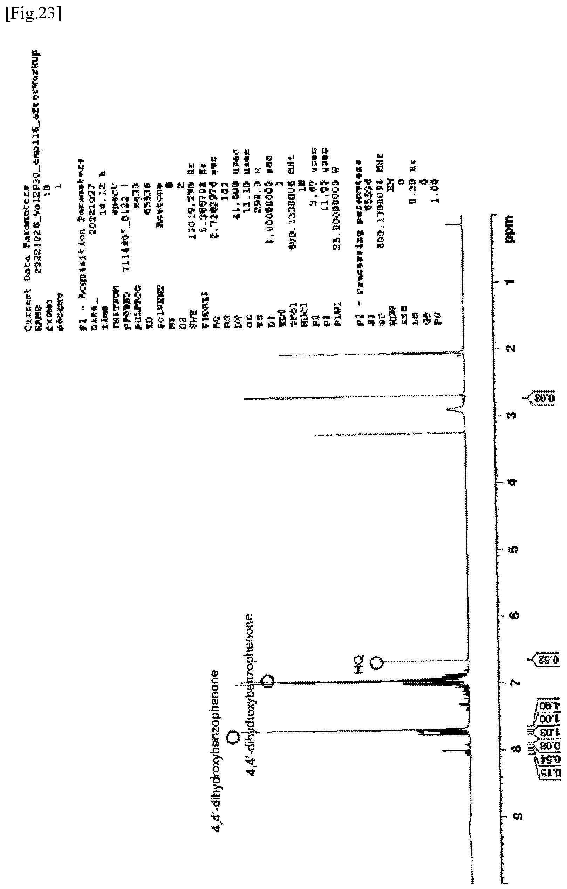

Analysis of the crude product by ¹H NMR confirmed that the target substance, 4,4'-dihydroxybenzophenone (corresponding to the compound (21)), was obtained (yield: 53%). This crude product was mixed with hydroquinone (corresponding to the compound (22)) (yield: approximately 5%), which was the other target substance. The results of ¹H NMR analysis in this case are shown in Fig. 23. 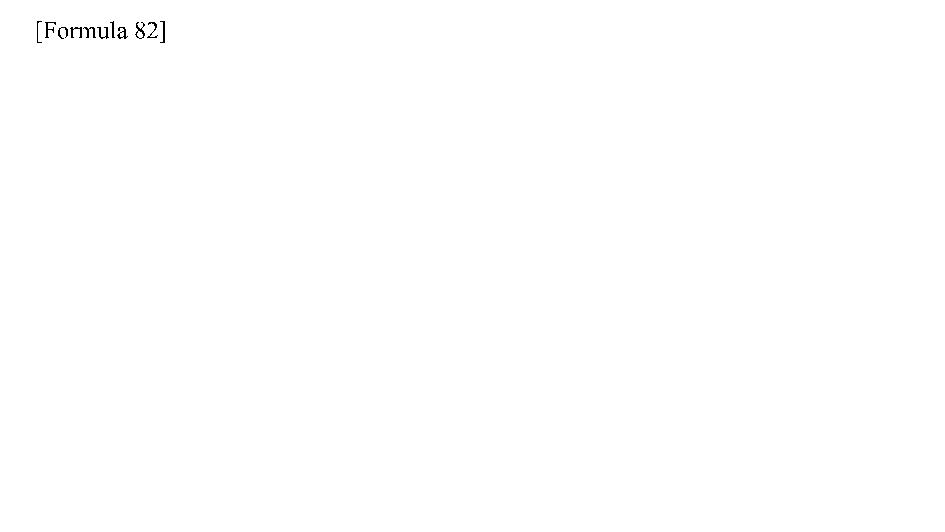 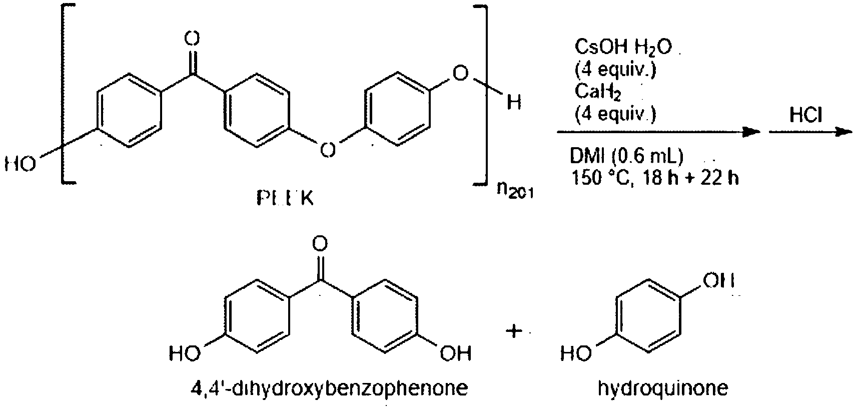 (wherein n₂₀₁ is an integer (preferably 10 to 200)).

### <Depolymerization of polyphenylsulfone (depolymerization method (1))>>

### [Example 22]

Under an argon atmosphere, to platy polyphenylsulfone (45 mg; the amount of repeating unit in the general formula (1) of 0.1 mmol) prepared by cutting a PPSU baby feeding bottle, manufactured by manufactured by CHU CHU HEALTHCARE LIMITED into small pieces, were added cesium hydroxide monohydrate (CsOH·H₂O)

(68 mg, 0.4 mmol, 4 times the amount by mole (4 equivalents) of repeating unit in the general formula (1)), calcium hydride (CaH₂, dehydrating agent) (18 mg, 0.4 mmol, 4 times the amount by mole (4 equivalents) of repeating unit in the general formula (1)), and 1,3-dimethyl-2-imidazolidinone (DMI) (0.2 mL) in sequence, and the resulting mixture was heated at 150°C to dissolve the polyphenylsulfone. Thereafter, the resulting mixture was stirred at 150°C for 24 hours. Cesium hydroxide monohydrate was dried by heating at 150°C for 5 hours under reduced pressure (266.644 Pa (2 mmHg)) before use.

Next, the temperature of the reaction mixture was returned to room temperature, and 1M concentration hydrochloric acid (3.6 mL) was added, followed by the addition of water (2 mL) and ethyl acetate (5 mL) in that order to extract the organic layer. The organic layer was then washed successively with water and a saturated sodium chloride aqueous solution. The obtained organic layer was dried over anhydrous magnesium sulfate, and then distilled under reduced pressure to obtain a crude product.

The crude product was purified by thin layer chromatography to isolate bisphenol S (corresponding to the compound (110)) (yield: 15.9 mg, 64%) and 4,4'-dihydroxybiphenyl (corresponding to the compound (123)) (yield: 14.3 mg, 77%). The results of ¹H NMR analysis of bisphenol S and 4,4'-dihydroxybiphenyl obtained were the same as those in Example 19. 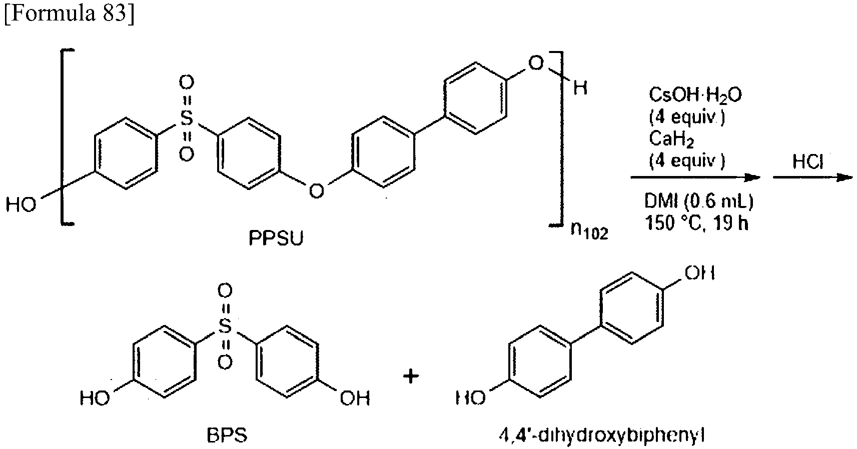 (wherein n₁₀₂ is an integer (preferably 10 to 200).)

### <<Depolymerization of polyether ether sulfone (depolymerization method (5))>>

### [Example 22]

Under an argon atmosphere, to pellet-shaped polyether ether sulfone (982 mg; the amount of repeating unit in the general formula (1) of 3.0 mmol, manufactured by Sigma-Aldrich Co. LLC, Cat. No.79421) were added cesium hydroxide monohydrate (CsOH·H₂O) (704 mg; 12.6 mmol, 4 times the amount by mole (4 equivalents) of repeating unit in the general formula (1)), calcium hydride (CaH₂) (513 mg; 12 mmol, 4 times the amount by mole (4 equivalents) of repeating unit in the general formula (1)), and 1,3-dimethyl-2-imidazolidinone (DMI) (6.0 mL) in sequence, and the mixture was stirred at 150°C for 20 hours. Cesium hydroxide monohydrate was dried by heating at 150°C for 5 hours under reduced pressure (266.644 Pa (2 mmHg)) before use.

Next, the temperature of the reaction mixture was returned to room temperature, and 2M concentration hydrochloric acid (15 mL) was added. Thereto was further added N,N-dimethylformamide (DMF) (5.0 µL, 0.065mmol), and the analysis by ¹H NMR confirmed that the target substances, bisphenol S (corresponding to the compound (11)), hydroquinone (corresponding to the compound (122)), and 4-((4-(4-Hydroxyphenoxy)phenyl)sulfonyl)phenol (BPS-HQ), were obtained.

To the reaction mixture obtained above were then added ethyl acetate (50 mL) and water (50 mL), and the mixture was stirred to extract the organic layer. The obtained organic layer was dried over anhydrous magnesium sulfate, and then distilled under reduced pressure to obtain a crude product.

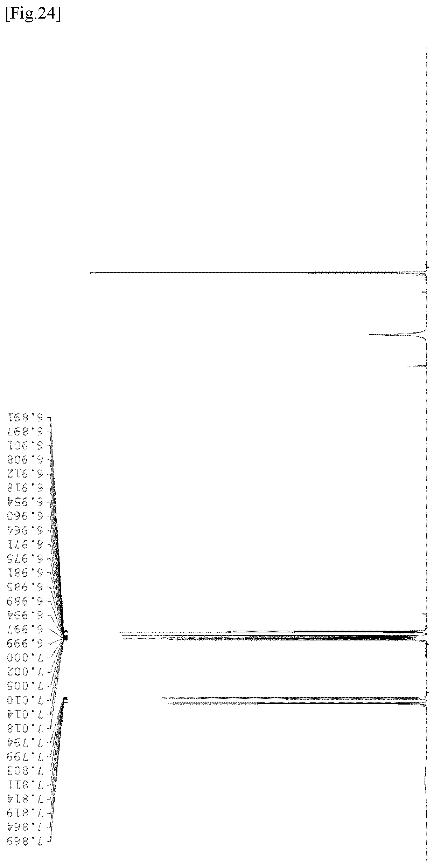

The resulting crude product was purified by silica gel column chromatography to isolate bisphenol S (BPS) (yield: 111 mg, 14%) and 4-((4-(4-hydroxyphenoxy)phenyl)sulfonyl)phenol (BPS-HQ) (yield: 822 mg, 79%). The results of ¹H NMR analysis of the obtained bisphenol S were the same as in Example 20. The results of ¹H NMR analysis of the obtained 4-((4-(4-hydroxyphenoxy)phenyl)sulfonyl)phenol are shown in Fig. 24. 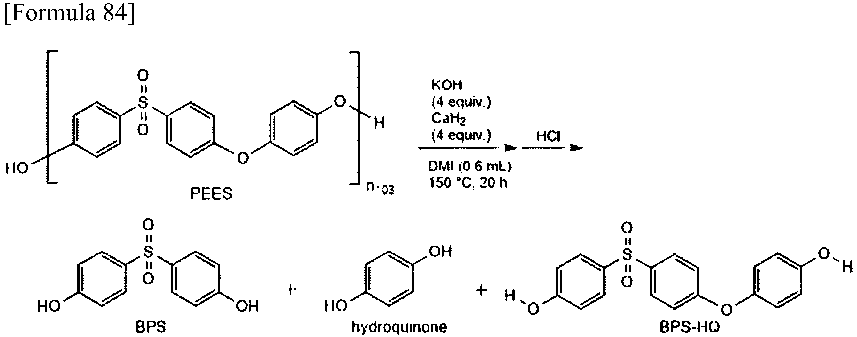 (wherein n₁₀₃ is an integer (preferably 10 to 200).)

### <<Depolymerization of polyether ether ketone (depolymerization method (6))>>

### [Example 23]

Under an argon atmosphere, to pellet-shaped polyether ether ketone (144 mg; the amount of repeating unit in the general formula (2) of 0.50 mmol, manufactured by Sigma-Aldrich Co. LLC, Cat. No.79421) were added cesium hydroxide monohydrate (CsOH·H₂O) (335 mg; 2.0 mmol, 4 times the amount by mole (4 equivalents) of repeating unit in the general formula (2)) and 1,3-dimethyl-2-imidazolidinone (DMI) (1.0 mL) in sequence, and the resulting mixture was stirred at 150°C for 19 hours. Cesium hydroxide monohydrate was dried by heating at 150°C for 5 hours under reduced pressure (266.644 Pa (2 mmHg)) before use.

Next, the temperature of the reaction mixture was returned to room temperature, and 2M concentration hydrochloric acid (1 mL) was added. Thereto was further added mesitylene (5.0 µL, 0.036 mmol), and the mixture was analyzed by ¹H NMR, confirming that the target substances, 4,4'-dihydroxybenzophenone (corresponding to the compound (21)), hydroquinone (corresponding to the compound (122)), and (4-(4-hydroxyphenoxy)-phenyl)(4-hydroxyphenyl)methanone, were obtained.

To the reaction mixture obtained above were then added ethyl acetate (30 mL) and water (30 mL), and the mixture was stirred to extract the organic layer. The obtained organic layer was dried over anhydrous magnesium sulfate, and then distilled under reduced pressure to obtain a crude product.

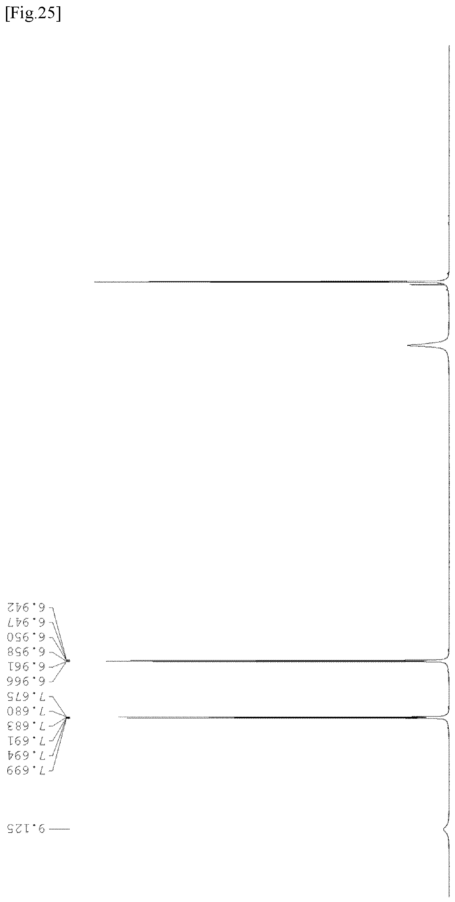

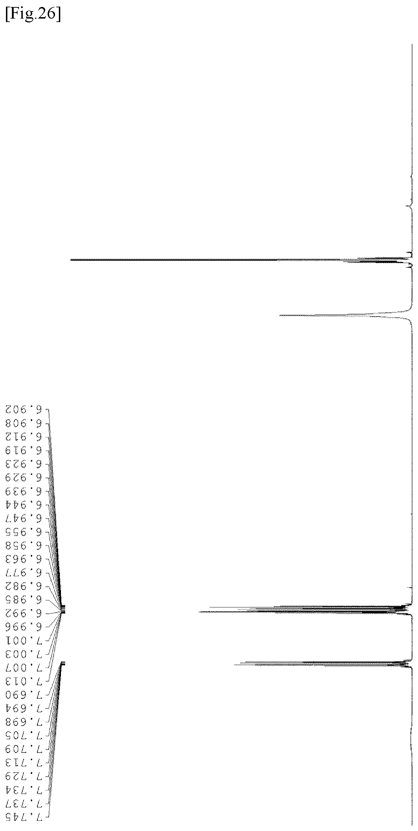

The resulting crude product was purified by silica gel column chromatography to isolate 4,4'-dihydroxybenzophenone (yield: 15.6 mg, 14%) and (4-(4-hydroxyphenoxy)-phenyl)(4-hydroxyphenyl)methanone (yield: 35.2 mg, 23%). The results of ¹H NMR analysis of the obtained 4,4'-dihydroxybenzophenone are shown in Fig. 25, and the results of ¹H NMR analysis of 4-((4-(4-hydroxyphenoxy)phenyl)sulfonyl)phenol are shown in Fig. 26. 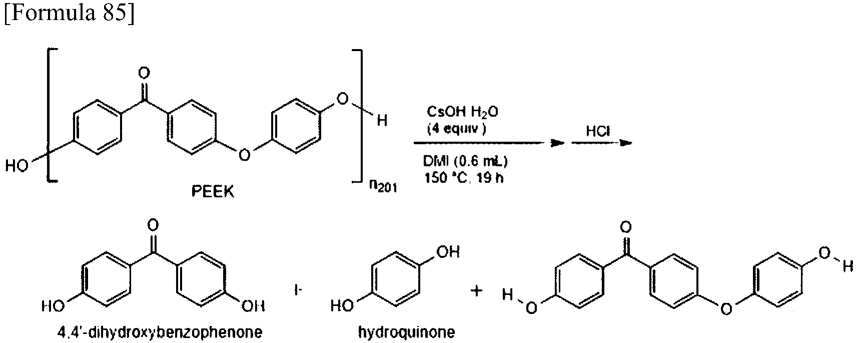 (wherein n₂₀₁ is an integer (preferably 10 to 200).)

### Industrial Applicability

The present invention can be used for depolymerization of polysulfone and its derivative, polyether ether sulfone and its derivative, polyphenyl sulfone and its derivative, polyether ether ketone and its derivative, polyetherimide and its derivative, and polyether sulfone and its derivative.

## Claims

1. A method for decomposing a compound, comprising a decomposition step of reacting a first compound represented by the following general formula (1): 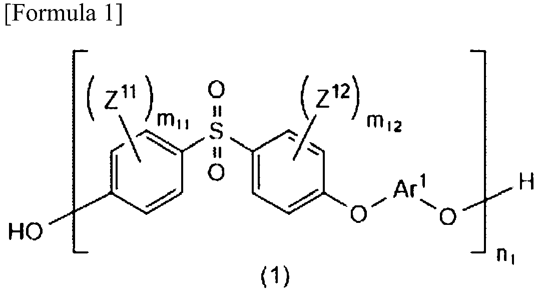
(wherein n₁ is an integer of 2 or greater; Z¹¹ and Z¹² are each independently a group other than a hydrogen atom; m₁₁ and m₁₂ are each independently an integer of 0 to 4, and when m₁₁ is an integer of 1 or greater, n₁ × m₁₁ Z¹¹ groups are optionally the same or different from each other, and when m₁₂ is an integer of 1 or greater, n₁ × m₁₂ Z¹² groups are optionally the same or different from each other; Ar¹ is represented by the following general formula (91), (92) or (93), 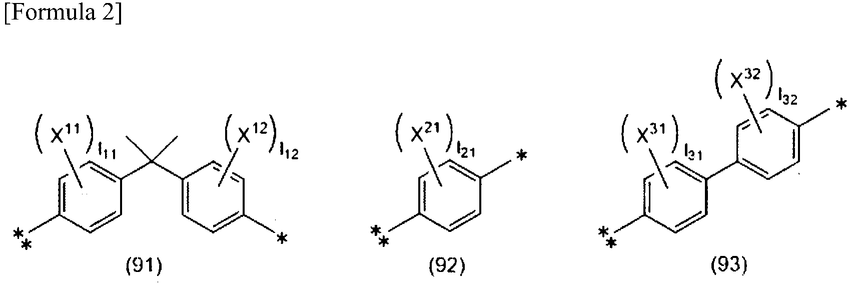
(wherein X¹¹, X¹², X²¹, X³¹, and X³² are each independently a group other than a hydrogen atom; l₁₁, l₁₂, l₂₁, l₃₁, and l₃₂ are each independently an integer of 0 to 4, and when 1₁₁ is an integer of 1 or greater, n₁ × l₁₁ X¹¹ groups are optionally the same or different from each other, when l₁₂ is an integer of 1 or greater, n₁ × l₁₂ X¹² groups are optionally the same or different from each other, when l₂₁ is an integer of 1 or greater, n₁ × l₂₁ X²¹ groups are optionally the same or different from each other, when l₃₁ is an integer of 1 or greater, n₁ × l₃₁ X³¹ groups are optionally the same or different from each other, and when l₃₂ is an integer of 1 or greater, n₁ × l₃₂ X³² groups are optionally the same or different from each other.), and the bond attached with the sign * and the bond attached with the sign ** in the general formulae (91), (92) and (93) are each formed to an oxygen atom in the general formula (1).)
and
a second compound represented by the following general formula (8):
R⁸-SH (8)
(wherein R⁸ is an alkyl group, an aryl group, or an aralkyl group, and one or two or more hydrogen atoms in the alkyl group, the aryl group, or the aralkyl group are optionally substituted with a halogen atom, a hydroxyl group, a mercapto group, or a trialkoxysilyl group, and when the alkyl group, the aryl group, or the aralkyl group has one or two or more trimethylene groups therein, a central methylene group in the trimethylene group is optionally substituted with an oxycarbonyl group or a carbonyloxy group.),
or a hydroxide in the co-presence of a base to decompose the first compound.

2. A method for decomposing a compound, comprising a decomposition step of reacting a third compound represented by the following general formula (2): 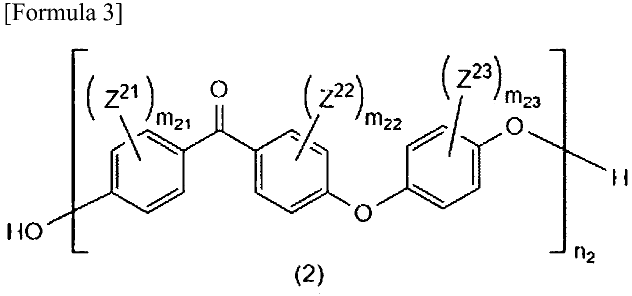
(wherein n₂ is an integer of 2 or greater; Z²¹, Z²², and Z²³ are each independently a group other than a hydrogen atom; m₂₁, m₂₂, and m₂₃ are each independently an integer of 0 to 4, and when m₂₁ is an integer of 1 or greater, n₂ × m₂₁ Z²¹ groups are optionally the same or different from each other, when m₂₂ is an integer of 1 or greater, n₂ × m₂₂ Z²² groups are optionally the same or different from each other, and when m₂₃ is an integer of 1 or greater, n₂ × m₂₃ Z²³ groups are optionally the same or different from each other.)
and
a second compound represented by the following general formula (8):
R³-SH (8)
(wherein R⁸ is an alkyl group, an aryl group, or an aralkyl group, and one or two or more hydrogen atoms in the alkyl group, the aryl group, or the aralkyl group are optionally substituted with a halogen atom, a hydroxyl group, a mercapto group, or a trialkoxysilyl group, and when the alkyl group, the aryl group, or the aralkyl group has one or two or more trimethylene groups therein, a central methylene group in the trimethylene group is optionally substituted with an oxycarbonyl group or a carbonyloxy group.),
or a hydroxide in the co-presence of a base to decompose the third compound.

3. A method for decomposing a compound, comprising a decomposition step of reacting a fourth compound represented by the following general formula (4): 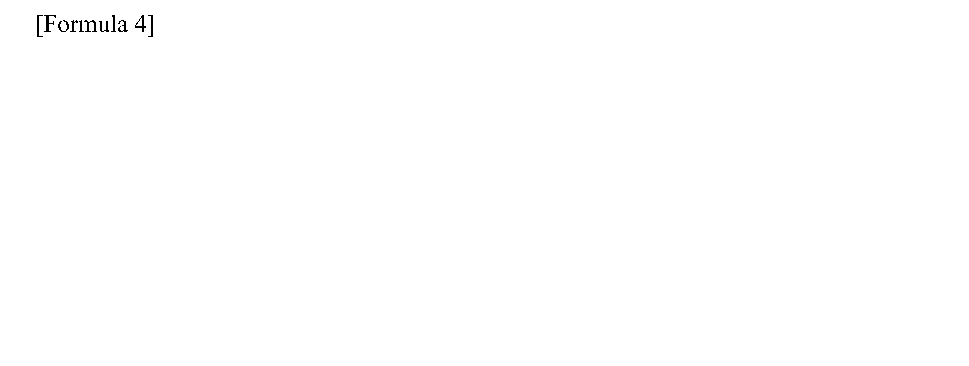 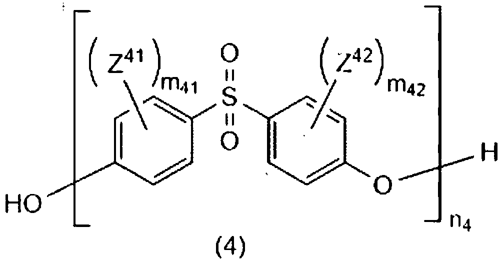
(wherein n₄ is an integer of 2 or greater; Z⁴¹ and Z⁴² are each independently a group other than a hydrogen atom; m₄₁ and m₄₂ are each independently an integer of 0 to 4, and when m₄₁ is an integer of 1 or greater, n₄ × m₄₁ Z⁴¹ groups are optionally the same or different from each other, and when m₄₂ is an integer of 1 or greater, n₄ × m₄₂ Z⁴² are optionally the same or different from each other.)
and
a second compound represented by the following general formula (8):
R⁸-SH (8)
(wherein R⁸ is an alkyl group, an aryl group, or an aralkyl group, and one or two or more hydrogen atoms in the alkyl group, the aryl group, or the aralkyl group are optionally substituted with a halogen atom, a hydroxyl group, a mercapto group, or a trialkoxysilyl group, and when the alkyl group, the aryl group, or the aralkyl group has one or two or more trimethylene groups therein, a central methylene group in the trimethylene group is optionally substituted with an oxycarbonyl group or a carbonyloxy group.),
or a hydroxide in the co-presence of a base to decompose the fourth compound.

4. The method for depolymerizing a compound according to any one of claims 1 to 3, comprising using at least a phosphazene base or sodium tert-butoxide as the base.

5. The method for decomposing a compound according to any one of claims 1 to 3, wherein the second compound represented by the general formula (8) above is one or two or more selected from the group consisting of an alkyl mercaptan having 1 to 15 carbon atoms, an aryl mercaptan having 6 to 12 carbon atoms, an aralkyl mercaptan having 7 to 14 carbon atoms, a halogenated alkyl mercaptan having 1 to 15 carbon atoms, a halogenated aryl mercaptan having 6 to 12 carbon atoms, a hydroxyalkyl mercaptan having 1 to 15 carbon atoms, a polymercaptoalkane having 1 to 15 carbon atoms, a (mercaptoalkyl)trialkoxysilane having 4 to 15 carbon atoms, and an alkoxycarbonylalkanethiol having 3 to 15 carbon atoms.

6. The method for decomposing a compound according to any one of claims 1 to 3, further comprising using a dehydrating agent in the decomposition step.

7. The method for decomposing a compound according to any one of claims 1 to 3, wherein the hydroxide is an alkali metal hydroxide.

8. The method for decomposing a compound according to claim 6, wherein the dehydrating agent is one or two or more selected from the group consisting of sodium hydride, potassium hydride, calcium hydride, calcium oxide, cesium chloride, calcium chloride, magnesium sulfate, and zeolite,

9. A method for producing an ether compound, comprising a step of etherifying one or more phenolic hydroxyl groups or one or more groups in which the phenolic hydroxyl group has formed its salt, in one or two or more selected from the group consisting of a compound represented by the following general formula (11): 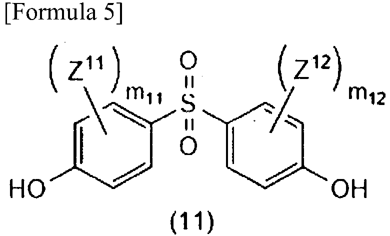
(wherein Z¹¹, Z¹², m₁₁, and m₁₂ are the same as described above.) or a salt thereof,
a compound represented by the following general formula (121): 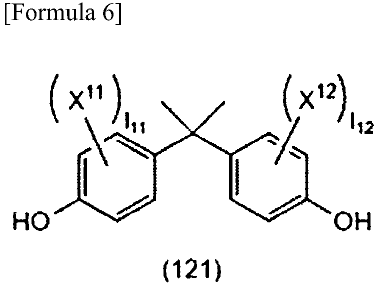
(wherein X¹¹, X¹², l₁₁ and l₁₂ are the same as described above.) and a salt thereof, a compound represented by the following general formula (122): 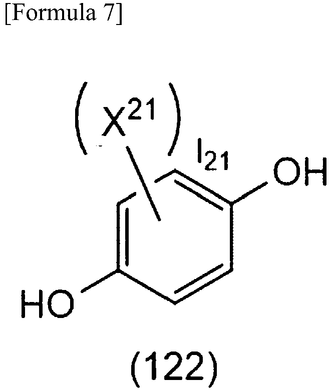 (wherein X²¹ and l₂₁ are the same as described above.) and a salt thereof, as well as
a compound represented by the following general formula (123): 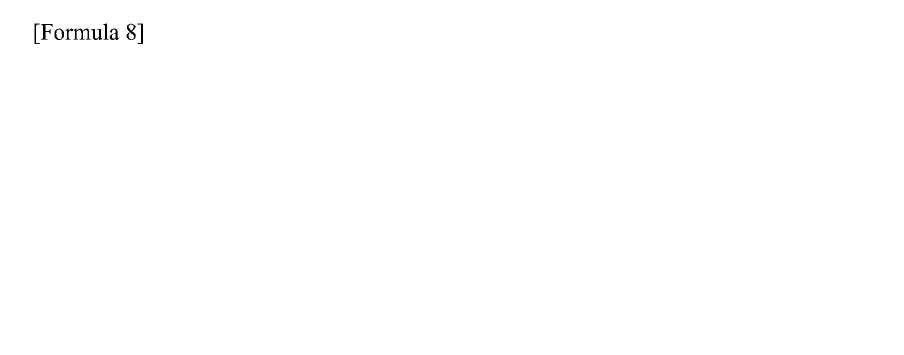 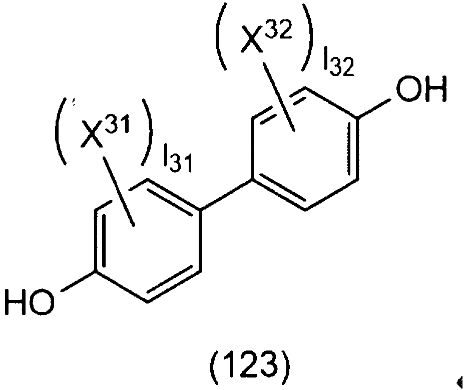 (wherein X³¹, X³², l₃₁ and l₃₂ are the same as described above.) and a salt thereof,
one or two or more selected from a compound represented by the following general formula (21): 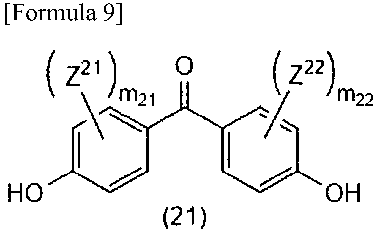
(wherein Z²¹, Z²², m₂₁, and m₂₂ are the same as described above.) and a compound represented by the following general formula (22): 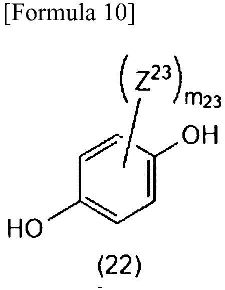
(wherein Z²³ and m₂₃ are the same as described above.) and a salt thereof,
or one or two or more selected from the group consisting of a compound represented by the following general formula (41): 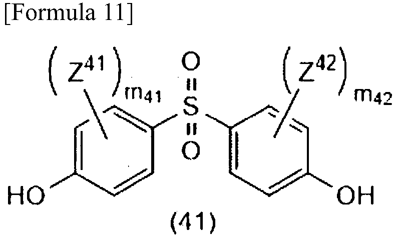
(wherein Z⁴¹, Z⁴², m₄₁, and m₄₂ are the same as described above.) and a salt thereof,
which have been obtained by the method for decomposing a compound according to claims 1 to 3, to obtain the ether compound.

10. A method for decomposing a compound, comprising a decomposition step of reacting a fifth compound represented by the following general formula (3): 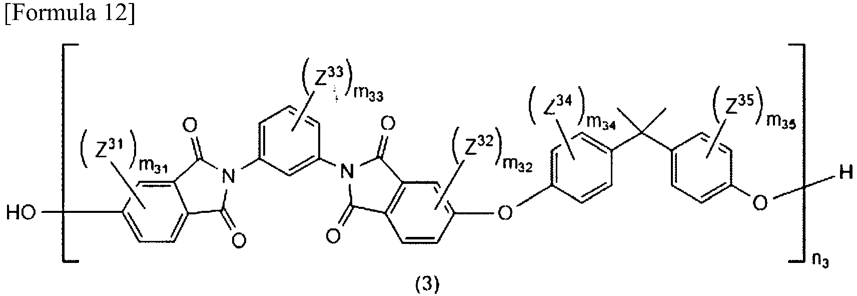
(wherein, n₃ is an integer of 2 or greater; Z³¹, Z³², Z³³, Z³⁴, and Z³⁵ are each independently a group other than a hydrogen atom; m₃₁ and m₃₂ are each independently an integer of 0 to 3, and m₃₃, m₃₄, and m₃₅ are each independently an integer of 0 to 4, and when m₃₁ is an integer of 1 or greater, n₃ × m₃₁ Z³¹ group are optionally be the same or different from each other, when m₃₂ is an integer of 1 or greater, n₃ × m₃₂ Z³² groups are the same or different from each other, when m₃₃ is an integer of 1 or greater, n₃ × m₃₃ Z³³ groups are optionally the same or different from each other, when m₃₄ is an integer of 1 or greater, n₃ × m₃₄ Z³⁴ groups are optionally the same or different from each other, and when m₃₅ is an integer of 1 or greater, n₃ × m₃₅ Z³⁵ groups are optionally the same or different from each other.)
with
a second compound represented by the following general formula (8):
R⁸-SH (8)
(wherein R⁸ is an alkyl group, an aryl group, or an aralkyl group, and one or two or more hydrogen atoms in the alkyl group, the aryl group, or the aralkyl group are optionally substituted with a halogen atom, a hydroxyl group, a mercapto group, or a trialkoxysilyl group, and when the alkyl group, the aryl group, or the aralkyl group has one or two or more trimethylene groups therein, a central methylene group in the trimethylene group is optionally substituted with an oxycarbonyl group or a carbonyloxy group.),
in the co-presence of a base to decompose the fifth compound.
